# EUROPEAN PATENT SPECIFICATION

(11) **EP 1 202 724 B1**
(45) Date of publication and mention of the grant of the patent: **01.10.2003**
(21) Application number: 00945140.2
(22) Date of filing: 05.07.2000
(51) Int. Cl.: A61K 31/18, A61K 31/185, A61K 31/63, A61K 31/196, A61K 31/166, A61K 31/4164, A61K 31/433, A61P 25/04

(54) **METHOD FOR TREATING CHRONIC PAIN USING MEK INHIBITORS**
VERFAHREN ZUR BEHANDLUNG VON CHRONISCHEM SCHMERZ DURCH VERABREICHUNG VON EINEM MEK HEMMER
TRAITEMENT DE DOULEURS CHRONIQUES AU MOYEN D'INHIBITEURS DE MEK

(30) Priority: 16.07.1999 US 144419 P; 16.07.1999 US 144280 P; 16.07.1999 US 144320 P; 16.07.1999 US 144658 P; 16.07.1999 US 144659 P; 16.07.1999 US 144655 P
(43) Date of publication of application: 08.05.2002
(73) Proprietor: WARNER-LAMBERT COMPANY, Morris Plains, New Jersey 07950 (US)
(72) Inventor: BRIDGES, Alexander, James, Saline, MI 48176 (US); BOOTH, Richard, John, Ann Arbor, MI 48105 (US); TECLE, Haile, Ann Arbor, MI 48108 (US); SCAGGS, Yvonne, Ypsilanti, MI 48197 (US); KAUFMAN, Michael, Ypsilanti, MI 48197 (US); BARRETT, Stephen, Douglas, Livonia, MI 48154 (US); DIXON, Alistair, Cambridge CB1 2LL (GB); LEE, Kevin, Cambridge CB1 9YT (GB); PINNOCK, Robert, Denham, Cambridge CB1 9YT (GB)
(74) Representative: Tesch, Rudolf, Dr.
(86) International application number: US0018348
(87) International publication number: WO01005393

(56) References cited:
- EP-A- 0 316 630
- WO-A-00/41505
- WO-A-00/41994
- WO-A-00/42002
- WO-A-00/42003
- WO-A-98/37881
- WO-A-99/01421
- US-A- 2 502 451
- FUJIMURA H ET AL: "HYDROXAMIC ACID DERIVATIVES" CHEMICAL ABSTRACTS + INDEXES,AMERICAN CHEMICAL SOCIETY. COLUMBUS,US, vol. 70, no. 3, 20 January 1969 (1969-01-20), XP002142605 ISSN: 0009-2258 & JP 42 024578 A (TAKEDA CHEMICAL INDUSTRIAL LTD) 1967
- MOKHORT N A: "DEPENDENCE BETWEEN STRUCTURE, ANTIINFLAMMATORY, ANALGESIC, AND ANTIPYR" CHEMABS, AN =1972:121461, 1972, XP002142610
- HIRANO HIROSHI ET AL: "NOVEL N-PHENYLANTHRANILIC ACID DERIVATIVES" CHEMABS, AN = 1968:418858, 1968, XP002142611
- JI RU-RONG ET AL: "Nociceptive-specific activation of ERK in spinal neurons contributes to pain hypersensitivity." NATURE NEUROSCIENCE, vol. 2, no. 12, December 1999 (1999-12), pages 1114-1119, XP000978586 ISSN: 1097-6256
- DUESBERY N S ET AL: "MEK WARS, A NEW FRONT IN THE BATTLE AGAINST CANCER" NATURE MEDICINE,US,NATURE PUBLISHING, CO, vol. 5, no. 7, 1999, pages 736-737, XP000907246 ISSN: 1078-8956
- DUNCIA J V ET AL: "MEK inhibitors: the chemistry and biological activity of U0126, its analogs, and cyclization products" BIOORGANIC & MEDICINAL CHEMISTRY LETTERS,GB,OXFORD, vol. 8, no. 20, 20 October 1998 (1998-10-20), pages 2839-2844, XP004139571 ISSN: 0960-894X

## Description

### BACKGROUND

The invention features medicaments for treating chronic pain using MEK inhibitors. Chronic pain includes neuropathic pain, and chronic inflammatory pain.

Abnormality anywhere in a nerve pathway disrupts nerve signals, which in turn are abnormally interpreted in the brain, causing neuropathic pain. Neuropathic pain may be, for example, a deep ache, a burning sensation, or hypersensitivity to touch. Diseases or conditions associated with neuropathic pain include, without limitation, diabetic neuropathy, causalgia, plexus avulsion, neuroma, vasculitis, crush injury, viral infections (e.g., herpes virus infection or HIV), constriction injury, tissue injury, nerve injury from the periphery to the central nervous system, limb amputation, hypothyroidism, uremia, chronic alcoholism, post-operative pain, arthritis, back pain, and vitamin deficiencies.

Infections such as herpes zoster (shingles) can cause nerve inflammation and produce postherpetic neuralgia, a chronic burning localized to the area of viral infection. Hyperalgesia is when an already noxious stimulus becomes more painful, and allodynia, when a previously non-noxious stimulus becomes painful (such as contact of clothing or a breeze). Reflex sympathetic dystrophy is accompanied by swelling and sweating or changes in local blood flow, tissue atrophy, or osteoporosis. Causalgia, including severe burning pain and swelling, sweating, and changes in blood flow, may follow an injury or disease of a major nerve such as the sciatic nerve. Some types of chronic low back pain can have a neuropathic component (e.g., sciatica, postpoliomyelitis and CPRM). Neuropathic pain may also be induced by cancer or chemotherapy.

Neuropathic pain is currently treated with anticonvulsants such as carbamazepine and antidepressants such as amitryptaline. NSAIDS and opioids generally have little effect *(Fields et al 1994 Textbook of Pain p 991-996 (pub: Churchill Livingstone), James & Page 1994 J.Am.Pediatr.Med.Assoc, 8: 439-447, Galer, 1995 Neurology 45 S17-S25*. Neuropathic conditions that have been treated with gabapentin include: postherpetic neuralgia, postpoliomyelitis, CPRM, HIV-related neuropathy, trigeminal neuralgia, and reflex sympathetic dystrophy (RSD). The generally weak efficacy of antiinflammatory agents suggests that the mechanism for chronic pain is separate from hyperalgesia.

### SUMMARY OF THE INVENTION

The invention features medicaments for treating chronic pain, which method includes the step of administering a composition including a MEK inhibitor to a patient in need of such treatment. Chronic pain includes neuropathic pain, idiopathic pain, and pain associated with vitamin deficiencies, uremia, hypothyroidism post-operative pain, arthritis, back pain, and chronic alcoholism. The invention also features compositions as disclosed, formulated for the treatment of chronic pain. Such a composition may include one or more MEK inhibitor compounds having a structure disclosed in patent applications USSN 60/115,652, filed January 13, 1999, USSN 60/115,670, filed January 13, 1999. USSN 60/115,876 filed January 13, 1999, USSN 60/115,874, PCT/US99/30417, international filing date December 21, 1999, PCT/US99/30418, international filing date December 21, 1999, PCT/US99/30491, international filing date December 21, 1999, and PCT/US99/30435, international filing date December 21, 1999.

Examples of MEK inhibitors include a compound having the formula (I) below:

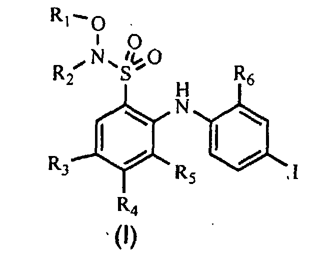

R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl, (CH₂)₂₋₄ OR_{C} or (CH₂)₂₋₄ NR_{C}R_{D}. R₂ is H, C ₁₋₄ alkyl, phenyl, C ₃₋₆ cycloalkyl, C ₃₋₆ heterocyclic radical, or (C ₃₋₆ cycloalkyl) methyl. Each of R₃ and R₄ is independently selected from H, F, NO₂, Br and Cl. R₅ is selected from H and F. R₆ is H, F, Cl or CH₃. Each of R_{C} and R_{D} is independently selected from H, C ₁₋₄ alkyl, C ₃₋₄ alkenyl, C ₃₋ ₄ alkynyl, C ₃₋₆ cycloalkyl, and phenyl; or NR_{C}R_{D} may be a piperidino, morpholino, or N-(C ₁₋₆ alkyl)piperazino ring. Each hydrocarbon radical above is optionally substituted with between 1 and 3 substituents independently selected from halo, hydroxyl, amino, (amino)sulfonyl, and NO₂. Each heterocyclic radical above is optionally substituted with between 1 and 3 substituents independently selected from halo, C ₁₋₄ alkyl, C ₃₋₆ cycloalkyl, C ₃₋₄ alkenyl, C ₃₋₄ alkynyl, phenyl, hydroxyl, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxyl, amino, and NO₂. The invention also includes a pharmaceutically acceptable salt or C ₁₋₈ ester of a disclosed compound. For example, the disclosed alcohol compounds may form esters having the structure obtained by replacing the H of a hydroxyl group with a -C(=O)C ₁₋₇ acyl group.

The invention also relates to the manufacture of a pharmaceutical composition including (a) a compound of formula (I) and (b) a pharmaceutically-acceptable carrier.

The invention also features the use of compounds of formulae (II)A below, such as formula (I)A: 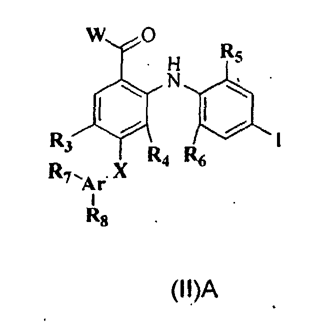 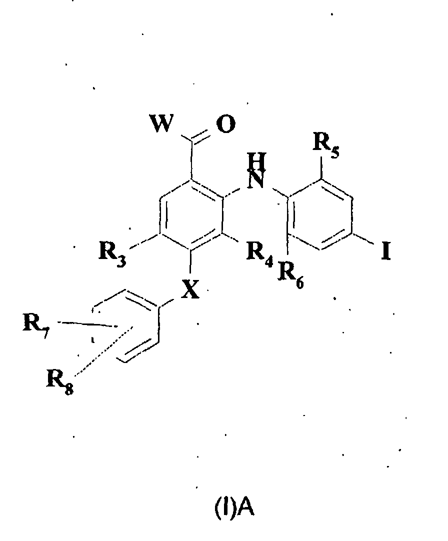 In formulae (I)A and (II)A, W is OR₁, NR₂OR₁, NR_{A}R_{B}, NR₂NR_{A}R_{B}, or NR₂(CH₂)₂₋₄ NR_{A}R_{B}. R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl, or (CH₂)₂₋₄NR_{A}R_{B}. R₂ is H, phenyl, C ₁₋₄ alkyl, C₃₋₄ alkenyl C ₃₋₈ alkynyl, C ₃₋₈ cycloalkyl, or (C ₃₋₈ cycloalkyl)C ₁₋₄ alkyl. R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl. R_{B} is H, C ₁₋₈ alkyl, C ₃₋₈ alkenyl, C ₃₋₈ alkynyl, C ₃₋₈ cycloalkyl, or C ₆₋₈ aryl. R₃ is halo, NO₂, SO₂NR_{I}(CH₂)₂₋₄NR_{E}R_{F}, SO₂NR_{I}R_{K} or (CO)T. T is C ₁₋₈ alkyl, C ₃₋ ₈ cycloalkyl, (NR_{E}R_{F})C ₁₋₄ alkyl, OR_{F}, NR_{I}(CH₂)₂₋₄NR_{E}R_{F}, or NR_{E}R_{F}. R₄ is H or F; R₅ is H, methyl, halo, or NO₂; and R₆ is H, methyl, halo, or NO₂. In formula (II)A, Ar is phenyl, 2-pyridyl, 3-pyridyl, or 4-pyridyl. Each of R₇ and R₈ is independently selected from H, halo, C ₁₋₄ alkyl, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H}, and (CO)NR_{G}R_{H}. However, where Ar is a pyridyl, each of R₇ and R₈ is H. Each of R_{C}, R_{D}, R_{E}, R_{F}, R_{G}, and R_{H} is independently selected from H, C ₁₋₄ alkyl, C ₃₋₄ alkenyl, C ₃₋₄ alkynyl, C ₃₋₆ cycloalkyl, and phenyl. Each of NR_{C}R_{D}, NR_{E}R_{F}, and NR_{G}R_{H} can also be independently morpholinyl, piperazinyl, pyrrolidinyl, or piperadinyl. Each of R_{I} and R_{J} is independently H, methyl, or ethyl. R_{K} is C ₁₋₄ alkyl, C ₃₋₄ alkenyl, C ₃₋₄ alkynyl, C ₃₋₆ cycloalkyl, or phenyl. X is O, S, or NH. Finally, each hydrocarbon radical or heterocyclic radical above is optionally substituted with between 1 and 3 substituents independently selected from halo, C ₁₋₄ alkyl, C ₃₋₆ cycloalkyl, C ₁₋₄ alkenyl, C ₁₋₄ alkynyl, phenyl, hydroxyl, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxyl, amino, and NO₂. In addition to the above compounds, the invention also provides a pharmaceutically acceptable salt or C ₁₋₇ ester thereof.

The invention also features the use of a compound having the formula (I)B below: 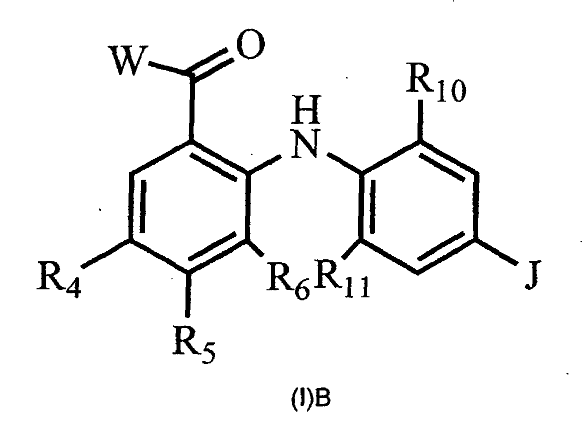 In formula (I)B, W is OR₁, NR₂OR₁, NR_{A}R_{B}, NR₂NR_{A}R_{B}, O(CH₂)₁₋₄NR_{A}R_{B}, or NR₂(CH₂)₁₋₄ NR_{A}R_{B}. R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)-C ₃₋₄ alkynyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical) C ₁₋₄ alkyl, (C ₃₋₈ heterocyclic radical)C ₃₋₄ alkenyl, or (C ₃₋₈ heterocyclic radical)C ₃₋₄ alkynyl. Each of R₂ and R₃ is independently H, phenyl, C ₁₋₄ alkyl, C ₃₋₈ alkenyl, C ₃₋₈ alkynyl, C ₃₋₈ cycloalkyl, or (C ₃₋₈ cycloalkyl)C ₁₋₄ alkyl. Each of R₄, R₅ and R₆ is independently H, F, Br, Cl, or NO₂. R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, (aminosulfonyl)-phenyl] C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl. R_{B} is H, C ₁₋₈ alkyl, C ₃₋₈ alkenyl, C ₃₋₈ alkynyl, C ₃₋₈ cycloalkyl, or phenyl. J is SR_{C}, OR_{C}, SO₂R_{C}, SOR_{C}, SO₂NR_{D}R_{E}, C ₁₋₈ alkyl, C ₃₋₈ alkenyl, C ₃₋₈ alkynyl, C ₃₋₈ cycloalkyl, C ₅₋₈ cycloalkenyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)-C ₃₋₄ alkynyl, C ₃₋₈ heterocyclic radical (e.g., 1,2,5-thiadiazol-3-yl), (C ₃₋₈ heterocyclic radical) C ₁₋₄ alkyl, -M'E'G', (heterocyclic radical)-M'-E'-G', or (cycloalkyl)-M'-E'-G'. M' is O, SO, SO₂, NR_{E}, (CO)NR_{E}, NR_{E} (CO), SO₂NR_{E}, NR_{E}SO₂, or CH₂. E' is absent (in other words, a covalent bond), (CH₂)₁₋₄ or (CH₂)ₘ O(CH₂)ₚ where 1 ≤ (each of m and p independently) ≤ 3 and 2 ≤ (m + p) ≤ 4. G' is OR₃, SOR_{C}, SO₂R_{C,} or NR_{F}R_{G}; provided that where p = 1, then G' is H. Each of R_{C}, R_{D}, R_{E}, R_{F} and R_{G} is independently selected from H, C ₁₋₆ alkyl, C ₃₋₄ alkenyl, C ₃₋₄ alkynyl, C ₃₋₆ cycloalkyl, C ₃₋₆ heterocyclic radical, and phenyl; NR_{F}R_{G} and NR_{D}R_{E} can each also independently be selected from morpholinyl, pyrazinyl, piperazinyl, pyrrolidinyl, or piperadinyl. R₁₀ is H, C ₁₋₄ alkyl, halo, NO₂, or SO₂NR_{H}R_{I}. R₁₁ is H, halo, or NO₂.

Each hydrocarbon radical or heterocyclic radical above is optionally substituted with between 1 and 3 substituents independently selected from halo, C ₁₋₄ alkyl, C ₃₋₆ cycloalkyl, C ₃₋₄ alkenyl, C ₃₋₄ alkynyl, phenyl, hydroxy, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxy, amino, and NO₂. The invention also encompasses a pharmaceutically acceptable salt or C ₁₋ ₇ ester of a compound of formula (I)B.

The invention also features the use of a compound having the formula (I)C below: 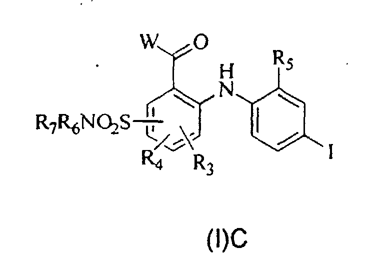 W is OR₁, NR₂OR₁, NR_{A}R_{B}, NR₂NR_{A}R_{B}, or NR₂(CH₂)₂₋₄ NR_{A}R_{B}. R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl, or (CH₂)₂₋₄NR_{A}R_{B}. R₂ is H, phenyl, C ₁₋₄ alkyl, C₃₋₄ alkenyl C ₃₋₈ alkynyl, C ₃₋₈ cycloalkyl, or (C ₃₋₈ cycloalkyl)C ₁₋₄ alkyl. R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl. R_{B} is H, C ₁₋₈ alkyl, C ₃₋₈ alkenyl, C ₃₋₈ alkynyl, C ₃₋₈ cycloalkyl, or C ₆₋₈ aryl. R₃ is H, F, Cl, Br, or NO₂. R₄ is H or F. R₅ is H, methyl or Cl. R₆ is H, C ₁₋₄ alkyl, hydroxyethyl, hydroxypropyl, (CH₂)₂₋₄(NR_{C}R_{D}), phenyl, 2-pyridyl, 3-pyridyl, 4-pyridyl or CH₂Ar, where Ar is phenyl, 2-pyridyl, 3-pyridyl, or 4-pyridyl. R₇ is H, C₁₋₄ alkyl, hydroxyethyl, hydroxypropyl, (CH₂)₂₋₄(NR_{C}R_{D}), phenyl, 2-pyridyl, 3-pyridyl, 4-pyridyl, or CH₂Ar , where Ar is phenyl, 2-pyridyl, 3-pyridyl, or 4-pyridyl. Each of R_{C} and R_{D} is independently selected from H, C ₁₋₆ alkyl, C ₃₋₄ alkenyl, C ₃₋₄ alkynyl, C ₃₋₆ cycloalkyl, C ₃₋₆ heterocyclic radical, and phenyl. NR_{C}R_{D} can also be selected from morpholinyl, piperazinyl, pyrrolidinyl, or piperadinyl. Each hydrocarbon radical or heterocyclic radical above is optionally substituted with between 1 and 3 substituents independently selected from halo, C ₁₋₄ alkyl, C ₃₋₆ cycloalkyl, C ₂₋₄ alkenyl, C ₂₋₄ alkynyl, phenyl, hydroxy, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxy, amino, and NO₂. The invention also features pharmaceutically acceptable salts and C ₁₋₇ esters thereof.

Preferred compounds include PD 297764, 3,4-Difluoro-2-(4-iodophenylamino)-N-methoxy-5-(4-pyridin-2-yl-piperazine-1-sulfonyl)-benzamide; PD 297765, N-Allyloxy-3,4-difluoro-2-(4-iodo-phenylamino)-5-(4-methylpiperazine-1-sulfonyl)-benzamide; PD297766, N-Allyloxy-5-[(2-diethylamino-ethyl)-methyl-sulfamoyl]-3,4-difluoro-2-(4-iodo-phenylamino)-benzamide; PD297767, N-Allyloxy-5-[(3-dimethylamino-propyl)-methyl-sulfamoyl]-3,4-difluoro-2-(4-iodo-phenylamino)-benzamide; PD297768, N-Cyclopropylmethoxy-3,4-difluoro-2-(4-iodo-phenylamino)-5-(4-methyl-piperazine-1-sulfonyl)-benzamide; PD297769, N-Cyclopropylmethoxy-5-[(2-diethylamino-ethyl)-methyl-sulfamoyl]-3,4-difluoro-2-(4-iodo-phenylamino)-benzamide; PD297770, N-Cyclopropylmethoxy-3,4-difluoro-2-(4-iodo-phenylamino)-5-[methyl-(2-pyridin-2-yl-ethyl)-sulfamoyl]-benzamide; PD297771, N-Cyclopropylmethoxy-3,4-difluoro-2-(4-iodo-phenylamino)-5-(4-pyridin-2-yl-piperazine-1-sulfonyl)-benzamide; PD297772, 5-[Benzyl-(2-dimethylamino-ethyl)-sulfamoyl]-N-cyclopropylmethoxy-3,4-difluoro-2-(4-iodo-phenylamino)-benzamide; PD297773, 3,4-Difluoro-2-(4-iodo-2-methyl-phenylamino)-N-methoxy-5-(4-pyridin-2-yl-piperazine-1-sulfonyl)-benzamide; and PD297774, 1-[5-Allyloxycarbamoyl-2,3-difluoro-4-(4-iodo-2-methyl-phenylamino)-benzenesulfonyl]-piperidine-3-carboxylic acid amide.

Preferred embodiments of the invention include methods using one or more of the following compounds:
(a) said MEK inhibitor has a structure selected from: 2-(2-chloro-4-iodophenylamino)-*N*-cyclopropylmethoxy-3,4-difluoro-benzenesulfonamide; and 2-(2-chloro-4-iodo-phenylamino)-cycloproplmethoxy-3,4-difluorobenzenesulfonamide;
(b) said MEK inhibitor has a structure selected from: 2,4-bis-(2-chloro-4-iodophenylamino)-3-fluoro-5-nitro-benzoic acid;
(c) said MEK inhibitor has a structure selected from: 2-(4-ethynyl-2-methylphenylamino)-4-fluoro-benzoic acid; and 2-(3',5'-dichloro-biphenyl-4-ylamino)-benzoic acid; and
(d) said MEK inhibitor has a structure selected from: 2-(2-chloro-4-iodophenylamino)-*N*-cyclopropylmethoxy-3,4-difluoro-5-sulfamoyl-benzamide; 2-(2-chloro-4-iodo-phenylamino)-3,4-difluoro-*N*-hydroxy-5-sulfamoyl-benzamide; *C*-(2-chloro-4-iodo-phenylamino)-*N*-cyclopropylmethoxy-dimethylsulfamoyl-difluorobenzamide; *N*-cyclopropylmethoxy-dimethylsulfamoyl-difluoro-*C*-(4-iodo-2-methyl-phenylamino)-benzamide; and *C*-(2-chloro-4-iodo-phenylamino)-difluoro-(methoxy-methyl-sulfamoyl)-*N*-(2-morpholin-4-yl-ethoxy)benzamide.

### BRIEF DESCRIPTION OF THE FIGURES

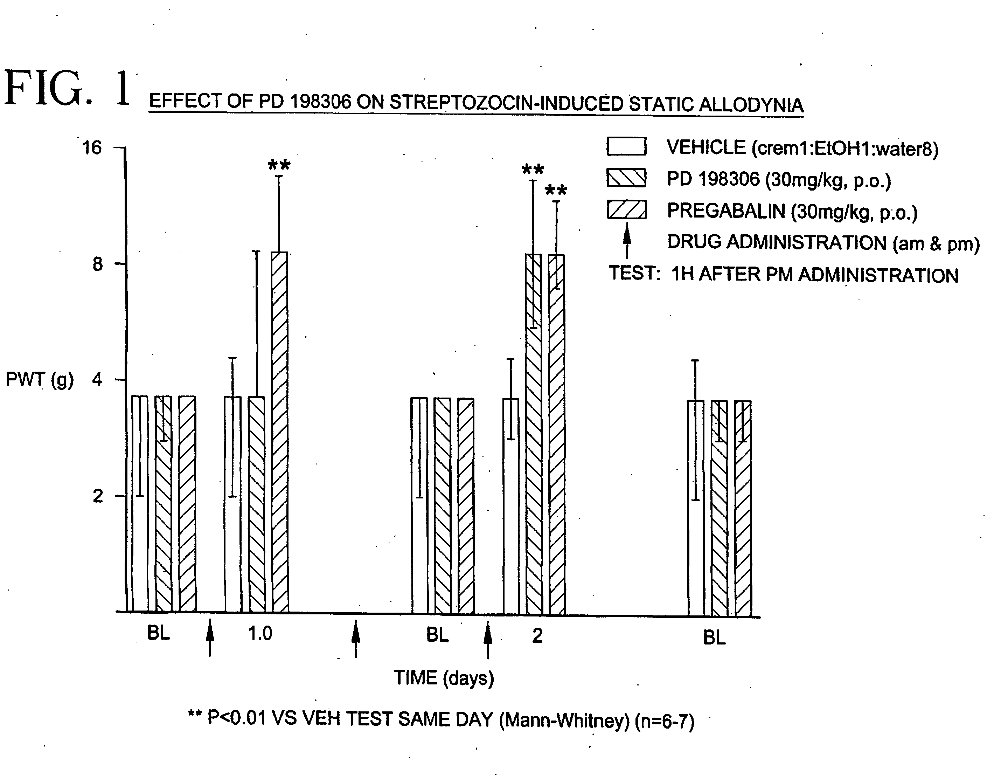

FIG. 1 is a bar graph representing the paw withdrawal threshold (PWT) in grams as a function of time in days. The empty, cross-hatched, and single-hatched bars are vehicle, PD 198306, and pregabalin, respectively. The arrows indicate time of drug administration (30 mg/kg, p.o.).

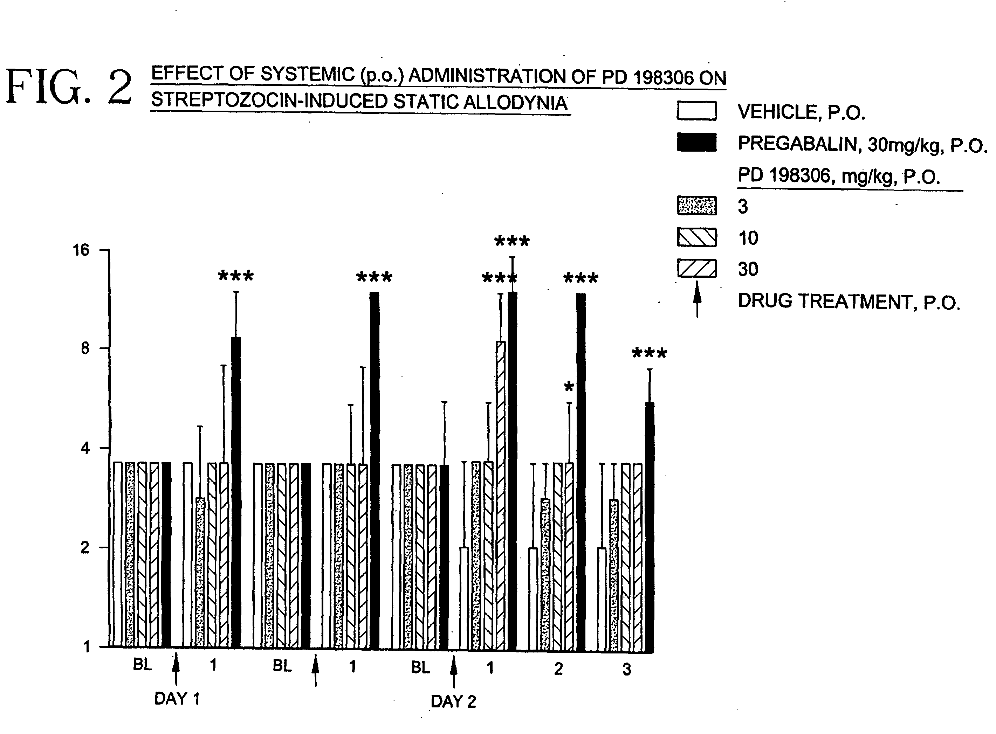

FIG 2. is a bar graph representing the force required in grams to elicit paw withdrawal using von Frey hair filaments as a function of time in days. Baseline (BL) measurements were taken before treatment. Animals received a single p.o. administration of PD 198306 (3-30mg/kg), or pregabalin (30mg/kg) and withdrawal thresholds were re-assessed 1 h after treatment. Treatments were repeated twice a day for two days. Results are expressed median ± 1^{st} and 3^{rd} quartiles. *P<0.05, **P<0.01, ***P<0.001 significantly different from vehicle treated animals (Mann-Whitney t test; n=7-8).

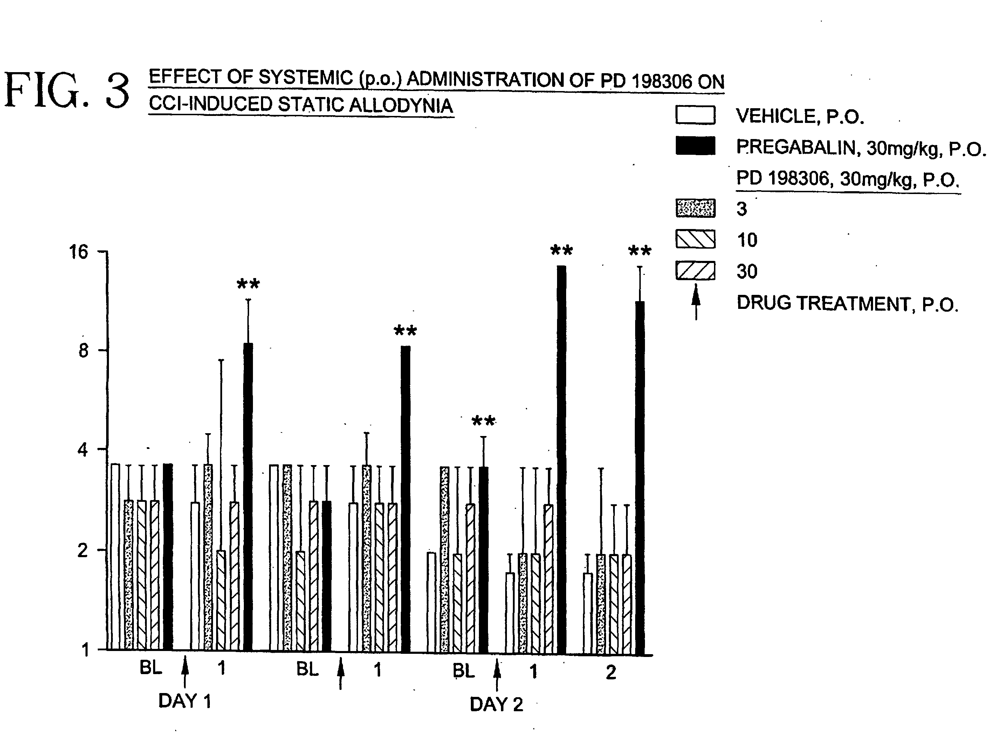

FIG. 3. is a bar graph representing the force required in grams to elicit paw withdrawal using von Frey hair filaments as a function of time in days. Baseline (BL) measurements were taken before treatment. Animals received a single p.o. administration of PD 198306 (3-30mg/kg), or pregabalin (30mg/kg) and withdrawal thresholds were re-assessed 1h after treatment. Treatments were repeated twice a day for two days. Results are expressed median ± 1^{st} and 3^{rd} quartiles. **P<0.01 significantly different from vehicle treated animals (Mann-Whitney t test; n=6).

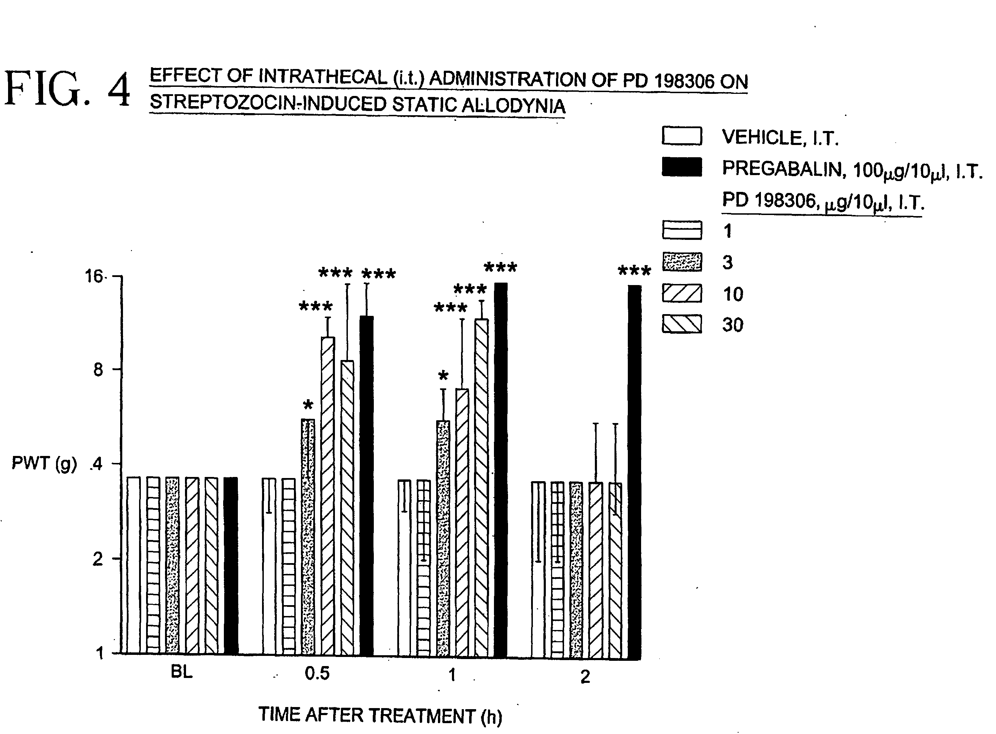

FIG. 4. is a bar graph representing the force required in grams to elicit paw withdrawal using von Frey hair filaments as a function of time in days. Baseline (BL) measurements were taken before treatment. Animals received a single i.t. administration of PD 198306 (1-30µg/10µl), or pregabalin (100µg/10µl) and withdrawal thresholds were re-assessed at 30min, 1h and 2h after treatment. Results are expressed median ± 1^{st} and 3^{rd} quartiles. *P<0.05, ***P<0.001 significantly different from vehicle treated animals (Mann-Whitney t test; n=7-9).

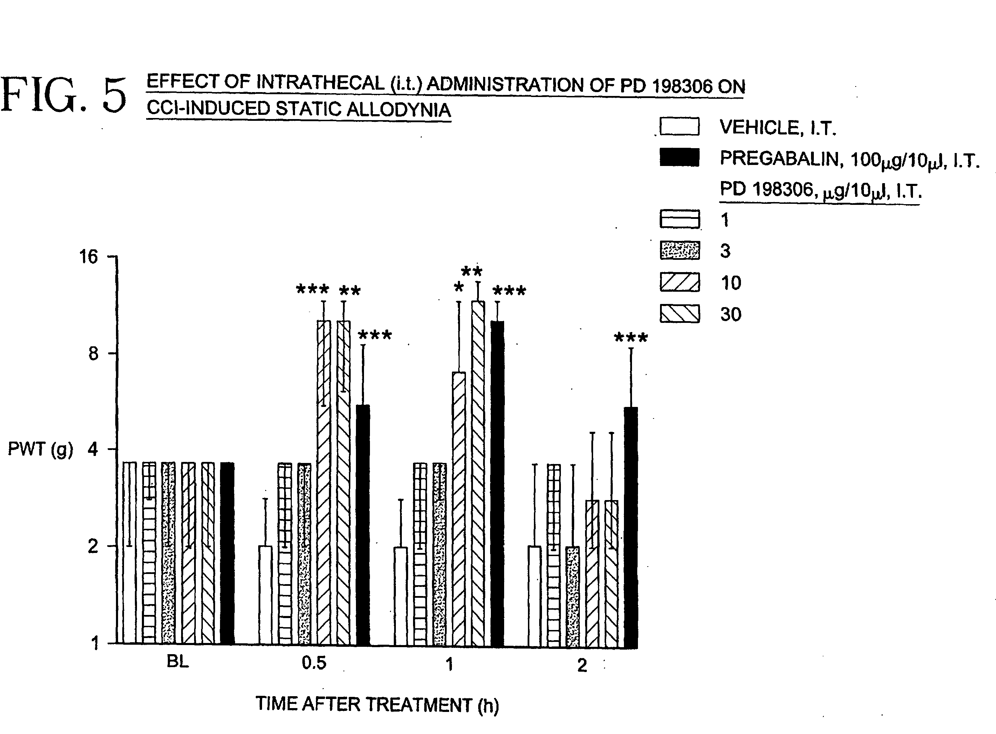

FIG. 5. is a bar graph representing the force required in grams to elicit paw withdrawal using von Frey hair filaments as a function of time in days. Baseline (BL) measurements were taken before treatment. Animals received a single i.t. administration of PD 198306 (1-30µg/10µl), or pregabalin (100µg/10µl) and withdrawal thresholds were re-assessed at 30min, 1h and 2h after treatment. Results are expressed median ± 1^{st} and 3^{rd} quartiles. *P<0.05, **P<0.01, ***P<0.001 significantly different from vehicle treated animals (Mann-Whitney t test; n=6-8).

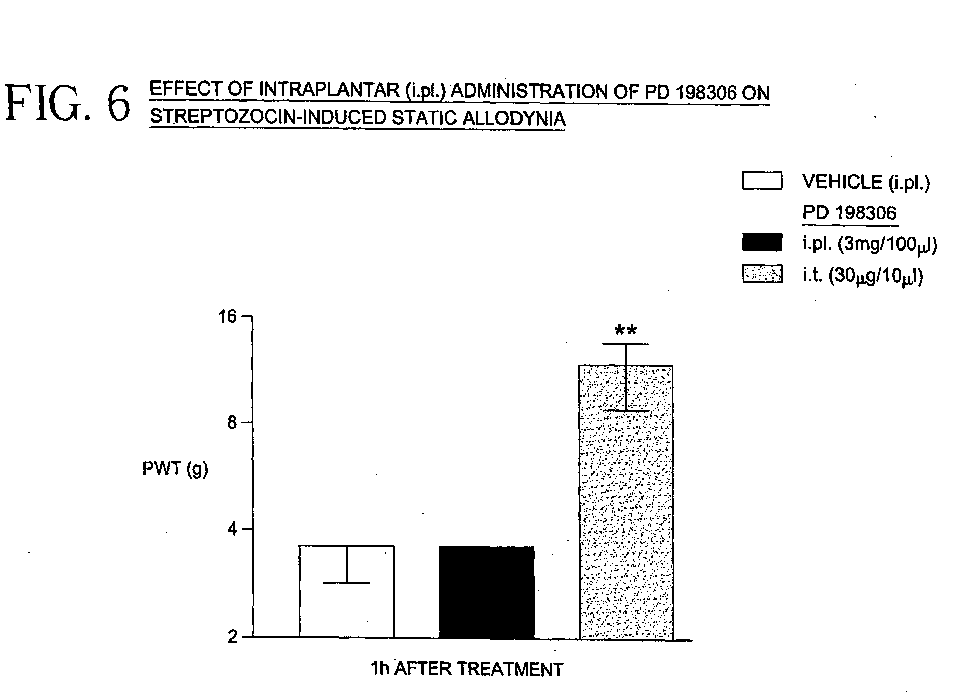

FIG. 6 is a bar graph representing the force required in grams to elicit paw withdrawal using von Frey hair filaments as a function of time in days . Animals received a single intraplantar (i.pl.) administration of PD 198306 (3mg/100µl), or an intrathecal injection of PD 198306 (30µg/10µl) and withdrawal thresholds were re-assessed 1h after treatment. Results are expressed median ± 1^{st} and 3^{rd} quartiles. **P<0.01 significantly different from vehicle treated animals (Mann-Whitney t test; n=6-9).

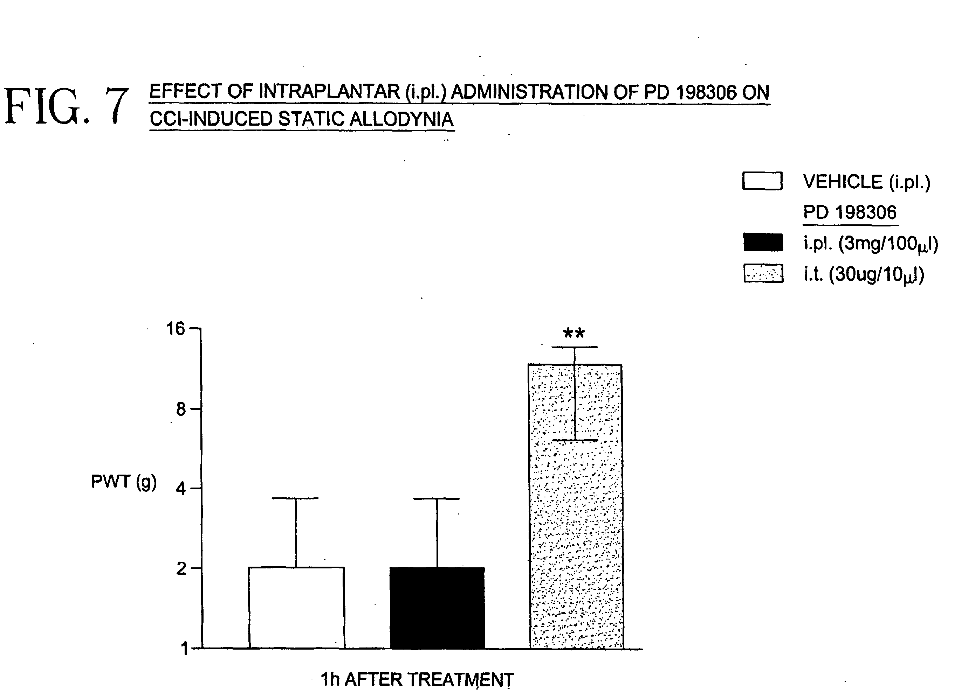

FIG. 7. is a bar graph representing the force required in grams to elicit paw withdrawal using von Frey hair filaments as a function of time in days. Animals received a single intraplantar (i.pl.) administration of PD 198306 (3mg/100µl), or an intrathecal injection of PD 198306 (30µg/10µl) and withdrawal thresholds were re-assessed 1h after treatment. Results are expressed median ± 1^{st} and 3^{rd} quartiles. **P<0.01 significantly different from vehicle treated animals (Mann-Whitney t test; n=6).

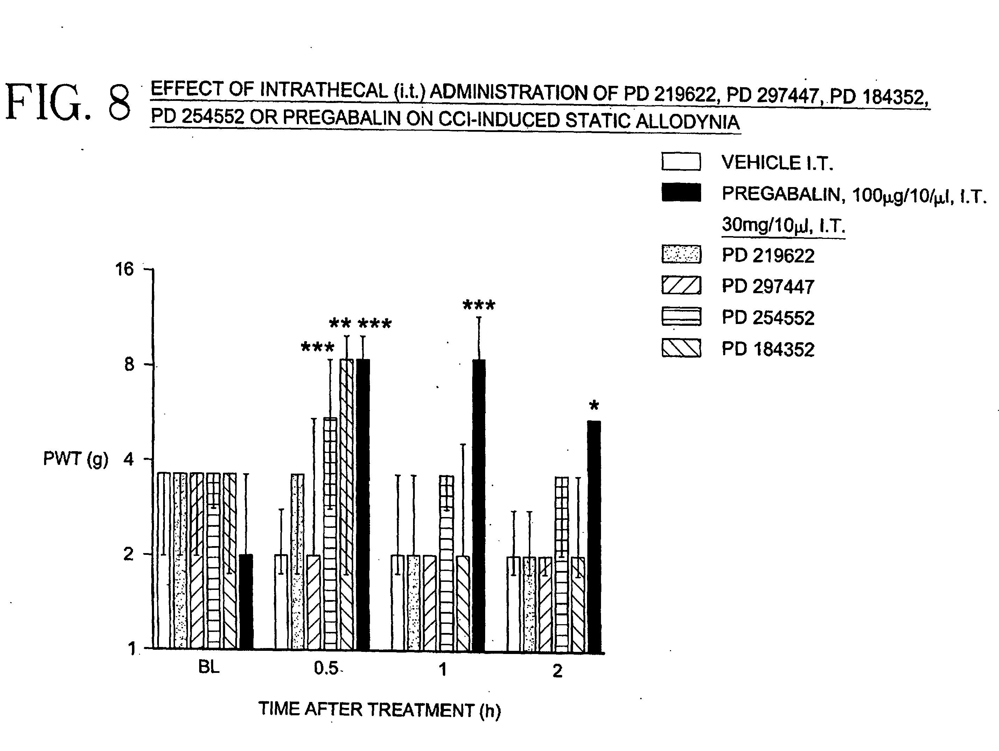

FIG. 8 is a bar graph representing the force required in grams to elicit paw withdrawal using von Frey hair filaments. Baseline (BL) measurements were taken before treatment. Animals received a single i.t. administration of PD219622, PD297447, PD 184352, or PD 254552 (30µg/10µl), or pregabalin (100µg/10µl) and withdrawal thresholds were re-assessed at 30min, 1h and 2h after treatment. Results are expressed median ± 1^{st} and 3^{rd} quartiles. *P<0.05, **P<0.01, ***P<0.001 significantly different from vehicle treated animals (Mann-Whitney t test; n=7-8).

### DETAILED DESCRIPTION

The compounds disclosed herein are pharmaceutically active, for example, they inhibit MEK. MEK enzymes are dual specificity kinases involved in, for example, immunomodulation, inflammation, and proliferative diseases such as cancer and restenosis.

Proliferative diseases are caused by a defect in the intracellular signaling system, or the signal transduction mechanism of certain proteins. Defects include a change either in the intrinsic activity or in the cellular concentration of one or more signaling proteins in the signaling cascade . The cell may produce a growth factor that binds to its own receptors, resulting in an autocrine loop, which continually stimulates proliferation. Mutations or overexpression of intracellular signaling proteins can lead to spurious mitogenic signals within the cell. Some of the most common mutations occur in genes encoding the protein known as Ras, a G-protein that is activated when bound to GTP, and inactivated when bound to GDP. The above-mentioned growth factor receptors, and many other mitogenic receptors, when activated, lead to Ras being converted from the GDP-bound state to the GTP-bound state. This signal is an absolute prerequisite for proliferation in most cell types. Defects in this signaling system, especially in the deactivation of the Ras-GTP complex, are common in cancers, and lead to the signaling cascade below Ras being chronically activated.

Activated Ras leads in turn to the activation of a cascade of serine/threonine kinases. One of the groups of kinases known to require an active Ras-GTP for its own activation is the Raf family. These in turn activate MEK (e.g., MEK₁ and MEK₂) which then activates MAP kinase, ERK (ERK₁ and ERK₂). Activation of MAP kinase by mitogens appears to be essential for proliferation; constitutive activation of this kinase is sufficient to induce cellular transformation. Blockade of downstream Ras signaling, for example by use of a dominant negative Raf-1 protein, can completely inhibit mitogenesis, whether induced from cell surface receptors or from oncogenic Ras mutants. Although Ras is not itself a protein kinase, it participates in the activation of Raf and other kinases, most likely through a phosphorylation mechanism. Once activated, Raf and other kinases phosphorylate MEK on two closely adjacent serine residues, S²¹⁸ and S²²² in the case of MEK-1, which are the prerequisite for activation of MEK as a kinase. MEK in turn phosphorylates MAP kinase on both a tyrosine, Y¹⁸⁵, and a threonine residue, T¹⁸³, separated by a single amino acid.

This double phosphorylation activates MAP kinase at least 100-fold. Activated MAP kinase can then catalyze the phosphorylation of a large number of proteins, including several transcription factors and other kinases. Many of these MAP kinase phosphorylations are mitogenically activating for the target protein, such as a kinase, a transcription factor, or another cellular protein. In addition to Raf-1 and MEKK, other kinases activate MEK, and MEK itself appears to be a signal integrating kinase. Current understanding is that MEK is highly specific for the phosphorylation of MAP kinase. In fact, no substrate for MEK other than the MAP kinase , ERK, has been demonstrated to date and MEK does not phosphorylate peptides based on the MAP kinase phosphorylation sequence, or even phosphorylate denatured MAP kinase. MEK also appears to associate strongly with MAP kinase prior to phosphorylating it, suggesting that phosphorylation of MAP kinase by MEK may require a prior strong interaction between the two proteins. Both this requirement and the unusual specificity of MEK are suggestive that it may have enough difference in its mechanism of action to other protein kinases that selective inhibitors of MEK, possibly operating through allosteric mechanisms rather than through the usual blockade of the ATP binding site, may be found.

The effect of the MEK inhibitor PD 198306 has been investigated in two animal models of neuropathic pain by assessing static allodynia with von Frey hairs.

Oral administration of PD 198306 (3-30mg/kg) had no effect in the model of chronic constriction injury of the sciatic nerve (CCI). However, after repeated administration (3 doses over two days) it had a transient effect in the diabetic neuropathy model (streptozocin). This may be due to disorders of the blood-brain barrier induced by the diabetic condition in these animals, thus allowing central action of the compound. Intrathecal administration of PD 198306 (1-30µg) dose-dependently blocked static allodynia in both the streptozocin and the CCI models of neuropathic pain, with minimum effective doses (MED) of 3 and 10µg respectively. The highest dose used (30µg) totally blocked the maintenance of static allodynia, for up to 1h. Intraplantar administration of PD 198306 (3mg/100µl) at a dose 100-fold higher than the dose shown to be effective intrathecally (30µg/10µl) had no effect on static allodynia in either of the neuropathic pain models. This finding confirms the lack of effect seen after systemic administration and suggests a central site of action for the compound.

From this study we can suggest the use of MEK inhibitors as potential new therapeutic tools for chronic pain. The study of potential side-effects, especially related to memory, of future brain-penetrant MEK inhibitors will indicate the therapeutic window for this novel class of compounds in the treatment of pain.

### A. Terms

Certain terms are defined below and by their usage throughout this disclosure.

Alkyl groups include aliphatic (i.e., hydrocarbyl or hydrocarbon radical structures containing hydrogen and carbon atoms) with a free valence. Alkyl groups are understood to include straight chain and branched structures. Examples include methyl, ethyl, propyl, isopropyl, butyl, n-butyl, isobutyl, t-butyl, pentyl, isopentyl, 2,3-dimethylpropyl, hexyl, 2,3-dimethylhexyl, 1,1-dimethylpentyl, heptyl, and octyl. Cycloalkyl groups include cyclopropyl, cyclobutyl, cyclopentyl, cyclohexyl, cycloheptyl, and cyclooctyl.

Alkyl groups can be substituted with 1, 2, 3 or more substituents which are independently selected from halo (fluoro, chloro, bromo, or iodo), hydroxy, amino, alkoxy, alkylamino, dialkylamino, cycloalkyl, aryl, aryloxy, arylalkyloxy, heterocyclic radical, and (heterocyclic radical)oxy. Specific examples include fluoromethyl, hydroxyethyl, 2,3-dihydroxyethyl, (2- or 3-furanyl)methyl, cyclopropylmethyl, benzyloxyethyl, (3-pyridinyl)methyl, (2- or 3-furanyl)methyl, (2-thienyl)ethyl, hydroxypropyl, aminocyclohexyl, 2-dimethylaminobutyl, methoxymethyl, *N*-pyridinylethyl, diethylaminoethyl, and cyclobutylmethyl.

Alkenyl groups are analogous to alkyl groups, but have at least one double bond (two adjacent sp² carbon atoms). Depending on the placement of a double bond and substituents, if any, the geometry of the double bond may be *entgegen* (E), or *zusammen* (Z), *cis,* or *trans.* Similarly, alkynyl groups have at least one triple bond (two adjacent sp carbon atoms). Unsaturated alkenyl or alkynyl groups may have one or more double or triple bonds, respectively, or a mixture thereof; like alkyl groups, unsaturated groups may be straight chain or branched, and they may be substituted as described both above for alkyl groups and throughout the disclosure by example. Examples of alkenyls, alkynyls, and substituted forms include cis-2-butenyl, trans-2-butenyl, 3-butynyl, 3-phenyl-2-propynyl, 3-(2'-fluorophenyl)-2-propynyl, 3-methyl(5-phenyl)-4-pentynyl, 2-hydroxy-2-propynyl, 2-methyl-2-propynyl, 2-propenyl, 4-hydroxy-3-butynyl, 3-(3-fluorophenyl)-2-propynyl, and 2-methyl-2-propenyl. In formula (I), alkenyls and alkynyls can be C ₂₋₄ or C ₂₋₈, for example, and are preferably C ₃₋₄ or C ₃₋₈.

More general forms of substituted hydrocarbon radicals include hydroxyalkyl, hydroxyalkenyl, hydroxyalkynyl, hydroxycycloalkyl, hydroxyaryl, and corresponding forms for the prefixes amino-, halo- (e.g., fluoro-, chloro-, or bromo-), nitro-, alkyl-, phenyl-, cycloalkyl- and so on, or combinations of substituents. According to formula (I), therefore, substituted alkyls include hydroxyalkyl, aminoalkyl, nitroalkyl, haloalkyl, alkylalkyl (branched alkyls, such as methylpentyl), (cycloalkyl)alkyl, phenylalkyl, alkoxy, alkylaminoalkyl, dialkylaminoalkyl, arylalkyl, aryloxyalkyl, arylalkyloxyalkyl, (heterocyclic radical)alkyl, and (heterocyclic radical)oxyalkyl. R₁ thus includes hydroxyalkyl, hydroxyalkenyl, hydroxyalkynyl, hydroxycycloalkyl, hydroxyaryl, aminoalkyl, aminoalkenyl, aminoalkynyl, aminocycloalkyl, aminoaryl, alkylalkenyl, (alkylaryl)alkyl, (haloaryl)alkyl, (hydroxyaryl)alkynyl, and so forth. Similarly, R_{A} includes hydroxyalkyl and aminoaryl, and R_{B} includes hydroxyalkyl, aminoalkyl, and hydroxyalkyl(heterocyclic radical)alkyl.

Heterocyclic radicals, which include but are not limited to heteroaryls, include: furyl, oxazolyl, isoxazolyl, thiophenyl, thiazolyl, pyrrolyl, imidazolyl, 1,3,4-triazolyl, tetrazolyl, pyridinyl, pyrimidinyl, pyridazinyl, indolyl, and their nonaromatic counterparts. Further examples of heterocyclic radicals include piperidyl, quinolyl, isothiazolyl, piperidinyl, morpholinyl, piperazinyl, tetrahydrofuryl, tetrahydropyrrolyl, pyrrolidinyl, octahydroindolyl, octahydrobenzothiofuranyl, and octahydrobenzofuranyl.

Selective MEK 1 or MEK 2 inhibitors are those compounds which inhibit the MEK 1 or MEK 2 enzymes, respectively, without substantially inhibiting other enzymes such as MKK3, PKC, Cdk2A, phosphorylase kinase, EGF, and PDGF receptor kinases, and C-src. In general, a selective MEK 1 or MEK 2 inhibitor has an IC₅₀ for MEK 1 or MEK 2 that is at least one-fiftieth (1/50) that of its IC₅₀ for one of the above-named other enzymes. Preferably, a selective inhibitor has an IC₅₀ that is at least 1/100, more preferably 1/500, and even more preferably 1/1000, 1/5000, or less than that of its IC₅₀ for one or more of the above-named enzymes.

### B. Compounds

One aspect of the invention features the use of disclosed compounds shown in formula (I) in the Summary section.

Embodiments of the invention include compounds wherein: (a) R₃ is bromo or chloro; (b) R₄ is fluoro; (c) R₅ is H; (d) each of R₄ and R₅ is H; (e) each of R₄ and R₅ is fluoro; (f) R₃ is bromo; (g) R₃ is fluoro; (h) R₄ is nitro; (i) R₅ is H; (j) R₆ is chloro; (k) R₆ is methyl; (I) R₁ is H or C ₁₋₄ alkyl, and R₂ is H; (m) R₁ is (C ₃₋₆ cycloalkyl)methyl; (n) R₁ is H; (o) R₁ is (CH₂) ₂₋₄OR_{C} or (CH₂) ₂₋₄ NR_{C}R_{D}; (p) R₆ is chloro or methyl; (q) R₆ is H; or combinations thereof.

Preferably, when R₁, R_{C}, or R_{D} is an alkenyl or alkynyl, the double or triple bond, respectively, is not adjacent the point of attachment when the point of attachment is a heteroatom. For example, R₁ is preferably prop-2-ynyl, or but-2 or 3-enyl, and less preferably prop-1-ynyl or but-1-enyl.

Examples of compounds of formula (I) include: 4-fluoro-2-(4-iodo-2-methyl-phenylamino)-benzenesulfonic acid; 4-fluoro-N-hydroxy-2-(4-iodo-2-methyl-phenylamino)-benzenesulfonamide; N-cyclopropylmethoxy-4-fluoro-2-(4-iodo-2-methyl-phenylamino)-benzenesulfonamide; 3,4-difluoro-2-(4-iodo-2-methyl-phenylamino)-benzenesulfonic acid; 3,4-difluoro-N-hydroxy-2-(4-iodo-2-methyl-phenylamino)-benzenesulfonamide; N-cyclopropylmethoxy-3,4-difluoro-2-(4-iodo-2-methyl-phenylamino)-benzenesulfonamide; 3,4,5-trifluoro-2-(4-iodo-2-methyl-phenylamino)-benzenesulfonic acid; 3,4,5-trifluoro-N-hydroxy-2-(4-iodo-2-methyl-phenylamino)-benzenesulfonamide; N-cyclopropylmethoxy-3,4,5-trifluoro-2-(4-iodo-2-methyl-phenylamino)-benzenesulfonamide; 5-bromo-3,4-difluoro-2-(4-iodo-2-methyl-phenylamino)-benzenesulfonic acid; 5-bromo-3,4-difluoro-N-hydroxy-2-(4-iodo-2-methyl-phenylamino)-benzenesulfonamide; 5-bromo-N-cyclopropylmethoxy-3,4-difluoro-2-(4-iodo-2-methyl-phenylamino)-benzenesulfonamide; 2-(4-iodo-2-methyl-phenylamino)-4-nitro-benzenesulfonic acid; N-hydroxy-2-(4-iodo-2-methyl-phenylamino)-4-nitro-benzenesulfonamide; or N-cyclopropylmethoxy-2-(4-iodo-2-methyl-phenylamino)-4-nitrobenzenesulfonamide.

Further examples of compounds include: 2-(2-chloro-4-iodophenylamino)-4-fluoro-benzenesulfonic acid; 2-(2-chloro-4-iodo-phenylamino)-4-fluoro-N-hydroxy-benzenesulfonamide; 2-(2-chloro-4-iodo-phenylamino)-N-cyclopropylmethoxy-4-fluoro-benzenesulfonamide; 2-(2-chloro-4-iodophenylamino)-3,4-difluoro-benzenesulfonic acid; 2-(2-chloro-4-iodophenylamino)-3,4-difluoro-N-hydroxy-benzenesulfonamide; 2-(2-chloro-4-iodophenylamino)-N-cyclopropylmethoxy-3,4-difluoro-benzenesulfonamide; 2-(2-chloro-4-iodo-phenylamino)-3,4,5-trifluoro-benzenesulfonic acid; 2-(2-chloro-4-iodo-phenylamino)-3,4,5-trifluoro-N-hydroxy-benzenesulfonamide; 2-(2-chloro-4-iodo-phenylamino)-N-cyclopropylmethoxy-3,4,5-trifluoro-benzenesulfonamide; 5-bromo-2-(2-chloro-4-iodo-phenylamino)-3,4-difluoro-benzenesulfonic acid; 5-bromo-2-(2-chloro-4-iodo-phenylamino)-3,4-difluoro-N-hydroxybenzenesulfonamide; 5-bromo-2-(2-chloro-4-iodo-phenylamino)-N-cyclopropylmethoxy-3,4-difluoro-benzenesulfonamide; 2-(2-chloro-4-iodophenyiamino)-4-nitro-benzenesulfonic acid; 2-(2-chloro-4-iodo-phenylamino)-N-hydroxy-4-nitro-benzenesulfonamide; or 2-(2-chloro-4-iodo-phenylamino)-N-cyclopropylmethoxy-4-nitro-benzenesulfonamide.

A second aspect of the invention features the use of compounds shown in formulae (I)A and (II)A in the Summary section. Embodiments of the invention includes compounds of formula (I)A wherein: (a) R₃ is NO₂; (b) R₄ is fluoro; (c) each of R₃ and R₄ is independently selected from H and fluoro; (d) R₅ is methyl, fluoro, or chloro; (e) R₆ is methyl, chloro, fluoro, nitro, or hydrogen; (f) R₆ is H; (g) R₆ is fluoro; (h) R_{K} is methyl or ethyl; (i) R₁ is H, methyl, ethyl, propyl, isopropyl, isobutyl, benzyl, phenyl, phenethyl, allyl, C ₃₋₅ alkenyl, C ₃₋₆ cycloalkyl, (C ₃₋₅ cycloalkyl)C ₁₋₂ alkyl, (C ₃₋₅ heterocyclic radical)C ₁₋₂ alkyl, or (CH₂)₂₋₄ NR_{C}R_{D}; (j) R₁ is H or (C ₃₋₄ cycloalkyl)C ₁₋₂ alkyl; (k) R₂ is H or methyl; (I) R_{A} has at least one hydroxyl substituent; (m) R_{A} is H, methyl, ethyl, isobutyl, hydroxyethyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl; (n) W is NR_{A}R_{B} or NR₂NR_{A}R_{B}; (o) W is NR₂(CH₂)₂₋₄ NR_{A}R_{B} or O(CH₂)₂₋₃ NR_{A}R_{B}; (p) W is NR₂OR₁; (q) W is OR_{B}; (r) R₇ is in the para position relative to X; (s) R₇ is iodo; (t) R₈ is in the ortho position relative to X; (u) or combinations thereof.

In additional embodiments, if R₆ is H, then R₅ is nitro; or R₆ is methyl, halo, or nitro; or R₃ is SO₂NR_{I}(CH₂)₂₋₄NR_{E}R_{F}, SO₂NR_{I}R_{K} or (CO)T. In some embodiments, Ar is phenyl (e.g., formula (I)A), and in other embodiments, Ar is 2-pyridyl, 3-pyridyl, or 4-pyridyl. Preferably, where one of R₁, R₂, R_{A}, R_{B}, R_{C}, and R_{D} is an alkenyl or alkynyl group, the double or triple bond, respectively, is not adjacent the point of attachment. For example, where W is NR₂OR₁, R₂ is preferably prop-2-ynyl, or but-2 or 3-enyl, and less preferably prop-1-ynyl or but-1-enyl. Some embodiments include the formula 2,4-bis-(2-chloro-4-iodophenylamino)-3-fluoro-5-nitro-benzoic acid, the compounds in the following list, and 2-methyl (instead of 2-chloro) analogs thereof.
1. 2-(2-Chloro-4-iodo-phenylamino)-3-fluoro-5-nitro-4-(4-sulfamoylphenylamino)-benzoic acid;
2. 2-(2-Chloro-4-iodo-phenylamino)-3-fluoro-5-nitro-4-phenylamino-benzoic acid;
3. 2-(2-Chloro-4-iodo-phenylamino)-3-fluoro-5-nitro-4-phenoxy-benzoic acid;
4. 2-(2-Chloro-4-iodo-phenylamino)-3-fluoro-5-nitro-4-phenylsulfanyl-benzoic acid;
5. 2-(2-Chloro-4-iodo-phenylamino)-3-fluoro-4-(methyl-phenyl-amino)-5-nitrobenzoic acid;
6. 2-[(2-chloro-4-iodophenyl)amino]-3-fluoro-4-[[4-[[(2-hydroxyethyl)amino]-carbonyl]phenyl]amino]-5-nitro-benzoic acid;
7. 2-[(2-chloro-4-iodophenyl)amino]-4-[[4-[(dimethylamino)carbonyl] phenyl]amino]-3-fluoro-5-nitro-benzoic acid;
8. 2-(2-Chloro-4-iodo-phenylamino)-3,5-difluoro-4-phenylamino-benzoic acid;
9. 2-(2-Chloro-4-iodo-phenylamino)-3-fluoro-5-nitro-4-(3-sulfamoylphenylamino)-benzoic acid;
10. 2-(2-Chloro-4-iodo-phenylamino)-3-fluoro-5-nitro-4-(2-sulfamoylphenylamino)-benzoic acid;
11. 2-(2-Chloro-4-iodo-phenylamino)-3-fluoro-N-hydroxy-5-nitro-4-(4-sulfamoyl-phenylamino)-benzamide;
12. 2-(2-Chloro-4-iodo-phenylamino)-3-fluoro-N-hydroxy-5-nitro-4-phenylamino-benzamide;
13. 2-(2-Chloro-4-iodo-phenylamino)-3-fluoro-N-hydroxy-5-nitro-4-phenoxybenzamide;
14. 2-(2-Chloro-4-iodo-phenylamino)-3-fluoro-N-hydroxy-5-nitro-4-phenylsulfanyl-benzamide;
15. 2-(2-Chloro-4-iodo-phenylamino)-3-fluoro-N-hydroxy-4-(methyl-phenylamino)-5-nitro-benzamide;
16. 2-[(2-chloro-4-iodophenyl)amino]-3-fluoro-N-hydroxy-4-[[4-[[(2-hydroxyethyl)amino]-carbonyl]phenyl]amino]-5-nitro-benzamide;
17. 2-[(2-chloro-4-iodophenyl)amino]-4-[[4-[(dimethylamino)carbonyl] phenyl]amino]-3-fluoro-N-hydroxy-5-nitro-benzamide;
18. 2-(2-Chloro-4-iodo-phenylamino)-3,5-difluoro-N-hydroxy-4-phenylaminobenzamide;
19. 2-(2-Chloro-4-iodo-phenylamino)-3-fluoro-N-hydroxy-5-nitro-4-(3-sulfamoyl-phenylamino)-benzamide;
20. 2-(2-Chloro-4-iodo-phenylamino)-3-fluoro-N-hydroxy-5-nitro-4-(2-sulfamoyl-phenylamino)-benzamide;
21. 2-(2-Chloro-4-iodo-phenylamino)-N-cyclopropylmethoxy-3-fluoro-5-nitro-4-(4-sulfamoyl-phenylamino)-benzamide;
22. 2-(2-Chloro-4-iodo-phenylamino)-N-cyclopropylmethoxy-3-fluoro-5-nitro-4-phenylamino-benzamide;
23. 2-(2-Chloro-4-iodo-phenylamino)-N-cyclopropylmethoxy-3-fluoro-5-nitro-4-phenoxy-benzamide;
24. 2-(2-Chloro-4-iodo-phenylamino)-N-cyclopropylmethoxy-3-fluoro-5-nitro-4-phenylsulfanyl-benzamide;
25. 2-(2-Chloro-4-iodo-phenylamino)-N-cyclopropylmethoxy-3-fluoro-4-(rnethyl-phenyl-amino)-5-nitro-benzamide;
26. 2-[(2-chloro-4-iodophenyl)amino]-3-fluoro-N-cyclopropylmethoxy-4-[[4-[[(2-hydroxyethyl)amino]-carbonyl]phenyl]amino]-5-nitro-benzamide;
27. 2-[(2-chloro-4-iodophenyl)amino]-4-[[4-[(dimethylamino)carbonyl]phenyl]-amino]-3-fluoro-N-cyclopropylmethoxy-5-nitro-benzamide;
28. 2-(2-Chloro-4-iodo-phenylamino)-N-cyclopropylmethoxy-3,5-difluoro-4-phenylamino-benzamide;
29. 2-(2-Chloro-4-iodo-phenylamino)-N-cyclopropylmethoxy-3-fluoro-5-nitro-4-(3-sulfamoyl-phenylamino)-benzamide; and
30. 2-(2-Chloro-4-iodo-phenylamino)-N-cyclopropylmethoxy-3-fluoro-5-nitro-4-(2-sulfamoyl-phenylamino)-benzamide.

In the scheme below, W can also be any of the values described herein for formula (I)A or (II)A in the section describing preferred values for W. The compound numbers provided in the scheme correspond to the numbers provided in the above list; these compounds are illustrative, not limitative, of the invention.

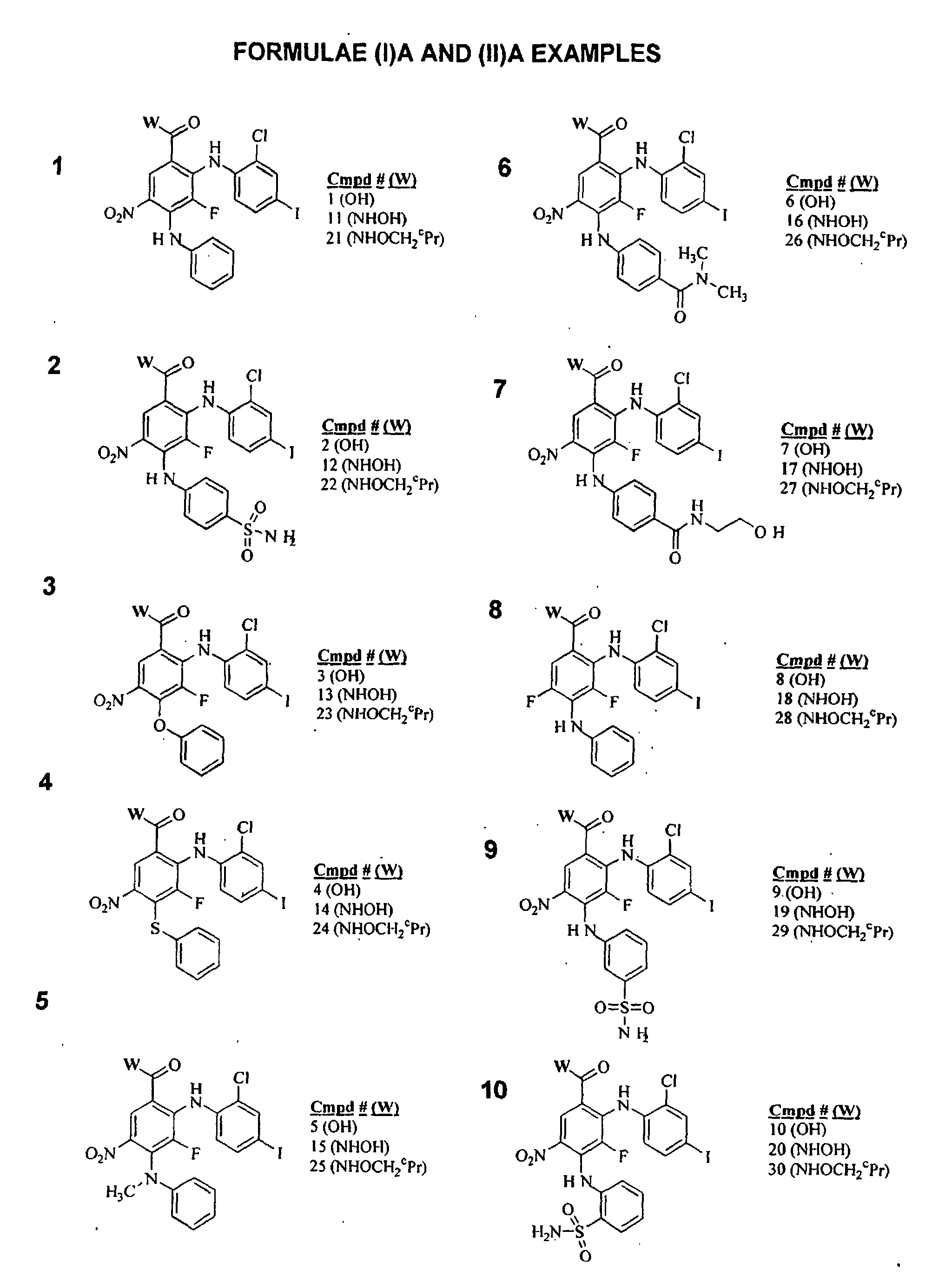

A third aspect of the invention features the use of compounds shown in formula (I)B in the Summary section. Embodiments of the invention include compounds wherein: (a) R_{C} is C ₁₋₂ alkyl; (b) W is OH, or W is NHOR₁ (c) R₁₀ is methyl or chloro; (d) R₁₁ is fluoro; (e) R₁₁ is H; (f) J is trihalomethyl or methylthio; (g) J is SO₂CH₃; (h) J is SOCH₃; (i) J is C ₃₋₈ alkynyl where the triple bond is between the carbon atoms alpha and beta to the phenyl group; (j) R₁ has at least. one hydroxy substituent; (k) R₁ is H, methyl, ethyl, propyl, isopropyl, isobutyl, benzyl, phenethyl, allyl, C ₃₋₅ alkenyl, C ₃₋₅ alkynyl, C ₃₋₆ cycloalkyl, (C ₃₋₅ cycloalkyl)C ₁₋₂ alkyl, or (C ₃₋₅ heterocyclic radical)C ₁₋₂ alkyl; (I) R₁ is H or (C ₃₋₄ cycloalkyl)C ₁₋₂ alkyl; (m) R₂ is H, methyl, C ₃₋₄ alkynyl, C ₃₋₅ cycloalkyl, or (C ₃₋₅ cycloalkyl)methyl; (n) R_{A} is H, methyl, ethyl, isobutyl, hydroxyethyl, hydroxypropyl, cyclopropylmethyl, cyclobutylmethyl, C ₃₋₄ alkynyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl; (o) each of R₄ and R₆ is H, and R₅ is F; (p) each of R₄, R₅, and R₆ is F; (q) R₅ is F; (r) each R₅ and R₆ is F and R₆ is Br; (s) each R₅ and R₆ is F and R₆ is H; (t) J is 1,2,5-thiadiazol-3-yl; or a combination thereof.

Preferably, where one of R₁, R₂, R_{A}, R_{B}, R_{C}, R_{D}, R_{E}, R_{F}, and R_{G}, for example, is an alkenyl or alkynyl group, its double or triple bond, respectively, is not adjacent the point of attachment. For example, where W is NR₂OR₁, R₂ is preferably prop-2-ynyl, or but-2 or 3-enyl, and less preferably prop-1-ynyl or but-1-enyl.

Examples of compounds of formula (I)B include: 4-fluoro-2-(2-methyl-4-methylsulfanyl-phenylamino)-benzoic acid; 5-bromo-3,4-difluoro-2-(2-methyl-4-methylsulfanyl-phenylamino)-benzoic acid; 3,4-difluoro-2-(4-methanesulfinyl-2-methyl-phenylamino)-benzoic acid; 2-(4-methanesulfinyl-2-methyl-phenylamino)-4-nitro-benzoic acid; 3,4,5-trifluoro-2-(4-methanesulfonyl-2-methyl-phenylamino)-benzoic acid; 3,4-difluoro-2-(2-methyl-4-methylsulfanyl-phenylamino)-benzoic acid; 2-(2-methyl-4-methylsulfanyl-phenylamino)-4-nitro-benzoic acid; 3,4,5-trifluoro-2-(4-methanesulfinyl-2-methyl-phenylamino)-benzoic acid; 4-fluoro-2-(4-methanesulfinyl-2-methyl-phenylamino)-benzoic acid; 5-bromo-3,4-difluoro-2-(4-methanesulfonyl-2-methyl-phenylamino)-benzoic acid; 3,4,5-trifluoro-2-(2-methyl-4-methylsulfanyl-phenylamino)-benzoic acid; 4-fluoro-2-(4-methane-sulfinyl-2-methyl-phenylamino)-benzoic acid; 5-bromo-3,4-difluoro-2-(4-methanesulfinyl-2-methyl-phenylamino)-benzoic acid; 3,4-difluoro-2-(4-methanesulfonyl-2-methylphenylamino)-benzoic acid; and 2-(4-methanesulfonyl-2-methyl-phenylamino)-4-nitro-benzoic acid; and the corresponding hydroxamic acid or cyclopropylhydroxamic acid of each.

Preferred examples of compounds of formula (I)B are: 4-Fluoro-2-(4-methanesulfanyl-phenylamino)-benzoic acid (**1**); 4-Fluoro-2-(4-methanesulfinylphenylamino)-benzoic acid (**2**); 4-Fluoro-2-(4-methanesulfonyl-phenylamino)-benzoic acid (**3**); 4-Fluoro-2-(2-methyl-4-trimethylsilanylethynyl-phenylamino)-benzoic acid (**6**); 4-Fluoro-2-(2-methyl-4-ethynyl-phenylamino)-benzoic acid (**7**). Biological data on these seven compounds is given on page 17; full characterization of the compounds - MP, NMR, MS, IR and CHN- is given on pages 28-31.

Additional preferred compounds include the following: (a) 5-Bromo-2-(4-ethynyl-2-methyl-phenylamino)-3,4-difluoro-benzoic acid; N-Cyclopropylmethoxy-2-(4-ethynyl-2-methyl-phenylamino)-3,4-difluoro-benzamide; 2-(4-Ethynyl-2-methyl-phenylamino)-3,4-difluoro-benzoic acid; N-Cyclopropylmethoxy-2-(4-ethynyl-2-methyl-phenylamino)-3,4,5-trifluoro-benzamide; 2-(4-Ethynyl-2-methylphenylamino)-3,4,5-trifluoro-benzoic acid; 5-Bromo-N-cyclopropylmethoxy-2-(4-ethynyl-2-methyl-phenylamino)-3,4-difluoro-benzamide; (b) 5-Bromo-2-(4-ethynyl-CI-methyl-phenylamino)-3,4-difluoro-benzoic acid; N-Cyclopropylmethoxy-2-(4-ethynyl-CI-methyl-phenylamino)-3,4-difluoro-benzamide; 2-(4-Ethynyl-CI-methylphenylamino)-3,4-difluoro-benzoic acid; N-Cyclopropylmethoxy-2-(4-ethynyl-CI-methyl-phenylamino)-3,4,5-trifluoro-benzamide; 2-(4-Ethynyl-CI-methylphenylamino)-3,4,5-trifluoro-benzoic acid; 5-Bromo-N-cyclopropylmethoxy-2-(4-ethynyl-CI-methyl-phenylamino)-3,4-difluoro-benzamide; (c) 5-bromo-3,4-difluoro-2-(2-methyl-4-methylsulfanyl-phenylamino)-benzoic acid; 3,4-difluoro-2-(4-methanesulfinyl-2-methyl-phenylamino)-benzoic acid; 3,4,5-trifluoro-2-(4-methanesulfonyl-2-methyl-phenylamino)-benzoic acid; 3,4-difluoro-2-(2-methyl-4-methylsulfanyl-phenylamino)-benzoic acid; 5-bromo-3,4-difluoro-2-(4-methanesulfonyl-2-methyl-phenylamino)-benzoic acid; 3,4-difluoro-2-(4-methanesulfonyl-2-methyl-phenylamino)-benzoic acid; (d) 5-bromo-N-cyclopropylmethoxy-3,4-difluoro-2-(2-methyl-4-methylsulfanyl-phenylamino)-benzamide; N-cyclopropylmethoxy-3,4-difluoro-2-(4-methanesulfinyl-2-methylphenylamino)-benzamide; N-cyclopropylmethoxy-3,4,5-trifluoro-2-(4-methanesulfonyl-2-methyl-phenylamino)-benzamide; N-cyclopropylmethoxy-3,4-difluoro-2-(2-methyl-4-methylsulfanyl-phenylamino)-benzamide; N-cyclopropylmethoxy-3,4,5-trifluoro-2-(4-methanesulfinyl-2-methylphenylamino)-benzamide; 5-bromo-N-cyclopropylmethoxy-3,4-difluoro-2-(4-methanesulfonyl-2-methyl-phenylamino)-benzamide; N-cyclopropylmethoxy-3,4,5-trifluoro-2-(2-methyl-4-methylsulfanyl-phenylamino)-benzamide; N-cyclopropylmethoxy-3,4-difluoro-2-(4-methanesulfonyl-2-methyl-phenylamino)-benzamide; (e) N-cyclopropylmethoxy-3,4-difluoro-2-(4-imidazol-1-yl-2-methylphenylamino)-benzamide; (f) N-cyclopropylmethoxy-3,4,5-trifluoro-2-(2-methyl-4-[1,2,5]thiadiazol-3-yl-phenylamino)-benzamide; 2-[4-(4-chloro-[1,2,5]thiadiazol-3-yl)-2-methyl-phenylamino]-3,4,5-trifluoro-benzoic acid; 2-[4-(4-chloro-[1,2,5]thiadiazol-3-yl)-2-methyl-phenylamino]-N-cyclopropylmethoxy-3,4,5-trifluoro-benzamide; (g) 2-{4-[4-(2-dimethylamino-ethoxy)-[1,2,5]thiadiazol-3-yl]-2-methyl-phenylamino}-3,4,5-trifluoro-benzoic acid; (h) N-cyclopropylmethoxy-3,4,5-trifluoro-2-{2-methyl-4-[4-(2-piperidin-1-yl-ethoxy)-[1,2,5]thiadiazol-3-yl]-phenylamino}-benzamide.

Further preferred compounds include: (a) 5-bromo-2-(2-chloro-4-methylsulfanyl-phenylamino)-3,4-difluoro-benzoic acid; 2-(2-chloro-4-methanesulfinyl-phenylamino)-3,4-difluoro-benzoic acid; 2-(2-chloro-4-methanesulfonyl-phenylamino)-3,4,5-trifluoro-benzoic acid; 2-(2-chloro-methylsulfanyl-phenylamino)-3,4-difluoro-benzoic acid; 5-bromo-2-(2-chloro-4-methanesulfonyl-phenylamino)-3,4-difluoro-benzoic acid; 2-(2-Chloro-4-methanesulfonyl-phenylamino)-3,4-difluoro-benzoic acid; (b) 5-bromo-2-(2-chloro-4-methylsulfanyl-phenylamino)-N-cyclopropylmethoxy-3,4-difluorobenzamide; 2-(2-chloro-4-methanesulfinyl-phenylamino)-N-cyclopropylmethoxy-3,4-difluoro-benzamide; 2-(2-chloro-4-methanesulfonyl-phenylamino)- N-cyclopropylmethoxy-3,4,5-trifluoro-benzamide; 2-(2-chloro-4-methylsulfanylphenylamino)- N-cyclopropylmethoxy-3,4-difluoro-benzamide; 2-(2-chloro-4-methanesulfinyl-phenylamino)- N-cyclopropylmethoxy-3,4,5-trifluoro-benzamide; 5-bromo-2-(2-chloro-4-methanesulfonyl-phenylamino)-N-cyclopropylmethoxy-3,4-difluoro-benzamide; 2-(2-chloro-4-methylsulfanyl-phenylamino)-N-cyclopropylmethoxy-3,4,5-trifluoro-benzamide; 2-(2-chloro-4-methanesulfonylphenylamino)-N-cyclopropylmethoxy-3,4-difluoro-benzamide; and (c) 2-[2-chloro 4-(3H-imidazol-1-yl)-phenylamino]-N-cyclopropylmethoxy-3,4-difluoro-benzamide; 2-(2-chloro-4-[1,2,5]thiadiazol-3-yl-phenylamino)-N-cyclopropylmethoxy-3,4,5-trifluoro-benzamide; 2-[4-(2-chloro-4-chloro-[1,2,5]thiadiazol-3-yl)-phenylamino]-3,4,5-trifluoro-benzoic acid; 2-[2-chloro-4-(4-chloro-[1,2,5]thiadiazol-3-yl)-phenylamino]-N-cyclopropylmethoxy-3,4,5-trifluoro-benzamide; 2-{4-[4-(2-dimethylamino-ethoxy)-[1,2,5]thiadiazol-3-yl]-2-methyl-phenylamino}-3,4,5-trifluoro-benzoic acid; 2-{2-chloro-4-[4-(2-piperidin-1-yl-ethoxy)-[1,2,5]thiadiazol-3-yl]-phenylamino}-N-cyclopropylmethoxy-3,4,5-trifluoro-benzamide;

Additional preferred compounds include: (a) 2-(2-Chloro-4-ethynylphenylamino)-4-fluoro-benzoic acid; 5-Bromo-2-(2-chloro-4-ethynylphenylamino)-3,4-difluoro-benzoic acid; 2-(2-Chloro-4-ethynyl-phenylamino)- N-cyclopropylmethoxy-3,4-difluoro-benzamide; 2-(2-Chloro-4-ethynyl-phenylamino)-N-cyclopropylmethoxy-4-nitro-benzamide; 2-(2-Chloro-4-ethynyl-phenylamino)-N-hydroxy-3,4,5-trifluoro- benzamide; 2-(2-Chloro-4-ethynyl-phenylamino)-3,4-difluoro-benzoic acid; 2-(4-Ethynyl-2-chloro-phenylamino)-4-nitro-benzoic acid; 2-(2-Chloro-4-ethynyl-phenylamino)- N-Cyclopropylmethoxy-3,4,5-trifluorobenzamide; 2-(2-chloro-4-methanesulfinyl-phenylamino)-4-fluoro-N-hydroxybenzamide; 5-Bromo-2-(4-ethynyl-2-chloro-phenylamino)-3,4-difluoro-N-hydroxybenzamide; (b) 2-(2-Chloro-4-ethynyl-phenylamino)-3,4,5-trifluoro-benzoic acid; 2-(2-Chloro-4-ethynyl-phenylamino)- N-cyclopropylmethoxy-4-fluoro-benzamide; 5-Bromo-2-(2-chloro-4-ethynyl-phenylamino)-N-cyclopropylmethoxy-3,4-difluorobenzamide; 2-(4-Ethynyl-2-chloro-phenylamino)-3,4-difluoro-N-hydroxybenzamide; 2-(4-Ethynyl-2-chloro-phenylamino)-N-hydroxy-4-nitro-benzamide; and (c) 2-(2-Chloro-4-ethynyl-phenylamino)-4-fluoro-benzoic acid; 2-(2-Chloro-4-ethynyl-phenylamino)- N-cyclopropylmethoxy-4-fluoro-benzamide; 2-(2-Chloro-4-methanesulfinyl-phenylamino)- 4-fluoro-N-hydroxy-benzamide; 2-(2-chloro-4-imidazol-1-yl-phenylamino)- 3,4-Difluoro-benzoic acid.

A fourth aspect of the invention features the use of compounds shown in formula (I)C in the Summary section.

Examples of compounds of formula (I)C have structures wherein:
(a) the sulfamoyl group is *meta* to W(CO)- and *para* to the bridging NH; (b) the sulfamoyl group is *para* to W (CO)- and *meta* to the bridging NH; (c) R₄ is fluoro; (d) R₃ is fluoro; (e) R₃ is H; (f) W is OH; (g) W is NR₂OR₁; (h) each of R₃ and R₄ is fluoro; (i) R₁ has at least one hydroxy substituent; (k) R₁ is H, methyl, ethyl, propyl, isopropyl, isobutyl, benzyl, phenethyl, allyl, C ₂₋₅ alkenyl, C ₂₋₅ alkynyl, C ₃₋₆ cycloalkyl, (C ₃₋₅ cycloalkyl)C ₁₋₂ alkyl, (C ₃₋₅ heterocyclic radical)-C ₁₋₂ alkyl, or (CH₂)₂₋₄NR_{A}R_{B}; (I) R₁ is H or (C ₃₋₄ cycloalkyl)C ₁₋₂ alkyl; (m) R₂ is H, methyl, C ₂₋₄ alkynyl, C ₃₋₅ cycloalkyl, or (C ₃₋₅ cycloalkyl)methyl; (n) R_{A} is H, methyl, ethyl, isobutyl, hydroxyethyl, hydroxypropyl, cyclopropylmethyl, cyclobutylmethyl, C ₃₋₄ alkynyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl; (o) R₇ is (CH₂)₂₋₄(NR_{C}R_{D}); (p) NR_{C}R_{D} is selected from morpholinyl, piperazinyl, pyrrolidinyl, or piperadinyl; (q) R_{C} is methyl, ethyl, hydroxyethyl, or hydroxypropyl; (r) R₅ is methyl or chloro; (s) R_{D} is methyl, ethyl, hydroxyethyl, or hydroxypropyl; (t) or combinations thereof, such as wherein each of R_{C} and R_{D} is methyl or ethyl.

Preferably, where one of R₁, R₂, R_{A}, R_{B}, R_{C}, or R_{D} is an alkenyl or alkynyl group, the double or triple bond, respectively, is not adjacent the point of attachment. For example, where W is NR₂OR₁, R₂ is preferably prop-2-ynyl, or but-2 or 3-enyl, and less preferably prop-1-ynyl or but-1-enyl.

Examples of compounds of formula (I)C include: 2-(2-chloro-4-iodophenylamino)-4-sulfamoyl-benzoic acid; 2-(2-chloro-4-iodo-phenylamino)-N-hydroxy-4-sulfamoyl-benzamide; 2-(2-chloro-4-iodo-phenylamino)-N-cyclopropylmethoxy-4-sulfamoyl-benzamide; 2-(2-chloro-4-iodo-phenylamino)-4-(2-morpholin-4-yl-ethylsulfamoyl)-benzoic acid; 2-(2-chloro-4-iodo-phenylamino)-N-hydroxy-4-(2-morpholin-4-yl-ethylsulfamoyl)-benzamide; 2-(2-chloro-4-iodophenylamino)-N-cyclopropylmethoxy-4-(2-morpholin-4-yl-ethylsulfamoyl)-benzamide; 2-(2-chloro-4-iodo-phenylamino)-3,4-difluoro-5-sulfamoyl-benzoic acid; 2-(2-chloro-4-iodo-phenylamino)-3,4-difluoro-N-hydroxy-5-sulfamoylbenzamide; 2-(2-chloro-4-iodo-phenylamino)-N-cyclopropylmethoxy-3,4-difluoro-5-sulfamoyl-benzamide; 2-(2-chloro-4-iodo-phenylamino)-3,4-difluoro-5-(2-morpholin-4-yl-ethylsulfamoyl)-benzoic acid; 2-(2-chloro-4-iodo-phenylamino)-3,4-difluoro-N-hydroxy-5-(2-morpholin-4-yl-ethylsulfamoyl)-benzamide; and 2-(2-chloro-4-iodo-phenylamino)-N-cyclopropylmethoxy-3,4-difluoro-5-(2-morpholin-4-yl-ethylsulfamoyl)-benzamide.

Other examples include 5-[bis-(4-methoxy-benzyl)-sulfamoyl]-2-(2-chloro-4-iodo-phenylamino)-3,4-difluoro-benzoic acid; and 2-(2-chloro-4-iodophenylamino)-5-dimethylsulfamoyl-3,4-difluoro-benzoic acid methyl ester.

Additional examples include 5-(bis-pyridin-3-ylmethyl-sulfamoyl)-3,4-difluoro-2-(4-iodo-phenylamino)-benzoic acid; 5-(bis-pyridin-3-ylmethylsulfamoyl)-N-cyclopropylmethoxy-3,4-difluoro-2-(4-iodo-phenylamino)-benzamide; N-cyclopropylmethoxy-3,4-difluoro-2-(4-iodo-phenylamino)-5-(methylpyridin-3-ylmethyl-sulfamoyl)-benzamide; N-cyclopropylmethoxy-3,4-difluoro-2-(4-iodo-phenylamino)-5-[(pyridin-3-ylmethyl)-sulfamoyl]-benzamide; N-cyclopropylmethoxy-5-[(3-diethylamino-propyl)-pyridin-3-ylmethyl-sulfamoyl]-3,4-difluoro-2-(4-iodo-phenylamino)-benzamide; N-cyclopropylmethoxy-3,4-difluoro-5-[(3-hydroxy-propyl)-pyridin-3-ylmethyl-sulfamoyl]-2-(4-iodo-phenylamino)-benzamide; N-cyclopropylmethoxy-5-(ethyl-pyridin-3-ylmethyl-sulfamoyl)-3,4-difluoro-2-(4-iodo-phenylamino)-benzamide; N-cyclopropylmethoxy-3,4-difluoro-5-[(2-hydroxy-ethyl)-pyridin-3-ylmethyl-sulfamoyl]-2-(4-iodo-phenylamino)-benzamide; 5-(bis-pyridin-2-ylmethyl-sulfamoyl)-3,4-difluoro-2-(4-iodophenylamino)-benzoic acid; 5-(bis-pyridin-2-ylmethyl-sulfamoyl)-N-cyclopropylmethoxy-3,4-difluoro-2-(4-iodo-phenylamino)-benzamide; N-cyclopropylmethoxy-3,4-difluoro-2-(4-iodo-phenylamino)-5-(methyl-pyridin-2-ylmethyl-sulfamoyl)-benzamide; N-cyclopropylmethoxy-3,4-difluoro-2-(4-iodophenylamino)-5-[(pyridin-2-ylmethyl)-sulfamoyl]-benzamide; 5-(bis-pyridin-3-ylmethyl-sulfamoyl)-3,4-difluoro-2-(4-iodo-2-methyl-phenylamino)-benzoic acid; 5-(bis-pyridin-3-ylmethyl-sulfamoyl)-N-cyclopropylmethoxy-3,4-difluoro-2-(4-iodo-2-methyl-phenylamino)-benzamide; N-cyclopropylmethoxy-3,4-difluoro-2-(4-iodo-2-methyl-phenylamino)-5-(methyl-pyridin-3-ylmethyl-sulfamoyl)-benzamide; N-cyclopropylmethoxy-3,4-difluoro-2-(4-iodo-2-methyl-phenylamino)-5-[(pyridin-3-ylmethyl)-sulfamoyl]-benzamide; N-cyclopropylmethoxy-5-[(3-diethylaminopropyl)-pyridin-3-ylmethyl-sulfamoyl]-3,4-difluoro-2-(4-iodo-2-methylphenylamino)-benzamide; N-cyclopropylmethoxy-3,4-difluoro-5-[(3-hydroxypropyl)-pyridin-3-ylmethyl-sulfamoyl]-2-(4-iodo-2-methyl-phenylamino)-benzamide; N-cyclopropylmethoxy-5-(ethyl-pyridin-3-ylmethyl-sulfamoyl)-3,4-difluoro-2-(4-iodo-2-methyl-phenylamino)-benzamide; N-cyclopropylmethoxy-3,4-difluoro-5-[(2-hydroxy-ethyl)-pyridin-3-ylmethyl-sulfamoyl]-2-(4-iodo-2-methylphenylamino)-benzamide; 5-(bis-pyridin-2-ylmethyl-sulfamoyl)-3,4-difluoro-2-(4-iodo-2-methyl-phenylamino)-benzoic acid; 5-(bis-pyridin-2-ylmethyl-sulfamoyl)-N-cyclopropylmethoxy-3,4-difluoro-2-(4-iodo-2-methyl-phenylamino)-benzamide; N-cyclopropylmethoxy-3,4-difluoro-2-(4-iodo-2-methyl-phenylamino)-5-(methylpyridin-2-ylmethyl-sulfamoyl)-benzamide; N-cyclopropylmethoxy-3,4-difluoro-2-(4-iodo-2-methyl-phenylamino)-5-[(pyridin-2-ylmethyl)-sulfamoyl]-benzamide; 5-(bis-pyridin-3-ylmethyl-sulfamoyl)-2-(2-chloro-4-iodo-phenylamino)-3,4-difluorobenzoic acid, 5-(bis-pyridin-3-ylmethyl-sulfamoyl)-2-(2-chloro-4-iodophenylamino)-N-cyclopropylmethoxy-3,4-difluoro-benzamide; 2-(2-chloro-4-iodo-phenylamino)-N-cyclopropylmethoxy-3,4-difluoro-5-(methylpyridin-3-ylmethyl-sulfamoyl)-benzamide; 2-(2-chloro-4-iodo-phenylamino)-N-cyclopropylmethoxy-3,4-difluoro-5-[(pyridin-3-ylmethyl)-sulfamoyl]-benzamide; 2-(2-chloro-4-iodo-phenylamino)-N-cyclopropylmethoxy-5-[(3-diethylaminopropyl)-pyridin-3-ylmethyl-sulfamoyl]-3,4-difluoro-benzamide; 2-(2-chloro-4-iodophenylamino)-N-cyclopropylmethoxy-3,4-difluoro-5-[(3-hydroxy-propyl)-pyridin-3-ylmethyl-sulfamoyl]-benzamide; 2-(2-chloro-4-iodo-phenylamino)-N-cyclopropylmethoxy-5-(ethyl-pyridin-3-ylmethyl-sulfamoyl)-3,4-difluorobenzamide; 2-(2-chloro-4-iodo-phenylamino)-N-cyclopropylmethoxy-3,4-difluoro-5-[(2-hydroxy-ethyl)-pyridin-3-ylmethyl-sulfamoyl]-benzamide; 5-(bis-pyridin-2-ylmethyl-sulfamoyl)-2-(2-chloro-4-iodo-phenylamino)-3,4-difluoro-benzoic acid; 5-(bis-pyridin-2-ylmethyl-sulfamoyl)-2-(2-chloro-4-iodo-phenylamino)-N-cyclopropylmethoxy-3,4-difluoro-benzamide; 2-(2-chloro-4-iodo-phenylamino)-N-cyclopropylmethoxy-3,4-difluoro-5-(methyl-pyridin-2-ylmethyl-sulfamoyl)-benzamide; 2-(2-chloro-4-iodo-phenylamino)-N-cyclopropylmethoxy-3,4-difluoro-5-[(pyridin-2-ylmethyl)-sulfamoyl]-benzamide; N-cyclopropylmethoxy-3,4-difluoro-5-[(3-hydroxy-propyl)-pyridin-2-ylmethyl-sulfamoyl]-2-(4-iodo-phenylamino)-benzamide; N-cyclopropylmethoxy-3,4-difluoro-5-[(2-hydroxy-ethyl)-pyridin-2-ylmethyl-sulfamoyl]-2-(4-iodo-phenylamino)-benzamide; 5-(benzyl-pyridin-2-ylmethyl-sulfamoyl)-N-cyclopropylmethoxy-3,4-difluoro-2-(4-iodo-phenylamino)-benzamide; N-cyclopropylmethoxy-3,4-difluoro-2-(4-iodo-phenylamino)-5-[(pyridin-4-ylmethyl)-sulfamoyl]-benzamide; N-cyclopropylmethoxy-5-(ethylpyridin-4-ylmethyl-sulfamoyl)-3,4-difluoro-2-(4-iodo-phenylamino)-benzamide: N-cyclopropylmethoxy-3,4-difluoro-2-(4-iodo-phenylamino)-5-(methyl-pyridin-4-ylmethyl-sulfamoyl)-benzamide; N-cyclopropylmethoxy-3,4-difluoro-5-[(3-hydroxypropyl)-pyridin-4-ylmethyl-sulfamoyl]-2-(4-iodo-phenylamino)-benzamide; N-cyclopropylmethoxy-3,4-difluoro-5-[(2-hydroxy-ethyl)-pyridin-4-ylmethylsulfamoyl]-2-(4-iodo-phenylamino)-benzamide; N-cyclopropylmethoxy-3,4-difluoro-2-(4-iodo-phenylamino)-5-(methyl-phenyl-sulfamoyl)-benzamide; N-cyclopropylmethoxy-3,4-difluoro-2-(4-iodo-phenylamino)-5-phenylsulfamoylbenzamide; N-Cyclopropylmethoxy-3,4-difluoro-2-(4-iodo-phenylamino)-5-(pyridin-3-ylsulfamoyl)-benzamide; N-cyclopropylmethoxy-3,4-difluoro-5-[(3-hydroxy-propyl)-pyridin-2-ylmethyl-sulfamoyl]-2-(4-iodo-2-methyl-phenylamino)-benzamide; N-cyclopropylmethoxy-3,4-difluoro-5-[(2-hydroxy-ethyl)-pyridin-2-ylmethyl-sulfamoyl]-2-(4-iodo-2-methyl-phenylamino)-benzamide; 5-(benzylpyridin-2-ylmethyl-sulfamoyl)-N-cyclopropylmethoxy-3,4-difluoro-2-(4-iodo-2-methyl-phenylamino)-benzamide; N-cyclopropylmethoxy-3,4-difluoro-2-(4-iodo-2-methyl-phenylamino)-5-[(pyridin-4-ylmethyl)-sulfamoyl]-benzamide; N-cyclopropylmethoxy-5-(ethyl-pyridin-4-ylmethyl-sulfamoyl)-3,4-difluoro-2-(4-iodo-2-methyl-phenylamino)-benzamide; N-cyclopropylmethoxy-3,4-difluoro-2-(4-iodo-2-methyl-phenylamino)-5-(methyl-pyridin-4-ylmethyl-sulfamoyl)-benzamide; N-cyclopropylmethoxy-3,4-difluoro-5-[(3-hydroxy-propyl)-pyridin-4-ylmethylsulfamoyl]-2-(4-iodo-2-methyl-phenylamino)-benzamide; N-cyclopropylmethoxy-3,4-difluoro-5-[(2-hydroxy-ethyl)-pyridin-4-ylmethyl-sulfamoyl]-2-(4-iodo-2-methylphenylamino)-benzamide; N-cyclopropylmethoxy-3,4-difluoro-2-(4-iodo-2-methylphenylamino)-5-(methyl-phenyl-sulfamoyl)-benzamide; N-cyclopropylmethoxy-3,4-difluoro-2-(4-iodo-2-methyl-phenylamino)-5-phenylsulfamoyl-benzamide; N-cyclopropylmethoxy-3,4-difluoro-2-(4-iodo-2-methyl-phenylamino)-5-(pyridin-3-ylsulfamoyl)-benzamide; 2-(2-chloro-4-iodo-phenylamino)-N-cyclopropylmethoxy-3,4-difluoro-5-[(3-hydroxy-propyl)-pyridin-2-ylmethyl-sulfamoyl]-benzamide; 2-(2-chloro-4-iodo-phenylamino)-N-cyclopropylmethoxy-3,4-difluoro-5-[(2-hydroxyethyl)-pyridin-2-ylmethyl-sulfamoyl]-benzamide; 5-(benzyl-pyridin-2-ylmethylsulfamoyl)-2-(2-chloro-4-iodo-phenylamino)-N-cyclopropylmethoxy-3,4-difluorobenzamide; 2-(2-chloro-4-iodo-phenylamino)-N-cyclopropylmethoxy-3,4-difluoro-5-[(pyridin-4-ylmethyl)-sulfamoyl]-benzamide; 2-(2-chloro-4-iodo-phenylamino)-N-cyclopropylmethoxy-5-(ethyl-pyridin-4-ylmethyl-sulfamoyl)-3,4-difluorobenzamide; 2-(2-chloro-4-iodo-phenylamino)-N-cyclopropylmethoxy-3,4-difluoro-5-(methyl-pyridin-4-ylmethyl-sulfamoyl)-benzamide; 2-(2-chloro-4-iodophenylamino)-N-cyclopropylmethoxy-3,4-difluoro-5-[(3-hydroxy-propyl)-pyridin-4-ylmethyl-sulfamoyl]-benzamide; 2-(2-chloro-4-iodo-phenylamino)-N-cyclopropylmethoxy-3,4-difluoro-5-[(2-hydroxy-ethyl)-pyridin-4-ylmethylsulfamoyl]-benzamide; 2-(2-chloro-4-iodo-phenylamino)-N-cyclopropylmethoxy-3,4-difluoro-5-(methyl-phenyl-sulfamoyl)-benzamide; 2-(2-chloro-4-iodophenylamino)-N-cyclopropylmethoxy-3,4-difluoro-5-phenylsulfamoyl-benzamide; 2-(2-chloro-4-iodo-phenylamino)-N-cyclopropylmethoxy-3,4-difluoro-5-(pyridin-3-ylsulfamoyl)-benzamide; N-cyclopropylmethoxy-2-(4-iodo-phenylamino)-4-phenylsulfamoyl-benzamide; N-cyclopropylmethoxy-2-(4-iodo-phenylamino)-4-(pyridin-3-ylsulfamoyl)-benzamide; N-cyclopropylmethoxy-2-(4-iodophenylamino)-4-[(pyridin-3-ylmethyl)-sulfamoyl]-benzamide; 4-(bis-pyridin-3-ylmethyl-sulfamoyl)-N-cyclopropylmethoxy-2-(4-iodo-phenylamino)-benzamide; N-cyclopropylmethoxy-4-[(2-hydroxy-ethyl)-pyridin-4-ylmethyl-sulfamoyl]-2-(4-iodo-phenylamino)-benzamide; -cyclopropylmethoxy-2-(4-iodo-phenylamino)-4-(methyl-pyridin-3-ylmethyl-sulfamoyl)-benzamide; N-cyclopropylmethoxy-4-[(3-diethylamino-propyl)-pyridin-3-ylmethyl-sulfamoyl]-2-(4-iodo-phenylamino)-benzamide; N-cyclopropylmethoxy-2-(4-iodo-2-methyl-phenylamino)-4-phenylsulfamoyl-benzamide; N-cyclopropylmethoxy-2-(4-iodo-2-methylphenylamino)-4-(pyridin-3-ylsulfamoyl)-benzamide; N-cyclopropylmethoxy-2-(4-iodo-2-methyl-phenylamino)-4-[(pyridin-3-ylmethyl)-sulfamoyl]-benzamide; 4-(bis-pyridin-3-ylmethyl-sulfamoyl)-N-cyclopropylmethoxy-2-(4-iodo-2-methylphenylamino)-benzamide; N-cyclopropylmethoxy-4-[(2-hydroxy-ethyl)-pyridin-4-ylmethyl-sulfamoyl]-2-(4-iodo-2-methyl-phenylamino)-benzamide; N-cyclopropylmethoxy-2-(4-iodo-2-methyl-phenylamino)-4-(methyl-pyridin-3-ylmethyl-sulfamoyl)-benzamide; N-cyclopropylmethoxy-4-[(3-diethylaminopropyl)-pyridin-3-ylmethyl-sulfamoyl]-2-(4-iodo-2-methyl-phenylamino)-benzamide; 2-(2-chloro-4-iodo-phenylamino)-N-cyclopropylmethoxy-4-phenylsulfamoyl-benzamide; 2-(2-chloro-4-iodo-phenylamino)-N-cyclopropylmethoxy-4-(pyridin-3-ylsulfamoyl)-benzamide; 2-(2-chloro-4-iodophenylamino)-N-cyclopropylmethoxy-4-[(pyridin-3-ylmethyl)-sulfamoyl]-benzamide; 4-(bis-pyridin-3-ylmethyl-sulfamoyl)-2-(2-chloro-4-iodo-phenylamino)-N-cyclopropylmethoxy-benzamide; 2-(2-chloro-4-iodo-phenylamino)-N-cyclopropylmethoxy-4-[(2-hydroxy-ethyl)-pyridin-4-ylmethyl-sulfamoyl]-benzamide; 2-(2-chloro-4-iodo-phenylamino)-N-cyclopropylmethoxy-4-(methylpyridin-3-ylmethyl-sulfamoyl)-benzamide; and 2-(2-chloro-4-iodo-phenylamino)-N-cyclopropylmethoxy-4-[(3-diethylamino-propyl)-pyridin-3-ylmethyl-sulfamoyl]-benzamide.

Further examples include: PD 298469, 2-(2-Chloro-4-iodo-phenylamino)-3,4-difluoro-N-methoxy-5-(4-methyl-piperazine-1-sulfonyl)-benzamide; PD 298470, 2-(2-Chloro-4-iodo-phenylamino)-5-[(2-diethylamino-ethyl)-methylsulfamoyl]-3,4-difluoro-N-methoxy-benzamide; PD 298450, 2-(2-Chloro-4-iodophenylamino)-3,4-difluoro-N-methoxy-5-(methyl-prop-2-ynyl-sulfamoyl)-benzamide; PD 298451, 1-[4-(2-Chloro-4-iodo-phenylamino)-2,3-difluoro-5-methoxycarbamoyl-benzenesulfonyl]-piperidine-3-carboxylic acid amide; PD 298452, 2-(2-Chloro-4-iodo-phenylamino)-3,4-difluoro-N-methoxy-5-[methyl-(2-pyridin-2-yl-ethyl)-sulfamoyl]-benzamide; PD 298453, 2-(2-Chloro-4-iodophenylamino)-5-[(3-dimethylamino-propyl)-methyl-sulfamoyl]-3,4-difluoro-N-methoxy-benzamide; PD 298454, 2-(2-Chloro-4-iodo-phenylamino)-3,4-difluoro-N-methoxy-5-(4-pyridin-2-yl-piperazine-1-sulfonyl)-benzamide; PD 298455, 5-[Bis-(2-methoxy-ethyl)-sulfamoyl]-2-(2-chloro-4-iodo-phenylamino)-3,4-difluoro-N-methoxy-benzamide; PD 298456, 5-[Benzyl-(2-dimethylamino-ethyl)-sulfamoyl]-2-(2-chloro-4-iodo-phenylamino)-3,4-difluoro-N-methoxy-benzamide; and PD 298457, N-Allyloxy-2-(2-chloro-4-iodo-phenylamino)-5-dimethylsulfamoyl-3,4-difluoro-benzamide; PD 298461, N-Allyloxy-2-(2-chloro-4-iodo-phenylamino)-3,4-difluoro-5-(methyl-prop-2-ynyl-sulfamoyl)-benzamide; PD 298462, N-Allyloxy-2-(2-chloro-4-iodo-phenylamino)-3,4-difluoro-5-[4-(4-fluoro-phenyl)-piperazine-1-sulfonyl]-benzamide; PD 298466, N-Allyloxy-5-[benzyl-(2-dimethylamino-ethyl)-sulfamoyl]-2-(2-chloro-4-iodo-phenylamino)-3,4-difluoro-benzamide; PD 298468, 2-(2-Chloro-4-iodo-phenylamino)-N-cyclopropylmethoxy-3,4-difluoro-5-(4-methylpiperazine-1-sulfonyl)-benzamide; and PD 298449, 2-(2-Chloro-4-iodophenylamino)-3,4-difluoro-5-(methoxy-methyl-sulfamoyl)-N-(2-morpholin-4-yl-ethoxy)-benzamide.

Particularly preferred compounds include: PD 298458, N-Allyloxy-2-(2-chloro-4-iodo-phenylamino)-3,4-difluoro-5-(4-methyl-piperazine-1-sulfonyl)-benzamide; PD 298459, N-Allyloxy-2-(2-chloro-4-iodo-phenylamino)-3,4-difluoro-5-(methyl-phenyl-sulfamoyl)-benzamide; PD 298460, 5-(Allyl-methyl-sulfamoyl)-N-allyloxy-2-(2-chloro-4-iodo-phenylamino)-3,4-difluoro-benzamide; PD 298463, 1-[5-Allyloxycarbamoyl-4-(2-chloro-4-iodo-phenylamino)-2,3-difluorobenzenesulfonyl]-piperidine-3-carboxylic acid amide; PD 298464, N-Allyloxy-2-(2-chloro-4-iodo-phenylamino)-5-[(3-dimethylamino-propyl)-methyl-sulfamoyl]-3,4-difluoro-benzamide; PD 298465, N-Allyloxy-2-(2-chloro-4-iodo-phenylamino)-3,4-difluoro-5-(4-pyridin-2-yl-piperazine-1-sulfonyl)-benzamide; and PD 298467, N-Allyloxy-2-(2-chloro-4-iodo-phenylamino)-3,4-difluoro-5-(methoxy-methylsulfamoyl)-benzamide.

### C. Synthesis

The disclosed compounds can be synthesized according to Scheme 1 below. 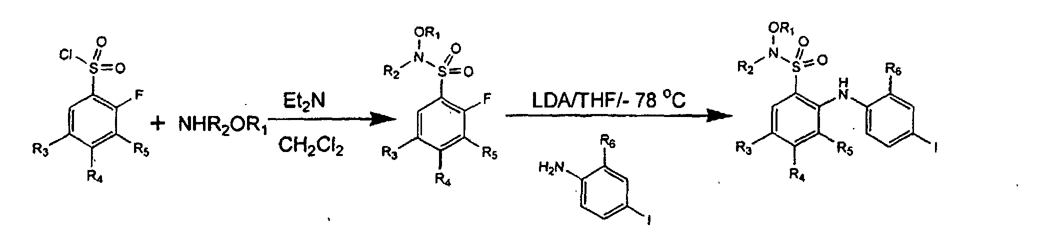 One equivalent of appropriately substituted sulfonyl chloride is added to a solution of one equivalent of appropriately substituted hydroxylamine and excess triethylamine in CH₂Cl₂ or Et₂O and stirred for 30 minutes. The triethylamine hydrochloride precipitate is separated by filtration and discarded. If necessary, the product is further purified by chromatography on silica column. The pure 2-fluor hydroxamic or hydroxamate product is then added to a solution of appropriately substituted lithium anilide prepared by adding LDA to the aniline in THF at-78 °C. After stirring at room temperature for 16 hours, the reaction mixture is poured in to Et₂O-HCl. Any precipitated solid is separated by filtration and discarded. The filtrate is concentrated and the resulting crude product is purified on silica column to give the desired target product.

The disclosed compounds can also be made by other synthetic organic methods, as well as automated or combinatorial methods.

The disclosed compounds can be synthesized according to the following two Schemes, or variants thereof (see also Example 1A).

Regarding the first step of synthetic Scheme 1A, the reaction of the aniline and the benzoic acid derivative generally is accomplished by mixing the benzoic acid with an equimolar quantity or excess of the aniline in an unreactive organic solvent such as tetrahydrofuran, or toluene, in the presence of a base such as lithium diisopropylamide, lithium hexamethyldisilazide, n-butyl lithium, sodium hydride, or sodium amide. The reaction generally is carried out at a temperature of about -78 °C to about 25 °C, and normally is complete within 2 hours to about 4 days. The product can be isolated by removing the solvent, for example by evaporation under reduced pressure, and further purified, if desired, by standard methods such as chromatography, crystallization, or distillation.

Turning to the second step, the 2-phenylamino benzoic acid derivative is next reacted with an equimolar quantity or excess of a nucleophile such as an aniline, a phenol, or a thiophenol by mixing in an unreactive organic solvent such as tetrahydrofuran, or toluene, in the presence of a base such as lithium diisopropylamide, lithium hexamethyldisilazide, n-butyl lithium, sodium hydride, or sodium amide. The reaction generally is carried out at a temperature of about -78 °C to boiling, and normally is complete within 2 hours to about 4 days. The product can be isolated by removing the solvent, for example by evaporation under reduced pressure, and further purified, if desired, by standard methods such as chromatography, crystallization, or distillation.

Finally, regarding step 3, the 4-arylheteroatom-2-phenylamino benzoic acid derivative next is reacted with a nucleophile such as ammonia, an amine, an alcohol, hydrazine, a hydrazine derivative, or a hydroxylamine derivative in the presence of a peptide coupling reagent. Amines that can be employed include monomethylamine and aniline. Alcohols that can be employed include cyclobutylmethanol and phenol. Hydrazine derivatives that can be employed include N,N-dimethylhydrazine and 1-aminopiperidine. Hydroxylamine derivatives that can be employed include methoxylamine, N-ethyl-isopropoxy amine, and tetrahydrooxazine. Typical coupling reagents include 2-ethoxy-1-ethoxycarbonyl-1,2-dihydroquinoline (EEDQ), 1,3-dicyclohexylcarbodiimide (DCC), bromo-tris-(pyrrolidino)-phosphonium hexafluorophosphate (PyBrOP) and (benzotriazolyloxy) tripyrrolidino phosphonium hexafluorophosphate (PyBOP). The 4-arylheteroatom-2-phenylamino benzoic acid derivative and the nucleophile normally are mixed in approximately equimolar quantities in an unreactive solvent such as dichloromethane, tetrahydrofuran, chloroform, or xylene, and an equimolar quantity of the coupling reagent is added. A base such as triethylamine or diisopropylethylamine can be added to act as an acid scavenger if desired. The coupling reaction generally is complete after about 10 minutes to 2 hours, and the product is readily isolated by removing the reaction solvent, for instance by evaporation under reduced pressure, and purifying the product by standard methods such as chromatography or crystallizations from solvents such as acetone diethyl ether or ethanol.

Referring to synthetic Scheme 2A, an alternative method for making the compounds of the invention involves first coupling the benzoic acid derivative with the arylheteroatom nucleophile, and then reacting this 4-arylheteroatom benzoic acid derivative with an aniline. The final step involves the coupling of the 4-arylheteroatom-2-phenylamino benzoic acid derivative with the ammonia, amine, alcohol, hydrazine, hydrazine derivative, or hydroxylamine derivative with a peptide coupling reagent. The general reaction conditions for all of the steps in Scheme 2A are similar to those described above for synthetic Scheme 1A.

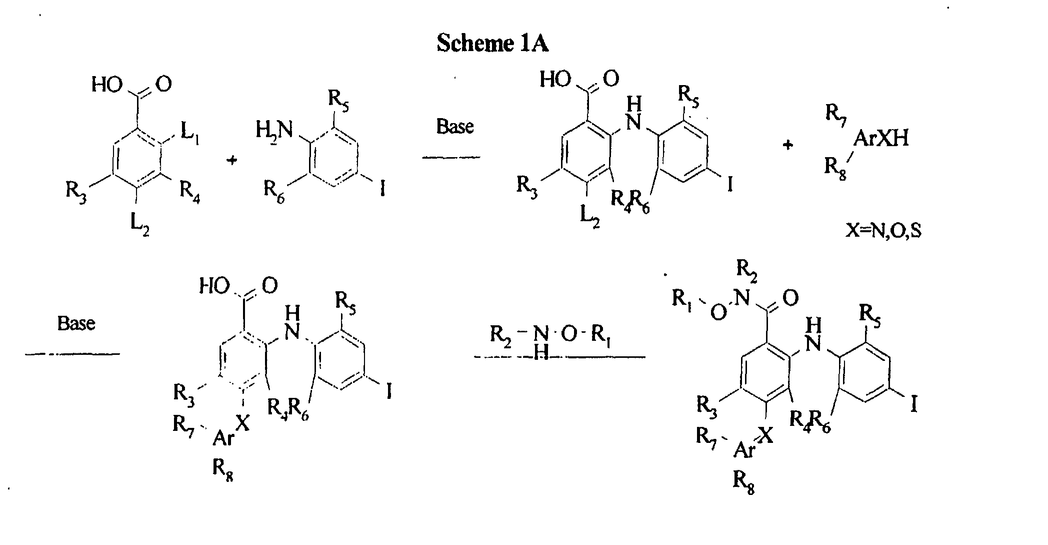

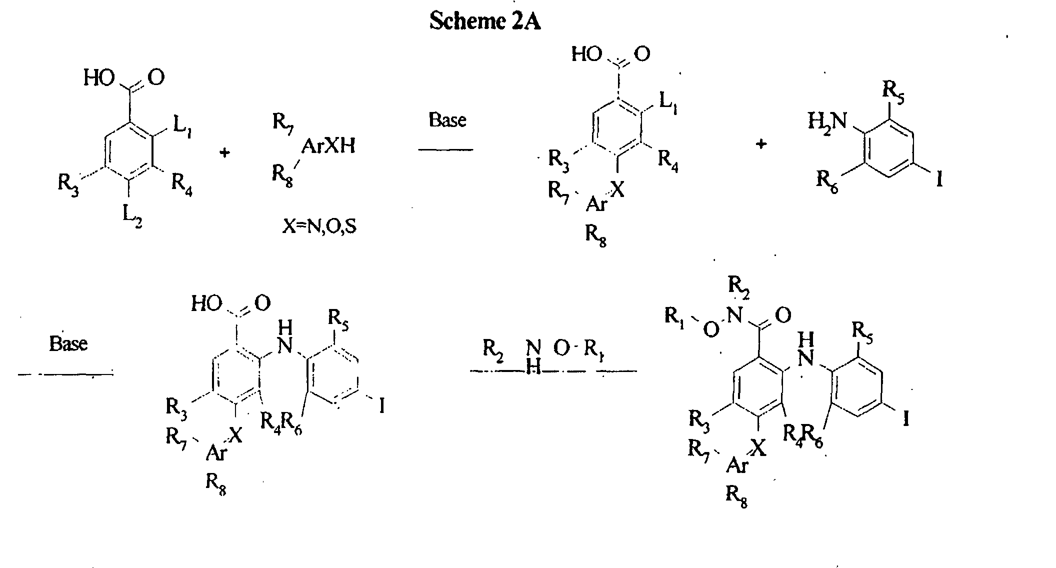

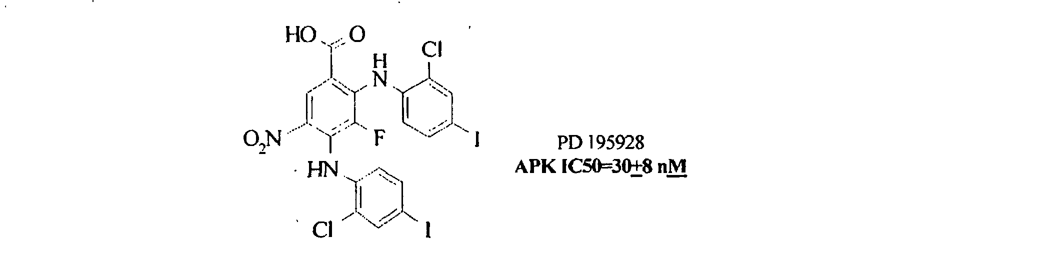

The disclosed compounds can be synthesized according to the following five schemes, or variants thereof. The abbreviation PyBOP is (benzotriazolyloxy)-tripyrrolidino phosphonium hexafluorophosphate. These synthetic strategies are further exemplified in Examples 1B-5B below:

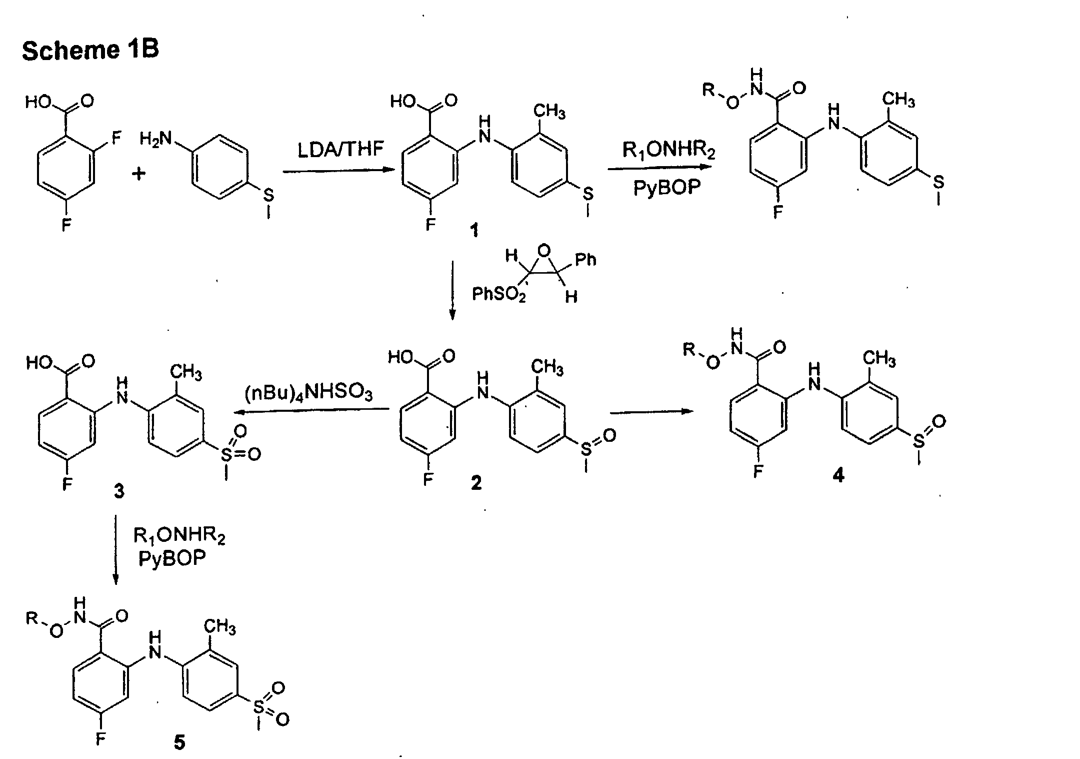

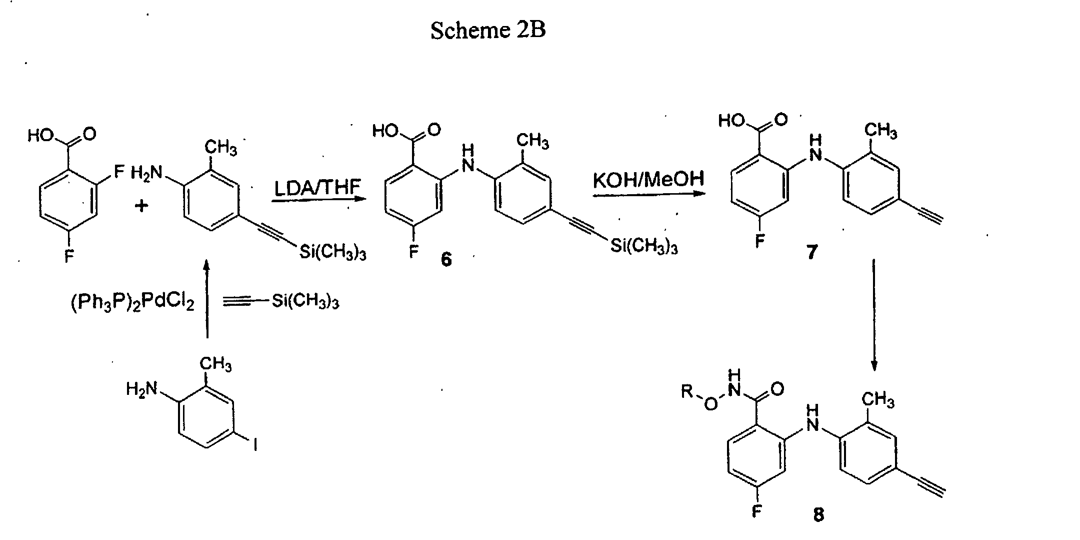

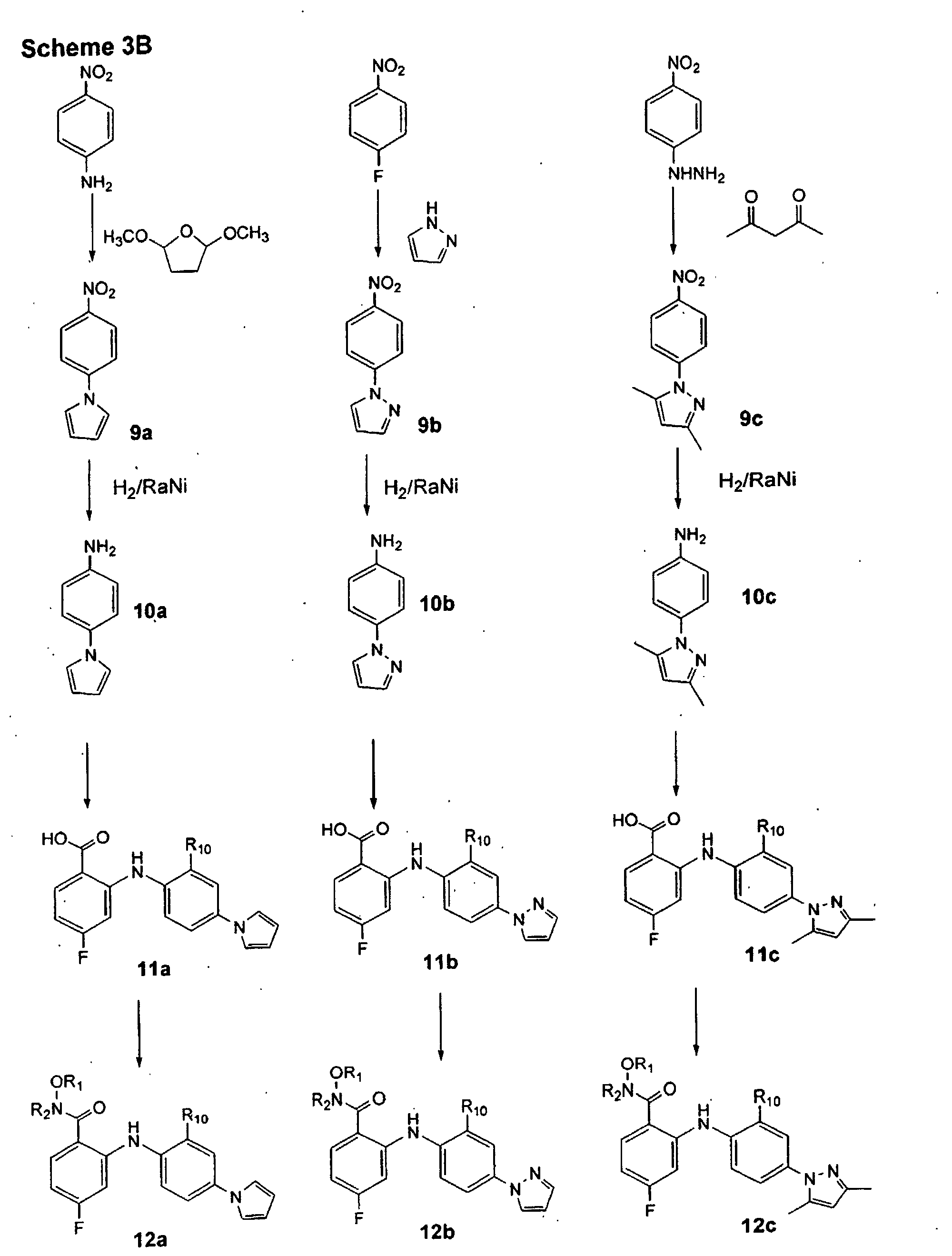

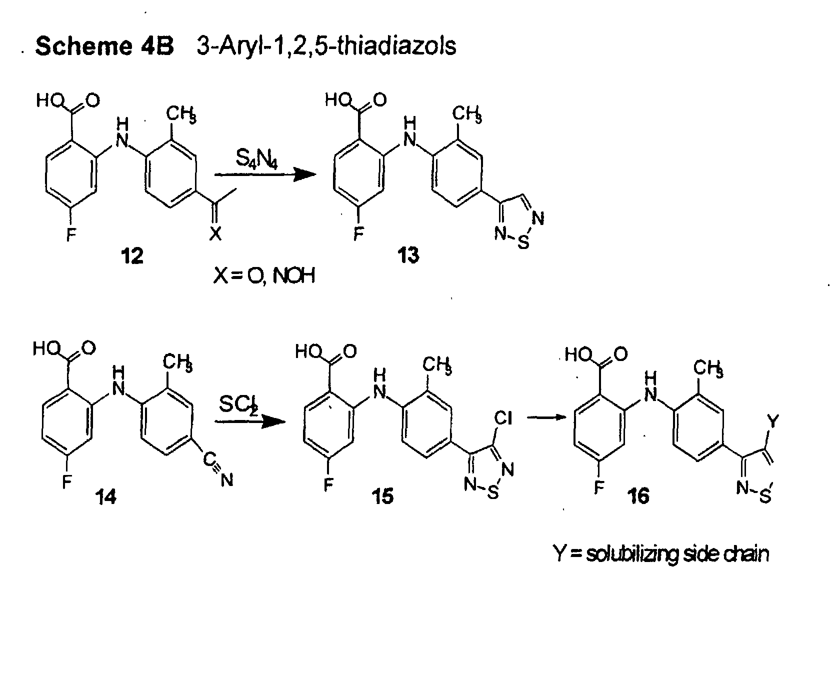

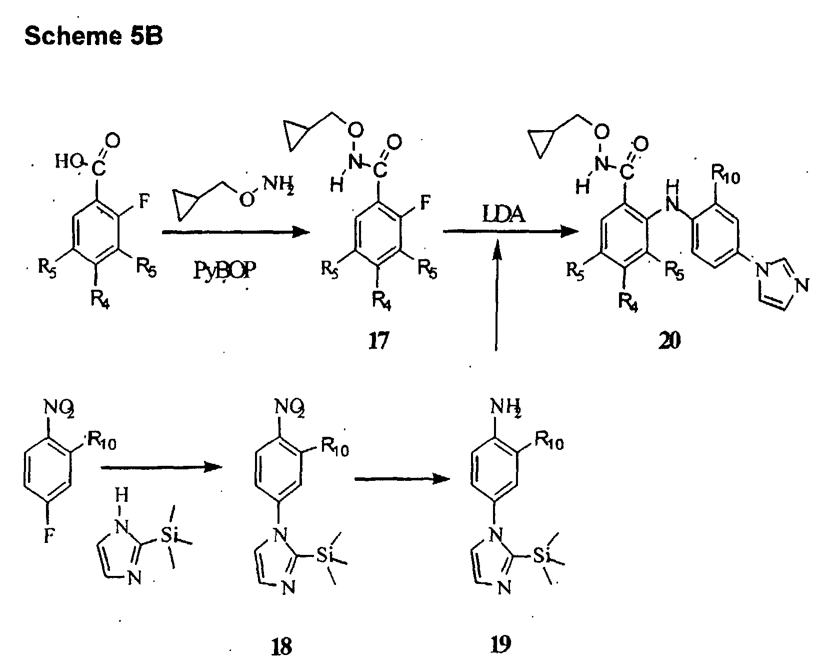

The disclosed compounds can be synthesized according to the following four Schemes, or variants thereof. These synthetic strategies, which are suitable for conventional or combinatorial synthetic methods, are further exemplified in Examples 1C-4C below. 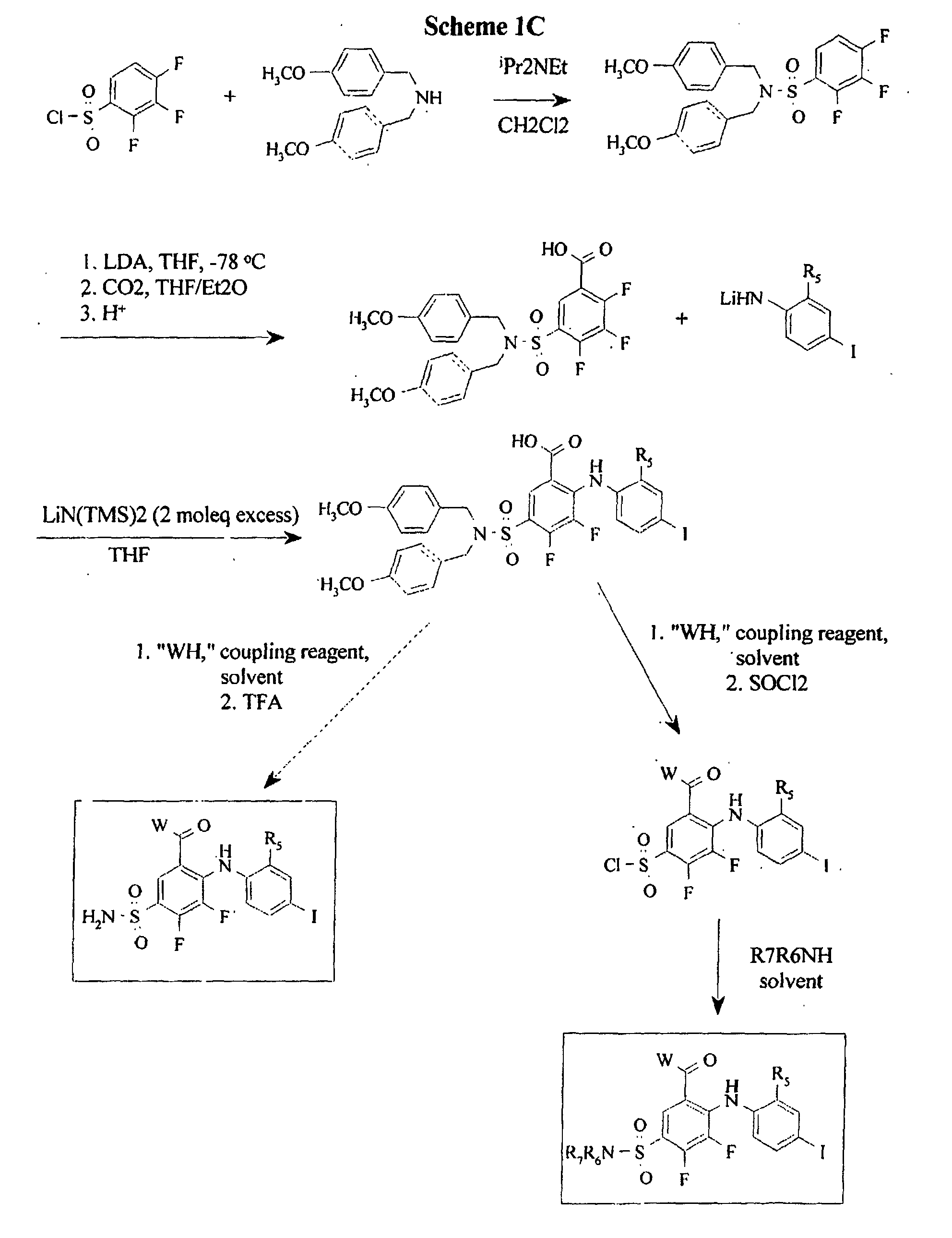 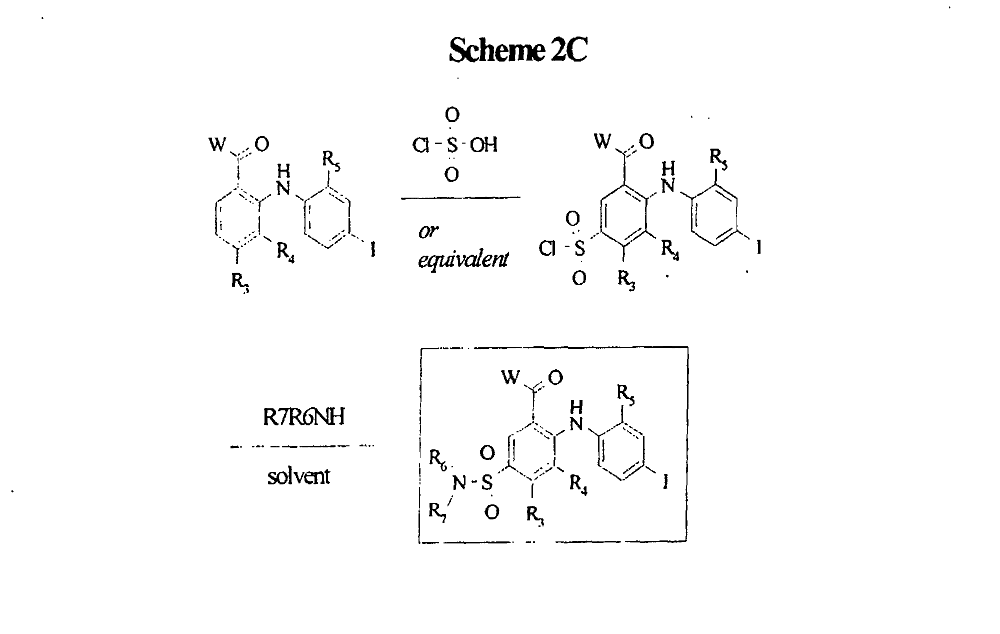 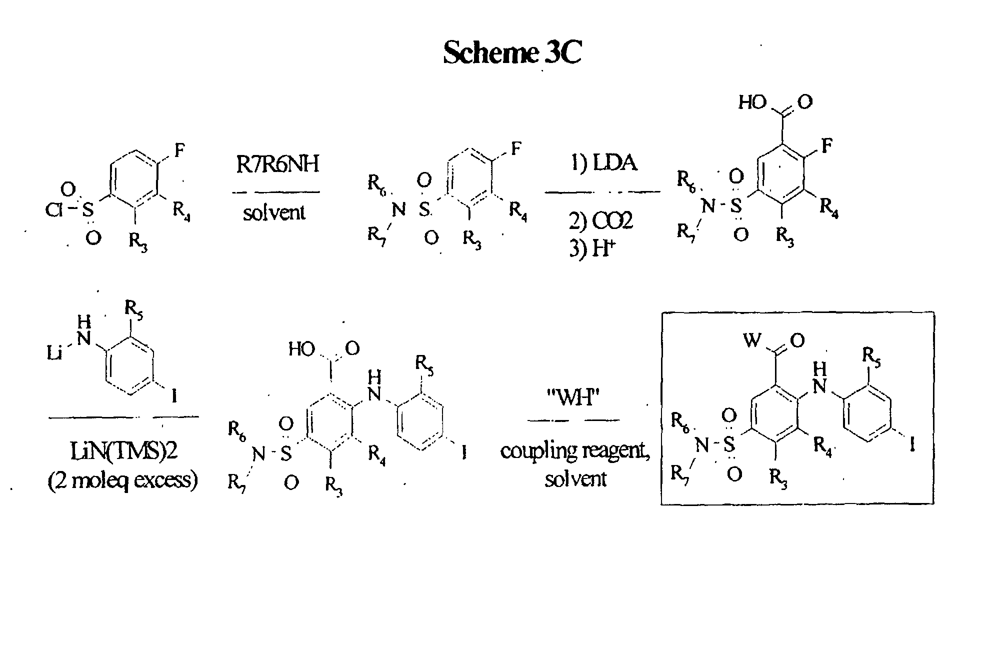 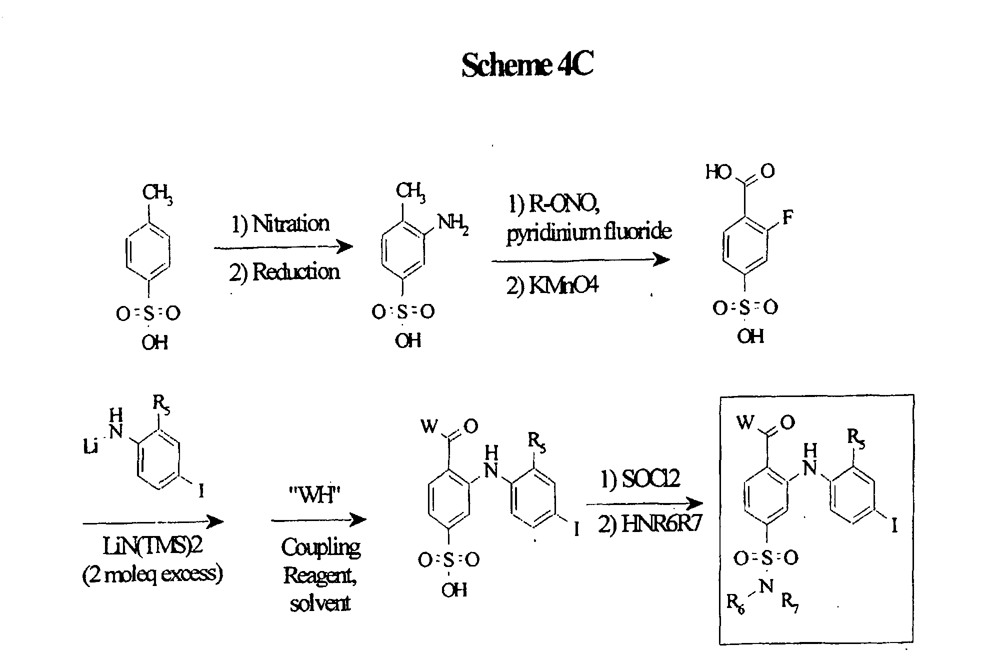 Amine reagents such as R₆R₇NH in the schemes above are either commercially available or through straightforward modification of commercially available intermediates. Examples of such amine reagents, which can be reacted with the appropriate intermediate in a combinatorial or matrix method, are provided below. For example, in section B (Compounds), starting at page 8, line 16, there are three sets of thirty (one set each for R₅ = H, Me, and Cl). The table below provides a number (corresponding to order that the name is found in the text; for example, "1" corresponds to compounds 1, 31, and 61 in the list of 90 compounds); the amine reagent name; and a Chemical Abstracts number. Where a PD number is listed, the amine reagent was prepared from commercially available starting materials.

| Number (position in subset of 30) | **Amine reagent name** | CAS # or PD # |
|---|---|---|
| 1 | 3,3'-dipicolylamine | 1656-94-6 |
| 2 | *3,3'-dipicolylamine* | *1656-94-6* |
| 3 | 3-(methylaminomethyl)pyridine | 20173-04-0 |
| 4 | 3-(aminomethyl)pyridine | 3731-52-0 |
| 5 | "N-(3-diethylaminopropyl)-N-(pyridin-3-ylmethyl)amine" | PD 0096419 |
| 6 | 3-(3-pyridylmethylamino)-1-propanol | 6951-00-4 |
| 7 | 3-(ethylaminomethyl)pyridine | PD 0133573 |
| 8 | 2-(3-pyridylmethylamino)ethanol hydrochloride | PD 0018185-0002 |
| 9 | di-(2-picolyl)amine | 1539-42-0 |
| 10 | *di-(2-picolyl)amine* | *1539-42-0* |
| 11 | 2-(methylaminomethyl)pyridine | PD 0091430 |
| 12 | 2-(aminomethyl)pyridine | 3731-51-9 |
| 13 | 3-(2-pyridylmethylamino)-1-propanol | 6950-99-8 |
| 14 | 2-(2-pyridylmethylamino)ethanol | PD 0018354 |
| 15 | 2-(N-benzylaminomethyl)pyridine | PD 0054372 |
| 16 | 4-(aminomethyl)pyridine | 3731-53-1 |
| 17 | 4-(ethylaminomethyl)pyridine | 33403-97-3 |
| 18 | 4-(methylaminomethyl)pyridine | PD 0111199 |
| 19 | 3-(4-pyridylmethylamino)-1-propanol | 7251-62-9 |
| 20 | 2-(4-pyridylmethylamino)ethanol hydrochloride | PD 0018008-0002 |
| 21 | N-methylaniline | 100-61-8 |
| 22 | aniline | 62-53-3 |
| *23* | *3-aminopyridine* | *462-08-8* |
| *24* | *aniline* | *62-53-3* |
| *25* | *3-aminopyridine* | *462-08-8* |
| *26* | *3-(aminomethyl)pyridine* | *3731-52-0* |
| *27* | *3,3'-dipicolylamine* | *1656-94-6* |
| *28* | *2-(4-pyridylmethylamino)ethanol hydrochloride* | *PD 0018008-0002* |
| *29* | *3-(methylaminomethyl)pyridine* | *20173-04-0* |
| *30* | *"N-(3-diethylaminopropyl)-N-(pyridin-3-ylmethyl)amine"* | *PD 0096419* |

Additional compounds within claim 1 can be made with the following amine reagents. The corresponding CAS number is provided.

| | |
|---|---|
| 2-(methylamino)pyridine | 4597-87-9 |
| 2-benzylaminopyridine | 6935-27-9 |
| 2-allylaminopyridine | 5866-28-4 |
| 2,2'-dipyridylamine | 1202-34-2 |
| 2-anilinopyridine | 6631-37-4 |
| 2-aminopyridine | 504-29-0 |
| 4-aminopyridine | 504-24-5 |
| 2-benzylaminopyridine | 6935-27-9 |
| 2-(4-methoxybenzyl)aminopyridine | 52818-63-0 |
| 2-methylaminopyridine | 4597-87-9 |

### Combinatorial Synthesis

The following stock solutions were prepared:
1) An acetonitrile (anhydrous) stock solution 0.05 M in 5-chlorosulfonyl-2,3,4-trifluoro-benzoyl chloride.
2) Acetonitrile (anhydrous) stock solutions 0.05 M in each of the four appropriate hydroxylamine hydrochlorides (see list A) and 0.3 M in 2,6-lutidine.
3) Acetonitrile stock solutions 0.05 M in each of the 25 appropriate amines (see list B). Note that amine salts that were not soluble were also 0.1 M in 2,6-lutidine.
4) Acetonitrile (anhydrous) stock solutions in each of the 3 appropriate anilines (see list C) and 0.88 M in lithium bis(trimethylsilyl)amide (1.0 M in tetrahydrofuran).

An array which treated 4 hydroxylamine hydrochlorides independently with 5-chlorosulfonyl-2,3,4-trifluoro-benzoyl chloride, 25 amines, and 1 aniline was prepared to yield a total of 100 reactions. A liquid handling robot was used to transfer the reagents in such a manner as to insure that all possible combinations were achieved. The appropriate hydroxylamine hydrochloride solution (0.05 mmol, 1 mL) was added to a 2-dram vial, and each vial was treated with 5-chlorosulfonyl-2,3,4-trifluoro-benzoyl chloride solution (0.05 mmol, 1 mL). After 20 minutes the appropriate amine solution (0.05 mmol, 1 mL) was added sequentially. After a further 20 minutes the vials were treated with the solution of 4-iodoaniline (0.055 mmol, 1 mL). The vials were capped and shaken overnight at room temperature. The reactions were quenched with 1 mL of a 1 M aqueous ammonium chloride solution. The vials were concentrated to dryness under a stream of nitrogen and purified by reverse phase HPLC using a 30x100 mm YMC ODS-A (C18) column. The mobile phase was acetonitrile/water (both with 0.05% trifluoroacetic acid) at 25 mL/min and a linear gradient of 10-100% over 6.5 min and then 3.5 min at 100%, detection was at 214 nm.

An array which treated 4 hydroxylamine hydrochlorides independently with 5-chlorosulfonyl-2,3,4-trifluoro-benzoyl chloride, 25 amines, and 1 aniline was prepared to yield a total of 100 reactions. A liquid handling robot was used to transfer the reagents in such a manner as to insure that all possible combinations were achieved. The appropriate hydroxylamine hydrochloride solution (0.05 mmol, 1 mL) was added to a 2-dram vial, and each vial was treated with 5-chlorosulfonyl-2,3,4-trifluoro-benzoyl chloride solution (0.05 mmol, 1 mL). After 20 minutes the appropriate amine solution (0.05 mmol, 1 mL) was added sequentially. After a further 20 minutes the vials were treated with the solution of 4-iodo-2-methylaniline (0.05 mmol, 0.91 mL). The vials were capped and shaken overnight at room temperature. The reactions were quenched with 1 mL of a 1 M aqueous ammonium chloride solution. The vials were concentrated to dryness under a stream of nitrogen and purified by reverse phase HPLC using a 30x100 mm YMC ODS-A (C18) column. The mobile phase was acetonitrile/water (both with 0.05% trifluoroacetic acid) at 25 mL/min and a linear gradient of 10-100% over 6.5 min and then 3.5 min at 100%, detection was at 214 nm.

An array which treated 4 hydroxylamine hydrochlorides independently with 5-chlorosulfonyl-2,3,4-trifluoro-benzoyl chloride, 25 amines, and 1 aniline was prepared to yield a total of 100 reactions. A liquid handling robot was used to transfer the reagents in such a manner as to insure that all possible combinations were achieved. The appropriate hydroxylamine hydrochloride solution (0.05 mmol, 1 mL) was added to a 2-dram vial, and each vial was treated with 5-chlorosulfonyl-2,3,4-trifluoro-benzoyl chloride solution (0.05 mmol, 1 mL). After 20 minutes the appropriate amine solution (0.05 mmol, 1 mL) was added sequentially. After a further 20 minutes the vials were treated with the solution of 2-chloro-4-iodoaniline (0.05 mmol, 0.91 mL). The vials were capped and shaken overnight at room temperature. The reactions were quenched with 1 mL of a 1 M aqueous ammonium chloride solution. The vials were concentrated to dryness under a stream of nitrogen and purified by reverse phase HPLC using a 30x100 mm YMC ODS-A (C18) column. The mobile phase was acetonitrile/water (both with 0.05% trifluoroacetic acid) at 25 mL/min and a linear gradient of 10-100% over 6.5 min and then 3.5 min at 100%, detection was at 214 nm.

### Combinatorial Synthesis

### Table of Example Reagents

### List A-Hydroxylamines:

1. *O*-methyl-hydroxylamine
2. *O*-allyl-hydroxylamine hydrochloride monohydrate (Aldrich)
3. *O*-cyclopropylmethyl-hydroxylamine hydrochloride
4. *O*-(2-morpholin-4-yl-ethyl)-hydroxylamine hydrochloride

### List B-Amines:

1. dimethylamine
2. diethylamine
3. isopropyl-methyl-amine
4. diisopropylamine
5. methylhydrazine
6. 1-methylpiperazine
7. *N*,*N*-diethyl-*N*'-methylethane-1,2-diamine
8. benzylmethylamine
9. dibenzylamine
10. methyl-phenyl-amine
11. allyl-methyl-amine
12. methyl-prop-2-ynyl-amine
13. methylamino-acetonitrile hydrochloride
14.1-(4-fluoro-phenyl)-piperazine
15. furan-2-ylmethyl-methyl-amine
16. piperidine-3-carboxylic acid amide
17. methyl-phenethyl-amine
18. methyl-(2-pyridin-2-yl-ethyl)-amine
19. *N,N,N*'-trimethyl-propane-1,3-diamine
20.methyl-(1-methyl-piperidin-4-yl)-amine
21.1-pyridin-2-yl-piperazine
22. bis-(2-methoxy-ethyl)-amine
23. *N*'-benzyl-*N*,*N*-dimethyl-ethane-1,2-diamine
24. methylamino-acetic acid *tert*-butyl ester hydrochloride
25. *O*,*N*-dimethyl-hydroxylamine hydrochloride

### List C-Anilines:

1. 4-iodoaniline
2. 2-chloro-4-iodoaniline
3. 4-iodo-2-methylaniline

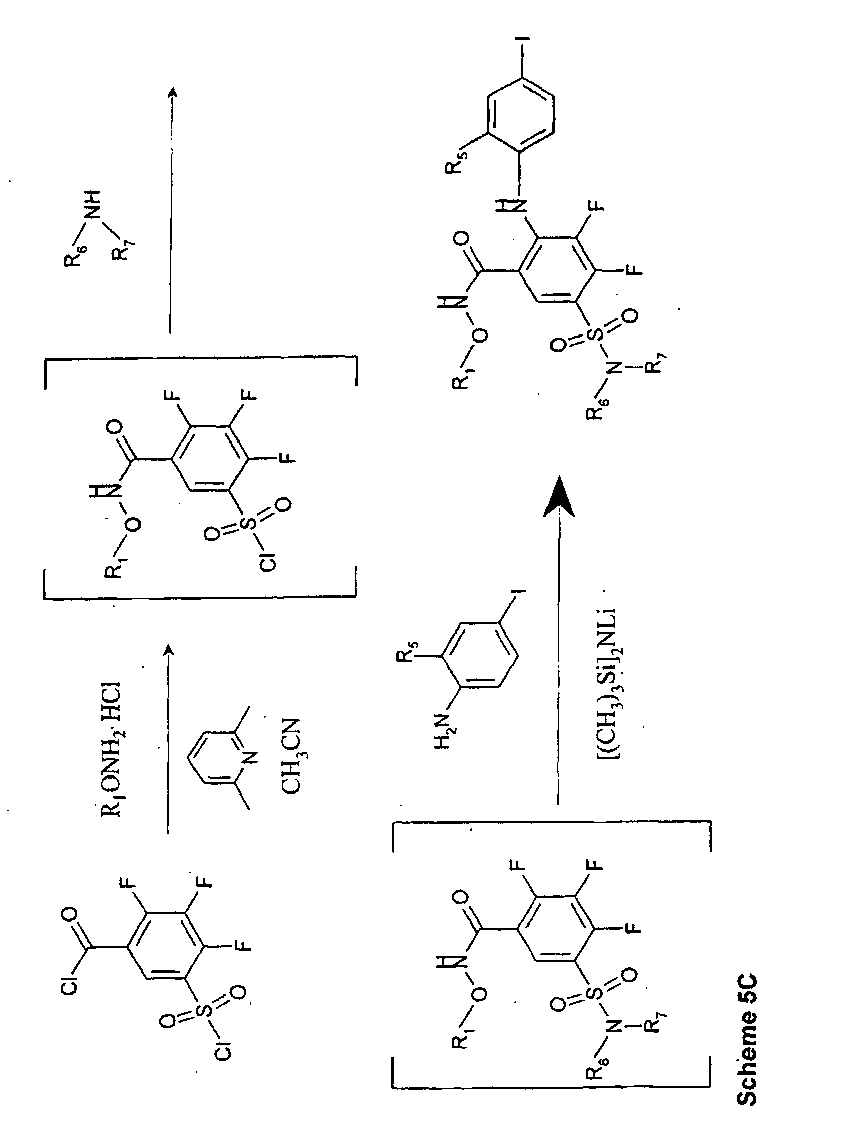

### Chemical Examples

### Example 1

### Preparation of 2-(2-Chloro-4-iodo-phenylamino)-N-cyclopropylmethoxy-3,4-difluoro-benzenesulfonamide (PD 0297447)

### N-cyclopropylmethoxy-2,3,4-trifluoro-benzenesulfonamide.

To a stirring suspension comprised of *O*-cyclopropylmethyl-hydroxylamine hydrochloride (5.40 g, 0.0437 mol) in dichloromethane (20 ml) at ambient temperature under a nitrogen atmosphere was added diisopropylethylamine (10.8 ml, 0.062 mol). A solution comprised of 2,3,4-trifluorobenzenesulfonyl chloride (Oakwood Products, Inc., 1.00 g, 4.34 x 10⁻³ mol) in dichloromethane (120 ml) was added dropwise to the reaction vessel containing the stirring suspension over a 12 minute period. The reaction mixture was stirred for another 12 minutes and was quenched with 10 % aqueous hydrochloric acid (140 ml). The biphasic mixture was stirred vigorously for 16 hours. The layers were separated and the organic phase was dried (MgSO₄) and concentrated to 6 ml volume. The concentrated solution was administered to a flash silica column (Biotage, 90 g of silica gel). Elution with dichloromethane afforded 0.8283 g of a white amorphous solid; 68 % yield; ¹H-NMR (400 MHz; CDCl₃ signal offset to δ 7.03; values reported are uncorrected) δ 7.50 (m, 1H), 7.10 (s, 1H), 6.95 (m, 1H), 3.59 (d, 2H, J=7.2 Hz), 0.80 (m, 1H), 0.31 (m, 2H), 0.02 (m, 2H); ¹⁹F-NMR (376 MHz; CDCl₃) δ-122.65 (m, 1F), -129.37 (m, 1F), -156.20 (m, 1F); MS (APCI-) 280 (M-1, 100), 210 (55), 195 (45).

### 2-(2-Chloro-4-iodo-phenylamino)-N-cyclopropylmethoxy-3,4-difluorobenzenesulfonamide (PD 0297447).

To a stirring solution comprised of 2-chloro-4-iodoaniline in tetrahydrofuran (10 ml) at -78 °C under a nitrogen atmosphere was added a 1.0 M tetrahydrofuran solution of lithium *bis*trimethylsilylamide (6.2 ml, 6.2 x 10⁻³ mol) to form a green suspension. The suspension was stirred for five minutes before a stirring suspension comprised of lithiated *N*-cyclopropylmethoxy-2,3,4-trifluoro-benzenesulfonamide (prepared by adding 3.0 ml of the 1.0 M lithium *bis*trimethylsilylamide solution to a stirring solution comprised of *N*-cyclopropylmethoxy-2,3,4-trifluoro-benzenesulfonamide in 10 ml of tetrahydrofuran at -78 °C under nitrogen gas) was added via canula. The cold bath was removed and the stirring suspension was stirred for one hour. The reaction mixture was quenched with 10 % aqueous hydrochloric acid (50 ml) and the biphasic mixture was concentrated *in vacuo* to an aqueous suspension that was extracted with diethyl ether (200 ml). The organic phase was dried (MgSO₄) an d was concentrated *in vacuo* to afford a tan oil. The crude product was purified by flash chromatography. Elution with a gradient (hexanes-ethyl acetate 99:1 → (2 min) 9:1 → (25 min) 3:1 afforded 1.10 g of a white amorphous foam; 73 % yield; ¹H-NMR (400 MHz; DMSO) δ 7.69 (m, 1H), 7.59 (d, 1H, J=1.9 Hz), 7.34 (dd, 1H, J=8.7, 1.9 Hz), 7.27 (s, 1H), 7.00 (s, 1H), 6.95 (m, 1H), 6.43 (dd, 1H, J=8.7, 5.8 Hz), 3.52 (d, 2H, J=7.5 Hz), 0.74 (m, 1H), 0.34 (m, 2H), 0.02 (m, 2H); ¹⁹F-NMR (376 MHz; CDCl₃) δ -124.76 (m, 1F), -136.69 (d, 1F, J=18.3 Hz); MS (APCl+) 515 (M+1, 100); (APCI-) 513 (M-1, 50), 443 (73), 428 (100); IR (KBr) 1491 cm⁻¹; Anal. Calcd/found for C₁₆H₁₄ClF₂lN₂O₃S C, 37.34/36.54; H, 2.74/2.71; N, 5.44/5.15; F, 7.38/7.57.

The APK IC₅₀ for PD 0297447 is 0.965 µM.

### EXAMPLE 1A

### Preparation of 2,4-bis-(2-chloro-4-iodo-phenylamino)-3-fluoro-5-nitrobenzoic acid

### Step a: Preparation of 5-nitro-2,3,4-trifluorobenzoic acid

To gently stirring concentrated sulfuric acid (50 ml) was added fuming nitric acid (3.4 ml, 0.076 mol). Solid 2,3,4-trifluorobenzoic acid (10.00 g, 0.05565 mol) was added directly in increments. After stirring 45 minutes, the reaction mixture had become an orange homogeneous solution which was then poured over chilled water (400 ml). The resulting aqueous suspension was extracted with diethyl ether (3 x 200 ml). The combined extracts were dried with anhydrous magnesium sulfate and concentrated *in vacuo* to yield 12.30 g of a dull, light-yellow solid. Recrystallization from chloroform (50 ml) afforded 9.54 g of the pale yellow microcrystalline product; 78 % yield; m.p. ; ¹H-NMR (400 MHz; DMSO) δ 14.29 (broad s, 1H), 8.43-8.38 (m, 1H); ¹³C-NMR (100 MHz; DMSO) δ 162.41, 154.24 (dd, J_{C-F}=270.1, 10.7 Hz), 148.35 (dd, J_{C-F}=267.0, 9.2 Hz), 141.23 (dt, J_{C-F}=253.4 Hz), 133.95, 123.30 (d, J_{C-} _{F}=2.2 Hz), 116.92 (dd, J_{C-F}=18.2, 3.8 Hz); ¹⁹F-NMR (376 MHz; DMSO) δ-120.50 to -120.63 (m), -131.133 to -131.27 (m), -153.63 to -153.74 (m).

### Step b: Preparation of 2,4-bis-(2-chloro-4-iodo-phenylamino)-3-fluoro-5-nitrobenzoic acid

To a stirring solution comprised of 2-chloro-4-iodoaniline (Lancaster, 98 %, 12.33 g, 0.04864 mol) in tetrahydrofuran (20 ml) at -78 °C under nitrogen was added a 2.0 M lithium diisopropylamide solution in tetrahydrofuranheptane-ethylbenzene (Aldrich, 35 ml, 0.070 mol) with a syringe. The addition formed a thick suspension. After five minutes of stirring, a solution comprised of 5-nitro-2,3,4-trifluorobenzoic acid (5.00 g, 0.0226 mol) in tetrahydrofuran (30 ml) was added with a syringe to give a dark reaction mixture. The cold bath was removed and the reaction mixture stirred for 20 minutes. The cool reaction mixture was poured into ether (600 ml) containing an excess of hydrogen chloride. The red solution instantly turned to a yellow suspension as a precipitate formed. This precipitate was removed by vacuum filtration. The filtrate was concentrated *in vacuo* to a red powder (10.5 g). The red powder was triturated with boiling chloroform (800 ml). The triturated solids were collected by vacuum filtration to give an orange powder (2.42 g). The mother liquor from the trituration was concentrated *in vacuo* to give a red-orange solid (ca. 10 g undried). This solid was loaded onto a flash silica column. Elution with dichloromethane removed some impurities. Continuing elution with 1 % methanol in dichloromethane afforede ca. 4 g of a red solid. This red solid was dissolved in hot absolute ethanol (100 ml). The solution was boiled down to 50 ml before dilution to 300 ml with hexanes. This solution was boiled to 150 ml and rediluted to 300 ml with hexanes to produce slight turbidity. The mixture was cooled in the refrigerator for three days, affording a yellow precipitate. The precipitate was collected by vacuum filtration and was dried with suction to afford 0.15 g of a yellow solid; 1 % yield; ¹H-NMR (400 MHz; DMSO) δ 8.94 (s, 1H), 8.55 (s, 1H), 7.79 (d, 2H, J=2.0 Hz), 7.61-7.57 (m, 2H), 6.90 (dd, 1H, J=8.5, 3.9 Hz), 6.84 (dd, 1H, J=8.3, 6.6 Hz); ¹⁹F-NMR (376 MHz; DMSO) δ -122.62 (s); MS (APCI+) 692 (6), 691 (8), 690 (31), 689 (10), 688 (55), 171 (47), 130 (100); (APCI-) 691 (4), 690 (12), 689 (14), 688 (70), 687 (32), 686 (100), 506 (50), 453 (97); IR (KBr) 1523 cm⁻¹; Anal. calcd/found for: C₁₉H₁₀Cl₂FI₂N₃O₄ C, 33.17/33.32; H, 1.47/1.73; N, 6.11/5.73; Cl, 10.31/10.04; F, 2.76/3.70; I, 36.89/34.32.

The APK IC₅₀ for 2,4-bis-(2-chloro-4-iodo-phenylamino)-3-fluoro-5-nitrobenzoic acid is 29.6 nM.

### EXAMPLE 1B

### 4-Fluoro-2-(4-methanesulfanyl-phenylamino)-benzoic acid (1).

To a solution of 4-(methylmercapto)aniline (3.1622 g, 0.02 mole) in THF at ⁻78°C, a solution of LDA in THF (2M, 30 ml, 0.06 mole) was added and the reaction mixture stirred for 30 minutes at ⁻78°C (Scheme 1E). Solid 2,4-diflluoro benzoic acid (3.1622 g, 0.02 mole) was added and the reaction stirred for 16 hours while it wormed up to room temperature. The reaction mixture was pour in to ether saturated with HCI gas. HCI gas was bubbled into until precipitation of salts ceased. The precipitated salts were separated by filtration and discarded. The ether layer was concentrated to give **1** as a white solid. Yield 5.63 g (100%); mp 173-179 °C (DEC); ¹H-NMR (400 MHz; CDCl₃) δ 9.39 (s, 1H), 8.04 (dd, 1H, J=9.2, 6.8 Hz), 7.32-7.17 (AB quartet, 4H), 6.74 (dd, 1H, J=12.1, 2.4 Hz), 6.46-6.41 (m, 1H), 2.51 (s, 3H); ¹³C-NMR (100 MHz; CDCl₃) δ 172.79, 167.57 (d, J_{C-F}=253.4 Hz), 151.55 (d, J_{C-F}=12.2 Hz), 136.83, 135.40 (d, J_{C-F}=12.2 Hz), 134.72, 128.31, 124.60, 106.51, 105.12 (d, J_{C-F}=22.9 Hz), 99.79 (d, J_{C-F}=26.7 Hz), 16.51; ¹⁹F-NMR (376 MHz; CDCl₃) δ -101.39 to -101.46 (m); MS (APCI+) 278 (M+1, 100); IR (KBr) 3319, 1664, 1589, 1258 cm⁻¹; Anal. calcd/found for: C₁₄H₁₂FNO₂S C, 60.64/60.99; H, 4.36/4.63; N, 5.05/4.80; S, 11.56/10.97.

### EXAMPLE 2B

### 4-Fluoro-2-(4-methanesulfinyl-phenylamino)-benzoic acid (2).

A mixture of **1** (Scheme 1B) (0.286 g, 0.001031 mole) and oxaziridine (0.235 g, 0.0009 mole) in CHCl₃ (30 ml) at room temperature for 2 hours. The solvent was removed and the resulting brown oil chromatographed on silica column. Elution with CH₂Cl₂ removed fast moving byproduct. Further elution with CH₂Cl₂:CH₃OH (9.5:05), R_{f} = 0.27, gave pure **2** as a light brown solid. Yield 132.8 mg (50%); mp 191-192 °C; ¹H-NMR (400 MHz; CDCl₃) δ 9.77 (s, 1H), 8.08 (dd, 1H, J=8.9, 6.7 Hz), 7.70-7.39 (AB quartet, 4H), 6,98 (dd, 1H, J=11.6, 2.4 Hz), 6.57-6.52 (m, 1H), 2.80 (s, 3H); ¹³C-NMR (100 MHz; CDCl₃) δ 170.76, 167.18 (d, J_{C-F}=253.3 Hz), 149.33 (d, J_{C-F}=12.2 Hz), 143.02, 139.50, 135.37 (d, J_{C-F}=12.2 Hz), 125.47, 122.32, 108.22, 106.35 (d, J_{C-F}=22.8 Hz), 100.69, (d, J_{C-F}=25.9 Hz), 43.75; MS (APCI+) 294 (M+1, 100); IR (KBr) 1673, 1592, 1228 cm⁻¹; Anal. calcd/found for: C₁₄H₁₂FNO₃S C, 57.33/57.48; H, 4.12/4.27; N, 4.78/4.67.

### EXAMPLE 3B

### 4-Fluoro-2-(4-methanesulfonyl-phenylamino)-benzoic acid (3).

A solution of **1** (Scheme 1B) (0.4458 g, 0.00152 mole) and tetrabutylammonium oxon (1.1 g, 0.0030 mole) in CH₂Cl₂ (20 ml) was stirred at room temperature for 16 hours. TLC showed the presence of starting material; so additional 1.1 g (0.0030 mole) of the tetrabutylammonium oxon was added and reaction mixture stirred for 16 more hours. The reaction mixture was loaded on to a silica column and eluted with CH₂Cl₂:CH₃OH (9.75:0.25) and the fast moving fraction collected and concentrated to give **3** as a white solid. Yield, 0.3856 g (82%); mp 200-202 °C; ¹H-NMR (400 MHz; CDCl₃) δ 9.78 (s, 1H), 8.13 (dd, 1H, J=8.9, 6.5 Hz), 7.94-7.38 (AB quartet, 4H), 7.10 (dd, 1H, J=11.3, 2.4 Hz), 6.66-6.61 (m, 1H), 3.09 (s, 3H); ¹³C-NMR (100 MHz; CDCl₃) δ 171.52, 167.28 (d, J_{C-F}=254.9 Hz), 148.32, 145.21, 135.59 (d, J_{C-F}=11.5 Hz), 134.50, 129.39, 120.62, 108.74, 107.46 (d, J_{C-F}=22.8 Hz), 101.61 (d, J_{C-} _{F}=26.7 Hz), 44.78; ¹⁹F-NMR (376 MHz; CDCl₃) δ -100.29 to -100.45 (m); MS (APCI+) 310 (M+1, 100); (APCI-) 308 (M-1, 100); Anal. calcd/found for: C₁₄H₁₂FNO₄S·0.75 H₂O C, 52.08/52.36; H, 4.22/3,88; N,4.34/4.26.

### EXAMPLE 4B

### 2-methyl-4-trimethylsilanylethynyl-aniline (5)

To a solution of 4-iodo-2-methyl-aniline (2.33g, 10 mmol), bis(triphenylphosphine)palladium(II)chloride (1.4g, 0.2 mmol), Cul (0.19 g, 0.1 mmol) in Et₃N (40 ml) at ice-bath temperature, (trimethylsilyl)acetylene (1.18 g, 12 mmol) was added dropwise (Scheme 2B). After an hour stirring, the ice-bath was removed and the reaction mixture heated at 40°C (oil-bath temperature) for one hour; cooled to room temperature and the solvent removed. The residue was partitioned between H₂O and Et₂O. The Et₂O layer was separated, dried (MgSO₄) and concentrated to give an oil. The oil was purified by silica column, eluting with CH₂Cl₂. The fraction with R_{f} = 0.37 was collected and concentrated to give 2-methyl-4-trimethylsilanylethynylaniline as a dark brown oil.
Yield 1.50 g (83%).

### EXAMPLE 5B

### 4-Fluoro-2-(2-methyl-4-trimethylsilanylethynyl-phenylamino)-benzoic acid (6)

Continuing after Example 4B, to a solution of 2-methyl-4-trimethylsilanylethynyl aniline (1.50 g, 0.008 mole) in THF (10 ml) at ⁻78°C, LDA (2 M in THF, 6 ml, 0.012 mole) was added and the mixture was stirred at ⁻78°C for 30 minutes. Solid 2,4-difluoro-benzoic acid (0.633 g, 0.004 mole) was added and the stirred for 16 hours while it warmed up to room temperature. The solvents were removed and water (30 ml) and Et₂O (50 ml) added to the oil residue. The mixture was stirred vigorously and the Et₂O layer separated, dried (MgSO₄) and concentrated to give a brown solid. The solid was purified on silica column, eluted with CH₂Cl₂. The fraction with R_{f} = 0.37 was collected and concentrated to give a light brown solid. The solid was added to pentane; some insoluble brown particulate was separated by filtration and discarded. The pentane layer was concentrated to give **6** as a light yellow solid. Yield 0.65 g (47%); mp 170-171°C; ¹H-NMR (400 MHz; CDCl₃) δ 9.33 (s, 1H), 8.05 (dd, 1H, J=8.9, 6.8 Hz), 7.43 (d, 1H, J=1.2 Hz), 7.35 (dd, 1H, J=8.2, 1.7 Hz), 7.25 (d, 1H, J=8.2 Hz), 6.53 (dd, 1H, J=11.8, 2.4 Hz), 6.47-6.42 (m, 1H), 2.25 (s, 3H), 0.26 (s, 9H); ¹³C-NMR (100 MHz; CDCl₃) δ 172.86, 167.61 (d, J_{C-} _{F}=253.3), 151.24 (d, J_{C-F}=12.3 Hz), 138.28, 135.38 (d, J_{C-F}=11.4 Hz), 134.85, 132.82, 130.63, 123.81, 119.91, 106.63, 105.23 (d, J_{C-F}=22.8 Hz), 104.77, 99.98 (d, J_{C-F}=26.7 Hz), 94.05, 17.78, 0.00; MS (APCI+) 342 (M+1, 100); IR (KBr) 2151, 1661, 1249 cm⁻¹; Anal. calcd/found for: C₁₉H₂₀FNO₂Si C, 66.83/67.02; H, 5.90/6.00; N, 4.10/4.09; F, 5.56/5.45.

### EXAMPLE 6B

### 4-Fluoro-2-(2-methyl-4-ethynyl-phenylamino)-benzoic acid (7).

To a solution of **6** in CH₃OH (30 ml), aqueous 1N KOH (10 ml) was added. After stirring at room temperature for 16 hours, the CH₃OH was removed and the aqueous layer was acidified with 6N HCI (Scheme 2B). The resulting white precipitation was extracted in to Et₂O, the Et₂O layer was dried (MgSO₄) and concentrated to give **7** as tan colored solid. Yield 0.4274 g (91%); mp 177-178 °C; ¹H-NMR (400 MHz; CDCl₃) δ 9.35 (s, 1H), 8.08-8.04 (m, 1H), 7.44 (s, 1H), 7.38-7.25 (m, 2H), 6.57 (d, 1H, J=11.8 Hz), 6.48-6.44 (m, 1H), 3.08 (s, 1H), 2.27 (s, 3H); ¹³C-NMR (100 MHz; CDCl₃) δ 172.84, 167.61 (d, J_{C-F}=253.3), 151.15 (d, J_{C-F}=12.3 Hz), 138.63, 135.40 (d, J_{C-F}=12.3 Hz), 135.00, 132.87, 130.81, 123.76, 118.79, 106.75, 105.33 (d, J_{C-F}=22.8 Hz), 100.03 (d, J_{C-F}=26.0 Hz), 83.37, 17.83, 0.00; ¹⁹F-NMR (376 MHz; CDCl₃) δ-101.24 to -101.31 (m); MS (APCI+) 270 (M+1, 100); IR (KBr) 3315, 1672, 1594, 1253 cm⁻¹; Anal. calcd/found for: C₁₆H₁₂FNO₂ C, 71.37/71.08; H, 4.49/4.82; N, 5.20/5.09.

### EXAMPLE 7B

### 1-(4-nitro-phenyl)-1H-pyrrole (9a)

To a gently refluxing mixture of 4-nitroaniline (6.906 g, 0.05 mole), and sodium acetate (23 g, 0.28 mole) in acetic acid (100 ml) was added 2,5-dimethoxytetrahydrofuran (7.26 g, 7.12 ml, 0.055 mole) dropwise (Scheme 3B). After refluxing for 3 hours, the reaction mixture was poured on to crushed ice (∼250 ml), basified with 10 % sodium hydroxide (250 ml) and extracted with CH₂Cl₂. The CH₂Cl₂ layer was dried (K₂CO₃) to afford the product as a dark brown oil. Yield 9.40 g (100 %).

### EXAMPLE 8B

### 1-(4-nitro-phenyl)-1H-pyrazole (9b)

A mixture of pyrrazole (6.808 g, 0.1 mole) tetrabutylammonium bromide (3.22 g, 0.01 mole) and KOH (11.22 g, 0.2 mole) were ground together and sonicated for 16 hours. To this 1-fluoro-4-nitrobenzene (15.521 g, 11.67 ml, 0.11 mole) was added and the mixture sonicated for 24 hours. The reaction mixture was extracted with CH₂Cl₂. The CH₂Cl₂ layer was dried (MgSO₄) and concentrated to give dark brown solid. This was purified by silica column chromatography. Elution with CH₂Cl₂ (R_{f} = 0.44) gave the product as a light brown solid. Yield 8.80 g (47 %); mp 171-172 °C; Anal. calcd/found for: C₉H₇N₃O₂ C, 57.14/56.52; H, 3.73/3.62; N, 22.21/21.95.

### EXAMPLE 9B

### 3,5-dimethyl-1-(4-nitro-phenyl)-1H-pyrazole (9c)

To a solution of 4-nitro-phenyl-hydrazine (15.3 g, 0.1 mole) and 2,4-pentanedione (10.01 g, 10.27 ml, 0.1 mole) in EtOH (200 ml) were added 5 drops of concentrated HCl. The mixture was refluxed for 15 minutes; and the solvent removed to give a gummy product. This was purified by silica column chromatography. Elution with CH₂Cl₂ gave the desired product (R_{f} = 0.10) as a brown solid. Yield 7.22 g (33 %).

### EXAMPLE 10B

### 4-Pyrrol-1-yl-phenylamine (10a)

Catalytic reduction (H₂/RaNi (5 g) /THF) of 1-(4-nitro-phenyl)- 1H-pyrrole (9.69 g, 0.05149 mole) at 51 psi gave crude product as an oil (Scheme 3B). The product was purified by silica column chromatography. Elution with CH₂Cl₂ (R_{f} = 0.13) gave the pure product as white solid. Yield 8.06 g (99 %); mp 80-81 °C.

### EXAMPLE 11B

In a manner similar to the preparation of 4-pyrrol-1-yl-phenylamine, the following were prepared:
4-1H-Pyrazol-1-yl-phenylamine (10b). Dark brown oil, yield 6.26 g (100 %).
Benzenamine, 4-(3,5-dimethyl-1H-pyrazzol-1-yl) (10c). Dark brown oil.
Yield 6.45 g (100 %).

### EXAMPLE 12B

### 4-Fluoro-2-(4-pyrrol-1-yl-phenylamino)-benzoic acid (11a)

To a solution of 4-pyrrol-1-yl-phenylamine (3.16 g, 0.02 mole) in THF (30 ml) at ⁻78°C, a solution of LDA (2M, 15 ml, 0.03 mole) was added and the mixture stirred for 30 minutes. Solid 2,4-difluorobenzoic acid was added and the reaction mixture stirred for 16 hours as it warmed up to room temperature. The solvent was removed and ether (100 ml) added to the dark oily residue. This was stirred vigorously and the insoluble gummy precipitate separated by filtration. The gamy residue was dissolved in H₂O, acidified to pH 1 with 10% HCI, and extracted with Et₂O. The Et₂O layer was dried (MgSO₄) and concentrated to give the target compound as a brown solid. Yield 2.74 g (93 %); mp 223-225 °C (DEC); ¹⁹F-NMR (376 MHz; CDCl₃) δ -101.44 (s); MS (APCI+) 297 (M+1, 100); IR (KBr) 1658, 1526, 1254 cm⁻¹.

In a manner similar to the preparation of 4-Fluoro-2-(4-pyrrol-1-yl-phenylamino)-benzoic acid, the following were prepared:
4-Fluoro-2-(4-pyrazol-1-yl-phenylamino)-benzoic acid (11b). Light brown solid, mp 212-213 °C.
2-[4-(3,5-Dimethyl-pyrazol-1-yl)-phenylamino]-4-Fluoro benzoic acid (11c). Tan powder, mp 198 -200 °C.

### EXAMPLE 1C

### Preparation of 2-(2-chloro-4-iodo-phenylamino)-5-dimethylsulfamoyl-3,4-difluoro-benzoic acid methyl ester (APK IC₅₀=222 nM)

### Step a: Preparation of 1-dimethylsulfamoyl-2,3,4-trifluorobenzene

To a gently stirring solution comprised of 2,3,4-trifluorobenzenesulfonyl chloride (5.70 g, 0.0247 mol) in 1,2-dichloroethane (200 ml) was introduced by bubbling gaseous anhydrous dimethylamine. The mixture became cloudy after several minutes and was subsequently washed with water (200 ml), 6 N aqueous hydrochloric acid (200 ml), brine (200 ml), was dried over anhydrous magnesium sulfate, and was concentrated *in vacuo* to obtain a yellow oil. The crude product was purified by flash chromatography. Elution with dichloromethane afforded 3.40 g of a white solid; 58 % yield; ¹H-NMR (400 MHz; CDCl₃) δ 7.63-7.56 (m, 1H), 7.12-7.04 (m, 1H), 2.812 (s, 3H), 2.807 (s, 3H); ¹⁹F-NMR (376 MHz; CDCl₃) δ -124.91 to -125.03 (m), -127.98 to-128.03 (m), -156.41 to -156.53.

### Step b: Preparation of 5-dimethvlsulfamovl-2,3,4-trifluoro-benzoic acid

To a cold (-78 °C) stirring solution comprised of 1-dimethylsulfamoyl-2,3,4-trifluorobenzene in anhydrous tetrahydrofuran (60 ml) under a nitrogen atmosphere was added a commercially available lithium diisopropylamide solution (Aldrich, 2.0 M in tetrahydrofuran/heptane/ethylbenzene, 7.5 ml, 0.0150 mol). After stirring for about ten minutes, the purple solution was transferred via canula to a cold, stirring, saturated carbon dioxide in diethyl ether solution (200 ml). The reaction mixture took on a dull burgundy color. The cold bath was removed and the reaction mixture warmed to ambient temperature over one hour. The mixture was then carefully quenched with 10 % aqueous hydrochloric acid (200 ml). The layers were separated. The organic phase was extracted twice (200, 100 ml portions) with 10 % (wt.) aqueous sodium hydroxide. The combined aqueous alkaline extracts were treated with concentrated aqueous hydrochloric acid (100 ml) to pH 0. A white precipitate formed. The suspension was allowed to cool, then was extracted with diethyl ether (600 ml). The organic extract was dried over anhydrous magnesium sulfate and was concentrated *in vacuo* to afford 2.70 g of an off-white solid; 67.5 % yield; mp 225-228 °C; ¹H-NMR (400 MHz; DMSO) δ 14.08 (broad s, 1H), 8.02-7.97 (m, 1H), 2.75 (s, 3H), 2.74 (s, 3H) ¹⁹F-NMR (376 MHz; DMSO) δ -122.50 to -122.63 (m), -122.95 to -123.08 (m), -154.49 to -154.61 (m); MS (APCI+) 284 (M+1, 22), 238 (100); (APCI-) 282 (M-1, 85), 259 (94), 238 (46), 216 (91), 195 (100); IR (KBr) 1702 cm⁻¹; Anal. calcd/found for: C₉H₈F₃NO₄S C, 38.17/38.40; H, 2.85/2.90; N, 4.95/4.80; F, 20.12/19.75; S, 11.32/11.12.

### Step c: Preparation of 5-dimethylsulfamoyl-2,3,4-trifluoro-benzoic acid methyl ester

The solid 5-dimethylsulfamoyl-2,3,4-trifluoro-benzoic acid (1.47 g, 0.00519 mol) and *p*-toluenesulfonic acid catalyst (17.1 mg) were dissolved in methanol (125 ml). The stirring mixture was brought to reflux under a nitrogen atmosphere for 51 hours. The reaction mixture was concentrated *in vacuo* to give a solid. The product was partitioned between diethyl ether (200 ml) and saturated aqueous potassium carbonate (75 ml). The layers were separated and the organic phase was washed with water (75 ml), brine (75 ml), was dried over anhydrous potassium carbonate, and was concentrated *in vacuo* to afford 0.15 g of an off-white solid; 10% yield; ¹H-NMR (400 MHz; CDCl₃) δ 8.23-8.19 (m, 1H), 3.92 (s, 3H), 2.83 (s, 6H); ¹⁹F-NMR (376 MHz; CDCl₃) δ-120.79 to -121.02 (m), -153.69 to -153.80.

### Step d: Preparation of 2-(2-chloro-4-iodo-phenylamino)-5-dimethylsulfamoyl-3,4-difluoro-benzoic acid methyl ester

To a stirring cold (-78 °C) solution comprised of 2-chloro-4-iodoaniline (0.143 g, 5.64x10⁻⁴ mol) in anhydrous tetrahydrofuran (5 ml) under a nitrogen atmosphere was added a commercially available lithium diisopropylamide solution (Aldrich, 2.0 M in tetrahydrofuran/heptane/ethylbenzene, 0.300 ml, 6.0x10⁻⁴ mol). After stirring for 5 minutes, a solution comprised of 5-dimethylsulfamoyl-2,3,4-trifluoro-benzoic acid methyl ester (0.15 g, 5.0x10⁻⁴ mol) in tetrahydrofuran (10 ml) was added via syringe. The cold bath was removed and the reaction mixture was stirred for 2 hours. The reaction mixture was then partitioned between diethyl ether (125 ml) and saturated aqueous sodium bicarbonate (125 ml). The aqueous bicarbonate phase was extracted with an additional portion (125 ml) of diethyl ether. The combined organic phases were dried over anhydrous magnesium sulfate and concentrated *in vacuo* to give a yellow oil. The oil was crystallized from heptane-ethyl acetate to afford 0.060 g of an off-white powder; 23 % yield; mp 154-156 °C; ¹H-NMR (400 MHz; CDCl₃) δ 9.74 (s, 1H), 8.30 (d, 1H, J=7.1 Hz), 7.72 (s, 1H), 7.49 (d, 1H, J=8.3 Hz), 6.73-6.69 (m, 1H), 3.92 (s, 3H), 2.84 (s, 3H), 2.83 (s, 3H); ¹⁹F-NMR (376 MHz; CDCl₃) δ -123.90 (d), -139.55 (d).

### EXAMPLE 2C

### Preparation of 2-(2-chloro-4-iodo-phenylamino)-N-cyclopropylmethoxy-3,4-difluoro-5-sulfamoyl-benzamide (PD 219622)

### Step a: Preparation of 1-bis-(4-methoxybenzyl)sulfamoyl-2,3,4-trifluorobenzene

To a stirring solution comprised of *bis*-4-methoxybenzylamine (2.5 g, 9.7x10⁻³ mol) and diisopropylethylamine (1.7 ml, 9.7x10⁻³ mol) in dichloromethane (50 ml) at 0 °C under nitrogen atmosphere was added liquid 2,3,4-trifluorobenzenesulfonyl chloride (2.26 g, 9.5x10⁻³ mol) directly. The mixture was stirred cold for ten minutes. The ice-water bath was removed and the mixture was stirred for an additional 15 minutes and was then diluted with dichloromethane to 350 ml volume and was washed with saturated aqueous ammonium chloride (200 ml). The organic phase was dried (MgSO₄) and concentrated *in vacuo* to afford 4.99 g of a sticky white solid. The crude product was recrystallized from hexanes-acetone to afford 3.00 g of white needles; 70 % yield; mp 87-90 °C; ¹H-NMR (400 MHz; CDCl₃) δ 7.64-7.58 (m, 1H), 7.04-6.99 (m, 1H), [6.97 (d, 4H, J=8.5 Hz), 6.75 (d, 4H, J=8.8 Hz) AB q], 4.33 (s, 4H), 3.76 (s, 6H); ¹⁹F-NMR (376 MHz; CDCl₃) δ -125.44 to -125.56 (m), -128.61 to -128.72 (m), -156.91 to -157.03 (m); MS (APCl+) 121 (M-330, 100); (APCl-) 330 (M-121, 18), 195 (M-256, 100); IR (KBr) 1612, 1517, 1506, 1465, 1258, 1240, 1156, 1037, 1030 cm⁻¹; Anal. calcd/found for: C₂₂H₂₀F₃NO₄S C, 58.53/57.98; H, 4.47/4.61; N, 3.10/2.85.

### Step b: Preparation of 5-bis-(4-methoxybenzyl)sulfamoyl-2,3,4-trifluorobenzoic acid

To a stirring solution comprised of 1-*bis*-(4-methoxybenzyl)sulfamoyl-2,3,4-trifluorobenzene (2.95 g, 6.5x10⁻³ mol) in tetrahydrofuran (60 ml) at -78 °C was added a solution comprised of 2.0 M lithium diisopropylamide in tetrahydrofuran/heptane/ethylbenzene (Aldrich, 3.35 ml, 6.7x10⁻³ mol). After several minutes of stirring, the dark solution was transferred via canula over five minutes to a stirring solution comprised of carbon dioxide (excess) in diethyl ether at -78 °C. A white precipitate immediately formed. The cold bath was removed and the reaction mixture was stirred at ambient temperature for 18 hours. The reaction mixture was quenched with 200 ml of dilute aqueous hydrochloric acid. The layers were separated and the organic phase was dried (MgSO₄) and concentrated *in vacuo* to give 2.82 g of an off-white solid. Recrystallization from dichloromethane (150 ml) afforded 2.10g of the white powder product; 65 % yield; mp 158-161 °C; ¹H-NMR (400 MHz; DMSO) δ 7.80-7.76 (m, 1H), 7.05-6.74 (AB q, 8H, J=8.6 Hz), 4.33 (s, 4H), 3.66 (s, 6H); ¹⁹F-NMR (376 MHz; DMSO) δ-123.28 to -123.36 (m), -124.12 to -124.21 (m), -155.41 to -155.53 (m); MS (APCI-) 494 (M-1, 47), 216 (89), 195 (100); IR (KBr) 3420, 2954, 2838, 1695, 1613, 1512, 1347, 1238, 1152, 1079 cm⁻¹; Anal. calcd/found for: C₂₃H₂₀F₃NO₆S C, 55.76/55.85; H, 4.07/4.02; N, 2.83/2.71; F, 11.50/11.41; S, 6.47/6.25.

### Step c: Preparation of 5-bis-(4-methoxybenzyl)sulfamoyl-2-(2-chloro-4-iodophenylamino)-3,4-difluorobenzoic acid (PD 215729)

To a stirring solution comprised of 2-chloro-4-iodoaniline (0.53 g, 2.0x10⁻³ mol) in tetrahydrofuran (10 ml) at -78 °C under a nitrogen atmosphere was added a solution comprised of 1.0 M lithium bis(trimethylsilyl)amide in tetrahydrofuran (Aldrich, 4.1 ml, 4.1x10⁻³ mol). Within several minutes the solution became a thick light-green suspension. To this mixture was added a solution comprised of lithium 5-*bis*-(4-methoxybenzyl)sulfamoyl-2,3,4-trifluorobenzoate in tetrahydrofuran, which was prepared by adding 2.0 ml of the Aldrich lithium bis(trimethylsilyl)amide solution (0.0020 mmol) to a solution comprised of 5-*bis*-(4-methoxybenzyl)sulfamoyl-2,3,4-trifluorobenzoic acid (1.00 g, 2.0x10⁻³ mol) in tetrahydrofuran (10 ml) at -78 °C. The reaction mixture was stirred for 15 minutes and was then concentrated *in vacuo* to a crude semisolid. The semisolid was taken up into diethyl ether (250 ml) and was washed with 1 % aqueous hydrochloric acid (150 ml). The ether phase was then washed with neutral water (200ml, pH 4 after wash), a second portion of water (200 ml, pH 6 after wash), and brine (200 ml). The organic phase was then dried (MgSO₄) and was concentrated *in vacuo* to give 1.88 g of a sticky residue which was crystallized from toluene-heptane to afford 1.12 g of an off-white powder; 76 % yield; mp 162-166 °C; ¹H-NMR (400 MHz; DMSO) δ 9.86 (s, 1H), 7.92 (d, 1H, J=6.8 Hz), 7.86 (d, 1H, J=1.7 Hz), 7.60 (dd, 1H, J=8.5, 1.7 Hz), 7.06-7.04/6.78-6.75 (AB q, 8H, J=8.5 Hz), 6.93-6.89 (m, 1H), 4.31 (s, 4H), 3.66 (s, 6H); ¹⁹F-NMR (376 MHz; DMSO) δ-127.22 (d), -141.36 (d); MS (APCI+) 729 (M+1, 1), 256 (50), 121 (100); (APCI-) 727 (M-1, 100); IR (KBr) 1698, 1673, 1513, 1251 cm⁻¹; Anal. calcd/found for: C₂₉H₂₄ClF₂IN₂O₆S C, 47.78/47.93; H, 3.32/3.33; N, 3.84/3.80; Cl, 4.86/4.84; F, 5.21/5.46; I, 17.41/17.16; S, 4.40/4.29.

### Step d: Preparation of 5-bis-(4-methoxybenzyl)sulfamoyl-2-(2-chloro-4-iodophenylamino)-N-cyclopropylmethoxy-3,4-difluorobenzamide (PD 218774)

To a stirring solution comprised of 5-*bis*-(4-methoxybenzyl)sulfamoyl-2-(2-chloro-4-iodophenylamino)-3,4-difluorobenzoic acid (0.935 g, 1.28x10⁻³ mol), cyclopropylmethoxylamine hydrochloride (0.175 g, 1.42x10⁻³ mol), and diisopropylethylamine (0.75 ml, 4.26x10⁻³ mol) in a 1:1 v/v tetrahydrofuran-dichloromethane mixture (50 ml) was added solid PyBOP ([benzotriazolyloxy]tripyrrolidino phosphonium hexafluorophosphate, Advanced ChemTech, 0.76 g, 1.46x10⁻³ mol). The reaction mixture was stirred for one hour, was then evaporated to a crude residue which was purified by flash silica column chromatography. Elution with a gradient (25 % dichloromethane to 75 % dichloromethane in hexanes) afforded 0.63 g of the off-white powder product; 62 % yield; mp 70->300 °C; ¹H-NMR (400 MHz; DMSO) δ 11.92 (s, 1H), 9.35 (s, 1H), 7.60 (s, 1H), 7.50-7.45 (m, 1H), 7.34 (d, 1H, J=8.5 Hz), 6.82-6.54 (AB q, 8H, J=8.3 Hz), 6.59-6.54 (m, 1H), 4.09 (s, 4H), 3.46 (s, 6H), 0.90-0.80 (m, 1H), 0.30-0.25 (m, 2H), 0.03-0.00 (m, 2H); ¹⁹F-NMR (376 MHz; DMSO) δ-129.05 (s), -140.23 (d, J=18.3 Hz); MS (APCI+) 798 (M+1, 70); (APCI-) 796 (M-1, 15), 726 (50), 131 (100); IR (KBr) 1642, 1611, 1584, 1513, 1478 cm⁻¹; Anal. calcd/found for: C₃₃H₃₁ClF₂lN₃O₆S C, 49.67/49.88; H, 3.92/3.95; N, 5.27/5.19.

### Step e: Preparation of 2-(2-chloro-4-iodophenylamino)-N-cyclopropylmethoxy-3,4-difluoro-5-sulfamoyl-benzamide (PD 219622)

A reaction solution comprised of 5-*bis*-(4-methoxybenzyl)sulfamoyl-2-(2-chloro-4-iodophenylamino)-N-cyclopropylmethoxy-3,4-difluorobenzamide (0.1010 g, 1.266x10⁻⁴ mol) in trifluoroacetic acid (4 ml) was stirred at ambient temperature for 24 hours. The mixture was vacuum filtered and the precipitate rinsed with hexanes to afford 28.6 mg of a pale lavender powder; 42 % yield; mp 219-227 °C DEC; ¹H-NMR (400 MHz; DMSO) δ 11.89 (s, 1H), 9.08 (s, 1H), 7.60 (s, 3H), 7.55 (d, 1H, J=6.9 Hz), 7.32 (d, 1H, J=8.6 Hz), 6.63-6.59 (m, 1H), 3.40 (d, 2H, J=6.6 Hz), 0.90-0.80 (m, 1H), 0.30-0.26 (m, 2H), 0.05-0.00 (m, 2H); ¹⁹F-NMR (376 MHz; DMSO) δ
-130.61 (s), -140.38 (d, J=21.4 Hz); MS (APCI+) 558 (M+1, 70), 282 (100); (APCI-) 556 (M-1, 73), 486 (100); IR (KBr) 3390, 3283, 1652, 1513, 1477, 1163 cm⁻¹; Anal. calcd/found for: C₁₇H₁₅CIF₂IN₃O₄S 0.1 C₂HF₃O₂ C, 36.30/36.31; H, 2.67/2.55; N, 7.38/7.00.

### EXAMPLE 3C

### Preparation of 2-(2-chloro-4-iodo-phenylamino)-3,4-difluoro-N-hvdroxy-5-sulfamoyl-benzamide (PD 224213)

To a stirring solution comprised of 5-*bis*-(4-methoxybenzyl)sulfamoyl-2-(2-chloro-4-iodophenylamino)-3,4-difluorobenzoic acid (0.67 g, 9.2x10⁻⁴ mol), O-(tetrahydro-2H-pyran-2-yl)-hydroxylamine (0.113 g, 9.65x10⁻⁴ mol), and diisopropylethylamine (0.50 ml, 2.9x10⁻³ mol) in a 1:1 v/v tetrahydrofuran-dichloromethane mixture (20 ml) was added solid PyBOP ([benzotriazolyloxy]tripyrrolidino phosphonium hexafluorophosphate, Advanced ChemTech, 0.52 g, 1.0x10⁻³ mol). The reaction mixture was stirred for 30 minutes, was concentrated *in vacuo* to a yellow oil, and was crystallized from methanol to afford 0.35 g of the off-white amorphous intermediate; 46 % yield; the intermediate was dissolved in trifluoroacetic acid (10 ml) and was stirred at ambient temperature for 16 hours. The mixture was vacuum filtered to collect the precipitate, which was recrystallized from methanol-chloroform to afford 0.055 g of the tan powder product; 26 % yield from intermediate; mp 230-236 °C DEC; ¹H-NMR (400 MHz; DMSO) δ 11.73 (s, 1H), 9.46 (s, 1H), 9.38 (s, 1H), 7.80-7.75 (m, 2H), 7.79 (s, 2H), 7.50 (d, 1H, J=8.5 Hz), 6.82-6.78 (m, 1H); ¹⁹F-NMR (376 MHz; DMSO) δ -130.83 (s), -139.24 (s); MS (APCI+) 504 (M+1, 53), 488 (90), 471 (100); (APCI-) 502 (M-1, 12), 486 (100); IR (KBr) 3295, 1652, 1636, 1519, 1477, 1315, 1157 cm⁻¹; Anal. calcd/found for: C₁₃H₉ClF₂IN₃O₄S 0.41 CHCl₃ C, 29.15/29.05; H, 1.72/1.66; N, 7.60/7.21.

### EXAMPLE 4C

### Preparation of 2-(2-chloro-4-iodo-phenylamino)-3,4-difluoro-5-sulfamoylbenzoic acid (PD 215730)

Solid 5-*bis*-(4-methoxybenzyl)sulfamoyl-2-(2-chloro-4-iodophenylamino)-3,4-difluorobenzoic acid (0.0995 g, 1.36x10⁻⁴ mol) was dissolved in trifluoroacetic acid (5 ml) under a nitrogen atmosphere. The reaction mixture was stirred at ambient temperature for 65 hours. The mixture was vacuum filtered to isolate 55.2 mg of a fine white precipitate. The crude product was recystallized from chloroform to afford 31.8 mg of the fluffy white solid product; 48 % yield; mp 295-296 °C DEC; ¹H-NMR (400 MHz; DMSO) δ 9.77 (s, 1H), 8.16 (d, 1H, J=7.3 Hz), 7.82 (s, 3H), 7.56 (d, 1H, J=8.5 Hz), 6.97-6.92 (m, 1H); ¹⁹F-NMR (376 MHz; DMSO) δ -128.47 (s), -141.13 (d, 19.8 Hz); MS (APCI+) 489 (M+1, 5), 102 (100); (APCI-) 491 (32), 490 (18), 489 (100), 488 (18), 487 (M-1, 75); IR (KBr) 3372, 3244, 1688 cm⁻¹; Anal. calcd/found for: C₁₃H₈ClF₂IN₂O₄S C, 31.96/32.19; H, 1.65/1.81; N, 5.73/5.37.

### EXAMPLE 5C

### Preparation of 2-(2-chloro-4-iodo-phenylamino)-N-cyclopropylmethoxy-3,4-difluoro-5-dimethylsulfamoyl-benzamide (PD 250253)

### Step a: Preparation of 2-(2-chloro-4-iodo-phenylamino)-3,4-difluoro-5-dimethylsulfamoyl-benzoic acid (PD 224339)

To a stirring solution comprised of 5-dimethylsulfamoyl-2,3,4-trifluorobenzoic acid (1.00 g, 3.53x10⁻³ mol) in tetrahydrofuran (15 ml) at -78 °C under a nitrogen atmosphere was added a 1.0 M solution of lithium *bis*(trimethylsilyl)amide in tetrahydrofuran (Aldrich, 3.6 ml, 3.6x10⁻³ mol). A lithium 2-chloro-4-iodoanilide suspension formed by adding a 1.0 M solution of lithium *bis*(trimethylsilyl)amide solution (7.2 ml, 7.2x10⁻³ mol) to a solution comprised of 2-chloro-4-iodoaniline (0.94 g, 3.63x10⁻³ mol) in tetrahydrofuran (15 ml) at -78 °C was added via canula to the lithium 5-dimethylsulfamoyl-2,3,4-trifluorobenzoate suspension. The cold bath was removed and the reaction mixture was stirred for one hour. The mixture was concentrated *in vacuo* to a crude solid. The crude product was suspended in diethyl ether (200 ml), to which suspension hydrogen chloride gas was introduced to produce a white precipitate. The precipitate was removed by vacuum filtration. The filtrate was concentrated *in vacuo* to give a dull-colored solid, which was triturated with hexanes-dichloromethane to afford 1.31 g of the white powder product; 72 % yield; mp 218-222 °C; ¹H-NMR (400 MHz; DMSO) δ 9.89 (s, 1H), 8.06 (d, 1H, J=6.1 Hz), 7.85 (d, 1H, J=1.9 Hz), 7.58 (dd, 1H, J=8.5, 1.9 Hz), 7.03 (dd, 1H, J=8.3, 6.6 Hz), 2.71 (s, 6H); ¹⁹F-NMR (376 MHz; DMSO) δ -125.58 (d, J=18.3 Hz), -140,14 (d, J=16.8 Hz); MS (APCl+) 519 (40), 518 (15), 517 (M+1, 100); (APCI-) 517 (6), 516 (2), 515 (M-1, 5), 480 (45), 127 (100); IR (KBr) 3346, 1665, 1487, 1283 cm⁻¹; Anal. calcd/found for: C₁₅H₁₂ClF₂lN₂O₄S C, 34.87/34.98; H, 2.34/2.32; N, 5.42/5.32.

### Step b: Preparation of 2-(2-chloro-4-iodo-phenylamino)-N-cyclopropylmethoxy-3,4-difluoro-5-dimethylsulfamoyl-benzamide

To a suspension comprised of 2-(2-chloro-4-iodo-phenylamino)-3,4-difluoro-5-dimethylsulfamoyl-benzoic acid (0.5 g, 9.68x10⁻⁴ mol) and cyclopropylmethoxylamine hydrochloride (0.13 g, 1.05x10⁻³ mol) in a 1:1 v/v mixture of dichloromethane-tetrahydrofuran (10 ml) was added diisopropylethylamine (0.65 ml, 3.73x10⁻³ mol) followed by the addition of solid PyBOP (0.55 g, 1.06x10⁻³ mol). The reaction mixture was stirred at ambient temperature for three days. The mixture was concentrated *in vacuo* to a red oil. The crude product was treated with 10 % aqueous hydrochloric acid (150 ml) and was extracted with diethyl ether (150 ml). The organic phase was dried (MgSO₄) and concentrated *in vacuo* to a crude solid. The solid was triturated with dichloromethane-hexanes and recovered by vacuum filtration to afford 0.3558 g of the white powder product; 63 % yield; mp 222-225 °C DEC; ¹H-NMR (400 MHz; DMSO) δ 11.97 (s, 1H), 9.32 (s, 1H), 7.60 (d, 1H, J=1.9 Hz), 7.49 (d, 1H, J=5.8 Hz), 7.33 (dd, 1H, J=8.4, 1.9 Hz), 6.70 (dd, 1H, 8.4, 6.3 Hz), 3.43 (d, 2H, J=7.2 Hz), 2.53 (s, 6H), 0.87-0.83 (m, 1H), 0.30-0.25 (m, 2H), 0.03-0.00 (m, 2H); ¹⁹F-NMR (3.76 MHz; DMSO) δ-127.67 (d, J=19.8 Hz), -139.32 (d, J=19.8 Hz); MS (APCI+) 586 (M+1, 100); (APCI-) 584 (M-1, 40), 514 (100); IR (KBr) 3263, 1644, 1585, 1507, 1480 cm⁻¹; Anal. calcd/found for: C₁₉H₁₉ClF₂IN₃O₄S C, 38.96/39.08; H, 3.27/3.18; N, 7.17/7.17.

### EXAMPLE 6C

### Preparation of N-cyclopropylmethoxy-3,4-difluoro-5-dimethylsulfamoyl-2-(4-iodo-2-methyl-phenylamino)-benzamide (PD 252745)

### Step a: Preparation of 3,4-difluoro-5-dimethylsulfamoyl-2-(4-iodo-2-methylphenylamino)-benzoic acid (PD 224340)

Same procedure and same scale as Example 4C, Step a, except 4-iodo-2-methylaniline was used instead of 2-chloro-4-iodoaniline; afforded 0.9592 g of the off-white powder product; 55 % yield; mp 235-238 °C; ¹H-NMR (400 MHz; DMSO) δ 9.69 (s, 1H), 8.04 (d, 1H, J=6.1 Hz), 7.60 (d, 1H, J=1.5 Hz), 7.45 (dd, 1H, J=8.3, 1.7 Hz), 6.88 (dd, 1H, J=8.3, 5.4 Hz), 2.70 (s, 6H), 2.21 (s, 3H); ¹⁹F-NMR (376 MHz; DMSO) δ -126.25 (d, J=16.8 Hz), -142.74 (d, J=19.8 Hz); MS (APCl+) 497 (M+1, 69), 357 (70), 316 (100); (APCI-) 495 (M-1, 3), 127 (100); IR (KBr) 3240, 1686, 1512, 1473, 1341, 1151 cm⁻¹; Anal. calcd/found for: C₁₆H₁₅F₂IN₂O₄S C, 38.72/38.70; H, 3.05/3.01; N, 5.64/5.49.

### Step b: Preparation of N-cyclopropylmethoxy-3,4-difluoro-5-dimethylsulfamoyl-2-(4-iodo-2-methyl-phenylamino)-benzamide

Same procedure and same scale as Example 4C, Step b, except the product was purified by recrystallization from absolute ethanol to afford 0.1718 g of the pale yellow microcrystalline product; 28 % yield; mp 171-172 °C; ¹H-NMR (400 MHz; DMSO) δ 11.79 (s, 1H), 8.91 (s, 1H), 7.40 (d, 1H, J=4.3 Hz), 7.36 (s, 1H), 7.21 (d, 1H, J=8.2 Hz), 6.54 (dd, 1H, 8.2, 4.3 Hz), 3.30 (d, 2H, J=6.5 Hz), 2.52 (s, 6H), 2.00 (s, 3H), 0.85-0.75 (m, 1H), 0.29 (d, 2H, J=7.7 Hz), 0.01 (d, 2H, J=4.1 Hz); ¹⁹F-NMR (376 MHz; DMSO) δ -128.94 (s),-143.32 (d, J=19.8 Hz); MS (APCI+) 566 (M+1, 100); (APCl-) 564 (M-1, 85), 494 (100); IR (KBr) 1649, 1609, 1588, 1512, 1475 cm⁻¹; Anal. calcd/found for: C₂₀H₂₂F₂IN₃O₄S C, 42.49/42.42; H, 3.92/3.78; N, 7.43/7.40.

### EXAMPLE 7C

### Preparation of 2-(2-chloro-4-iodo-phenylamino)-N-hydroxy-4-dimethylsulfamoyl-benzamide

### Step a: Preparation of 4-methyl-benzene-N,N-dimethylsulfonamide

To a stirring solution comprised of *para*-toluenesulfonyl chloride in dichloromethane at 0 °C is introduced excess gaseous dimethylamine. The precipitate is removed by filtration and the filtrate is concentrated *in vacuo* to obtain the product.

### Step b: Preparation of 4-methyl-3-nitro-benzene-N,N-dimethylsulfonamide

To a gently stirring solution comprised of 1 molar equivalent of fuming nitric acid in excess concentrated sulfuric acid is added 1 molar equivalent of 4-methyl-benzene-N,N-dimethylsulfonamide in increments. The mixture is stirred for one hour and then poured over chilled water. The mixture is extracted with a suitable solvent like diethyl ether or dichloromethane. The organic phase is dried over a suitable drying agent like magnesium sulfate and concentrated *in vacuo* to afford a crude product which may be purified by normal methods such as chromatography or crystallization from a solvent like chloroform or heptane.

### Step c: Preparation of 3-amino-4-methyl-benzene-N,N-dimethylsulfonamide

The compound 4-methyl-3-nitro-benzene-N,N-dimethylsulfonamide is dissolved in ethanol. A catalyst like Raney nickel is added and the mixture hydrogenated in a shaker. The catalyst is removed by filtration. The solvent is removed *in vacuo* to give a product which may be purified if necessary by chromatography or crystallization from an appropriate solvent like chloroform or heptane-ethyl acetate.

### Step d: Preparation of 3-fluoro-4-methyl-benzene-N,N-dimethylsulfonamide

The compound 3-amino-4-methyl-benzene-N,N-dimethylsulfonamide is diazotized with an alkyl nitrite like *tert*-butyl nitrite under anhydrous conditions in a non-reactive solvent like tetrahydrofuran or dichloromethane. The intermediate diazonium species is then treated with pyridinium fluoride to give the product, which may be purified by chromatography or crystallization.

### Step e: Preparation of 4-dimethylsulfamoyl-2-fluoro-benzoic acid

A mixture comprised of 3-fluoro-4-methyl-benzene-N,N-dimethylsulfonamide and potassium permanganate (2.2 molar equivalents) in water is brought to reflux for four hours. The reaction mixture is filtered through celite. The filtrate is treated with activated carbon and refiltered through fresh celite. The second filtrate is acidified with concentrated hydrochloric acid to pH 0. The mixture is allowed to cool and is extracted with diethyl ether. The organic phase is dried over a drying agent like magnesium sulfate and is concentrated *in vacuo*. The product may be purified by recrystallization from an appropriate solvent like ethanol or chloroform.

### Step f: Preparation of 2-(2-chloro-4-iodo-phenylamino)-4-dimethylsulfamoylbenzoic acid

To a stirring cold (-78 °C) solution comprised of 2-chloro-4-iodoaniline (1 molar equivalent) in anhydrous tetrahydrofuran under a nitrogen atmosphere is added a commercially available lithium diisopropylamide solution (Aldrich, 2.0 M in tetrahydrofuran/heptane/ethylbenzene, 1 molar equivalent). After stirring for 5 minutes, a solution comprised of 4-dimethylsulfamoyl-2-fluoro-benzoic acid (1 molar equivalent) in tetrahydrofuran is added. The cold bath is removed and the reaction mixture is stirred for 2 hours. The reaction mixture is then partitioned between diethyl ether and dilute aqueous hydrochloric acid: The organic phase is washed with brine, dried over magnesium sulfate, and concentrated *in vacuo* to afford a product which may be purified by chromatography or recrystallization from an appropriate solvent like chloroform or heptane-ethanol.

### Step g: Preparation of 2-(2-chloro-4-iodo-phenylamino)-4-dimethylsulfamoylbenzoic acid O-(tetrahydro-2H-pyran-2-yl)-oxyamide

A solution comprised of 2-(2-chloro-4-iodo-phenylamino)-4-dimethylsulfamoyl-benzoic acid, O-(tetrahydro-2H-pyran-2-yl)-hydroxylamine (1.25 molar equivalents), benzotriazole-1-yl-oxy-*tris*-pyrrolidino-phosphonium hexafluorophosphate (1.25 molar equivalents), and diisopropylethylamine (3 molar equivalents) in 1:1 v/v tetrahydrofuran-dichloromethane is stirred for 30 minutes. The reaction mixture is concentrated *in vacuo* and the residue is purified by flash chromatography; elution with dichloromethane affords the desired product. The product may be recrystallized with an appropriate solvent like methanol if further purification is necessary.

### Step h: Preparation of 2-(2-chloro-4-iodo-phenylamino)-4-dimethylsulfamoyl-N-hydroxy-benzamide

The compound 2-(2-chloro-4-iodo-phenylamino)-4-dimethylsulfamoylbenzoic acid O-(tetrahydro-2H-pyran-2-yl)-oxyamide is dissolved in an appropriate hydrogen chloride-saturated solvent like methanol or ethanol. Once homogeneous, the solution is concentrated *in vacuo* to give the desired product. The product may be triturated with an appropriate solvent like chloroform or dichloromethane if further purification is necessary.

### D. Uses

The disclosed compositions are useful as both prophylactic and therapeutic treatments for diseases or conditions relating to chronic pain, including neuropathic pain, as provided in the Summary section, as well as diseases or conditions modulated by the MEK cascade. For example, in one embodiment, the disclosed method relates to postoperative pain, phantom limb pain, burn pain, gout, trigeminal neuralgia, acute herpetic and postherpetic pain, causalgia, diabetic neuropathy, plexus avulsion, neuroma, vasculitis, crush injury, constriction injury, tissue injury, post-surgical pain, arthritis pain, or limb amputation

For example, local injuries can be treated with local or topical administration. Chronic pain affecting the entire body, such as diabetic neuropathy can be treated with systemic administration (injection or orally) of a disclosed composition. Treatment for chronic pain (e.g., post-operative pain) confined to the lower body can be administered centrally, e.g., epidurally. Formulations and methods of administration can include the use of more than one MEK inhibitor, or a combination of a MEK inhibitor and another pharmaceutical agent, such as an anti-inflammatory, analgesic, muscle relaxing, or anti-infective agent. Preferred routes of administration are oral, intrathecal or epidural, subcutaneous, intravenous, intramuscular, and, for non-human mammals, intraplantar, and are preferably epidural.

### 1. Dosages

Those skilled in the art will be able to determine, according to known methods, the appropriate dosage for a patient, taking into account factors such as age, weight, general health, the type of pain requiring treatment, and the presence of other medications. In general, an effective amount will be between 0.1 and 1000 mg/kg per day, preferably between 1 and 300 mg/kg body weight, and daily dosages will be between 10 and 5000 mg for an adult subject of normal weight. Commercially available capsules or other formulations (such as liquids and film-coated tablets) of 100 mg, 200 mg, 300 mg, or 400 mg can be administered according to the disclosed methods.

### 2. Formulations

Dosage unit forms include tablets, capsules, pills, powders, granules, aqueous and nonaqueous oral solutions and suspensions, and parenteral solutions packaged in containers adapted for subdivision into individual doses. Dosage unit forms can also be adapted for various methods of administration, including controlled release formulations, such as subcutaneous implants. Administration methods include oral, rectal, parenteral (intravenous, intramuscular, subcutaneous), intracisternal, intravaginal, intraperitoneal, intravesical, local (drops, powders, ointments, gels, or cream), and by inhalation (a buccal or nasal spray).

Parenteral formulations include pharmaceutically acceptable aqueous or nonaqueous solutions, dispersion, suspensions, emulsions, and sterile powders for the preparation thereof. Examples of carriers include water, ethanol, polyols (propylene glycol, polyethylene glycol), vegetable oils, and injectable organic esters such as ethyl oleate. Fluidity can be maintained by the use of a coating such as lecithin, a surfactant, or maintaining appropriate particle size. Carriers for solid dosage forms include (a) fillers or extenders, (b) binders,
(c) humectants, (d) disintegrating agents, (e) solution retarders, (f) absorption acccelerators, (g) adsorbants, (h) lubricants, (i) buffering agents, and (j) propellants.

Compositions may also contain adjuvants such as preserving, wetting, emulsifying, and dispensing agents; antimicrobial agents such as parabens, chlorobutanol, phenol, and sorbic acid; isotonic agents such as a sugar or sodium chloride; absorption-prolonging agents such as aluminum monostearate and gelatin; and absorption-enhancing agents.

### 3. Related compounds

The invention provides the disclosed compounds and closely related, pharmaceutically acceptable forms of the disclosed compounds, such as salts, esters, amides, hydrates or solvated forms thereof; masked or protected forms; and racemic mixtures, or enantiomerically or optically pure forms.

Pharmaceutically acceptable salts, esters, and amides include carboxylate salts (e.g., C ₁₋₈ alkyl, cycloalkyl, aryl, heteroaryl, or non-aromatic heterocyclic), amino acid addition salts, esters, and amides which are within a reasonable benefit/risk ratio, pharmacologically effective, and suitable for contact with the tissues of patients without undue toxicity, irritation, or allergic response. Representative salts include hydrobromide, hydrochloride, sulfate, bisulfate, nitrate, acetate, oxalate, valerate, oleate, palmitate, stearate, laurate, borate, benzoate, lactate, phosphate, tosylate, citrate, maleate, fumarate, succinate, tartrate, naphthylate, mesylate, glucoheptonate, lactiobionate, and laurylsulfonate. These may include alkali metal and alkali earth cations such as sodium, potassium, calcium, and magnesium, as well as non-toxic ammonium, quaternary ammonium, and amine cations such as tetramethyl ammonium, methylamine, trimethylamine, and ethylamine. See, for example, S.M. Berge, et al., "Pharmaceutical Salts," *J. Pharm. Sci*., 1977, 66:1-19 which is incorporated herein by reference. Representative pharmaceutically acceptable amides of the invention include those derived from ammonia, primary C ₁₋₆ alkyl amines and secondary di (C ₁₋₆ alkyl) amines. Secondary amines include 5- or 6-membered heterocyclic or heteroaromatic ring moieties containing at least one nitrogen atom and optionally between 1 and 2 additional heteroatoms. Preferred amides are derived from ammonia, C ₁₋₃ alkyl primary amines, and di (C ₁₋₂ alkyl)amines. Representative pharmaceutically acceptable esters of the invention include C ₁₋₇ alkyl, C ₅₋₇ cycloalkyl, phenyl, and phenyl(C ₁₋₆)alkyl esters. Preferred esters include methyl esters.

The invention also includes disclosed compounds having one or more functional groups (e.g., hydroxyl, amino, or carboxyl) masked by a protecting group. Some of these masked or protected compounds are pharmaceutically acceptable; others will be useful as intermediates. Synthetic intermediates and processes disclosed herein, and minor modifications thereof, are also within the scope of the invention.

### HYDROXYL PROTECTING GROUPS

Hydroxyl protecting groups include: ethers, esters, and protection for 1,2- and 1,3-diols. The ether protecting groups include: methyl, substituted methyl ethers, substituted ethyl ethers, substituted benzyl ethers, silyl ethers and conversion of silyl ethers to other functional groups.

### Substituted Methyl Ethers

Substituted methyl ethers include: methoxymethyl, methylthiomethyl, *t*-utylthiomethyl, (phenyldimethylsilyl) methoxymethyl, benzyloxymethyl, *p*-ethoxybenzyloxymethyl, (4-methoxyphenoxy) methyl, guaiacolmethyl, *t*-butoxymethyl, 4-pentenyloxymethyl, siloxymethyl, 2-methoxyethoxymethyl, 2,2,2-trichloroethoxymethyl, bis(2-chloro- ethoxy)methyl, 2-(trimethylsilyl)ethoxymethyl, tetrahydropyranyl, 3-bromotetrahydro-pyranyl, tetrahydrothiopyranyl, 1-methoxycyclohexyl, 4-methoxytetrahydropyranyl, 4-methoxytetrahydrothio-pyranyl, 4-methoxytetrahydrothiopyranyl *S,S*-dioxido, 1-[(2-chloro-4-methyl)phenyl]-4-methoxypiperidin-4-yl, 1,4-dioxan-2-yl, tetrahydrofuranyl, tetrahydrothiofuranyl, and 2,3,3a,4,5,6,7,7a-octahydro-7,8,8-trimethyl-4,7-ethanobenzofuran-2-yl.

### Substituted Ethyl Ethers

Substituted ethyl ethers include: 1-ethoxyethyl, 1-(2,chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilyethyl, 2-(phenylselenyl)ethyl, *t*-butyl, allyl, *p*-chlorophenyl, *p*-methoxyphenyl, 2,4-dinitrophenyl, and benzyl.

### Substituted Benzyl Ethers

Substituted benzyl ethers include: *p*-methoxybenzyl, 3,4-dimethoxybenzyl, *o*-nitrobenzyl, *p*-nitrobenzyl, *p*-halobenzyl, 2,6-dichlorobenzyl, *p*-cyanobenzyl, *p*-phenylbenzyl, 2- and 4-picolyl, 3-methyl-2-picolyl *N*-oxido, diphenylmethyl, *p*, *p*'-dinitrobenzhydryl, 5-dibenzosuberyl, triphenylmethyl, α-naphthyldiphenylmethyl, *p*-methoxyphenyldiphenylmethyl, di(*p*-methoxyphenyl)phenylmethyl, tri-(*p*-methoxyphenyl)methyl, 4-(4'-bromophenacyloxy)phenyldiphenylmethyl, 4,4',4"-tris(4,5-dichlorophthalimidophenyl)methyl, 4,4',4"-tris(levulinoyloxyphenyl) methyl, 4,4',4"tris(benzoyloxyphenyl)methyl, 3-(imidazol-1-ylmethyl)bis(4',4"-dimethoxyphenyl)-methyl, 1,1-bis(4-methoxyphenyl)-1'-pyrenylmethyl, 9-anthryl, 9-(9-phenyl) xanthenyl, 9-(9-phenyl-10-oxo) anthryl, 1,3-benzodithiolan-2-yl, and benzisothiazolyl *S,S*-dioxido.

### Silyl Ethers

Silyl ethers include: trimethylsilyl, triethylsilyl, triisopropylsilyl, dimethylisopropylsilyl, diethylisopropylsilyl, dimethylthexylsilyl, *t*-butyldimethylsilyl, *t*-butyldiphenylsilyl, tribenzylsilyl, tri-*p*-xylylsilyl, triphenylsilyl, diphenylmethylsilyl, and *t*-butylmethoxyphenylsilyl.

### ESTERS

Esters protecting groups include: esters, carbonates, assisted cleavage, miscellaneous esters, and sulfonates.

### Esters

Examples of protective esters include: formate, benzoylformate, acetate, chloroacetate, dichloroacetate, trichloroacetate, trifluoroacetate, methoxyacetate, triphenylmethoxyacetate, phenoxyacetate, *p*-chlorophenoxyacetate, *p*-P-phenylacetate, 3-phenylpropionate, 4-oxopentanoate (levulinate), 4,4-(ethylenedithio) pentanoate, pivaloate, adamantoate,crotonate,4-methoxycrotonate, benzoate, *p*-phenylbenzoate, and 2,4,6-trimethylbenzoate (mesitoate).

### Carbonates

Carbonates include: methyl, 9-fluorenylmethyl, ethyl, 2,2,2-trichloroethyl, 2-(trimethylsilyl) ethyl, 2-(phenylsulfonyl), ethyl, 2-(triphenylphosphonio) ethyl, isobutyl, vinyl, allyl, *p*-nitrophenyl, benzyl, *p*-methoxybenzyl, 3,4-dimethoxybenzyl, *o*-nitrobenzyl, p-nitrobenzyl, S-benzyl thiocarbonate, 4-ethoxy-1-naphthyl, and methyl dithiocarbonate.

### Assisted Cleavage

Examples of assisted cleavage protecting groups include: 2-iodobenzoate, 4-azido-butyrate, 4-nitro-4-methylpentanoate, *o*-(dibromomethyl) benzoate, 2-formylbenzene-sulfonate, 2-(methylthiomethoxy) ethyl carbonate, 4-(methylthiomethoxymethyl) benzoate, and 2-(methylthiomethoxymethyl) benzoate.

### Miscellaneous Esters

In addition to the above classes, miscellaneous esters include: 2,6-dichloro-4-methylphenoxyacetate, 2,6-dichloro-4-(1,1,3,3-tetramethylbutyl) phenoxyacetate, 2,4-bis(1,1-dimethylpropyl) phenoxyacetate, chlorodiphenylacetate, isobutyrate, monosuccinoate, (*E*)-2-methyl-2-butenoate (tigloate), *o*-(methoxycarbonyl) benzoate, *p*-P-benzoate, α-naphthoate, nitrate, alkyl *N,N,N'N'*-tetramethylphosphorodiamidate, *N*-phenylcarbamate, borate, dimethylphosphinothioyl, and 2,4-dinitrophenylsulfenate.

### Sulfonates

Protective sulfates includes: sulfate, methanesulfonate(mesylate), benzylsulfonate, and tosylate.

### PROTECTION FOR 1,2- AND 1,3-DIOLS

The protection for 1,2 and 1,3-diols group includes: cyclic acetals and ketals, cyclic ortho esters, and silyl derivatives.

### Cyclic Acetals and Ketals

Cyclic acetals and ketals include: methylene, ethylidene, 1-*t*-butylethylidene, 1-phenylethylidene, (4-methoxyphenyl) ethylidene, 2,2,2-trichloroethylidene, acetonide (isopropylidene), cyclopentylidene, cyclohexylidene, cycloheptylidene, benzylidene, *p*-methoxybenzylidene, 2,4-dimethoxybenzylidene, 3,4-dimethoxybenzylidene, and 2-nitrobenzylidene.

### Cyclic Ortho Esters

Cyclic ortho esters include: methoxymethylene, ethoxymethylene, dimethoxymethylene, 1-methoxyethylidene, 1-ethoxyethylidine, 1,2-dimethoxyethylidene, α-methoxybenzylidene, 1-(*N,N*-dimethylamino)ethylidene derivative, α-(*N*,*N*-dimethylamino) benzylidene derivative, and 2-oxacyclopentylidene.

### PROTECTION FOR THE CARBOXYL GROUP

### ESTERS

Ester protecting groups include: esters, substituted methyl esters, 2-substituted ethyl esters, substituted benzyl esters, silyl esters, activated esters, miscellaneous derivatives, and stannyl esters.

### Substituted Methyl Esters

Substituted methyl esters include: 9-fluorenylmethyl, methoxymethyl, methylthiomethyl, tetrahydropyranyl, tetrahydrofuranyl, methoxyethoxymethyl, 2-(trimethylsilyl)ethoxy-methyl, benzyloxymethyl, phenacyl, *p*-bromophenacyl, α-methylphenacyl, *p*-methoxyphenacyl, carboxamidomethyl, and *N*-phthalimidomethyl.

### 2-Substituted Ethyl Esters

2-Substituted ethyl esters include: 2,2,2-trichloroethyl, 2-haloethyl, chloroalkyl, 2-(trimethylsily)ethyl, 2-methylthioethyl, 1,3-dithianyl-2-methyl, 2(*p*-nitrophenylsulfenyl)-ethyl, 2-(*p*-toluenesulfonyl)ethyl, 2-(2'-pyridyl)ethyl, 2-(diphenylphosphino)ethyl, 1-methyl-1-phenylethyl, *t*-butyl, cyclopentyl, cyclohexyl, allyl, 3-buten-1-yl, 4-(trimethylsily)-2-buten-1-yl, cinnamyl, α-methylcinnamyl, phenyl, *p*-(methylmercapto)-phenyl, and benzyl.

### Substituted Benzyl Esters

Substituted benzyl esters include: triphenylmethyl, diphenylmethyl, bis(*o*-nitrophenyl)methyl, 9-anthrylmethyl, 2-(9,10-dioxo)anthrylmethyl, 5-dibenzo-suberyl, 1-pyrenylmethyl,2-(trifluoromethyl)-6-chromylmethyl, 2,4,6-trimethylbenzyl, *p*-bromobenzyl, *o*-nitrobenzyl, *p*-nitrobenzyl, *p*-methoxybenzyl, 2,6-dimethoxybenzyl, 4-(methylsulfinyl)benzyl, 4-sulfobenzyl, piperonyl, and 4-P-benzyl.

### Silyl Esters

Silyl esters include: trimethylsilyl, triethylsilyl, *t*-butyldimethylsilyl, *i*-propyldimethylsilyl, phenyldimethylsilyl, and di- *t*-butylmethylsilyl.

### Miscellaneous Derivatives

Miscellaneous derivatives includes: oxazoles, 2-alkyl-1,3-oxazolines, 4-alkyl-5-oxo-1,3-oxazolidines, 5-alkyl-4-oxo-1,3-dioxolanes, ortho esters, phenyl group, and pentaaminocobalt(III) complex.

### Stannyl Esters

Examples of stannyl esters include: triethylstannyl and tri-*n*-butylstannyl.

### AMIDES AND HYDRAZIDES

Amides include: *N*,*N* -dimethyl, pyrrolidinyl, piperidinyl, 5,6-dihydrophenanthridinyl, *o*-nitroanilides, *N*-7-nitroindolyl, *N*-8-nitro-1,2,3,4-tetrahydroquinolyl, and *p*-P-benzenesulfonamides. Hydrazides include: *N*-phenyl, *N*,*N'*-diisopropyl and other dialkyl hydrazides.

### PROTECTION FOR THE AMINO GROUP

### CARBAMATES

Carbamates include: carbamates, substituted ethyl, assisted cleavage, photolytic cleavage, urea-type derivatives, and miscellaneous carbamates.

### Carbamates

Carbamates include: methyl and ethyl, 9-fluorenylmethyl, 9-(2-sulfo)fluorenylmethyl, 9-(2,7-dibromo)fluorenylmethyl, 2,7-di-*t*-butyl-[9-(10,10-dioxo-10,10,10,10-tetrahydro- thioxanthyl)]methyl, and 4-methoxyphenacyl.

### Substituted Ethyl

Substituted ethyl protective groups include: 2,2,2-trichloroethyl, 2-trimethylsilylethyl, 2-phenylethyl, 1-(1-adamantyl)-1-methylethyl, 1,1-dimethyl-2-haloethyl, 1,1dimethyl-2,2-dibromoethyl, 1,1-dimethyl-2,2,2-trichloroethyl, 1-methyl-1-(4-biphenylyl)ethyl, 1-(3,5-di-*t*-butylphenyl)-1-methylethyl, 2-(2'-and 4'-pyridyl)ethyl, 2-(*N*,*N*-icyclohexylcarboxamido)- ethyl, *t*-butyl, 1-adamantyl, vinyl, allyl, 1-isopropylallyl, connamyl, 4-nitrocinnamyl, quinolyl, *N*-hydroxypiperidinyl, alkyldithio, benzyl, *p*-methoxybenzyl, *p*-nitrobenzyl, *p*-bromobenzyl, *p*-chlorobenzyl, 2,4dichlorobenzyl, 4-methylsulfinylbenzyl, 9-anthrylmethyl, and diphenylmethyl.

### Assisted Cleavage

Protection via assisted cleavage includes: 2-methylthioethyl, 2-methylsulfonylethyl, 2-(*p*-toluenesulfonyl)ethyl, [2-(1,3-dithianyl)]methyl, 4-methylthiophenyl, 2,4-dimethyl-thiophenyl, 2-phosphonioethyl, 2-triphenylphosphonioisopropyl, 1,1-dimethyl-2cyanoethyl, *m*-chloro-*p*-acyloxybenzyl, *p*-(dihydroxyboryl)benzyl, 5-benzisoxazolyl-methyl, and 2-(trifluoromethyl)-6-chromonylmethyl.

### Photolytic Cleavage

Photolytic cleavage methods use groups such as: *m*-nitrophenyl, 3,5-dimethoxybenzyl, *o*-nitrobenzyl, 3,4-dimethoxy-6-nitrobenzyl, and phenyl(*o*-nitrophenyl)methyl.

### Urea-Type Derivatives

Examples of of urea-type derivatives include: phenothiazinyl-(10)-carbonyl derivative, *N'*-p-toluenesulfonylaminocarbonyl, and *N'*-phenylaminothiocarbonyl.

### Miscellaneous Carbamates

In addition to the above, miscellaneous carbamates include: *t*-amyl, *S*-benzyl thiocarbamate, *p*-cyanobenzyl, cyclobutyl, cyclohexyl, cyclopentyl, cyclopropylmethyl, *p*-decyloxy-benzyl, diisopropylmethyl, 2,2-dimethoxycarbonylvinyl, *o*-(*N*,*N*-dimethyl-carboxamido)-benzyl, 1,1-dimethyl-3(*N*,*N*-dimethylcarboxamido)propyl, 1,1-dimethyl-propynyl, di(2-pyridyl)methyl, 2-furanylmethyl, 2-iodoethyl, isobornyl, isobutyl, isonicotinyl, *p*(*p'*-methoxyphenyl- azo)benzyl, 1-methylcyclobutyl, 1-methylcyclohexyl, 1-methyl-1-cyclopropyl- methyl, 1-methyl-(3,5-dimethoxyphenyl)ethyl, 1-methyl-1 (*p*-henylazophenyl)- ethyl, 1-methyl-1-phenylethyl, 1-methyl-1-(4-pyridyl)ethyl, phenyl, *p*-(phenylazo)benzyl, 2,4,6-tri-*t*-butylphenyl, 4-(trimethylammonium) benzyl, and 2,4,6-trimethylbenzyl.

### AMIDES

### Amides

Amides includes: *N*-formyl, *N*-acetyl, *N*-chloroacetyl, *N*-trichloroacetyl, *N*-trifluoroacetyl, *N*-phenylacetyl, *N*-3-phenylpropionyl, *N*-picolinoyl, *N*-3-pyridyl-carboxamide, *N*-benzoylphenylalanyl derivative, *N*-benzoyl, and *N*-*p*-phenylbenzoyl.

### Assisted Cleavage

Assisted cleavage groups include: *N*-*o*-nitrophenylacetyl, *N*-*o*-nitrophenoxyacetyl, *N*-acetoacetyl, (*N'*-dithiobenzyloxycarbonylamino)acetyl, *N*-3-(*p*-hydroxphenyl) propionyl, *N*-3-(*o*-nitrophenyl)propionyl, *N*-2-methyl-2-(*o*-nitrophenoxy)propionyl, *N*-2-methyl-2-(*o*-phenylazophenoxy)propionyl, *N*-4-chlorobutyryl, *N*-3-methyl-3-nitrobutyryl, *N*-*o*-nitrocinnamoyl, *N*-acetylmethionine derivative, *N*-*o*-nitrobenzoyl, *N-o*-(benzoyloxymethyl)benzoyl, and 4,5-diphenyl-3-oxazolin-2-one.

### Cyclic Imide Derivatives

Cyclic imide derivatives include: *N*-phthalimide, *N*-dithiasuccinoyl, *N*-2,3-diphenyl-maleoyl, *N*-2,5-dimethylpyrrolyl, *N*-1,1,4,4-tetramethyldisilylazacyclopentane adduct, 5-substituted 1,3-dimethyl-1,3,5-triazacyclohexan-2-one, 5-substituted 1,3-dibenzyl-1,3,5-triazacyclohexan-2-one, and 1-substituted 3,5-dinitro-4-pyridonyl.

### SPECIAL -NH PROTECTIVE GROUPS

Protective groups for - NH include: *N*-alkyl and *N*-aryl amines, imine derivatives, enamine derivatives, and *N*-hetero atom derivatives (such as *N*-metal, N-N, N-P, N-Si, and N-S), *N*-sulfenyl, and *N*-sulfonyl.

### N-Alkyl and N-Aryl Amines

*N*-alkyl and *N*-aryl amines include: *N*-methyl, *N*-allyl, *N*-[2-(trimethylsilyl)ethoxyl]-methyl, *N*-3-acetoxypropyl, *N*-(1-isopropyl-4-nitro-2-oxo-3-pyrrolin-3-yl), quaternary ammonium salts, *N*-benzyl, *N*-di(4-methoxyphenyl)methyl, *N*-5-dibenzosuberyl, *N*-triphenylmethyl, *N*-(4-methoxyphenyl)diphenylmethyl, *N*-9-phenylfluorenyl, *N*-2,7-dichloro-9-fluorenylmethylene, *N*-ferrocenylmethyl, and *N*-2-picolylamine *N'*-oxide.

### Imine Derivatives

Imine derivatives include: *N*-1,1-dimethylthiomethylene, *N*-benzylidene, *N*-*p*-methoxybenzylidene, *N*-diphenylmethylene, *N*-[(2-pyridyl)mesityl]methylene, *N*-(*N'*,*N'*-dimethylaminomethylene), *N*,*N'*-isopropylidene, *N*-*p*-nitrobenzylidene,
*N*-salicylidene, *N*-5-chlorosalicylidene, *N*-(5-chloro-2-hydroxyphenyl)phenylmethylene, and *N*-cyclohexylidene.

### Enamine Derivative

An example of an enamine derivative is *N*-(5,5-dimethyl-3-oxo-1-cyclohexenyl).

### N-Hetero Atom Derivatives

N-metal derivatives include: *N*-borane derivatives, *N*-diphenylborinic acid derivative, *N*-[phenyl(pentacarbonylchromium- or -tungsten)]carbenyl, and *N*-copper or *N*-zinc chelate. Examples of *N-N* derivatives include: *N*-nitro, *N*-nitroso, and *N*-oxide. Examples of *N*-P derivatives include: *N*-diphenylphosphinyl, *N*-dimethylthiophosphinyl, *N*-diphenylthiophosphinyl, *N*-dialkyl phosphoryl, *N*-dibenzyl phosphoryl, and *N*-diphenyl phosphoryl. Examples of *N*-sulfenyl derivatives include: *N*-benzenesulfenyl, *N*-*o*-nitrobenzenesulfenyl, *N*-2,4-dinitrobenzenesulfenyl, *N*-pentachlorobenzenesulfenyl, *N*-2-nitro-4-methoxy-benzenesulfenyl, *N*-triphenylmethylsulfenyl, and *N*-3-nitropyridinesulfenyl. *N*-sulfonyl derivatives include: *N*-*p*-toluenesulfonyl, *N*-benzenesulfonyl, *N*-2,3,6-trimethyl-4-methoxybenzenesulfonyl, *N*-2,4,6-trimethoxybenzenesulfonyl, *N*-2,6-dimethyl-4-methoxy-benzenesulfonyl, *N*-pentamethylbenzenesulfonyl, *N*-2,3,5,6-tetramethyl-4-methoxybenzene- sulfonyl, *N*-4-methoxybenzenesulfonyl, *N*-2,4,6-trimethylbenzenesulfonyl, *N*-2,6-dimethoxy- 4-methylbenzenesulfonyl, *N*-2,2,5,7,8-pentamethylchroman-6-sulfonyl, *N*-methanesulfonyl, *N*-β-trimethylsilylethanesulfonyl, *N*-9-anthracenesulfonyl, *N*-4-(4',8'-dimethoxynaphthylmethyl)-benzenesulfonyl, *N*-benzylsulfonyl, *N*-trifluoromethylsulfonyl, and *N*-phenacylsulfonyl.

Disclosed compounds which are masked or protected may be prodrugs, compounds metabolized or otherwise transformed *in vivo* to yield a disclosed compound, e.g., transiently during metabolism. This transformation may be a hydrolysis or oxidation which results from contact with a bodily fluid such as blood, the action of acids, or liver, gastrointestinal, or other enzymes.

Features of the invention are further described in the examples below.

### E. Examples

### BIOLOGICAL EXAMPLES

### Example 1

### Effect of PD 198306 on streptozocin-induced static allodynia

### Animals

Male Sprague Dawley rats (250-300g), obtained from Bantin and Kingman, (Hull, U.K.) were housed in groups of 3. All animals were kept under a 12h light/dark cycle (lights on at 07h 00min) with food and water *ad libitum.* All experiments were carried out by an observer blind to drug treatments.

### Development of diabetes in the rat

Diabetes was induced in rats by a single i.p. injection of streptozocin (50 mg/kg) as described previously (Courteix et al., 1993).

### Evaluation of static allodynia

Mechanical hypersensitivity was measured using Semmes-Weinstein von Frey hairs (Stoelting, Illinois, U.S.A.). Animals were placed into wire mesh bottom cages allowing access to the underside of their paws. Animals were habituated to this environment prior to the start of the experiment. Mechanical hypersensitivity was tested by touching the plantar surface of the animals right hind paw with von Frey hairs in ascending order of force ( 0.7, 1.2, 1.5, 2, 3.6, 5.5, 8.5, 11.8, 15.1 and 29g) for up to 6 sec. Once a withdrawal response was established, the paw was re-tested, starting with the next descending von Frey hair until no response occurred. The highest force of 29 g lifted the paw as well as eliciting a response, thus represented the cut off point. The lowest amount of force required to elicit a response was recorded as the paw withdrawal threshold (PWT) in grams.

### Drugs

PD 198306 [N-Cyclopropylmethoxy-3,4,5-trifluoro-2-(4-iodo-2-methylphenylamino)-benzamide] and Cl-1008 (pregabalin) were synthesized at Parke-Davis (Ann Arbor, Ml, USA). PD 198306 was suspended in cremophor:ethanol:water (1:1:8) vehicle. Pregabalin was dissolved in water. Both compounds were administered orally. Streptozocin (Aldrich, UK) was dissolved in 0.9% w/v NaCI and administered intraperitoneally. Drug administrations were made in a volume of 1 ml/kg.

### Statistics

The static allodynia data were analysed using a Kruskall-Wallis ANOVA for non-parametric results, followed when significant by Mann-Whitney's t test.

### Experimental protocol

Static allodynia was assessed with von Frey hairs, before (baseline, BL) and 1h after oral administration of PD 198306 (30mg/kg, p.o.), vehicle (cremophor:ethanol:water, 1:1:8) or pregabalin (30mg/kg, p.o.) (test). Animals were administered again the same compounds on the following day, both in the morning and the afternoon. Static allodynia was assessed only before and 1h after the afternoon administration, in order to minimise the habituation of the animals to the testing conditions. Animals treated with pregabalin received water in the morning administration, in order to avoid the potential development of tolerance to the compound with repeated administration.

| Day 1: | | Day 2: | |
|---|---|---|---|
| | | a.m.: | PD 198306 |
| | | | Water |
| | | | Vehicle |
| p.m.: | **BL** | p:m.: | **BL** |
| | PD 198306 | | PD 198306 |
| | Pregabalin | | Pregabalin |
| | Vehicle | | Vehicle |
| | **Test** | | **Test** |

### RESULTS

A single administration of pregabalin (30mg/kg, p.o.) significantly blocked streptozocin-induced static allodynia 1h after administration. In contrast, a single administration of PD 198306 (30mg/kg, p.o) had no effect on streptozocin-induced static allodynia 1h after administration (see below). However, after the compound had been administered twice more on the following day, it significantly blocked streptozocin-induced static allodynia 1h after the third administration. The effects had disappeared by the following day (see FIG. 1).

### Example 2

### MATERIALS AND METHODS

### Animals

Male Sprague Dawley rats (250-300g), obtained from Charles River, Margate, U.K.) were housed in groups of 3-6. All animals were kept under a 12h light/dark cycle (lights on at 07h 00min) with food and water *ad libitum*. All experiments were carried out by an observer blind to drug treatments.

Diabetes was induced in rats by a single i.p. injection of streptozocin (50mg/kg) as described previously (Courteix et al., 1993).

### Development of Chronic Constriction Injury in the rat

Animals were anaesthetised with 2% isoflurane 1:4 O₂/N₂O mixture maintained during surgery via a nose cone. The sciatic nerve was ligated as previously described by Bennett and Xie, 1988. Animals were placed on a homeothermic blanket for the duration of the procedure. After surgical preparation the common sciatic nerve was exposed at the middle of the thigh by blunt dissection through biceps femoris. Proximal to the sciatic trifurcation, about 7mm of nerve was freed of adhering tissue and 4 ligatures (4-0 silk) were tied loosely around it with about 1mm spacing. The incision was closed in layers and the wound treated with topical antibiotics.

### Intrathecal injections

PD 198306 and pregabalin were administered intrathecally in a volume of 10 µl using a 100 µl Hamilton syringe by exposing the spine of the rats under brief isoflurane anaesthesia. Injections were made into the intrathecal space between lumbar region 5-6 with a 10 mm long 27 gauge needle. Penetrations were judged successful if there was a tail flick response. The wound was sealed with an autoclip and rats appeared fully awake within 2-3 min following injection.

### Evaluation of static allodynia

Mechanical hypersensitivity was measured using Semmes-Weinstein von Frey hairs (Stoelting, Illinois, U.S.A.). Animals were placed into wire mesh bottom cages allowing access to the underside of their paws. Animals were habituated to this environment prior to the start of the experiment. Mechanical hypersensitivity was tested by touching the plantar surface of the animals right hind paw with von Frey hairs in ascending order of force ( 0.7, 1.2, 1.5, 2, 3.6, 5.5, 8.5, 11.8, 15.1 and 29g) for up to 6sec. Once a withdrawal response was established, the paw was re-tested, starting with the next descending von Frey hair until no response occurred. The highest force of 29g lifted the paw as well as eliciting a response, thus represented the cut off point. The lowest amount of force required to elicit a response was recorded as the paw withdrawal threshold (PWT) in grams.

### Experimental protocol

Static allodynia was assessed with von Frey hairs, before (baseline, BL) and 0.5h, 1h and 2h after intrathecal or intraplantar administration of PD 198306 (1-30µg, i.t.), vehicle (cremophor:ethanol:water, 1:1:8) or pregabalin (10µg, i.t). For oral administration experiments, static allodynia was assessed with von Frey hairs, before (baseline, BL) and 1h after oral administration of PD 198306 (3-30mg/kg, p.o.), vehicle (cremophor:ethanol:water, 1:1:8) or pregabalin (30mg/kg, p.o.). Animals were administered again the same compounds on the following day, both in the morning and the afternoon. Static allodynia was assessed before and 1h after the morning administration. In the afternoon static allodynia was assessed before, 1h, 2h and 3h after administration for streptozocin treated animals. CCI animals were assessed before, 1h and 2h after administration

### Drugs used

PD 198306 and pregabalin were synthesised at Parke-Davis (Ann Arbor, Ml, USA). PD 198306 was suspended in cremophor:ethanol:water (1:1:8) vehicle. Pregabalin was dissolved in water. Both compounds were administered orally, intrathecally or intraplantar in volumes of 1ml/kg, 10µl and 100µl respectively. Streptozocin (Aldrich, UK) was dissolved in 0.9% w/v NaCl and administered intraperitoneally in a volume of 1ml/kg.

### Statistics

Data were analysed using a Kruskall-Wallis ANOVA for non-parametric results, followed when significant by Mann-Whitney's t test *vs* vehicle group.

### RESULTS

### 1. Effects of PD 198306 on static allodynia, following systemic administration

### 1.1. Effect of PD198306 on streptozocin-induced static allodynia

A single administration of pregabalin (30mg/kg, p.o.) significantly blocked streptozocin-induced static allodynia 1h after administration. In contrast, a single administration of PD 198306 (3-30mg/kg, p.o) had no effect on streptozocin-induced static allodynia 1h after administration (FIG. 2). However, after the compound had been administered twice more on the following day, PD 198306 (30mg/kg) significantly blocked streptozocin-induced static allodynia for 2h after the third administration (FIG. 2).

### 1.2. Effect of PD198306 on CCI-induced static allodynia

A single administration of pregabalin (30mg/kg, p.o.) significantly blocked CCI-induced static allodynia 1h after administration. In contrast, neither a single or multiple administration of PD 198306 (3-30mg/kg, p.o) had any effect on CCI-induced static allodynia (FIG. 3).

### 2. Effects of PD 198306 on static allodynia, following intrathecal administration

Intrathecally administered PD198306 (1-30µg) dose-dependently blocked the maintenance of static allodynia in both streptozocin (FIG. 4) and CCI animals (FIG. 5) with respective MEDs of 3 and 10 µg. This antiallodynic effect lasted for 1h.

### 3. Effects of PD 198306 on static allodynia, following intraplantar administration

An intrathecal administration of PD 198306 (30µg) significantly blocked static allodynia in both neuropathic pain models (FIGS. 6,7). In contrast, a single administration of PD 198306 at a dose 100-fold higher (3mg/100µl) directly into the paw had no effect on streptozocin (FIG. 6) or CCI-induced static allodynia (FIG. 7).

### REFERENCES

Bennett GJ, Xie Y-K. A peripheral mononeuropathy in rat that produces disorders of pain sensation like those seen in man. Pain 1988;33:87-107.
Courteix C, Eschalier A and Lavarenne J. Streptozocin -induced rats: behavioural evidence for a model of chronic pain. Pain 1993;53:81-8

### Example 3

### Effect of other MEK inhibitors in a neuropathic pain model in the rat

### SUMMARY

The effect of several MEK inhibitors, with different binding affinities, has been investigated in the CCI model of neuropathic pain in the rat, by assessing static allodynia with von Frey hairs. Intrathecal administration of PD219622 or PD297447 (30µg) had no significant effect on allodynia. This lack of effect may reflect the low affinity or solubility of the compounds. However, intrathecal administration of PD 254552 or PD 184352 (30µg), which posses higher binding affinities, blocked the maintenance of static allodynia in CCI animals. The antiallodynic effect was only evident for 30min post-injection and thus, shorter than the one observed for pregabalin (100µg). The magnitude of the effect was similar for 30µg of PD 184352 and 100µg of pregabalin. From this study it is concluded that MEK inhibitors exert an antiallodynic effect in CCI-induced neuropathic rats when administered intrathecally, and that the antiallodynic effect correlates with the affinity of the compounds.

The animals and methods for developing chronic constriction injury in the rat, injecting test compounds, and evaluation of static allodynia were according to Example 2 above. PD219622, PD297447, PD 184352, PD 254552 and pregabalin were administered intrathecally at doses of 30µg for all PD compounds and 100µg for pregabalin. Static allodynia was assessed with von Frey hairs, before (baseline, BL) and 0.5h, 1h and 2h after intrathecal administration of the compounds

### Drugs used

PD297447, PD219622, PD 254552, PD 184352 (CI-1040), and pregabalin were synthesised at Parke-Davis (Ann Arbor, Ml, USA). PD297447, PD219622, PD 254552 and PD 184352 were suspended in cremophor:ethanol:water (1:1:8) vehicle. Pregabalin was dissolved in water. All compounds were administered intrathecally in a 10µl volume.

### Statistics

Data were analysed using a Kruskall-Wallis ANOVA for non-parametric results, followed when significant by Mann-Whitney's t test *vs* vehicle group.

### RESULTS

Intrathecally administered PD297447 or PD219622 (30µg) had no significant effect on allodynia. This lack of effect may reflect the low affinity of the compounds (965nM and 100nM respectively). However, intrathecal administration of PD 184352 or PD 254552 (30µg) blocked the maintenance of static allodynia in CCI animals (see FIG. 8). These compounds possess higher affinity (2 and 5 nM respectively). The antiallodynic effect was only evident for 30min post-injection and thus, shorter than the one observed for pregabalin (100µg). The magnitude of the effect was similar for 30µg of PD 184352 and 100µg of pregabalin.

The results indicate that MEK inhibitors exert an antiallodynic effect in CCI-induced neuropathic rats when administered intrathecally, and that the antiallodynic effect correlates with the affinity of the compounds.

### CHEMICAL EXAMPLES

### Example 1

### Preparation of 2-(2-Chloro-4-iodo-phenylamino)-N-cyclopropylmethoxy-3,4-difluoro-benzenesulfonamide (PD 0297447)

### N-cyclopropylmethoxy-2,3,4-trifluoro-benzenesulfonamide.

To a stirring suspension comprised of *O*-cyclopropylmethyl-hydroxylamine hydrochloride (5.40 g, 0.0437 mol) in dichloromethane (20 ml) at ambient temperature under a nitrogen atmosphere was added diisopropylethylamine (10.8 ml, 0.062 mol). A solution comprised of 2,3,4-trifluorobenzenesulfonyl chloride (Oakwood Products, Inc., 1.00 g, 4.34 x 10⁻³ mol) in dichloromethane (120 ml) was added dropwise to the reaction vessel containing the stirring suspension over a 12 minute period. The reaction mixture was stirred for another 12 minutes and was quenched with 10 % aqueous hydrochloric acid (140 ml). The biphasic mixture was stirred vigorously for 16 hours. The layers were separated and the organic phase was dried (MgSO₄) and concentrated to 6 ml volume. The concentrated solution was administered to a flash silica column (Biotage, 90 g of silica gel). Elution with dichloromethane afforded 0.8283 g of a white amorphous solid; 68 % yield; ¹H-NMR (400 MHz; CDCI₃ signal offset to δ 7.03; values reported are uncorrected) δ 7.50 (m, 1H), 7.10 (s, 1H), 6.95 (m, 1H), 3.59 (d, 2H, J=7.2 Hz), 0.80 (m, 1H), 0.31 (m, 2H), 0.02 (m, 2H); ¹⁹F-NMR (376 MHz; CDCI₃) δ-122.65 (m, 1F), -129.37 (m, 1F), -156.20 (m, 1F); MS (APCI-) 280 (M-1, 100), 210 (55), 195 (45).

### 2-(2-Chloro-4-iodo-phenylamino)-N-cyclopropylmethoxy-3,4-difluorobenzenesulfonamide (PD 0297447).

To a stirring solution comprised of 2-chloro-4-iodoaniline in tetrahydrofuran (10 ml) at -78 °C under a nitrogen atmosphere was added a 1.0 M tetrahydrofuran solution of lithium *bis*trimethylsilylamide (6.2 ml, 6.2 x 10⁻³ mol) to form a green suspension. The suspension was stirred for five minutes before a stirring suspension comprised of lithiated *N*-cyclopropylmethoxy-2,3,4-trifluoro-benzenesulfonamide (prepared by adding 3.0 ml of the 1.0 M lithium *bis*trimethylsilylamide solution to a stirring solution comprised of *N*-cyclopropylmethoxy-2,3,4-trifluoro-benzenesulfonamide in 10 ml of tetrahydrofuran at -78 °C under nitrogen gas) was added via canula. The cold bath was removed and the stirring suspension was stirred for one hour. The reaction mixture was quenched with 10 % aqueous hydrochloric acid (50 ml) and the biphasic mixture was concentrated *in vacuo* to an aqueous suspension that was extracted with diethyl ether (200 ml). The organic phase was dried (MgSO₄) an d was concentrated *in vacuo* to afford a tan oil. The crude product was purified by flash chromatography. Elution with a gradient (hexanes-ethyl acetate 99:1 → (2 min) 9:1 → (25 min) 3:1 afforded 1.10 g of a white amorphous foam; 73 % yield; ¹H-NMR (400 MHz; DMSO) δ 7.69 (m, 1H), 7.59 (d, 1H, J=1.9 Hz), 7.34 (dd, 1H, J=8.7, 1.9 Hz), 7.27 (s, 1H), 7.00 (s, 1H), 6.95 (m, 1H), 6.43 (dd, 1H, J=8.7, 5.8 Hz), 3.52 (d, 2H, J=7.5 Hz), 0.74 (m, 1H), 0.34 (m, 2H), 0.02 (m, 2H); ¹⁹F-NMR (376 MHz; CDCl₃) δ-124.76 (m, 1F), -136.69 (d, 1F, J=18.3 Hz); MS (APCI+) 515 (M+1, 100); (APCI-) 513 (M-1, 50), 443 (73), 428 (100); IR (KBr) 1491 cm⁻¹; Anal. Calcd/found for C₁₆H₁₄ClF₂IN₂O₃S C, 37.34/36.54; H, 2.74/2.71; N, 5.44/5.15; F, 7.38/7.57.

The APK IC₅₀ for PD 0297447 is 0.965 µM.

### EXAMPLE 1A

### Preparation of 2,4-bis-(2-chloro-4-iodo-phenylamino)-3-fluoro-5-nitrobenzoic acid

### Step a: Preparation of 5-nitro-2,3,4-trifluorobenzoic acid

To gently stirring concentrated sulfuric acid (50 ml) was added fuming nitric acid (3.4 ml, 0.076 mol). Solid 2,3,4-trifluorobenzoic acid (10.00 g, 0.05565 mol) was added directly in increments. After stirring 45 minutes, the reaction mixture had become an orange homogeneous solution which was then poured over chilled water (400 ml). The resulting aqueous suspension was extracted with diethyl ether (3 x 200 ml). The combined extracts were dried with anhydrous magnesium sulfate and concentrated *in vacuo* to yield 12.30 g of a dull, light-yellow solid. Recrystallization from chloroform (50 ml) afforded 9.54 g of the pale yellow microcrystalline product; 78 % yield; m.p. ; ¹H-NMR (400 MHz; DMSO) δ 14.29 (broad s, 1H), 8.43-8.38 (m, 1H); ¹³C-NMR (100 MHz; DMSO) δ 162.41, 154.24 (dd, J_{C-F}=270.1, 10.7 Hz), 148.35 (dd, J_{C-F}=267.0. 9.2 Hz), 141.23 (dt, J_{C-F}=253.4 Hz), 133.95, 123.30 (d, J_{C-} _{F}=2.2 Hz), 116.92 (dd, J_{C-F}=18.2, 3.8 Hz); ¹⁹F-NMR (376 MHz; DMSO) δ-120.50 to -120.63 (m), -131.133 to -131.27 (m), -153.63 to -153.74 (m).

### Step b: Preparation of 2,4-bis-(2-chloro-4-iodo-phenylamino)-3-fluoro-5-nitrobenzoic acid

To a stirring solution comprised of 2-chloro-4-iodoaniline (Lancaster, 98 %, 12.33 g, 0.04864 mol) in tetrahydrofuran (20 ml) at -78 °C under nitrogen was added a 2.0 M lithium diisopropylamide solution in tetrahydrofuranheptane-ethylbenzene (Aldrich, 35 ml, 0.070 mol) with a syringe. The addition formed a thick suspension. After five minutes of stirring, a solution comprised of 5-nitro-2,3,4-trifluorobenzoic acid (5.00 g, 0.0226 mol) in tetrahydrofuran (30 ml) was added with a syringe to give a dark reaction mixture. The cold bath was removed and the reaction mixture stirred for 20 minutes. The cool reaction mixture was poured into ether (600 ml) containing an excess of hydrogen chloride. The red solution instantly turned to a yellow suspension as a precipitate formed. This precipitate was removed by vacuum filtration. The filtrate was concentrated *in vacuo* to a red powder (10.5 g). The red powder was triturated with boiling chloroform (800 ml). The triturated solids were collected by vacuum filtration to give an orange powder (2.42 g). The mother liquor from the trituration was concentrated *in vacuo* to give a red-orange solid (ca. 10 g undried). This solid was loaded onto a flash silica column. Elution with dichloromethane removed some impurities. Continuing elution with 1 % methanol in dichloromethane afforede ca. 4 g of a red solid. This red solid was dissolved in hot absolute ethanol (100 ml). The solution was boiled down to 50 ml before dilution to 300 ml with hexanes. This solution was boiled to 150 ml and rediluted to 300 ml with hexanes to produce slight turbidity. The mixture was cooled in the refrigerator for three days, affording a yellow precipitate. The precipitate was collected by vacuum filtration and was dried with suction to afford 0.15 g of a yellow solid; 1 % yield; ¹H-NMR (400 MHz; DMSO) δ 8.94 (s, 1H), 8.55 (s, 1H), 7.79 (d, 2H, J=2.0 Hz), 7.61-7.57 (m, 2H), 6.90 (dd, 1H, J=8.5, 3.9 Hz), 6.84 (dd, 1H, J=8.3, 6.6 Hz); ¹⁹F-NMR (376 MHz; DMSO) δ -122.62 (s); MS (APCI+) 692 (6), 691 (8), 690 (31), 689 (10), 688 (55), 171 (47), 130 (100); (APCI-) 691 (4), 690 (12), 689 (14), 688 (70), 687 (32), 686 (100), 506 (50), 453 (97); IR (KBr) 1523 cm⁻¹; Anal. calcd/found for: C₁₉H₁₀Cl₂Fl₂N₃O₄ C, 33.17/33.32; H, 1.47/1.73; N, 6.11/5.73; Cl, 10.31/10.04; F, 2.76/3.70; I, 36.89/34.32.

The APK IC₅₀ for 2,4-bis-(2-chloro-4-iodo-phenylamino)-3-fluoro-5-nitrobenzoic acid is 29.6 nM.

### EXAMPLE 1B

### 4-Fluoro-2-(4-methanesulfanyl-phenylamino)-benzoic acid (1).

To a solution of 4-(methylmercapto)aniline (3.1622 g, 0.02 mole) in THF at ⁻78°C, a solution of LDA in THF (2M, 30 ml, 0.06 mole) was added and the reaction mixture stirred for 30 minutes at ⁻78°C (Scheme 1E). Solid 2,4-diflluoro benzoic acid (3.1622 g, 0.02 mole) was added and the reaction stirred for 16 hours while it wormed up to room temperature. The reaction mixture was pour in to ether saturated with HCl gas. HCl gas was bubbled into until precipitation of salts ceased. The precipitated salts were separated by filtration and discarded. The ether layer was concentrated to give **1** as a white solid. Yield 5.63 g (100%); mp 173-179 °C (DEC); ¹H-NMR (400 MHz; CDCl₃) δ 9.39 (s, 1H), 8.04 (dd, 1H, J=9.2, 6.8 Hz), 7.32-7.17 (AB quartet, 4H), 6.74 (dd, 1H, J=12.1, 2.4 Hz), 6.46-6.41 (m, 1H), 2.51 (s, 3H); ¹³C-NMR (100 MHz; CDCl₃) δ 172.79, 167.57 (d, J_{C-F}=253.4 Hz), 151.55 (d, J_{C-F}=12.2 Hz), 136.83, 135.40 (d, J_{C-F}=12.2 Hz), 134.72, 128.31, 124.60, 106.51, 105.12 (d, J_{C-F}=22.9 Hz), 99.79 (d, J_{C-F}=26.7 Hz), 16.51; ¹⁹F-NMR (376 MHz; CDCl₃) δ -101.39 to -101.46 (m); MS (APCI+) 278 (M+1, 100); IR (KBr) 3319, 1664, 1589, 1258 cm⁻¹; Anal. calcd/found for: C₁₄H₁₂FNO₂S C, 60.64/60.99; H, 4.36/4.63; N, 5.05/4.80; S, 11.56/10.97.

### EXAMPLE 2B

### 4-Fluoro-2-(4-methanesulfinyl-phenylamino)-benzoic acid (2).

A mixture of **1** (Scheme 1B) (0.286 g, 0.001031 mole) and oxaziridine (0.235 g, 0.0009 mole) in CHCl₃ (30 ml) at room temperature for 2 hours. The solvent was removed and the resulting brown oil chromatographed on silica column. Elution with CH₂Cl₂ removed fast moving byproduct. Further elution with CH₂Cl₂:CH₃OH (9.5:05), R_{f} = 0.27, gave pure **2** as a light brown solid. Yield 132.8 mg (50%); mp 191-192 °C; ¹H-NMR (400 MHz; CDCl₃) δ 9.77 (s, 1H), 8.08 (dd, 1H, J=8.9, 6.7 Hz), 7.70-7.39 (AB quartet, 4H), 6.98 (dd, 1H, J=11.6, 2.4 Hz), 6.57-6.52 (m, 1H), 2.80 (s, 3H); ¹³C-NMR (100 MHz; CDCl₃) δ 170.76, 167.18 (d, J_{C-F}=253.3 Hz), 149.33 (d, J_{C-F}=12.2 Hz), 143.02, 139.50, 135.37 (d, J_{C-F}=12.2 Hz), 125.47,122.32, 108.22,106.35 (d, J_{C-F}=22.8 Hz), 100.69, (d, J_{C-F}=25.9 Hz), 43.75; MS (APCI+) 294 (M+1, 100); IR (KBr) 1673, 1592, 1228 cm⁻¹; Anal. calcd/found for: C₁₄H₁₂FNO₃S C, 57.33/57.48; H, 4.12/4.27; N, 4.78/4.67.

### EXAMPLE 3B

### 4-Fluoro-2-(4-methanesulfonyl-phenylamino)-benzoic acid (3).

A solution of **1** (Scheme 1B) (0.4458 g, 0.00152 mole) and tetrabutylammonium oxon (1.1 g, 0.0030 mole) in CH₂Cl₂ (20 ml) was stirred at room temperature for 16 hours. TLC showed the presence of starting material; so additional 1.1 g (0.0030 mole) of the tetrabutylammonium oxon was added and reaction mixture stirred for 16 more hours. The reaction mixture was loaded on to a silica column and eluted with CH₂Cl₂:CH₃OH (9.75:0.25) and the fast moving fraction collected and concentrated to give **3** as a white solid. Yield, 0.3856 g (82%); mp 200-202 °C; ¹H-NMR (400 MHz; CDCl₃) δ 9.78 (s, 1H), 8.13 (dd, 1H, J=8.9, 6.5 Hz), 7.94-7.38 (AB quartet, 4H), 7.10 (dd, 1H, J=11.3, 2.4 Hz), 6.66-6.61 (m, 1H), 3.09 (s, 3H); ¹³C-NMR (100 MHz; CDCl₃) δ 171.52, 167.28 (d, J_{C-F}=254.9 Hz), 148.32, 145.21, 135.59 (d, J_{C-F}=11.5 Hz), 134.50, 129.39, 120.62, 108.74, 107.46 (d, J_{C-F}=22.8 Hz), 101.61 (d, J_{C-} _{F}=26.7 Hz), 44.78; ¹⁹F-NMR (376 MHz; CDCl₃) δ-100.29 to -100.45 (m); MS (APCI+) 310 (M+1, 100); (APCI-) 308 (M-1, 100); Anal. calcd/found for: C₁₄H₁₂FNO₄S 0.75 H₂O C, 52.08/52.36; H, 4.22/3.88; N,4.34/4.26.

### EXAMPLE 4B

### 2-methyl-4-trimethylsilanylethynyl-aniline (5)

To a solution of 4-iodo-2-methyl-aniline (2.33g, 10 mmol), bis(triphenylphosphine)palladium(II)chloride (1.4g, 0.2 mmol), Cul (0.19 g, 0.1 mmol) in Et₃N (40 ml) at ice-bath temperature, (trimethylsilyl)acetylene (1.18 g, 12 mmol) was added dropwise (Scheme 2B). After an hour stirring, the ice-bath was removed and the reaction mixture heated at 40°C (oil-bath temperature) for one hour; cooled to room temperature and the solvent removed. The residue was partitioned between H₂O and Et₂O. The Et₂O layer was separated, dried (MgSO₄) and concentrated to give an oil. The oil was purified by silica column, eluting with CH₂Cl₂. The fraction with R_{f} = 0.37 was collected and concentrated to give 2-methyl-4-trimethylsilanylethynylaniline as a dark brown oil.
Yield 1.50 g (83%).

### EXAMPLE 5B

### 4-Fluoro-2-(2-methyl-4-trimethylsilanylethynyl-phenylamino)-benzoic acid (6)

Continuing after Example 4B, to a solution of 2-methyl-4-trimethylsilanylethynyl aniline (1.50 g, 0.008 mole) in THF (10 ml) at ⁻78°C, LDA (2 M in THF, 6 ml, 0.012 mole) was added and the mixture was stirred at ⁻78°C for 30 minutes. Solid 2,4-difluoro-benzoic acid (0.633 g, 0.004 mole) was added and the stirred for 16 hours while it warmed up to room temperature. The solvents were removed and water (30 ml) and Et₂O (50 ml) added to the oil residue. The mixture was stirred vigorously and the Et₂O layer separated, dried (MgSO₄) and concentrated to give a brown solid. The solid was purified on silica column, eluted with CH₂Cl₂. The fraction with R_{f} = 0.37 was collected and concentrated to give a light brown solid. The solid was added to pentane; some insoluble brown particulate was separated by filtration and discarded. The pentane layer was concentrated to give **6** as a light yellow solid. Yield 0.65 g (47%); mp 170-171°C; ¹H-NMR (400 MHz; CDCl₃) δ 9.33 (s, 1H), 8.05 (dd, 1H, J=8.9, 6.8 Hz), 7.43 (d, 1H, J=1.2 Hz), 7.35 (dd, 1H, J=8.2, 1.7 Hz), 7.25 (d, 1H, J=8.2 Hz), 6.53 (dd, 1H, J=11.8, 2.4 Hz), 6.47-6.42 (m, 1H), 2.25 (s, 3H), 0.26 (s, 9H); ¹³C-NMR (100 MHz; CDCl₃) δ 172.86, 167.61 (d, J_{C-} _{F}=253.3), 151.24 (d, J_{C-F}=12.3 Hz), 138.28, 135.38 (d, J_{C-F}=11.4 Hz), 134.85, 132.82, 130.63, 123.81, 119.91, 106.63, 105.23 (d, J_{C-F}=22.8 Hz), 104.77, 99.98 (d, J_{C-F}=26.7 Hz), 94.05, 17.78, 0.00; MS (APCI+) 342 (M+1, 100); IR (KBr) 2151, 1661, 1249 cm⁻¹; Anal. calcd/found for: C₁₉H₂₀FNO₂Si C, 66.83/67.02; H, 5.90/6.00; N, 4.10/4.09; F, 5.56/5.45.

### EXAMPLE 6B

### 4-Fluoro-2-(2-methyl-4-ethynyl-phenylamino)-benzoic acid (7).

To a solution of **6** in CH₃OH (30 ml), aqueous 1N KOH (10 ml) was added. After stirring at room temperature for 16 hours, the CH₃OH was removed and the aqueous layer was acidified with 6N HCI (Scheme 2B). The resulting white precipitation was extracted in to Et₂O, the Et₂O layer was dried (MgSO₄) and concentrated to give **7** as tan colored solid. Yield 0.4274 g (91%); mp 177-178 °C; ¹H-NMR (400 MHz; CDCl₃) δ 9.35 (s, 1H), 8.08-8.04 (m, 1H), 7.44 (s, 1H), 7.38-7.25 (m, 2H), 6.57 (d, 1H, J=11.8 Hz), 6.48-6.44 (m, 1H), 3.08 (s, 1H), 2.27 (s, 3H); ¹³C-NMR (100 MHz; CDCl₃) δ 172.84, 167.61 (d, J_{C-F}=253.3), 151.15 (d, J_{C-F}=12.3 Hz), 138.63, 135.40 (d, J_{C-F}=12.3 Hz), 135.00, 132.87, 130.81, 123.76, 118.79, 106.75, 105.33 (d, J_{C-F}=22.8 Hz), 100.03 (d, J_{C-F}=26.0 Hz), 83.37, 17.83, 0.00; ¹⁹F-NMR (376 MHz; CDCl₃) δ -101.24 to -101.31 (m); MS (APCI+) 270 (M+1, 100); IR (KBr) 3315, 1672, 1594, 1253 cm⁻¹; Anal. calcd/found for: C₁₆H₁₂FNO₂ C, 71.37/71.08; H, 4.49/4.82; N, 5.20/5.09.

### EXAMPLE 7B

### 1-(4-nitro-phenyl)-1H-pyrrole (9a)

To a gently refluxing mixture of 4-nitroaniline (6.906 g, 0.05 mole), and sodium acetate (23 g, 0.28 mole) in acetic acid (100 ml) was added 2,5-dimethoxytetrahydrofuran (7.26 g, 7.12 ml, 0.055 mole) dropwise (Scheme 3B). After refluxing for 3 hours, the reaction mixture was poured on to crushed ice (∼250 ml), basified with 10 % sodium hydroxide (250 ml) and extracted with CH₂Cl₂. The CH₂Cl₂ layer was dried (K₂CO₃) to afford the product as a dark brown oil. Yield 9.40 g (100 %).

### EXAMPLE 8B

### 1-(4-nitro-phenyl)-1H-pyrazole (9b)

A mixture of pyrrazole (6.808 g, 0.1 mole) tetrabutylammonium bromide (3.22 g, 0.01 mole) and KOH (11.22 g, 0.2 mole) were ground together and sonicated for 16 hours. To this 1-fluoro-4-nitrobenzene (15.521 g, 11.67 ml, 0.11 mole) was added and the mixture sonicated for 24 hours. The reaction mixture was extracted with CH₂Cl₂. The CH₂Cl₂ layer was dried (MgSO₄) and concentrated to give dark brown solid. This was purified by silica column chromatography. Elution with CH₂Cl₂ (R_{f} = 0.44) gave the product as a light brown solid. Yield 8.80 g (47 %); mp 171-172 °C; Anal. calcd/found for: C₉H₇N₃O₂ C, 57,14/56.52; H, 3.73/3.62; N, 22.21/21.95.

### EXAMPLE 9B

### 3,5-dimethyl-1-(4-nitro-phenyl)-1H-pyrazole (9c)

To a solution of 4-nitro-phenyl-hydrazine (15.3 g, 0.1 mole) and 2,4-pentanedione (10.01 g, 10.27 ml, 0.1 mole) in EtOH (200 ml) were added 5 drops of concentrated HCI. The mixture was refluxed for 15 minutes; and the solvent removed to give a gummy product. This was purified by silica column chromatography. Elution with CH₂Cl₂ gave the desired product (R_{f} = 0.10) as a brown solid. Yield 7.22 g (33 %).

### EXAMPLE 10B

### 4-Pyrrol-1-yl-phenylamine (10a)

Catalytic reduction (H₂/RaNi (5 g) /THF) of 1-(4-nitro-phenyl)- 1H-pyrrole (9.69 g, 0.05149 mole) at 51 psi gave crude'product as an oil (Scheme 3B). The product was purified by silica column chromatography. Elution with CH₂Cl₂
(R_{f} = 0.13) gave the pure product as white solid. Yield 8.06 g (99 %); mp 80-81 °C.

### EXAMPLE 11B

In a manner similar to the preparation of 4-pyrrol-1-yl-phenylamine, the following were prepared:
4-1H-Pyrazol-1-yl-phenylamine (10b). Dark brown oil, yield 6.26 g (100 %).
Benzenamine, 4-(3,5-dimethyl-1H-pyrazzol-1-yl) (10c). Dark brown oil.
Yield 6.45 g (100 %).

### EXAMPLE 12B

### 4-Fluoro-2-(4-pyrrol-1-yl-phenylamino)-benzoic acid (11a)

To a solution of 4-pyrrol-1-yl-phenylamine (3.16 g, 0.02 mole) in THF (30 ml) at ⁻78°C, a solution of LDA (2M, 15 ml, 0.03 mole) was added and the mixture stirred for 30 minutes. Solid 2,4-difluorobenzoic acid was added and the reaction mixture stirred for 16 hours as it warmed up to room temperature. The solvent was removed and ether (100 ml) added to the dark oily residue. This was stirred vigorously and the insoluble gummy precipitate separated by filtration. The gummy residue was dissolved in H₂O, acidified to pH 1 with 10% HCI, and extracted with Et₂O. The Et₂O layer was dried (MgSO₄) and concentrated to give the target compound as a brown solid. Yield 2.74 g (93 %); mp 223-225 °C (DEC); ¹⁹F-NMR (376 MHz; CDCI₃) δ -101.44 (s); MS (APCI+) 297 (M+1, 100); IR (KBr) 1658, 1526, 1254 cm⁻¹.

In a manner similar to the preparation of 4-Fluoro-2-(4-pyrrol-1-yl-phenylamino)-benzoic acid, the following were prepared:
4-Fluoro-2-(4-pyrazol-1-yl-phenylamino)-benzoic acid (11b). Light brown solid, mp 212-213 °C.
2-[4-(3,5-Dimethyl-pyrazol-1-yl)-phenylamino]- 4-Fluoro benzoic acid (11c). Tan powder, mp 198 -200 °C.

### EXAMPLE 1C

### Preparation of 2-(2-chloro-4-iodo-phenylamino)-5-dimethylsulfamoyl-3,4-difluoro-benzoic acid methyl ester (APK IC₅₀=222 nM)

### Step a: Preparation of 1-dimethylsulfamoyl-2,3,4-trifluorobenzene

To a gently stirring solution comprised of 2,3,4-trifluorobenzenesulfonyl chloride (5.70 g, 0.0247 mol) in 1,2-dichloroethane (200 ml) was introduced by bubbling gaseous anhydrous dimethylamine. The mixture became cloudy after several minutes and was subsequently washed with water (200 ml), 6 N aqueous hydrochloric acid (200 ml), brine (200 ml), was dried over anhydrous magnesium sulfate, and was concentrated *in vacuo* to obtain a yellow oil. The crude product was purified by flash chromatography. Elution with dichloromethane afforded 3.40 g of a white solid; 58 % yield; ¹H-NMR (400 MHz; CDCl₃) δ 7.63-7.56 (m, 1H), 7.12-7.04 (m, 1H), 2.812 (s, 3H), 2.807 (s, 3H); ¹⁹F-NMR (376 MHz; CDCl₃) δ -124.91 to-125.03 (m), -127.98 to-128.03 (m), -156.41 to -156.53.

### Step b: Preparation of 5-dimethylsulfamoyl-2,3,4-trifluoro-benzoic acid

To a cold (-78 °C) stirring solution comprised of 1-dimethylsulfamoyl-2,3,4-trifluorobenzene in anhydrous tetrahydrofuran (60 ml) under a nitrogen atmosphere was added a commercially available lithium diisopropylamide solution (Aldrich, 2.0 M in tetrahydrofuran/heptane/ethylbenzene, 7.5 ml, 0.0150 mol). After stirring for about ten minutes, the purple solution was transferred via canula to a cold, stirring, saturated carbon dioxide in diethyl ether solution (200 ml). The reaction mixture took on a dull burgundy color. The cold bath was removed and the reaction mixture warmed to ambient temperature over one hour. The mixture was then carefully quenched with 10 % aqueous hydrochloric acid (200 ml). The layers were separated. The organic phase was extracted twice (200, 100 ml portions) with 10 % (wt.) aqueous sodium hydroxide. The combined aqueous alkaline extracts were treated with concentrated aqueous hydrochloric acid (100 ml) to pH 0. A white precipitate formed. The suspension was allowed to cool, then was extracted with diethyl ether (600 ml). The organic extract was dried over anhydrous magnesium sulfate and was concentrated *in vacuo* to afford 2.70 g of an off-white solid; 67.5 % yield; mp 225-228 °C; ¹H-NMR (400 MHz; DMSO) δ 14.08 (broad s, 1H), 8.02-7.97 (m, 1H), 2.75 (s, 3H), 2.74 (s, 3H) ¹⁹F-NMR (376 MHz; DMSO) δ-122.50 to -122.63 (m), -122.95 to -123.08 (m), -154.49 to -154.61 (m); MS (APCI+) 284 (M+1, 22), 238 (100); (APCI-) 282 (M-1, 85), 259 (94), 238 (46), 216 (91), 195 (100); IR (KBr) 1702 cm⁻¹; Anal. calcd/found for: C₉H₈F₃NO₄S C, 38.17/38.40; H, 2.85/2.90; N, 4.95/4.80; F, 20.12/19.75; S, 11.32/11.12.

### Step c: Preparation of 5-dimethylsulfamoyl-2,3,4-trifluoro-benzoic acid methyl ester.

The solid 5-dimethylsulfamoyl-2,3,4-trifluoro-benzoic acid (1.47 g, 0.00519 mol) and *p*-toluenesulfonic acid catalyst (17.1 mg) were dissolved in methanol (125 ml). The stirring mixture was brought to reflux under a nitrogen atmosphere for 51 hours. The reaction mixture was concentrated *in vacuo* to give a solid. The product was partitioned between diethyl ether (200 ml) and saturated aqueous potassium carbonate (75 ml). The layers were separated and the organic phase was washed with water (75 ml), brine (75 ml), was dried over anhydrous potassium carbonate, and was concentrated *in vacuo* to afford 0.15 g of an off-white solid; 10 % yield; ¹H-NMR (400 MHz; CDCl₃) δ 8.23-8.19 (m, 1H), 3.92 (s, 3H), 2.83 (s, 6H); ¹⁹F-NMR (376 MHz; CDCl₃) δ-120.79 to -121.02 (m), -153.69 to -153.80.

### Step d: Preparation of 2-(2-chloro-4-iodo-phenylamino)-5-dimethylsulfamoyl-3,4-difluoro-benzoic acid methyl ester

To a stirring cold (-78 °C) solution comprised of 2-chloro-4-iodoaniline (0.143 g, 5.64x10⁻⁴ mol) in anhydrous tetrahydrofuran (5 ml) under a nitrogen atmosphere was added a commercially available lithium diisopropylamide solution (Aldrich, 2.0 M in tetrahydrofuran/heptane/ethylbenzene, 0.300 ml, 6.0x10⁻⁴ mol). After stirring for 5 minutes, a solution comprised of 5-dimethylsulfamoyl-2,3,4-trifluoro-benzoic acid methyl ester (0.15 g, 5.0x10⁻⁴ mol) in tetrahydrofuran (10 ml) was added via syringe. The cold bath was removed and the reaction mixture was stirred for 2 hours. The reaction mixture was then partitioned between diethyl ether (125 ml) and saturated aqueous sodium bicarbonate (125 ml). The aqueous bicarbonate phase was extracted with an additional portion (125 ml) of diethyl ether. The combined organic phases were dried over anhydrous magnesium sulfate and concentrated *in vacuo* to give a yellow oil. The oil was crystallized from heptane-ethyl acetate to afford 0.060 g of an off-white powder; 23 % yield; mp 154-156 °C; ¹H-NMR (400 MHz; CDCl₃) δ 9.74 (s, 1H), 8.30 (d, 1H, J=7.1 Hz), 7.72 (s, 1H), 7.49 (d, 1H, J=8.3 Hz), 6.73-6.69 (m, 1H), 3.92 (s, 3H), 2.84 (s, 3H), 2.83 (s, 3H); ¹⁹F-NMR (376 MHz; CDCl₃) δ -123.90 (d), -139.55 (d).

### EXAMPLE 2C

### Preparation of 2-(2-chloro-4-iodo-phenylamino)-N-cyclopropylmethoxy-3,4-difluoro-5-sulfamoyl-benzamide (PD 219622)

### Step a: Preparation of 1-bis-(4-methoxybenzyl)sulfamoyl-2,3,4-trifluorobenzene

To a stirring solution comprised of *bis*-4-methoxybenzylamine (2.5 g, 9.7x10⁻³ mol) and diisopropylethylamine (1.7 ml, 9.7x10⁻³ mol) in dichloromethane (50 ml) at 0 °C under nitrogen atmosphere was added liquid 2,3,4-trifluorobenzenesulfonyl chloride (2.26 g, 9.5x10⁻³ mol) directly. The mixture was stirred cold for ten minutes. The ice-water bath was removed and the mixture was stirred for an additional 15 minutes and was then diluted with dichloromethane to 350 ml volume and was washed with saturated aqueous ammonium chloride (200 ml). The organic phase was dried (MgSO₄) and concentrated *in vacuo* to afford 4.99 g of a sticky white solid. The crude product was recrystallized from hexanes-acetone to afford 3.00 g of white needles; 70 % yield; mp 87-90 °C; ¹H-NMR (400 MHz; CDCl₃) δ 7.64-7.58 (m, 1H), 7.04-6.99 (m, 1H), [6.97 (d, 4H, J=8.5 Hz), 6.75 (d, 4H, J=8.8 Hz) AB q], 4.33
(s, 4H), 3.76 (s, 6H); ¹⁹F-NMR (376 MHz; CDCl₃) δ-125.44 to -125.56 (m),-128.61 to -128.72 (m), -156.91 to -157.03 (m); MS (APCI+) 121 (M-330, 100); (APCI-) 330 (M-121, 18), 195 (M-256, 100); IR (KBr) 1612, 1517, 1506, 1465, 1258, 1240, 1156, 1037, 1030 cm⁻¹; Anal. calcd/found for: C₂₂H₂₀F₃NO₄S C, 58,53/57.98; H, 4.47/4.61; N, 3.10/2.85.

### Step b: Preparation of 5-bis-(4-methoxybenzyl)sulfamoyl-2,3,4-trifluorobenzoic acid

To a stirring solution comprised of 1-*bis*-(4-methoxybenzyl)sulfamoyl-2,3,4-trifluorobenzene (2.95 g, 6.5x10⁻³ mol) in tetrahydrofuran (60 ml) at -78 °C was added a solution comprised of 2.0 M lithium diisopropylamide in tetrahydrofuran/heptane/ethylbenzene (Aldrich, 3.35 ml, 6.7x10⁻³ mol). After several minutes of stirring, the dark solution was transferred via canula over five minutes to a stirring solution comprised of carbon dioxide (excess) in diethyl ether at -78 °C. A white precipitate immediately formed. The cold bath was removed and the reaction mixture was stirred at ambient temperature for 18 hours. The reaction mixture was quenched with 200 ml of dilute aqueous hydrochloric acid. The layers were separated and the organic phase was dried (MgSO₄) and concentrated *in vacuo* to give 2.82 g of an off-white solid. Recrystallization from dichloromethane (150 ml) afforded 2.10g of the white powder product; 65 % yield; mp 158-161 °C; ¹H-NMR (400 MHz; DMSO) δ 7.80-7.76 (m, 1H), 7.05-6.74 (AB q, 8H, J=8.6 Hz), 4.33 (s, 4H), 3.66 (s, 6H); ¹⁹F-NMR (376 MHz; DMSO) δ-123.28 to -123.36 (m), -124.12 to -124.21 (m), -155.41 to -155.53 (m); MS (APCI-) 494 (M-1, 47), 216 (89), 195 (100); IR (KBr) 3420, 2954, 2838, 1695, 1613, 1512, 1347, 1238, 1152, 1079 cm⁻¹; Anal. calcd/found for: C₂₃H₂₀F₃NO₆S C, 55.76/55.85; H, 4.07/4.02; N, 2.83/2.71; F, 11.50/11.41; S, 6.47/6.25.

### Step c: Preparation of 5-bis-(4-methoxybenzyl)sulfamoyl-2-(2-chloro-4-iodophenylamino)-3,4-difluorobenzoic acid (PD 215729)

To a stirring solution comprised of 2-chloro-4-iodoaniline (0.53 g, 2.0x10⁻³ mol) in tetrahydrofuran (10 ml) at -78 °C under a nitrogen atmosphere was added a solution comprised of 1.0 M lithium bis(trimethylsilyl)amide in tetrahydrofuran (Aldrich, 4.1 ml, 4.1x10⁻³ mol). Within several minutes the solution became a thick light-green suspension. To this mixture was added a solution comprised of lithium 5-*bis*-(4-methoxybenzyl)sulfamoyl-2,3,4-trifluorobenzoate in tetrahydrofuran, which was prepared by adding 2.0 ml of the Aldrich lithium bis(trimethylsilyl)amide solution (0.0020 mmol) to a solution comprised of 5-*bis*-(4-methoxybenzyl)sulfamoyl-2,3,4-trifluorobenzoic acid (1.00 g, 2.0x10⁻³ mol) in tetrahydrofuran (10 ml) at -78 °C. The reaction mixture was stirred for 15 minutes and was then concentrated *in vacuo* to a crude semisolid. The semisolid was taken up into diethyl ether (250 ml) and was washed with 1 % aqueous hydrochloric acid (150 ml). The ether phase was then washed with neutral water (200ml, pH 4 after wash), a second portion of water (200 ml, pH 6 after wash), and brine (200 ml). The organic phase was then dried (MgSO₄) and was concentrated *in vacuo* to give 1.88 g of a sticky residue which was crystallized from toluene-heptane to afford 1.12 g of an off-white powder; 76 % yield; mp 162-166 °C; ¹H-NMR (400 MHz; DMSO) δ 9.86 (s, 1H), 7.92 (d, 1H, J=6.8 Hz), 7.86 (d, 1H, J=1.7 Hz), 7.60 (dd, 1H, J=8.5, 1.7 Hz), 7.06-7.04/6.78-6.75 (AB q, 8H, J=8.5 Hz), 6.93-6.89 (m, 1H), 4.31 (s, 4H), 3.66 (s, 6H); ¹⁹F-NMR (376 MHz; DMSO) δ-127.22 (d), -141.36 (d); MS (APCI+) 729 (M+1, 1), 256 (50), 121 (100); (APCI-) 727 (M-1, 100); IR (KBr) 1698, 1673, 1513, 1251 cm⁻¹; Anal. calcd/found for: C₂₉H₂₄ClF₂IN₂O₆S C, 47.78/47.93; H, 3.32/3.33; N, 3.84/3.80; Cl, 4.86/4.84; F, 5.21/5.46; I, 17.41/17.16; S, 4.40/4.29.

### Step d: Preparation of 5-bis-(4-methoxybenzyl)sulfamoyl-2-(2-chloro-4-iodophenylamino)-N-cyclopropylmethoxy-3,4-difluorobenzamide (PD 218774)

To a stirring solution comprised of 5-*bis*-(4-methoxybenzyl)sulfamoyl-2-(2-chloro-4-iodophenylamino)-3,4-difluorobenzoic acid (0.935 g, 1.28x10⁻³ mol), cyclopropylmethoxylamine hydrochloride (0.175 g, 1.42x10⁻³ mol), and diisopropylethylamine (0.75 ml, 4.26x10⁻³ mol) in a 1:1 v/v tetrahydrofuran-dichloromethane mixture (50 ml) was added solid PyBOP ([benzotriazolyloxy]tripyrrolidino phosphonium hexafluorophosphate, Advanced ChemTech, 0.76 g, 1.46x10⁻³ mol). The reaction mixture was stirred for one hour, was then evaporated to a crude residue which was purified by flash silica column chromatography. Elution with a gradient (25 % dichloromethane to 75 % dichloromethane in hexanes) afforded 0.63 g of the off-white powder product; 62 % yield; mp 70->300 °C; ¹H-NMR (400 MHz; DMSO) δ 11.92 (s, 1H), 9.35 (s, 1H), 7.60 (s, 1H), 7.50-7.45 (m, 1H), 7.34 (d, 1H, J=8.5 Hz), 6.82-6.54 (AB q, 8H, J=8.3 Hz), 6.59-6.54 (m, 1H), 4.09 (s, 4H), 3.46 (s, 6H), 0.90-0.80 (m, 1H), 0.30-0.25 (m, 2H), 0.03-0.00 (m, 2H); ¹⁹F-NMR (376 MHz; DMSO) δ -129.05 (s), -140.23 (d, J=18.3 Hz); MS (APCI+) 798 (M+1, 70); (APCI-) 796 (M-1, 15), 726 (50), 131 (100); IR (KBr) 1642, 1611, 1584, 1513, 1478 cm⁻¹; Anal. calcd/found for: C₃₃H₃₁ClF₂IN₃O₆S C, 49.67/49.88; H, 3.92/3.95; N, 5.27/5.19.

### Step e: Preparation of 2-(2-chloro-4-iodophenylamino)-N-cyclopropylmethoxy-3,4-difluoro-5-sulfamoyl-benzamide (PD 219622)

A reaction solution comprised of 5-*bis*-(4-methoxybenzyl)sulfamoyl-2-(2-chloro-4-iodophenylamino)-N-cyclopropylmethoxy-3,4-difluorobenzamide (0.1010 g, 1.266x10⁻⁴ mol) in trifluoroacetic acid (4 ml) was stirred at ambient temperature for 24 hours. The mixture was vacuum filtered and the precipitate rinsed with hexanes to afford 28.6 mg of a pale lavender powder; 42 % yield; mp 219-227 °C DEC; ¹H-NMR (400 MHz; DMSO) δ 11.89 (s, 1H), 9.08 (s, 1H), 7.60 (s, 3H), 7.55 (d, 1H, J=6.9 Hz), 7.32 (d, 1H, J=8.6 Hz), 6.63-6.59 (m, 1H), 3.40 (d, 2H, J=6.6 Hz), 0.90-0.80 (m, 1H), 0.30-0.26 (m, 2H), 0.05-0.00 (m, 2H); ¹⁹F-NMR (376 MHz; DMSO) δ
-130.61 (s), -140.38 (d, J=21.4 Hz); MS (APCI+) 558 (M+1, 70), 282 (100); (APCI-) 556 (M-1, 73), 486 (100); IR (KBr) 3390, 3283, 1652, 1513, 1477, 1163 cm⁻¹; Anal. calcd/found for: C₁₇H₁₅ClF₂IN₃O₄S 0.1 C₂HF₃O₂ C, 36.30/36.31; H, 2.67/2.55; N, 7.38/7.00.

### EXAMPLE 3C

### Preparation of 2-(2-chloro-4-iodo-phenylamino)-3,4-difluoro-N-hydroxy-5-sulfamoyl-benzamide (PD 224213)

To a stirring solution comprised of 5-*bis*-(4-methoxybenzyl)sulfamoyl-2-(2-chloro-4-iodophenylamino)-3,4-difluorobenzoic acid (0.67 g, 9.2x10⁻⁴ mol), O-(tetrahydro-2H-pyran-2-yl)-hydroxylamine (0.113 g, 9.65x10⁻⁴ mol), and diisopropylethylamine (0.50 ml, 2.9x10⁻³ mol) in a 1:1 v/v tetrahydrofuran-dichloromethane mixture (20 ml) was added solid PyBOP ([benzotriazolyloxy]tripyrrolidino phosphonium hexafluorophosphate, Advanced ChemTech, 0.52 g, 1.0x10⁻³ mol). The reaction mixture was stirred for 30 minutes, was concentrated *in vacuo* to a yellow oil, and was crystallized from methanol to afford 0.35 g of the off-white amorphous intermediate; 46 % yield; the intermediate was dissolved in trifluoroacetic acid (10 ml) and was stirred at ambient temperature for 16 hours. The mixture was vacuum filtered to collect the precipitate, which was recrystallized from methanol-chloroform to afford 0.055 g of the tan powder product; 26 % yield from intermediate; mp 230-236 °C DEC; ¹H-NMR (400 MHz; DMSO) δ 11.73 (s, 1H), 9.46 (s, 1H), 9.38 (s, 1H), 7.80-7.75 (m, 2H), 7.79 (s, 2H), 7.50 (d, 1H, J=8.5 Hz), 6.82-6.78 (m, 1H); ¹⁹F-NMR (376 MHz; DMSO) δ-130.83 (s), -139.24 (s); MS (APCI+) 504 (M+1, 53), 488 (90), 471 (100); (APCI-) 502 (M-1, 12), 486 (100); IR (KBr) 3295, 1652, 1636, 1519, 1477, 1315, 1157 cm⁻¹; Anal. calcd/found for: C₁₃H₉ClF₂IN₃O₄S 0.41 CHCl₃ C, 29.15/29.05; H, 1.72/1.66; N, 7.60/7.21.

### EXAMPLE 4C

### Preparation of 2-(2-chloro-4-iodo-phenylamino)-3,4-difluoro-5-sulfamoylbenzoic acid (PD 215730)

Solid 5-*bis*-(4-methoxybenzyl)sulfamoyl-2-(2-chloro-4-iodophenylamino)-3,4-difluorobenzoic acid (0.0995 g, 1.36x10⁻⁴ mol) was dissolved in trifluoroacetic acid (5 ml) under a nitrogen atmosphere. The reaction mixture was stirred at ambient temperature for 65 hours. The mixture was vacuum filtered to isolate 55.2 mg of a fine white precipitate. The crude product was recystallized from chloroform to afford 31.8 mg of the fluffy white solid product; 48 % yield; mp 295-296 °C DEC; ¹H-NMR (400 MHz; DMSO) δ 9.77 (s, 1H), 8.16 (d, 1H, J=7.3 Hz), 7.82 (s, 3H), 7.56 (d, 1H, J=8.5 Hz), 6.97-6.92 (m, 1H); ¹⁹F-NMR (376 MHz; DMSO) δ -128.47 (s), -141.13 (d, 19.8 Hz); MS (APCI+) 489 (M+1, 5), 102 (100); (APCI-) 491 (32), 490 (18), 489 (100), 488 (18), 487 (M-1, 75); IR (KBr) 3372, 3244, 1688 cm⁻¹; Anal. calcd/found for: C₁₃H₈ClF₂lN₂O₄S C, 31.96/32.19; H, 1.65/1.81; N, 5.73/5.37.

### EXAMPLE 5C

### Preparation of 2-(2-chloro-4-iodo-phenylamino)-N-cyclopropylmethoxy-3,4-difluoro-5-dimethylsulfamoyl-benzamide (PD 250253)

### Step a: Preparation of 2-(2-chloro-4-iodo-phenylamino)-3,4-difluoro-5-dimethylsulfamoyl-benzoic acid (PD 224339)

To a stirring solution comprised of 5-dimethylsulfamoyl-2,3,4-trifluorobenzoic acid (1.00 g, 3.53x10⁻³ mol) in tetrahydrofuran (15 ml) at -78 °C under a nitrogen atmosphere was added a 1.0 M solution of lithium *bis*(trimethylsilyl)amide in tetrahydrofuran (Aldrich, 3.6 ml, 3.6x10⁻³ mol). A lithium 2-chloro-4-iodoanilide suspension formed by adding a 1.0 M solution of lithium *bis*(trimethylsilyl)amide solution (7.2 ml, 7.2x10⁻³ mol) to a solution comprised of 2-chloro-4-iodoaniline (0.94 g, 3.63x10⁻³ mol) in tetrahydrofuran (15 ml) at -78 °C was added via canula to the lithium 5-dimethylsulfamoyl-2,3,4-trifluorobenzoate suspension. The cold bath was removed and the reaction mixture was stirred for one hour. The mixture was concentrated *in* vacuo to a crude solid. The crude product was suspended in diethyl ether (200 ml), to which suspension hydrogen chloride gas was introduced to produce a white precipitate. The precipitate was removed by vacuum filtration. The filtrate was concentrated *in vacuo* to give a dull-colored solid, which was triturated with hexanes-dichloromethane to afford 1.31 g of the white powder product; 72 % yield; mp 218-222 °C; ¹H-NMR (400 MHz; DMSO) δ 9.89 (s, 1H), 8.06 (d, 1H, J=6.1 Hz), 7.85 (d, 1H, J=1.9 Hz), 7.58 (dd, 1H, J=8.5, 1.9 Hz), 7.03 (dd, 1H, J=8.3, 6.6 Hz), 2.71 (s, 6H); ¹⁹F-NMR (376 MHz; DMSO) δ-125.58 (d, J=18.3 Hz), -140.14 (d, J=16.8 Hz); MS (APCl+) 519 (40), 518 (15), 517 (M+1, 100); (APCI-) 517 (6), 516 (2), 515 (M-1, 5), 480 (45), 127 (100); IR (KBr) 3346, 1665, 1487, 1283 cm⁻¹; Anal. calcd/found for: C₁₅H₁₂CIF₂IN₂O₄S C, 34.87/34.98; H, 2.34/2.32; N, 5.42/5.32.

### Step b: Preparation of 2-(2-chloro-4-iodo-phenylamino)-N-cyclopropylmethoxy-3,4-difluoro-5-dimethylsulfamoyl-benzamide

To a suspension comprised of 2-(2-chloro-4-iodo-phenylamino)-3,4-difluoro-5-dimethylsulfamoyl-benzoic acid (0.5 g, 9.68x10⁻⁴ mol) and cyclopropylmethoxylamine hydrochloride (0.13 g, 1.05x10⁻³ mol) in a 1:1 v/v mixture of dichloromethane-tetrahydrofuran (10 ml) was added diisopropylethylamine (0.65 ml, 3.73x10⁻³ mol) followed by the addition of solid PyBOP (0.55 g, 1.06x10⁻³ mol). The reaction mixture was stirred at ambient temperature for three days. The mixture was concentrated *in vacuo* to a red oil. The crude product was treated with 10 % aqueous hydrochloric acid (150 ml) and was extracted with diethyl ether (150 ml). The organic phase was dried (MgSO₄) and concentrated *in vacuo* to a crude solid. The solid was triturated with dichloromethane-hexanes and recovered by vacuum filtration to afford 0.3558 g of the white powder product; 63 % yield; mp 222-225 °C DEC; ¹H-NMR (400 MHz; DMSO) δ 11.97 (s, 1H), 9.32 (s, 1H), 7.60 (d, 1H, J=1.9 Hz), 7.49 (d, 1H, J=5.8 Hz), 7.33 (dd, 1H, J=8.4, 1.9 Hz), 6.70 (dd, 1H, 8.4, 6.3 Hz), 3.43 (d, 2H, J=7.2 Hz), 2.53 (s, 6H), 0.87-0.83 (m, 1H), 0.30-0.25 (m, 2H), 0.03-0.00 (m, 2H); ¹⁹F-NMR (376 MHz; DMSO) δ -127.67 (d, J=19,8 Hz), -139.32 (d, J=19.8 Hz); MS (APCI+) 586 (M+1, 100); (APCI-) 584 (M-1, 40), 514 (100); IR (KBr) 3263, 1644, 1585, 1507, 1480 cm⁻¹; Anal. calcd/found for: C₁₉H₁₉ClF₂IN₃O₄S C, 38.96/39.08; H, 3.27/3.18; N, 7.17/7.17.

### EXAMPLE 6C

### Preparation of N-cyclopropylmethoxy-3,4-difluoro-5-dimethylsulfamoyl-2-(4-iodo-2-methyl-phenylamino)-benzamide (PD 252745)

### Step a: Preparation of 3,4-difluoro-5-dimethylsulfamoyl-2-(4-iodo-2-methylphenylamino)-benzoic acid (PD 224340)

Same procedure and same scale as Example 4C, Step a, except 4-iodo-2-methylaniline was used instead of 2-chloro-4-iodoaniline; afforded 0.9592 g of the off-white powder product; 55 % yield; mp 235-238 °C; ¹H-NMR (400 MHz; DMSO) δ 9.69 (s, 1H), 8.04 (d, 1H, J=6.1 Hz), 7.60 (d, 1H, J=1.5 Hz), 7.45 (dd, 1H, J=8.3, 1.7 Hz), 6.88 (dd, 1H, J=8.3, 5.4 Hz), 2.70 (s, 6H), 2.21 (s, 3H); ¹⁹F-NMR (376 MHz; DMSO) δ -126.25 (d, J=16.8 Hz), -142.74 (d, J=19.8 Hz); MS (APCI+) 497 (M+1, 69), 357 (70), 316 (100); (APCI-) 495 (M-1, 3), 127 (100); IR (KBr) 3240, 1686, 1512, 1473, 1341, 1151 cm⁻¹; Anal. calcd/found for: C₁₆H₁₅F₂IN₂O₄S C, 38.72/38.70; H, 3.05/3.01; N, 5.64/5.49.

### Step b: Preparation of N-cyclopropylmethoxy-3,4-difluoro-5-dimethylsulfamoyl-2-(4-iodo-2-methyl-phenylamino)-benzamide

Same procedure and same scale as Example 4C, Step b, except the product was purified by recrystallization from absolute ethanol to afford 0.1718 g of the pale yellow microcrystalline product; 28 % yield; mp 171-172 °C; ¹H-NMR (400 MHz; DMSO) δ 11.79 (s, 1H), 8.91 (s, 1H), 7.40 (d, 1H, J=4.3 Hz), 7.36 (s, 1H), 7.21 (d, 1H, J=8.2 Hz), 6.54 (dd, 1H, 8.2, 4.3 Hz), 3.30 (d, 2H, J=6.5 Hz), 2.52 (s, 6H), 2.00 (s, 3H), 0.85-0.75 (m, 1H), 0.29 (d, 2H, J=7.7 Hz), 0.01 (d, 2H, J=4.1 Hz); ¹⁹F-NMR (376 MHz; DMSO) δ -128.94 (s), -143.32 (d, J=19.8 Hz); MS (APCI+) 566 (M+1, 100); (APCl-) 564 (M-1, 85), 494 (100); IR (KBr) 1649, 1609, 1588, 1512, 1475 cm⁻¹; Anal. calcd/found for: C₂₀H₂₂F₂IN₃O₄S C, 42.49/42.42; H, 3.92/3.78; N, 7.43/7.40.

### EXAMPLE 7C

### Preparation of 2-(2-chloro-4-iodo-phenylamino)-N-hydroxy-4-dimethylsulfamoyl-benzamide

### Step a: Preparation of 4-methyl-benzene-N,N-dimethylsulfonamide

To a stirring solution comprised of *para*-toluenesulfonyl chloride in dichloromethane at 0 °C is introduced excess gaseous dimethylamine. The precipitate is removed by filtration and the filtrate is concentrated *in vacuo* to obtain the product.

### Step b: Preparation of 4-methyl-3-nitro-benzene-N,N-dimethylsulfonamide

To a gently stirring solution comprised of 1 molar equivalent of fuming nitric acid in excess concentrated sulfuric acid is added 1 molar equivalent of 4-methyl-benzene-N,N-dimethylsulfonamide in increments. The mixture is stirred for one hour and then poured over chilled water. The mixture is extracted with a suitable solvent like diethyl ether or dichloromethane. The organic phase is dried over a suitable drying agent like magnesium sulfate and concentrated *in vacuo* to afford a crude product which may be purified by normal methods such as chromatography or crystallization from a solvent like chloroform or heptane.

### Step c: Preparation of 3-amino-4-methyl-benzene-N,N-dimethylsulfonamide

The compound 4-methyl-3-nitro-benzene-N,N-dimethylsulfonamide is dissolved in ethanol. A catalyst like Raney nickel is added and the mixture hydrogenated in a shaker. The catalyst is removed by filtration. The solvent is removed *in vacuo* to give a product which may be purified if necessary by chromatography or crystallization from an appropriate solvent like chloroform or heptane-ethyl acetate.

### Step d: Preparation of 3-fluoro-4-methyl-benzene-N,N-dimethylsulfonamide

The compound 3-amino-4-methyl-benzene-N,N-dimethylsulfonamide is diazotized with an alkyl nitrite like *tert*-butyl nitrite under anhydrous conditions in a non-reactive solvent like tetrahydrofuran or dichloromethane. The intermediate diazonium species is then treated with pyridinium fluoride to give the product, which may be purified by chromatography or crystallization.

### Step e: Preparation of 4-dimethylsulfamoyl-2-fluoro-benzoic acid

A mixture comprised of 3-fluoro-4-methyl-benzene-N,N-dimethylsulfonamide and potassium permanganate (2.2 molar equivalents) in water is brought to reflux for four hours. The reaction mixture is filtered through celite. The filtrate is treated with activated carbon and refiltered through fresh celite. The second filtrate is acidified with concentrated ' hydrochloric acid to pH 0. The mixture is allowed to cool and is extracted with diethyl ether. The organic phase is dried over a drying agent like magnesium sulfate and is concentrated *in vacuo.* The product may be purified by recrystallization from an appropriate solvent like ethanol or chloroform.

### Step f: Preparation of 2-(2-chloro-4-iodo-phenylamino)-4-dimethylsulfamoylbenzoic acid

To a stirring cold (-78 °C) solution comprised of 2-chloro-4-iodoaniline (1 molar equivalent) in anhydrous tetrahydrofuran under a nitrogen atmosphere is added a commercially available lithium diisopropylamide solution (Aldrich, 2.0 M in tetrahydrofuran/heptane/ethylbenzene, 1 molar equivalent). After stirring for 5 minutes, a solution comprised of 4-dimethylsulfamoyl-2-fluoro-benzoic acid (1 molar equivalent) in tetrahydrofuran is added. The cold bath is removed and the reaction mixture is stirred for 2 hours. The reaction mixture is then partitioned between diethyl ether and dilute aqueous hydrochloric acid. The organic phase is washed with brine, dried over magnesium sulfate, and concentrated *in vacuo* to afford a product which may be purified by chromatography of recrystallization from an appropriate solvent like chloroform or heptane-ethanol.

### Step g: Preparation of 2-(2-chloro-4-iodo-phenylamino)-4-dimethylsulfamoylbenzoic acid O-(tetrahydro-2H-pyran-2-yl)-oxyamide

A solution comprised of 2-(2-chloro-4-iodo-phenylamino)-4-dimethylsulfamoyl-benzoic acid, O-(tetrahydro-2H-pyran-2-yl)-hydroxylamine (1.25 molar equivalents), benzotriazole-1-yl-oxy-*tris*-pyrrolidino-phosphonium hexafluorophosphate (1.25 molar equivalents), and diisopropylethylamine (3 molar equivalents) in 1:1 v/v tetrahydrofuran-dichloromethane is stirred for 30 minutes. The reaction mixture is concentrated *in vacuo* and the residue is purified by flash chromatography; elution with dichloromethane affords the desired product. The product may be recrystallized with an appropriate solvent like methanol if further purification is necessary.

### Step h: Preparation of 2-(2-chloro-4-iodo-phenylamino)-4-dimethylsulfamoyl-N-hydroxy-benzamide

The compound 2-(2-chloro-4-iodo-phenylamino)-4-dimethylsulfamoylbenzoic acid O-(tetrahydro-2H-pyran-2-yl)-oxyamide is dissolved in an appropriate hydrogen chloride-saturated solvent like methanol or ethanol. Once homogeneous, the solution is concentrated *in vacuo* to give the desired product. The product may be triturated with an appropriate solvent like chloroform or dichloromethane if further purification is necessary.

### F. Other Embodiments

From the above disclosure and examples, and from the claims below, the essential features of the invention are readily apparent. The scope of the invention also encompasses various modifications and adaptations within the knowledge of a person of ordinary skill. Examples include a disclosed compound modified by addition or removal of a protecting group, or an ester, pharmaceutical salt, hydrate, acid, or amide of a disclosed compound.

## Claims

1. Use of
a MEK inhibitor selected from: a compound of formula (I): 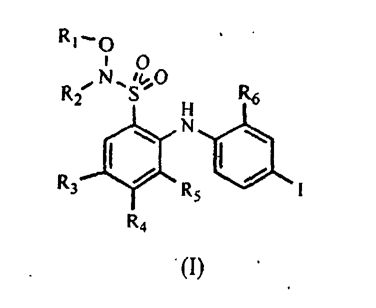
R₁ is H, C ₁₋₈ alkyl, C ₃₋₈ alkenyl, C ₃₋₈ alkynyl, C ₃₋₈ cycloalkyl phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl, (CH₂)₂₋₄ OR_{C} or (CH₂)₂₋₄ NR_{C}R_{D} ;
R₂ is H, C ₁₋₄ alkyl, phenyl, C ₃₋₆ cycloalkyl, C ₃₋₆ heterocyclic radical, or (C ₃₋₆ cycloalkyl) methyl;
each of R₃ and R₄ is independently selected from H, F, NO₂, Br and Cl;
R₅ is selected from H and F;
R₆ is H, F, Cl or CH₃;
each of R_{C} and R_{D} is independently selected from H, C ₁₋₄ alkyl, C ₃₋₄ alkenyl, C ₃₋₄ alkynyl, C ₃₋₆ cycloalkyl, and phenyl; or NR_{C}R_{D} may be a piperidino, morpholino, or N-(C ₁₋₆ alkyl)piperazino ring;
wherein each hydrocarbon radical above is optionally substituted with between 1 and 3 substituents independently selected from halo, hydroxyl, amino, (amino)sulfonyl, and NO₂; and
wherein each heterocyclic radical above is optionally substituted with between 1 and 3 substituents independently selected from halo, C ₁₋₄ alkyl, C ₃₋₆ cycloalkyl, C ₃₋₄ alkenyl, C ₃₋₄ alkynyl, phenyl, hydroxyl, amino, (amino)sulfonyl, and NO₂, wherein each substituent alkyl, cycloalkyl, alkenyl, alkynyl or phenyl is in turn optionally substituted with between 1 and 2 substituents independently selected from halo, C ₁₋₂ alkyl, hydroxyl, amino, and NO₂;
or a pharmaceutically acceptable salt or C ₁₋₈ ester thereof for the manufacturing of pharmaceuticals for the treatment of chronic pain.

2. The use of claim 1, wherein said chronic pain is selected from neuropathic pain, idiopathic pain, and pain associated with chronic alcoholism, vitamin deficiency, uremia, or hypothyroidism.

3. 'The use of claim 2, wherein said chronic pain is a type of neuropathic pain.

4. The use of claim 3, wherein said neuropathic pain is associated with one of the following: inflammation, postoperative pain, phantom limb pain, burn pain, gout, trigeminal neuralgia, acute herpetic and postherpetic pain, causalgia, diabetic neuropathy, plexus avulsion, neuroma, vasculitis, viral infection, crush injury, constriction injury, tissue injury, limb amputation, post-operative pain, arthritis pain, and any other nerve injury between the peripheral nervous system and the central nervous system, inclusively.

5. The use of claim 2, wherein said chronic pain is associated with chronic alcoholism, vitamin deficiency, uremia, or hypothyroidism.

6. The use of claim 2, wherein said chronic pain is associated with idiopathic pain.

7. The use of claim 1, wherein said chronic pain is associated with inflammation.

8. The use of claim 1, wherein said chronic pain is associated with arthritis.

9. The use of claim 1, wherein said chronic pain is associated with post-operative pain.

10. The use of claim 1, wherein R₃ is bromo or chloro.

11. The use of claim 1, wherein R₄ is fluoro.

12. The use of claim 1, wherein R₅ is H.

13. The use of claim 12, wherein each of R₄ and R₅ is H.

14. The use of claim 1, wherein each of R₄ and R₅ is fluoro.

15. The use of claim 14, wherein R₃ is bromo.

16. The use of claim 14, wherein R₃ is fluoro.

17. The use of claim 1, wherein R₄ is nitro.

18. The use of claim 16, wherein R₅ is H.

19. The use of claim 1, wherein R₆ is chloro.

20. The use of claim 1, wherein R₆ is methyl.

21. The use of claim 1, wherein R₁ is H or C ₁₋₄ alkyl, and R₂ is H.

22. The use of claim 1, wherein R₁ is (C ₃₋₆ cycloalkyl)methyl.

23. The use of claim 1, wherein R₁ is H.

24. The use of claim 1, wherein R₁ is (CH₂)₂₋₄OR_{C} or (CH₂)₂₋₄ NR_{C}R_{D}.

25. The use of claim 1, wherein said MEK inhibitor has a structure selected from: 4-fluoro-2-(4-iodo-2-methyl-phenylamino)-benzenesulfonic acid; 4-fluoro-N-hydroxy-2-(4-iodo-2-methyl-phenylamino)-benzenesulfonamide; N-cyclopropylmethoxy-4-fluoro-2-(4-iodo-2-methylphenylamino)-benzenesulfonamide; 3,4-difluoro-2-(4-iodo-2-methylphenylamino)-benzenesulfonicacid; 3,4-difluoro-N-hydroxy-2-(4-iodo-2-methyl-phenylamino)-benzenesulfonamide; N-cyclopropylmethoxy-3,4-difluoro-2-(4-iodo-2-methyl-phenylamino)-benzenesulfonamide; 3,4,5-trifluoro-2-(4-iodo-2-methyl-phenylamino)-benzenesulfonic acid; 3,4,5-trifluoro-N-hydroxy-2-(4-iodo-2-methyl-phenylamino)-benzenesulfonamide; N-cyclopropylmethoxy-3,4,5-trifluoro-2-(4-iodo-2-methyl-phenylamino)-benzenesulfonamide; 5-bromo-3,4-difluoro-2-(4-iodo-2-methyl-phenylamino)-benzenesulfonic acid; 5-bromo-3,4-difluoro-N-hydroxy-2-(4-iodo-2-methylphenylamino)-benzenesulfonamide; 5-bromo-N-cyclopropylmethoxy-3,4-difluoro-2-(4-iodo-2-methyl-phenylamino)-benzenesulfonamide; 2-(4-iodo-2-methyl-phenylamino)-4-nitro-benzenesulfonic acid; N-hydroxy-2-(4-iodo-2-methyl-phenylamino)-4-nitro-benzenesulfonamide; and N-cyclopropylmethoxy-2-(4-iodo-2-methyl-phenylamino)-4-nitrobenzenesulfonamide.

26. The use of claim 1, wherein said MEK inhibitor has a structure selected from: 2-(2-chloro-4-iodo-phenylamino)-4-fluoro-benzenesulfonic acid; 2-(2-chloro-4-iodo-phenylamino)-4-fluoro-N-hydroxybenzenesulfonamide; 2-(2-chloro-4-iodo-phenylamino)-N-cyclopropylmethoxy-4-fluoro-benzenesulfonamide; 2-(2-chloro-4-iodo-phenylamino)-3,4-difluorobenzenesulfonic acid; 2-(2-chloro-4-iodo-phenylamino)-3,4-difluoro-N-hydroxy-benzenesulfonamide; 2-(2-chloro-4-iodo-phenylamino)-N-cyclopropylmethoxy-3,4-difluoro-benzenesulfonamide; 2-(2-chloro-4-iodophenylamino)-3,4,5-trifluoro-benzenesulfonic acid; 2-(2-chloro-4-iodophenylamino)-3,4,5-trifluoro-N-hydroxy-benzenesulfonamide; 2-(2-chloro-4-iodo-phenylamino)-N-cyclopropylmethoxy-3,4,5-trifluoro-benzenesulfonamide; 5-bromo-2-(2-chloro-4-iodo-phenylamino)-3,4-difluoro-benzenesulfonic acid; 5-bromo-2-(2-chloro-4-iodo-phenylamino)-3,4-difluoro-N-hydroxybenzenesulfonamide; 5-bromo-2-(2-chloro-4-iodo-phenylamino)-N-cyclopropylmethoxy-3,4-difluoro-benzenesulfonamide; 2-(2-chloro-4-iodophenylamino)-4-nitro-benzenesulfonic acid; 2-(2-chloro-4-iodo-phenylamino)-N-hydroxy-4-nitro-benzenesulfonamide; and 2-(2-chloro-4-iodo-phenylamino)-N-cyclopropylmethoxy-4-nitro-benzenesulfonamide.

27. Use of a MEK inhibitor selected from a compound having the formula (II)A: 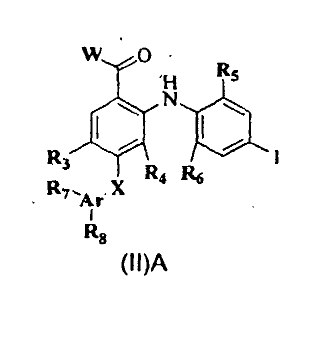
W is OR₁, NR₂OR₁, NR_{A}R_{B}, NR₂NR_{A}R_{B}, or NR₂(CH₂)₂₋₄ NR_{A}R_{B};
R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl or (CH₂)₂₋₄NR_{A}R_{B};
R₂ is H, phenyl, C ₁₋₄ alkyl, C₃₋₄ alkenyl, C ₃₋₈ alkynyl, C ₃₋₈ cycloalkyl, or (C ₃₋₈ cycloalkyl)-C ₁₋₄ alkyl;
R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl;
R_{B} is H, C ₁₋₈ alkyl, C ₃₋₈ alkenyl, C ₃₋₈ alkynyl, C ₃₋₈ cycloalkyl, or C ₆₋₈ aryl;
R₃ is halo, NO₂, SO₂NR₁ (CH₂)₂₋₄NR_{E}R_{F}, SO₂NR_{I} R_{K} or (CO)T;
T is C ₁₋₈ alkyl, C ₃₋₈ cycloalkyl, (NR_{E}R_{F})C ₁₋₄ alkyl, OR_{F}, NR_{I}(CH₂)₂₋₄NR_{E}R_{F}, or NR_{E}R_{F};
R₄ is H or F;
R₅ is H, methyl, halo, or NO₂;
R₆ is H, methyl, halo, or NO₂;
Ar is phenyl, 2-pyridyl, 3-pyridyl, or 4-pyridyl;
each of R₇ and R₈ is independently selected from H, halo, C ₁₋₄ alkyl, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H}, and (CO)NR_{G}R_{H}; provided that where Ar is a pyridyl, each of R₇ and R₈ is H;
each of R_{C}, R_{D}, R_{E}, R_{F}, R_{G}, and R_{H} is independently selected from H, C ₁₋₄ alkyl, C ₃₋₄ alkenyl, C ₃₋₄ alkynyl, C ₃₋₆ cycloalkyl, and phenyl; each of NR_{C}R_{D}, NR_{E}R_{F}, and NR_{G}R_{H} can also be independently morpholinyl, piperazinyl, pyrrolidinyl, or piperadinyl;
each of R_{I} and R_{J} is independently H, methyl, or ethyl;
R_{K} is C ₁₋₄ alkyl, C ₃₋₄ alkenyl, C ₃₋₄ alkynyl, C ₃₋₆ cycloalkyl, or phenyl;
X is O, S, or NH; and
wherein each hydrocarbon radical or heterocyclic radical above is optionally substituted with between 1 and 3 substituents independently selected from halo, C ₁₋₄ alkyl, C ₃₋₆ cycloalkyl, C ₂₋₄ alkenyl, C ₂₋₄ alkynyl, phenyl, hydroxyl, amino, (amino)sulfonyl, and NO₂, wherein each substituent alkyl, cycloalkyl, alkenyl, alkynyl or phenyl is in turn optionally substituted with between 1 and 2 substituents independently selected from halo, G ₁₋₂ alkyl, hydroxyl, amino, and NO₂;
or a pharmaceutically acceptable salt or C ₁₋₇ ester thereof for the manufacturing of pharmaceuticals for the treatment of chronic pain.

28. The use of claim 27, wherein said chronic pain is selected from neuropathic pain, idiopathic pain, and pain associated with chronic alcoholism, vitamin deficiency, uremia, or hypothyroidism.

29. The use of claim 28, wherein said chronic pain is a type of neuropathic pain.

30. The use of claim 29, wherein said neuropathic pain is associated with one of the following: inflammation, postoperative pain, phantom limb pain, burn pain, gout, trigeminal neuralgia, acute herpetic and postherpetic pain, causalgia, diabetic neuropathy, plexus avulsion, neuroma, vasculitis, viral infection, crush injury, constriction injury, tissue injury, limb amputation, post-operative pain, arthritis pain, and any other nerve injury between the peripheral nervous system and the central nervous system, inclusively.

31. The use of claim 28, wherein said chronic pain is associated with chronic alcoholism, vitamin deficiency, uremia, or hypothyroidism.

32. The use of claim 28, wherein said chronic pain is associated with idiopathic pain.

33. The use of claim 27, wherein said chronic pain is associated with inflammation.

34. The use of claim 27, wherein said chronic pain is associated with arthritis.

35. The use of claim 27, wherein said chronic pain is associated with post-operative pain.

36. A use of claim 27, having the following formula (I)A: 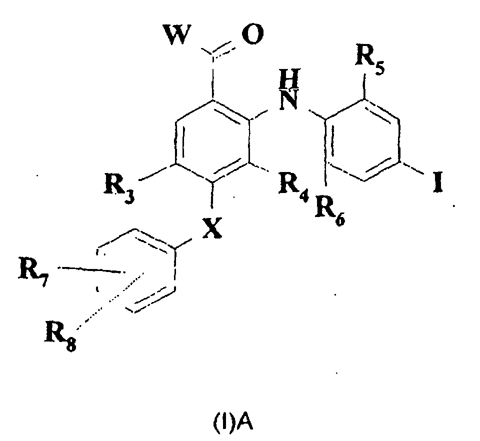 wherein
W is OR₁, NR₂OR₁, NR_{A}R_{B}, NR₂NR_{A}R_{B}, or NR₂(CH₂)₂₋₄NR_{A}R_{B};
R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl or (CH₂)₂₋₄NR_{A}R_{B};
R₂ is H, phenyl, C ₁₋₄ alkyl, C₃₋₄ alkenyl, C ₃₋₈ alkynyl, C ₃₋₈ cycloalkyl, or (C ₃₋₈ cycloalkyl)-C ₁₋₄ alkyl;
R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl;
R_{B} is H, C ₁₋₈ alkyl, C ₃₋₈ alkenyl, C ₃₋₈ alkynyl, C ₃₋₈ cycloalkyl, or C ₆₋₈ aryl;
R₃ is halo, NO₂, SO₂NR_{I}(CH₂)₂₋₄NR_{E}R_{F}, SO₂NR_{I} R_{K} or (CO)T;
T is C ₁₋₈ alkyl, C ₃₋₈ cycloalkyl, (NR_{E}R_{F})C ₁₋₄ alkyl, OR_{F}, NR_{I}(CH₂)₂₋₄NR_{E}R_{F}, or NR_{E}R_{F};
R₄ is H or F;
R₅ is H, methyl, halo, or NO₂;
R₆ is H, methyl, halo, or NO₂;
each of R₇ and R₈ is independently selected from H, halo, C ₁₋₄ alkyl, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H}, and (CO)NR_{G}R_{H};
each of R_{C}, R_{D}, R_{E}, R_{F}, R_{G}, and R_{H} is independently selected from H, C ₁₋₄ alkyl, C ₃₋₄ alkenyl, C ₃₋₄ alkynyl, C ₃₋₆ cycloalkyl, and phenyl; each of NR_{C}R_{D}, NR_{E}R_{F}, and NR_{G}R_{H} can also be independently morpholinyl, piperazinyl, pyrrolidinyl, or piperadinyl;
each of R_{I} and R_{J} is independently H, methyl, or ethyl;
R_{K} is C ₁₋₄ alkyl, C ₃₋₄ alkenyl, C ₃₋₄ alkynyl, C ₃₋₆ cycloalkyl, or phenyl;
X is O, S, or NH; and
wherein each hydrocarbon radical or heterocyclic radical above is optionally substituted with between 1 and 3 substituents independently selected from halo, C ₁₋₄ alkyl, C ₃₋₆ cycloalkyl, C ₂₋₄ alkenyl, C ₂₋₄ alkynyl, phenyl, hydroxyl, amino, (amino)sulfonyl, and NO₂, wherein each substituent alkyl, cycloalkyl, alkenyl, alkynyl or phenyl is in turn optionally substituted with between 1 and 2 substituents independently selected from halo, C ₁₋₂ alkyl, hydroxyl, amino, and NO₂;
or a pharmaceutically acceptable salt or C ₁₋₇ ester thereof.

37. A use of claim 27, wherein R₃ is NO₂.

38. A use of claim 27, wherein R₄ is fluoro.

39. A use of claim 27, wherein each of R₃ and R₄ is independently selected from Hand fluoro.

40. A use of claim 27, wherein R₅ is methyl, fluoro, or chloro.

41. A use of claim 27, wherein R₆ is methyl, chloro, fluoro, nitro, or hydrogen.

42. A use of claim 41, wherein R₆ is H.

43. A use of claim 41, wherein R₆ is fluoro.

44. A use of claim 27, wherein R_{K} is methyl or ethyl.

45. A use of claim 27, wherein R₁ is H, methyl, ethyl, propyl, isopropyl, isobutyl, benzyl, phenyl, phenethyl, allyl, C ₂₋₅ alkenyl, C ₃₋₆ cycloalkyl, (C ₃₋₅ cycloalkyl)C ₁₋₂ alkyl, (C ₃₋₅ heterocyclic radical)C ₁₋₂ alkyl, or (CH₂)₂₋₄ NR_{C}R_{D}.

46. A use of claim 45, wherein R₁ is H or (C ₃₋₄ cycloalkyl)-C ₁₋₂ alkyl.

47. A use of claim 27, wherein R₂ is H or methyl.

48. A use of claim 27, wherein R_{A} has at least one hydroxyl substituent.

49. A use of claim 27, wherein R_{A} is H, methyl, ethyl, isobutyl, hydroxyethyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl.

50. A use of claim 27, wherein W is NR_{A}R_{B} or NR₂NR_{A}R_{B}.

51. A use of claim 27, wherein W is NR₂(CH₂)₂₋₄ NR_{A}R_{B} or O(CH₂)₂₋₃ NR_{A}R_{B}.

52. A use of claim 27, wherein W is NR₂OR₁.

53. A use of claim 27, wherein W is OR_{B}.

54. A use of claim 27, wherein R₇ is in the para position relative to X.

55. A use of claim 54, wherein R₇ is iodo.

56. A use of claim 27, wherein R₈ is in the ortho position relative to X.

57. A use of claim 27 having the formula 2,4-bis-(2-chloro-4-iodo-phenylamino)-3-fluoro-5-nitro-benzoic acid.

58. A use of claim 27, wherein said MEK inhibitor has a structure selected from: 2-(2-chloro-4-iodo-phenylamino)-3-fluoro-5-nitro-4-(4-sulfamoyl-phenylamino)-benzoic acid; 2-(2-chloro-4-iodo-phenylamino)-3-fluoro-5-nitro-4-phenylamino-benzoic acid; 2-(2-chloro-4-iodo-phenylamino)-3-fluoro-5-nitro-4-phenoxy-benzoic acid; 2-(2-chloro-4-iodo-phenylamino)-3-fluoro-5-nitro-4-phenylsulfanyl-benzoic acid; 2-(2-chloro-4-iodo-phenylamino)-3-fluoro-4-(methyl-phenyl-amino)-5-nitro-benzoic acid; benzamide, 2-[(2-chloro-4-iodophenyl)amino]-3-fluoro-N-hydroxy-4-[[4-[[(2-hydroxyethyl)amino]-carbonyl]phenyl]amino]-5-nitro-; benzamide, 2-[(2-chloro-4-iodophenyl)amino]-4-[[4-[(dimethylamino)carbonyl]phenyl]amino]-3-fluoro-N-hydroxy-5-nitro-; 2-(2-chloro-4-iodo-phenylamino)-3,5-difluoro-4-phenylamino-benzoic acid; 2-(2-chloro-4-iodo-phenylamino)-3-fluoro-5-nitro-4-(3-sulfamoyl-phenylamino)-benzoic acid; and 2-(2-chloro-4-iodo-phenylamino)-3-fluoro-5-nitro-4-(2-sulfamoyl-phenylamino)-benzoic acid; and the corresponding hydroxamic acids and cyclopropylmethyl hydroxamates.

59. Use of a MEK inhibitor selected from a compound of formula (I)B: 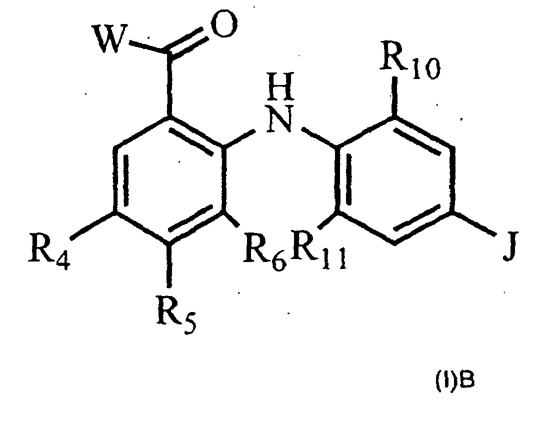 wherein
W is OR₁, NR₂OR₁, NR_{A}R_{B}, NR₂NR_{A}R_{B}, O(CH₂)₁₋₄NR_{A}R_{B}, or NR₂(CH₂)₁₋₄ NR_{A}R_{B}; O(CH₂)₁₋₄OR₁, or NR₂(CH₂)₁₋₄ OR₁;
R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, or (C ₃₋₈ heterocyclic radical)C ₃₋₄ alkynyl;
each of R₂ and R₃ is independently H, phenyl, C ₁₋₄ alkyl, C ₃₋₈ alkynyl, C ₃₋₈ cycloalkyl, or (C ₃₋₈ cycloalkyl)C ₁₋₄ alkyl;
each of R₄, R₅ and R₆ is independently H, Cl, F, or Br;
R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl;
R_{B} is H, C ₁₋₈ alkyl, C ₃₋₈ alkenyl, C ₃₋₈ alkynyl, C ₃₋₈ cycloalkyl, or phenyl;
J is SR_{C}, OR_{C}, SO₂R_{C}, SOR_{C}, SO₂NR_{D}R_{E}, C ₁₋₈ alkyl, C ₃₋₈ alkenyl, C ₃₋₈ alkynyl, C ₃₋₈ cycloalkyl, C ₅₋₈ cycloalkenyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, -M'E'G', (heterocyclic radical)-M'-E'-G', or (cycloalkyl)-M'-E'-G';
M' is O, SO, SO₂, NR_{E}, (CO)NR_{E}, NR_{E} (CO), SO₂NR_{E}, NR_{E}SO₂, or CH₂;
E' is absent (a covalent bond), (CH₂)₁₋₄ or (CH₂)ₘ O(CH₂)ₚ where 1 ≤ (each of m and p independently) ≤ 3 and 2 ≤ (m + p) ≤ 4;
G' is OR₃, SO₂R_{C,} or NR_{F}R_{G}; provided that where p = 1, then G' is H;
each of R_{C}, R_{D}, R_{E}, R_{F} and R_{G} is independently selected from H, C ₁₋₆ alkyl, C ₃₋₄ alkenyl, C ₃₋₄ alkynyl, C ₃₋₆ cycloalkyl, C ₃₋₆ heterocyclic radical, and phenyl; NR_{F}R_{G} and NR_{D}R_{E} can each also independently be selected from morpholinyl, pyrazinyl, piperazinyl, pyrrolidinyl, or piperadinyl;
R₁₀ is H, C ₁₋₄ alkyl, halo, NO₂, or SO₂NR_{H}R_{I}; and
R₁₁ is H, halo, or NO₂;
wherein each hydrocarbon radical or heterocyclic radical above is optionally substituted with between 1 and 3 substituents independently selected from halo, C ₁₋₄ alkyl, C ₃₋₆ cycloalkyl, C ₂₋₄ alkenyl, C ₂₋₄ alkynyl, phenyl, hydroxy, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxy, amino, and NO₂;
or a pharmaceutically acceptable salt or C ₁₋₇ ester thereof for the manufacturing of pharmaceutical compositions for the treatment of chronic pain.

60. The use of claim 59, wherein said chronic pain is selected from neuropathic pain, idiopathic pain, and pain associated with chronic alcoholism, vitamin deficiency, uremia, or hypothyroidism.

61. The use of claim 60, wherein said chronic pain is a type of neuropathic pain.

62. The use of claim 61, wherein said neuropathic pain is associated with one of the following: inflammation, postoperative pain, phantom limb pain, burn pain, gout, trigeminal neuralgia, acute herpetic and postherpetic pain, causalgia, diabetic neuropathy, plexus avulsion, neuroma, vasculitis, viral infection, crush injury, constriction injury, tissue injury, limb amputation, post-operative pain, arthritis pain, and any other nerve injury between the peripheral nervous system and the central nervous system, inclusively.

63. The use of claim 60, wherein said chronic pain is associated with chronic alcoholism, vitamin deficiency, uremia, or hypothyroidism.

64. The use of claim 60, wherein said chronic pain is associated with idiopathic pain.

65. The use of claim 59, wherein said chronic pain is associated with inflammation.

66. The use of claim 59, wherein said chronic pain is associated with arthritis.

67. The use of claim 59, wherein said chronic pain is associated with post-operative pain.

68. A use of claim 59, wherein R_{C} is C ₁₋₂ alkyl.

69. A use of claim 59, wherein W is OH.

70. A use of claim 59, wherein W is NHOH.

71. A use of claim 59, wherein W is NHO(cyclopropylmethyl).

72. A use of claim 59, wherein R₁₀ is methyl or chloro.

73. A use of claim 59, where R₁₁ is fluoro.

74. A use of claim 59, where R₁₁ is H.

75. A use of claim 59, wherein J is trihalomethyl or methylthio.

76. A use of claim 59, wherein J is 1,2,5-thiadiazol-3-yl.

77. A use of claim 59, wherein J is SO₂CH₃.

78. A use of claim 59, wherein J is SOCH₃.

79. A use of claim 59, wherein J is C ₂₋₈ alkynyl where the triple bond is between the carbon atoms alpha and beta to the phenyl group.

80. A use of claim 59, wherein R₁ has at least one hydroxy substituent.

81. A use of claim 59, wherein R₁ is H, methyl, ethyl, propyl, isopropyl, isobutyl, benzyl, phenethyl, allyl, C ₃₋₅ alkenyl, C ₃₋₅ alkynyl, C ₃₋₆ cycloalkyl, (C ₃₋₅ cycloalkyl)C ₁₋₂ alkyl, or (C ₃₋₅ heterocyclic radical)-C ₁₋₂ alkyl.

82. A use of claim 59, wherein R₁ is H or (C ₃₋₄ cycloalkyl)-C ₁₋₂ alkyl.

83. A use of claim 59, wherein R₂ is H, methyl, C ₃₋₄ alkynyl, C ₃₋ ₅ cycloalkyl, or (C ₃₋₅ cycloalkyl)methyl.

84. A use of claim 59, wherein R_{A} is H, methyl, ethyl, isobutyl, hydroxyethyl, hydroxypropyl, cyclopropylmethyl, cyclobutylmethyl, C ₂₋₄ alkynyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl.

85. A use of claim 59, wherein each of R₄ and R₆ is H, and R₅ is F.

86. A use of claim 59, wherein each of R₄, R₅, and R₆ is F.

87. A use of claim 59, wherein each of R₄ and R₅ is F and R₆ is Br.

88. A use of claim 59, wherein R₅ is F.

89. A use of claim 59, wherein said MEK inhibitor has a structure selected from: 4-fluoro-2-(2-methyl-4-methylsulfanyl-phenylamino)-benzoic acid; 5-bromo-3,4-difluoro-2-(2-methyl-4-methylsulfanylphenylamino)-benzoic acid; 3,4-difluoro-2-(4-methanesulfinyl-2-methylphenylamino)-benzoic acid; 2-(4-methanesulfinyl-2-methyl-phenylamino)-4-nitro-benzoic acid; 3,4,5-trifluoro-2-(4-methanesulfonyl-2-methylphenylamino)-benzoic acid; 3,4-difluoro-2-(2-methyl-4-methylsulfanylphenylamino)-benzoic acid; 2-(2-methyl-4-methylsulfanyl-phenylamino)-4-nitro-benzoic acid; 3,4,5-trifluoro-2-(4-methanesulfinyl-2-methyl-phenylamino)-benzoic acid; 4-fluoro-2-(4-methanesulfinyl-2-methyl-phenylamino)-benzoic acid; 5-bromo-3,4-difluoro-2-(4-methanesulfonyl-2-methyl-phenylamino)-benzoic acid; 3,4,5-trifluoro-2-(2-methyl-4-methylsulfanyl-phenylamino)-benzoic acid; 4-fluoro-2-(4-methane-sulfinyl-2-methyl-phenylamino)-benzoic acid; 5-bromo-3,4-difluoro-2-(4-methanesulfinyl-2-methyl-phenylamino)-benzoic acid; 3,4-difluoro-2-(4-methanesulfonyl-2-methyl-phenylamino)-benzoic acid; 2-(4-methanesulfonyl-2-methyl-phenylamino)-4-nitro-benzoic acid; N-cyclopropylmethoxy-4-fluoro-2-(2-methyl-4-methylsulfanyl-phenylamino)-benzamide; 5-bromo-N-cyclopropylmethoxy-3,4-difluoro-2-(2-methyl-4-methylsulfanyl-phenylamino)-benzamide; N-cyclopropylmethoxy-3,4-difluoro-2-(4-methanesulfinyl-2-methyl-phenylamino)-benzamide; N-cyclopropylmethoxy-2-(4-methanesulfinyl-2-methyl-phenylamino)-4-nitrobenzamide; N-cyclopropylmethoxy-3,4,5-trifluoro-2-(4-methanesulfonyl-2-methyl-phenylamino)-benzamide; N-cyclopropylmethoxy-3,4-difluoro-2-(2-methyl-4-methylsulfanyl-phenylamino)-benzamide; N-cyclopropylmethoxy-2-(2-methyl-4-methylsulfanyl-phenylamino)-4-nitro-benzamide; N-cyclopropylmethoxy-3,4,5-trifluoro-2-(4-methanesulfinyl-2-methylphenylamino)-benzamide; N-cyclopropylmethoxy-4-fluoro-2-(4-methanesulfinyl-2-methyl-phenylamino)-benzamide; 5-bromo-N-cyclopropylmethoxy-3,4-difluoro-2-(4-methanesulfonyl-2-methylphenylamino)-benzamide; N-cyclopropylmethoxy-3,4,5-trifluoro-2-(2-methyl-4-methylsulfanyl-phenylamino)-benzamide; N-cyclopropylmethoxy-4-fluoro-2-(4-methanesulfinyl-2-methyl-phenylamino)-benzamide; 5-bromo-N-cyclopropylmethoxy-3,4-difluoro-2-(4-methanesulfinyl-2-methyl-phenylamino)-benzamide; N-cyclopropylmethoxy-3,4-difluoro-2-(4-methanesulfonyl-2-methyl-phenylamino)-benzamide; and N-cyclopropylmethoxy-2-(4-methanesulfonyl-2-methyl-phenylamino)-4-nitro-benzamide.

90. A use of claim 59, wherein said MEK inhibitor has a structure selected from: 4-fluoro-N-hydroxy-2-(2-methyl-4-methylsulfanylphenylamino)-benzamide; 5-bromo-3,4-difluoro-N-hydroxy-2-(2-methyl-4-methylsulfanyl-phenylamino)-benzamide; 3,4-difluoro-N-hydroxy-2-(4-methanesulfinyl-2-methyl-phenylamino)-benzamide; N-hydroxy-2-(4-methanesulfinyl-2-methyl-phenylamino)-4-nitro-benzamide; 3,4,5-trifluoro-N-hydroxy-2-(4-methanesulfonyl-2-methyl-phenylamino)-benzamide; 3,4-difluoro-N-hydroxy-2-(2-methyl-4-methylsulfanyl-phenylamino)-benzamide; N-hydroxy-2-(2-methyl-4-methylsulfanyl-phenylamino)-4-nitro-benzamide; 8: 3,4,5-trifluoro-N-hydroxy-2-(4-methanesulfinyl-2-methyl-phenylamino)-benzamide; 4-fluoro-N-hydroxy-2-(4-methanesulfinyl-2-methyl-phenylamino)-benzamide; 5-bromo-3,4-difluoro-N-hydroxy-2-(4-methanesulfonyl-2-methylphenylamino)-benzamide; 3,4,5-trifluoro-N-hydroxy-2-(2-methyl-4-methylsulfanyl-phenylamino)-benzamide, 4-fluoro-N-hydroxy-2-(4-methanesulfinyl-2-methyl-phenylamino)-benzamide; 5-bromo-3,4-difluoro-N-hydroxy-2-(4-methanesulfinyl-2-methyl-phenylamino)-benzamide; 3,4-difluoro-N-hydroxy-2-(4-methanesulfonyl-2-methyl-phenylamino)-benzamide; and N-hydroxy-2-(4-methanesulfonyl-2-methyl-phenylamino)-4-nitro-benzamide.

91. A use of claim 59, wherein said MEK inhibitor has a structure selected from: 3,4-difluoro-2-(4-imidazol-1-yl-2-methylphenylamino)-benzoicacid;N-cyclopropylmethoxy-3,4-difluoro-2-(4-imidazol-1-yl-2-methyl-phenylamino)-benzamide;3,4-difluoro-N-hydroxy-2-(4-imidazol-1-yl-2-methyl-phenylamino)-benzamide; 3,4,5-trifluoro-2-(2-methyl-4-[1,2,5]thiadiazol-3-yl-phenylamino)-benzoicacid; N-cyclopropylmethoxy-3,4,5-trifluoro-2-(2-methyl-4-[1,2,5]thiadiazol-3-yl-phenylamino)-benzamide; 3,4,5-trifluoro-N-hydroxy-2-(2-methyl-4-[1,2,5]thiadiazol-3-yl-phenylamino)-benzamide; 2-[4-(4-chloro-[1,2,5]thiadiazol-3-yl)-2-methyl-phenylamino]-3,4,5-trifluoro-benzoic acid; 2-[4-(4-chloro-[1,2,5]thiadiazol-3-yl)-2-methylphenylamino]-N-cyclopropylmethoxy-3,4,5-trifluoro-benzamide; 2-[4-(4-chloro-[1,2,5]thiadiazol-3-yl)-2-methyl-phenylamino]-3,4,5-trifluoro-N-hydroxybenzamide;2-{4-[4-(2-dimethylamino-ethoxy)-[1,2,5]thiadiazol-3-yl]-2-methylphenylamino}-3,4,5-trifluoro-benzoic acid; N-cyclopropylmethoxy-3,4,5-trifluoro-2-{2-methyl-4-[4-(2-piperidin-1-yl-ethoxy)-[1,2,5]thiadiazol-3-yl]-phenylamino}-benzamide; and 3,4,5-trifluoro-N-hydroxy-2-{2-methyl-4-[4-(2-morpholin-4-yl-ethoxy)-[1,2,5]thiadiazol-3-yl]-phenylamino}-benzamide.

92. The use of claim 59, wherein said MEK inhibitor has a structure selected from: 5-bromo-2-(2-chloro-4-methylsulfanyl-phenylamino)-3,4-difluoro-benzoic acid; 2-(2-chloro-4-methanesulfinyl-phenylamino)-3,4-difluoro-benzoic acid; 2-(2-chloro-4-methanesulfonyl-phenylamino)-3,4,5-trifluoro-benzoic acid; 2-(2-chloro-methylsulfanyl-phenylamino)-3,4-difluorobenzoic acid; 5-bromo-2-(2-chloro-4-methanesulfonyl-phenylamino)-3,4-difluoro-benzoic acid; 2-(2-Chloro-4-methanesulfonyl-phenylamino)-3,4-difluoro-benzoic acid; 5-bromo-2-(2-chloro-4-methylsulfanyl-phenylamino)-N-cyclopropylmethoxy-3,4-difluoro-benzamide; 2-(2-chloro-4-methanesulfinylphenylamino)-N-cyclopropylmethoxy-3,4-difluoro-benzamide; 2-(2-chloro-4-methanesulfonyl-phenylamino)- N-cyclopropylmethoxy-3,4,5-trifluorobenzamide; 2-(2-chloro-4-methylsulfanyl-phenylamino)- N-cyclopropylmethoxy-3,4-difluoro-benzamide; 2-(2-chloro-4-methanesulfinyl-phenylamino)-N-cyclopropylmethoxy-3,4,5-trifluoro-benzamide; 5-bromo-2-(2-chloro-4-methanesulfonyl-phenylamino)-N-cyclopropylmethoxy-3,4-difluoro-benzamide;2-(2-chloro-4-methylsulfanylphenylamino)-N-cyclopropylmethoxy-3,4,5-trifluoro-benzamide; 2-(2-chloro-4-methanesulfonyl-phenylamino)-N-cyclopropylmethoxy-3,4-difluoro-benzamide; 2-[2-chloro 4-(3H-imidazol-1-yl)-phenylamino]-N-cyclopropylmethoxy-3,4-difluoro-benzamide; 2-(2-chloro-4-[1,2,5]thiadiazol-3-yl-phenylamino)-N-cyclopropylmethoxy-3,4,5-trifluoro-benzamide; 2-[4-(2-chloro-4-chloro-[1,2,5]thiadiazol-3-yl)-phenylamino]-3,4,5-trifluoro-benzoic acid; 2-[2-chloro-4-(4-chloro-[1,2,5]thiadiazol-3-yl)-phenylamino]-N-cyclopropylmethoxy-3,4,5-trifluoro-benzamide; 2-{4-[4-(2-dimethylamino-ethoxy)-[1,2,5]thiadiazol-3-yl]-2-methyl-phenylamino}-3,4,5-trifluoro-benzoic acid; and 2-{2-chloro-4-[4-(2-piperidin-1-yl-ethoxy)-[1,2,5]thiadiazol-3-yl]-phenylamino}-N-cyclopropylmethoxy-3,4,5-trifluoro-benzamide.

93. The use of claim 59, wherein said MEK inhibitor has a structure selected from: 2-(4-Ethynyl-2-methyl-phenylamino)-4-fluoro-benzoic acid; 5-Bromo-2-(4-ethynyl-2-methyl-phenylamino)-3,4-difluoro-benzoic acid; N-Cyclopropylmethoxy-2-(4-ethynyl-2-methyl-phenylamino)-3,4-difluorobenzamide; N-Cyclopropylmethoxy-2-(4-ethynyl-2-methyl-phenylamino)-4-nitro-Benzamide; 2-(4-Ethynyl-2-methyl-phenylamino)-3,4,5-trifluoro-N-hydroxy-benzamide; 2-(4-Ethynyl-2-methyl-phenylamino)-3,4-difluoro-benzoic acid; 2-(4-Ethynyl-2-methyl-phenylamino)-4-nitro-benzoic acid; N-Cyclopropylmethoxy-2-(4-ethynyl-2-methyl-phenylamino)-3,4,5-trifluorobenzamide; 4-Fluoro-N-hydroxy-2-(4-methanesulfinyl-2-methyl-phenylamino)-benzamide; 5-Bromo-2-(4-ethynyl-2-methyl-phenylamino)-3,4-difluoro-N-hydroxy-benzamide; 2-(4-Ethynyl-2-methyl-phenylamino)-3,4,5-trifluorobenzoic acid; N-Cyclopropylmethoxy-2-(4-ethynyl-2-methyl-phenylamino)-4-fluoro-benzamide; 5-Bromo-N-cyclopropylmethoxy-2-(4-ethynyl-2-methylphenylamino)-3,4-difluoro-benzamide; 2-(4-Ethynyl-2-methyl-phenylamino)-3,4-difluoro-N-hydroxy-benzamide; 2-(4-Ethynyl-2-methyl-phenylamino)-N-hydroxy-4-nitro-benzamide; 2-(4-Ethynyl-2-methyl-phenylamino)-4-fluorobenzoic acid; N-Cyclopropylmethoxy-2-(4-ethynyl-2-methyl-phenylamino)-4-fluoro-benzamide; and 4-Fluoro-N-hydroxy-2-(4-methanesulfinyl-2-methylphenylamino)-benzamide.

94. The use of claim 59, wherein said MEK inhibitor has a structure selected from: 2-(2-Chloro-4-ethynyl-phenylamino)-4-fluoro-benzoic acid; 5-Bromo-2-(2-chloro-4-ethynyl-phenylamino)-3,4-difluoro-benzoic acid; 2-(2-Chloro-4-ethynyl-phenylamino)-N-cyclopropylmethoxy-3,4-difluorobenzamide; 2-(2-Chloro-4-ethynyl-phenylamino)- N-cyclopropylmethoxy-4-nitro-benzamide; 2-(2-Chloro-4-ethynyl-phenylamino)-N-hydroxy-3,4,5-trifluoro- benzamide; 2-(2-Chloro-4-ethynyl-phenylamino)-3,4-difluoro-benzoic ethynyl-phenylamino)- N-Cyclopropylmethoxy-3,4,5-trifluoro-benzamide; 2-(2-ethynyl-phenylamino)-N-Cyclopropylmethoxy-3,4,5-trifluoro-benzamide; 2-(2-chloro-4-methanesulfinyl-phenylamino)- 4-fluoro-N-hydroxy-benzamide; 5-Bromo-2-(4-ethynyl-2-chloro-phenylamino)-3,4-difluoro-N-hydroxy-benzamide; 2-(2-Chloro-4-ethynyl-phenylamino)-3,4,5-trifluoro-benzoic acid; 2-(2-Chloro-4-ethynyl-phenylamino)-N-cyclopropylmethoxy-4-fluoro-benzamide; 5-Bromo-2-(2-chloro-4-ethynyl-phenylamino)-N-cyclopropylmethoxy-3,4-difluorobenzamide; 2-(4-Ethynyl-2-chloro-phenylamino)-3,4-difluoro-N-hydroxybenzamide; 2-(4-Ethynyl-2-chloro-phenylamino)-N-hydroxy-4-nitrobenzamide; 2-(2-Chloro-4-ethynyl-phenylamino)-4-fluoro-benzoic acid; 2-(2-Chloro-4-ethynyl-phenylamino)- N-cyclopropylmethoxy-4-fluoro-benzamide; 2-(2-Chloro-4-methanesulfinyl-phenylamino)-4-fluoro-N-hydroxy-benzamide; and 2-(2-chloro-4-imidazol-1-yl-phenylamino)- 3,4-Difluoro-benzoic acid.

95. Use of a MEK inhibitor selected from a compound of formula (I)C: 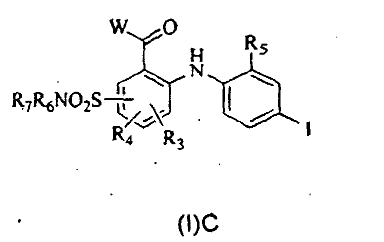 wherein
W is OR₁, NR₂OR₁, NR_{A}R_{B}, NR₂NR_{A}R_{B}, or NR₂(CH₂)₂₋₄ NR_{A}R_{B};
R₁ is H, C ₁₋₈ alkyl, C ₃₋₈ alkenyl, C ₃₋₈ alkynyl, C ₃₋₈ cycloalkyl, phenyl, (phenyl)C ₁₋₄ alkyl, (phenyl)C ₃₋₄ alkenyl, (phenyl)C ₃₋₄ alkynyl, (C ₃₋₈ cycloalkyl)-C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (C ₃₋₈ heterocyclic radical)C ₃₋₄ alkenyl, (C ₃₋₈ heterocyclic radical)C ₃₋₄ alkynyl or (CH₂)₂₋₄NR_{A}R_{B};
R₂ is H, phenyl, C ₁₋₄ alkyl, C₃₋₄ alkenyl, C ₃₋₈ alkynyl, C ₃₋₈ cycloalkyl, or (C ₃₋₈ cycloalkyl)-C ₁₋₄ alkyl;
R_{A} is H, C ₁₋₆ alkyl, C ₃₋₈ alkenyl, C ₃₋₈ alkynyl, C ₃₋₈ cycloalkyl, phenyl, (C ₃₋₈ cycloalkyl)C ₁₋₄ alkyl, (C ₃₋₈ cycloalkyl)C ₃₋₄ alkenyl, (C ₃₋₈ cycloalkyl)C ₃₋₄ alkynyl, C ₃₋₈ heterocyclic radical, (C ₃₋₈ heterocyclic radical)C ₁₋₄ alkyl, (aminosulfonyl)phenyl, [(aminosulfonyl)phenyl]C ₁₋₄ alkyl, (aminosulfonyl)C ₁₋₆ alkyl, (aminosulfonyl)C ₃₋₆ cycloalkyl, or [(aminosulfonyl)C ₃₋₆ cycloalkyl]C ₁₋₄ alkyl;
R_{B} is H, C ₁₋₈ alkyl, C ₃₋₈ alkenyl, C ₃₋₈ alkynyl, C ₃₋₈ cycloalkyl, or C ₆₋₈ aryl;
R₃ is H, F, Cl, Br, or NO₂;
R₄ is H or F;
R₅ is H, methyl or Cl;
R₆ is H, C ₁₋₄ alkyl, hydroxyethyl, hydroxypropyl, (CH₂)₂₋₄(NR_{C}R_{D}), phenyl, 2-pyridyl, 3-pyridyl, 4-pyridyl or CH₂Ar, where Ar is phenyl, 2-pyridyl, 3-pyridyl, or 4-pyridyl;
R₇ is H, C ₁₋₄ alkyl, hydroxyethyl, hydroxypropyl, (CH₂)₂₋₄(NR_{C}R_{D}), phenyl, 2-pyridyl, 3-pyridyl, 4-pyridyl, or CH₂Ar , where Ar is phenyl, 2-pyridyl, 3-pyridyl, or 4-pyridyl;
each of R_{C} and R_{D} is independently selected from H, C ₁₋₆ alkyl, C ₃₋₄ alkenyl, C ₃₋₄ alkynyl, C ₃₋₆ cycloalkyl, C ₃₋₆ heterocyclic radical, and phenyl; NR_{C}R_{D} can also be selected from morpholinyl, piperazinyl, pyrrolidinyl, or piperadinyl;
wherein each hydrocarbon radical or heterocyclic radical above is optionally substituted with between 1 and 3 substituents independently selected from halo, C ₁₋₄ alkyl, C ₃₋₆ cycloalkyl, C ₂₋₄ alkenyl, C ₂₋₄ alkynyl, phenyl, hydroxy, amino, (amino)sulfonyl, and NO₂, wherein each substituent alkyl, cycloalkyl, alkenyl, alkynyl or phenyl is in turn optionally substituted with between 1 and 3 substituents independently selected from halo, C ₁₋₂ alkyl, hydroxy, amino, and NO₂;
or a pharmaceutically-acceptable salt or C ₁₋₆ ester thereof for the manufacturing of pharmaceuticals for the treatment of chronic pain,

96. The use of claim 95, wherein said chronic pain is selected from neuropathic pain, idiopathic pain, and pain associated with chronic alcoholism, vitamin deficiency, uremia, or hypothyroidism.

97. The use of claim 96, wherein said chronic pain is type of neuropathic pain.

98. The use of claim 97, wherein said neuropathic pain is associated with one of the following: inflammation, postoperative pain, phantom limb pain, burn pain, gout, trigeminal neuralgia, acute herpetic and postherpetic pain, causalgia, diabetic neuropathy, plexus avulsion, neuroma, vasculitis, viral infection, crush injury, constriction injury, tissue injury, limb amputation, post-operative pain, arthritis pain, and any other nerve injury between the peripheral nervous system and the central nervous system, inclusively.

99. The use of claim 96, wherein said chronic pain is associated with chronic alcoholism, vitamin deficiency, uremia, or hypothyroidism.

100. The use of claim 96, wherein said chronic pain is associated with idiopathic pain.

101. The use of claim 95, wherein said chronic pain is associated with inflammation.

102. The use of claim 95, wherein said chronic pain is associated with arthritis.

103. The use of claim 95, wherein said chronic pain is associated with post-operative pain.

104. A use of claim 95, wherein the sulfamoyl group is *meta* to W(CO)- and *para* to the bridging NH.

105. A use of claim 95, wherein the sulfamoyl group is *para* to W (CO)- and *meta* to the bridging NH.

106. A use of claim 95, wherein R₄ is fluoro.

107. A use of claim 95, where R₃ is fluoro.

108. A use of claim 95, where R₃ is H.

109. A use of claim 95, wherein W is OH.

110. A use of claim 95, wherein W is NR₂OR₁.

111. A use of claim 109, wherein each of R₃ and R₄ is fluoro.

112. A use of claim 95, wherein R₁ has at least one hydroxy substituent.

113. A use of claim 95, wherein R₁ is H, methyl, ethyl, propyl, isopropyl, isobutyl, benzyl, phenethyl, allyl, C ₃₋₅ alkenyl, C ₃₋₅ alkynyl, C ₃₋₆ cycloalkyl, (C ₃₋₅ cycloalkyl)C ₁₋₂ alkyl, or (C ₃₋₅ heterocyclic radical)-C ₁₋₂ alkyl.

114. A use of claim 113, wherein R₁ is H or (C ₃₋₄ cycloalkyl)-C ₁₋₂ alkyl.

115. A use of claim 95, wherein R₂ is H, methyl, C ₃₋₄ alkynyl, C ₃₋ ₅ cycloalkyl, or (C ₃₋₅ cycloalkyl)methyl.

116. A use of claim 95, wherein R_{A} is H, methyl, ethyl, isobutyl, hydroxyethyl, hydroxypropyl, cyclopropylmethyl, cyclobutylmethyl, C ₃₋₄ alkynyl, phenyl, 2-piperidin-1-yl-ethyl, 2,3-dihydroxy-propyl, 3-[4-(2-hydroxyethyl)-piperazin-1-yl]-propyl, 2-pyrrolidin-1-yl-ethyl, or 2-diethylamino-ethyl; and R_{B} is H; or where R_{B} is methyl and R_{A} is phenyl.

117. A use of claim 95, wherein R₇ is (CH₂)₂₋₄(NR_{C}R_{D}).

118. A use of claim 95, wherein NR_{C}R_{D} is selected from morpholinyl, piperazinyl, pyrrolidinyl, or piperadinyl.

119. A use of claim 95, wherein R₅ is methyl or chloro.

120. A use of claim 95, wherein said MEK inhibitor has a structure selected from: 2-(2-chloro-4-iodo-phenylamino)-4-sulfamoyl-benzoic acid; 2-(2-chloro-4-iodo-phenylamino)-N-hydroxy-4-sulfamoyl-benzamide; 2-(2-chloro-4-iodo-phenylamino)-N-cyclopropylmethoxy-4-sulfamoyl-benzamide; 2-(2-chloro-4-iodo-phenylamino)-4-(2-morpholin-4-yl-ethylsulfamoyl)-benzoic acid; 2-(2-chloro-4-iodo-phenylamino)-N-hydroxy-4-(2-morpholin-4-yl-ethylsulfamoyl)-benzamide; 2-(2-chloro-4-iodo-phenylamino)-N-cyclopropylmethoxy-4-(2-morpholin-4-yl-ethylsulfamoyl)-benzamice; 2-(2-chloro-4-iodo-phenylamino)-3,4-difluoro-5-sulfamoyl-benzoic acid; 2-(2-chloro-4-iodo-phenylamino)-3,4-difluoro-N-hydroxy-5-sulfamoyl-benzamide; 2-(2-chloro-4-iodo-phenylamino)-N-cyclopropylmethoxy-3,4-difluoro-5-sulfamoylbenzamide; 2-(2-chloro-4-iodo-phenylamino)-3,4-difluoro-5-(2-morpholin-4-yl-ethylsulfamoyl)-benzoic acid; 2-(2-chloro-4-iodo-phenylamino)-3,4-difluoro-N-hydroxy-5-(2-morpholin-4-yl-ethylsulfamoyl)-benzamide; 2-(2-chloro-4-iodophenylamino)-N-cyclopropylmethoxy-3,4-difluoro-5-(2-morpholin-4-yl-ethylsulfamoyl)-benzamide; 5-(bis-pyridin-3-ylmethyl-sulfamoyl)-3,4-difluoro-2-(4-iodo-phenylamino)-benzoic acid; 5-(bis-pyridin-3-ylmethyl-sulfamoyl)-N-cyclopropylmethoxy-3,4-difluoro-2-(4-iodo-pheny)amino)-benzamide; N-cyclopropylmethoxy-3,4-difluoro-2-(4-iodo-phenylamino)-5-(methyl-pyridin-3-ylmethyl-sulfamoyl)-benzamide; N-cyclopropylmethoxy-3,4-difluoro-2-(4-iodophenylamino)-5-[(pyridin-3-yimethyl)-sulfamoyl]-benzamide; N-cyclopropylmethoxy-5-[(3-diethylamino-propyl)-pyridin-3-ylmethyl-sulfamoyl]-3,4-difluoro-2-(4-iodo-phenylamino)-benzamide; N-cyclopropylmethoxy-3,4-difluoro-5-[(3-hydroxy-propyl)-pyridin-3-ylmethyl-sulfamoyl]-2-(4-iodophenylamino)-benzamide; N-cyclopropylmethoxy-5-(ethyl-pyridin-3-ylmethylsulfamoyl)-3,4-difluoro-2-(4-iodo-phenylamino)-benzamide; N-cyclopropylmethoxy-3,4-difluoro-5-[(2-hydroxy-ethyl)-pyridin-3-ylmethylsulfamoyl]-2-(4-iodo-phenylamino)-benzamide; 5-(bis-pyridin-2-ylmethylsulfamoyl)-3,4-difluoro-2-(4-iodo-phenylamino)-benzoic acid; 5-(bis-pyridin-2-ylmethyl-sulfamoyl)-N-cyclopropylmethoxy-3,4-difluoro-2-(4-iodophenylamino)-benzamide; N-cyclopropylmethoxy-3,4-difluoro-2-(4-iodophenylamino)-5-(methyl-pyridin-2-ylmethyl-sulfamoyl)-benzamide; N-cyclopropylmethoxy-3,4-difluoro-2-(4-iodo-phenylamino)-5-[(pyridin-2-ylmethyl)-sulfamoyl]-benzamide; 5-(bis-pyridin-3-ylmethyl-sulfamoyl)-3,4-difluoro-2-(4-iodo-2-methyl-phenylamino)-benzoic acid; 5-(bis-pyridin-3-ylmethyl-sulfamoyl)-N-cyclopropylmethoxy-3,4-difluoro-2-(4-iodo-2-methylphenylamino)-benzamide; N-cyclopropylmethoxy-3,4-difluoro-2-(4-iodo-2-methyl-phenylamino)-5-(methyl-pyridin-3-ylmethyl-sulfamoyl)-benzamide; N-cyclopropylmethoxy-3,4-difluoro-2-(4-iodo-2-methyl-phenylamino)-5-[(pyridin-3-ylmethyl)-sulfamoyl]-benzamide; N-cyclopropylmethoxy-5-[(3-diethylaminopropyl)-pyridin-3-ylmethyl-sulfamoyl]-3,4-difluoro-2-(4-iodo-2-methylphenylamino)-benzamide; N-cyclopropylmethoxy-3,4-difluoro-5-[(3-hydroxypropyl)-pyridin-3-ylmethyl-sulfamoyl]-2-(4-iodo-2-methyl-phenylamino)-benzamide; N-cyclopropylmethoxy-5-(ethyl-pyridin-3-ylmethyl-sulfamoyl)-3,4-difluoro-2-(4-iodo-2-methyl-phenylamino)-benzamide; N-cyclopropylmethoxy-3,4-difluoro-5-[(2-hydroxy-ethyl)-pyridin-3-ylmethyl-sulfamoyl]-2-(4-iodo-2-methyl-phenylamino)-benzamide; 5-(bis-pyridin-2-ylmethyl-sulfamoyl)-3,4-difluoro-2-(4-iodo-2-methyl-phenylamino)-benzoic acid; 5-(bis-pyridin-2-ylmethyl-sulfamoyl)-N-cyclopropylmethoxy-3,4-difluoro-2-(4-iodo-2-methylphenylamino)-benzamide; N-cyclopropylmethoxy-3,4-difluoro-2-(4-iodo-2-methyl-phenylamino)-5-(methyl-pyridin-2-ylmethyl-sulfamoyl)-benzamide; N-cyclopropylmethoxy-3,4-difluoro-2-(4-iodo-2-methyl-phenylamino)-5-[(pyridin-2-ylmethyl)-sulfamoyl]-benzamide; 5-(bis-pyridin-3-ylmethyl-sulfamoyl)-2-(2-chloro-4-iodo-phenylamino)-3,4-difluoro-benzoic acid; 5-(bis-pyridin-3-ylmethyl-sulfamoyl)-2-(2-chloro-4-iodo-phenylamino)-N-cyclopropylmethoxy-3,4-difluoro-benzamide; 2-(2-chloro-4-iodo-phenylamino)-N-cyclopropylmethoxy-3,4-difluoro-5-(methyl-pyridin-3-ylmethyl-sulfamoyl)-benzamide; 2-(2-chloro-4-iodo-phenylamino)-N-cyclopropylmethoxy-3,4-difluoro-5-[(pyridin-3-ylmethyl)-sulfamoyl]-benzamide; 2-(2-chloro-4-iodophenylamino)-N-cyclopropylmethoxy-5-[(3-diethylamino-propyl)-pyridin-3-ylmethyl-sulfamoyl]-3,4-difluoro-benzamide; 2-(2-chloro-4-iodo-phenylamino)-N-cyclopropylmethoxy-3,4-difluoro-5-[(3-hydroxy-propyl)-pyridin-3-ylmethylsulfamoyl]-benzamide; 2-(2-chloro-4-iodo-phenylamino)-N-cyclopropylmethoxy-5-(ethyl-pyridin-3-ylmethyl-sulfamoyl)-3,4-difluorobenzamide; 2-(2-chloro-4-iodo-phenylamino)-N-cyclopropylmethoxy-3,4-difluoro-5-[(2-hydroxy-ethyl)-pyridin-3-ylmethyl-sulfamoyl]-benzamide; 5-(bis-pyridin-2-ylmethyl-sulfamoyl)-2-(2-chloro-4-iodo-phenylamino)-3,4-difluorobenzoic acid; 5-(bis-pyridin-2-ylmethyl-sulfamoyl)-2-(2-chloro-4-iodophenylamino)-N-cyclopropylmethoxy-3,4-difluoro-benzamide; 2-(2-chloro-4-iodo-phenylamino)-N-cyclopropylmethoxy-3,4-difluoro-5-(methyl-pyridin-2-ylmethyl-sulfamoyl)-benzamide; 2-(2-chloro-4-iodo-phenylamino)-N-methyl-phenylamino)-4-phenylsulfamoyl-benzamide; N-cyclopropylmethoxy-2-(4-iodo-2-methyl-phenylamino)-4-(pyridin-3-ylsulfamoyl)-benzamide; N-cyclopropylmethoxy-2-(4-iodo-2-methyl-phenylamino)-4-[(pyridin-3-ylmethyl)-sulfamoyl]-benzamide; 4-(bis-pyridin-3-ylmethyl-sulfamoyl)-N-cyclopropylmethoxy-2-(4-iodo-2-methyl-phenylamino)-benzamide; N-cyclopropylmethoxy-4-[(2-hydroxy-ethyl)-pyridin-4-ylmethyl-sulfamoyl]-2-(4-iodo-2-methyl-phenylamino)-benzamide; N-cyclopropylmethoxy-2-(4-iodo-2-methyl-phenylamino)-4-(methyl-pyridin-3-ylmethyl-sulfamoyl)-benzamide; N-cyclopropylmethoxy-4-[(3-diethylamino-propyl)-pyridin-3-ylmethyl-sulfamoyl]-2-(4-iodo-2-methyl-phenylamino)-benzamide; 2-(2-chloro-4-iodophenylamino)-N-cyclopropylmethoxy-4-phenylsulfamoyl-benzamide; 2-(2-chloro-4-iodo-phenylamino)-N-cyclopropylmethoxy-4-(pyridin-3-ylsulfamoyl)-benzamide; 2-(2-chloro-4-iodo-phenylamino)-N-cyclopropylmethoxy-4-[(pyridin-3-ylmethyl)-sulfamoyl]-benzamide; 4-(bis-pyridin-3-ylmethylsulfamoyl)-2-(2-chloro-4-iodo-phenylamino)-N-cyclopropylmethoxybenzamide;2-(2-chloro-4-iodo-phenylamino)-N-cyclopropy)methoxy-4-[(2-hydroxy-ethyl)-pyridin-4-ylmethyl-sulfamoyl]-benzamide; 2-(2-chloro-4-iodophenylamino)-N-cyclopropylmethoxy-4-(methyl-pyridin-3-ylmethyl-sulfamoyl)-benzamide; 2-(2-chloro-4-iodo-phenylamino)-N-cyclopropylmethoxy-4-[(3-diethylamino-propyl)-pyridin-3-ylmethyl-sulfamoyl]-benzamide; and 5-[bis-(4-methoxy-benzyl)-sulfamoyl]-2-(2-chloro-4-iodo-phenylamino)-3,4-difluorobenzoic acid; and 2-(2-chloro-4-iodo-phenylamino)-5-dimethylsulfamoyl-3,4-difluoro-benzoic acid methyl ester.

121. The use of claim 95, wherein said MEK inhibitor has a structure selected from: PD 298458, N-Allyloxy-2-(2-chloro-4-iodophenylamino)-3,4-difluoro-5-(4-methyl-piperazine-1-sulfonyl)-benzamide; PD 298459, N-Allyloxy-2-(2-chloro-4-iodo-phenylamino)-3,4-difluoro-5-(methylphenyl-sulfamoyl)-benzamide; PD 298460, 5-(Allyl-methyl-sulfamoyl)-N-allyloxy-2-(2-chloro-4-iodo-phenylamino)-3,4-difluoro-benzamide; PD 298463, 1-[5-Allyloxycarbamoyl-4-(2-chloro-4-iodo-phenylamino)-2,3-difluorobenzenesulfonyl]-piperidine-3-carboxylic acid amide; PD 298464, N-Allyloxy-2-(2-chloro-4-iodo-phenylamino)-5-[(3-dimethylamino-propyl)-methylsulfamoyl]-3,4-difluoro-benzamide; PD 298465, N-Allyloxy-2-(2-chloro-4-iodophenylamino)-3,4-difluoro-5-(4-pyridin-2-yl-piperazine-1-sulfonyl)-benzamide; and PD 298467, N-Allyloxy-2-(2-chloro-4-iodo-phenylamino)-3,4-difluoro-5-(methoxy-methyl-sulfamoyl)-benzamide.

122. The use of claim 1, wherein said MEK inhibitor has a structure selected from: 2-(2-chloro-4-iodo-phenylamino)-*N*-cyclopropylmethoxy-3,4-difluoro-benzenesulfonamide; and 2-(2-chloro-4-iodo-phenylamino)-cycloproplmethoxy-3,4-difluoro-benzenesulfonamide.

123. The use of claim 27, wherein said MEK inhibitor has a structure selected from: 2,4-bis-(2-chloro-4-iodo-phenylamino)-3-fluoro-5-nitro-benzoic acid.

124. The use of claim 59, wherein said MEK inhibitor has a structure selected from: 2-(4-ethynyl-2-methyl-phenylamino)-4-fluoro-benzoic acid; and 2-(3',5'-dichloro-biphenyl-4-ylamino)-benzoic acid.

125. The use of claim 95, wherein said MEK inhibitor has a structure selected from: 2-(2-chloro-4-iodo-phenylamino)-*N*-cyclopropylmethoxy-3,4-difluoro-5-sulfamoyl-benzamide; 2-(2-chloro-4-iodo-phenylamino)-3,4-difluoro-*N*-hydroxy-5-sulfamoyl-benzamide; *C*-(2-chloro-4-iodo-phenylamino)-*N*-cyclopropylmethoxy-dimethylsulfamoyl-difluoro-benzamide; *N*-cyclopropylmethoxy-dimethylsulfamoyl-difluoro-*C*-(4-iodo-2-methylphenylamino)-benzamide; and *C*-(2-chloro-4-iodo-phenylamino)-difluoro(methoxy-methyl-sulfamoyl)-*N*-(2-morpholin-4-yl-ethoxy)benzamide

## Patentansprüche

1. Verwendung eines MEK-Inhibitors, ausgewählt aus: einer Verbindung der Formel (I): 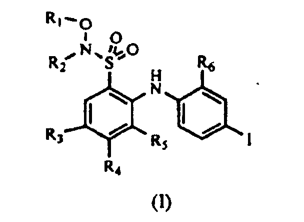
R₁ ist H, C₁₋₈-Alkyl, C₃₋₈-Alkenyl, C₃₋₈-Alkinyl, C₃₋₈-Cycloalkyl, Phenyl, (Phenyl)-C₁₋₄-alkyl, (Phenyl)-C₃₋₄-alkenyl, (Phenyl)-C₃₋₄-alkinyl, (C₃₋₈-Cycloalkyl)-C₁₋₄-alkyl, (C₃₋₈-Cycloalkyl)-C₃₋₄-alkenyl, (C₃₋₈-Cycloalkyl)-C₃₋₄-alkinyl, C₃₋₈-heterocyclischer Rest, (C₃₋₈-heterocyclischer Rest)-C₁₋₄-alkyl, (C₃₋₈heterocyclischer Rest)-C₃₋₄-alkenyl, (C₃₋₈-heterocyclischer Rest) -C₃₋₄-alkinyl, (CH₂)₂₋₄OR_{C} oder, (CH₂)₂₋₄NR_{C}R_{D};
R₂ ist H, C₁₋₄-Alkyl, Phenyl, C₃₋₆-Cycloalkyl, C₃₋₆-heterocyclischer Rest oder (C₃₋₆-Cycloalkyl)methyl;
jeder von R₃ und R₄ ist unabhängig ausgewählt aus H, F, NO₂, Br und Cl;
R₅ ist ausgewählt aus H und F;
R₆ ist H, F, Cl oder CH₃;
jeder von R_{C} und R_{D} ist unabhängig ausgewählt aus H, C₁₋₄-Alkyl, C₃₋₄-Alkenyl, C₃₋₄-Alkinyl, C₃₋₆-Cycloalkyl und Phenyl; oder NR_{C}R_{D} kann ein Piperidino-, Morpholino- oder N- (C₁₋₆-Alkyl)piperazinoring sein;
worin jeder vorstehend genannte Kohlenwasserstoffrest gegebenenfalls mit zwischen 1 und 3 Substituenten, unabhängig ausgewählt aus Halogen, Hydroxyl, Amino, (Amino)sulfonyl und NO₂, substituiert ist; und
worin jeder vorstehend genannte heterocyclische Rest gegebenenfalls mit zwischen 1 und 3 Substituenten, unabhängig ausgewählt aus Halogen, C₁₋₄-Alkyl, C₃₋₆-Cycloalkyl, C₃₋₄-Alkenyl, C₃₋₄-Alkinyl, Phenyl, Hydroxyl, Amino, (Amino)sulfonyl und NO₂, substituiert ist, worin jeder Substituent Alkyl, Cycloalkyl, Alkenyl, Alkinyl oder Phenyl wiederum gegebenenfalls mit zwischen 1 und 2 Substituenten, unabhängig ausgewählt aus Halogen, C₁₋₂-Alkyl, Hydroxyl, Amino und NO₂, substituiert ist;
oder einem pharmazeutisch verträglichen Salz oder C₁₋₈-Ester davon, zur Herstellung von Arzneimitteln für die Behandlung von chronischem Schmerz.

2. Verwendung nach Anspruch 1, wobei der chronische Schmerz ausgewählt ist aus neuropathischem Schmerz, idiopathischem Schmerz und Schmerz verbunden mit chronischem A1-koholismus, Vitaminmangel, Urämie oder Hypothyroidismus.

3. Verwendung nach Anspruch 2, wobei der chronische Schmerz eine Art von neuropathischem Schmerz ist.

4. Verwendung nach Anspruch 3, wobei der neuropathische Schmerz mit einem von den Nachstehenden verbunden ist: Entzündung, postoperativer Schmerz, Phantomgliederschmerz, Verbrennungsschmerz, Gicht, trigeminale Neuralgie, akuter herpetischer und postherpetischer Schmerz, Causalgie, diabetische Neuropathie, Plexusavulsion, Neurom, Vaskulitis, virale Infektion, Quetschverletzung, Konstriktionsschädigung, Gewebsschädigung, Gliedmaßenamputation, postoperativer Schmerz, Arthritisschmerz und jede andere Nervenschädigung zwischen dem peripheren Nervensystem und einschließlich des zentralen Nervensystems.

5. Verwendung nach Anspruch 2, wobei der chronische Schmerz mit chronischem Alkoholismus, Vitaminmangel, Urämie oder Hypothyroidismus verbunden ist.

6. Verwendung nach Anspruch 2, wobei der chronische Schmerz mit idiopathischem Schmerz verbunden ist.

7. Verwendung nach Anspruch 1, wobei der chronische Schmerz mit Entzündung verbunden ist.

8. Verwendung nach Anspruch 1, wobei der chronische Schmerz mit Arthritis verbunden ist.

9. Verwendung nach Anspruch 1, wobei der chronische Schmerz mit postoperativem Schmerz verbunden ist.

10. Verwendung nach Anspruch 1, wobei R₃ Brom oder Chlor darstellt.

11. Verwendung nach Anspruch 1, wobei R₄ Fluor darstellt.

12. Verwendung nach Anspruch 1, wobei R₅ H darstellt.

13. Verwendung nach Anspruch 12, wobei jeder von R₄ und R₅ H darstellt.

14. Verwendung nach Anspruch 1, wobei jeder von R₄ und R₅ Fluor darstellt.

15. Verwendung nach Anspruch 14, wobei R₃ Brom darstellt.

16. Verwendung nach Anspruch 14, wobei R₃ Fluor darstellt.

17. Verwendung nach Anspruch 1, wobei R₄ Nitro darstellt.

18. Verwendung nach Anspruch 16, wobei R₅ H darstellt.

19. Verwendung nach Anspruch 1, wobei R₆ Chlor darstellt.

20. Verwendung nach Anspruch 1, wobei R₆ Methyl darstellt.

21. Verwendung nach Anspruch 1, wobei R₁ H oder C₁₋₄-Alkyl darstellt und R₂ H darstellt.

22. Verwendung nach Anspruch 1, wobei R₁ (C₃₋₆-Cycloalkyl)methyl darstellt.

23. Verwendung nach Anspruch 1, wobei R₁ H darstellt.

24. Verwendung nach Anspruch 1, wobei R₁ (CH₂)₂₋₄OR_{C} oder (CH₂)₂₋₄NR_{C}R_{D} darstellt.

25. Verwendung nach Anspruch 1, wobei der MEK-Inhibitor eine Struktur aufweist, ausgewählt aus: 4-Fluor-2-(4-jod-2-methylphenylamino)benzolsulfonsäure, 4-Fluor-N-hydroxy-2-(4-jod-2-methylphenylamino)benzolsulfonamid, N-Cyclopropylmethoxy-4-fluor-2-(4-jod-2-methylphenylamino)benzolsulfonamid, 3,4-Difluor-2-(4-jod-2-methylphenylamino)benzolsulfonsäure, 3,4-Difluor-N-hydroxy-2-(4-jod-2-methylphenylamino)-benzolsulfonamid, N-Cyclopropylmethoxy-3,4-difluor-2-(4-jod-2-methylphenylamino)benzolsulfonamid, 3,4,5-Trifluor-2-(4-jod-2-methylphenylamino)benzolsulfonsäure, 3,4,5-Trifluor-N-hydroxy-2-(4-jod-2-methylphenylamino)benzolsulfonamid, N-Cyclopropylmethoxy-3,4,5-trifluor-2-(4-jod-2-methylphenylamino)benzolsulfonamid, 5-Brom-3,4-difluor-2-(4-jod-2-methylphenylamino)benzolsulfonsäure, 5-Brom-3,4-difluor-N-hydroxy-2-(4-jod-2-methylphenylamino)benzolsulfonamid, 5-Brom-N-cyclopropylmethoxy-3,4-difluor-2-(4-jod-2-methylphenylamino)benzolsulfonamid, 2-(4-Jod-2-methylphenylamino)-4-nitrobenzolsulfonsäure, N-Hydroxy-2-(4-jod-2-methylphenylamino)-4-nitrobenzolsulfonamid und N-Cyclopropylmethoxy-2-(4-jod-2-methylphenylamino)-4-nitrobenzolsulfonamid.

26. Verwendung nach Anspruch 1, wobei der MEK-Inhibitor eine Struktur aufweist, ausgewählt aus: 2-(2-Chlor-4-jodphenylamino)-4-fluorbenzolsulfonsäure, 2-(2-Chlor-4-jodphenylamino)-4-fluor-N-hydroxybenzolsulfonamid, 2-(2-Chlor-4-jodphenylamino)-N-cyclopropylmethoxy-4-fluorbenzolsulfonamid, 2-(2-Chlor-4-jodphenylamino)-3,4-difluorbenzolsulfonsäure, 2-(2-Chlor-4-jodphenylamino)-3,4-difluor-N-hydroxybenzolsulfonamid, 2-(2-Chlor-4-jodphenylamino)-N-cyclopropylmethoxy-3,4-difluorbenzolsulfonamid, 2-(2-Chlor-4-jodphenylamino)-3,4,5-trifluorbenzolsulfonsäure, 2-(2-Chlor-4-jodphenylamino)-3,4,5-trifluor-N-hydroxybenzolsulfonamid, 2-(2-Chlor-4-jodphenylamino)-N-cyclopropylmethoxy-3,4,5-trifluorbenzolsulfonamid, 5-Brom-2-(2-chlor-4-jodphenylamino)-3,4-difluorbenzolsulfonsäure, 5-Brom-2-(2-Chlor-4-jodphenylamino)-3,4-difluor-N-hydroxybenzolsulfonamid, 5-Brom-2-(2-chlor-4-jodphenylamino)-N-cyclopropylmethoxy-3,4-difluorbenzolsulfonamid, 2-(2-Chlor-4-jodphenylamino)-4-nitrobenzolsulfonsäure, 2-(2-Chlor-4-jodphenylamino)-N-hydroxy-4-nitrobenzolsulfonamid und 2-(2-Chlor-4-jodphenylamino)-N-cyclopropylmethoxy-4-nitrobenzolsulfonamid.

27. Verwendung eines MEK-Inhibitors, ausgewählt aus einer Verbindung mit der Formel (II)A 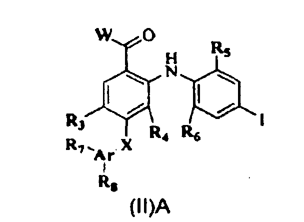
W ist OR₁, NR₂OR₁, NR_{A}R_{B}, NR₂NR_{A}R_{B} oder NR₂ (CH₂)₂₋₄NR_{A}R_{B};
R₁ ist H, C₁₋₈-Alkyl, C₃₋₈-Alkenyl, C₃₋₈-Alkinyl, C₃₋₈-Cycloalkyl, Phenyl, (Phenyl)C₁₋₄-alkyl, (Phenyl)C₃₋₄-alkenyl, (Phenyl)C₃₋₄-alkinyl, (C₃₋₈-Cycloalkyl)C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischer Rest, (C₃₋₈-heterocyclischer Rest)C₁₋₄-alkyl, (C₃₋₈heterocyclischer Rest)C₃₋₄-alkenyl, (C₃₋₈-heterocyclischer Rest) C₃₋₄-alkinyl oder (CH₂)₂₋₄NR_{A}R_{B};
R₂ ist H, Phenyl, C₁₋₄-Alkyl, C₃₋₄-Alkenyl, C₃₋₈-Alkinyl, C₃₋₈-Cycloalkyl oder (C₃₋₈-Cycloalkyl)C₁₋₄-alkyl;
R_{A} ist H, C₁₋₆-Alkyl, C₃₋₈-Alkenyl, C₃₋₈-Alkinyl, C₃₋₈-Cycloalkyl, Phenyl, (C₃₋₈-Cycloalkyl)C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischer Rest, (C₃₋₈-heterocyclischer Rest)C₁₋₄-alkyl, (Aminosulfonyl)phenyl, [(Aminosulfonyl)phenyl]C₁₋₄-alkyl, (Aminosulfonyl)C₁₋₆-alkyl, (Aminosulfonyl)C₃₋₆-cycloalkyl oder [(Aminosulfonyl)C₃₋₆-cycloalkyl]C₁₋₄-alkyl;
R_{B} ist H, C₁₋₈-Alkyl, C₃₋₈-Alkenyl, C₃₋₈-Alkinyl, C₃₋₈-Cycloalkyl oder C₆₋₈-Aryl;
R₃ ist Halogen, NO₂, SO₂NR_{I}(CH₂)₂₋₄NR_{E}R_{F}, SO₂NR_{I}R_{K} oder (CO)T;
T ist C₁₋₈-Alkyl, C₃₋₈-Cycloalkyl, (NR_{E}R_{F})C₁₋₄-alkyl, OR_{F}, NR_{I}(CH₂)₂₋₄NR_{E}R_{F} oder NR_{E}R_{F};
R₄ ist H oder F;
R₅ ist H, Methyl, Halogen oder NO₂;
R₆ ist H, Methyl, Halogen oder NO₂;
Ar ist Phenyl, 2-Pyridyl, 3-Pyridyl oder 4-Pyridyl;
wobei jeder von R₇ und R₈ unabhängig ausgewählt ist aus H, Halogen, C₁₋₄-Alkyl, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H} und (CO)NR_{G}R_{H}; mit der Maßgabe, dass, wenn Ar ein Pyridyl darstellt, jeder von R₇ und R₈ H ist;
jeder von R_{C}, R_{D}, R_{E}, R_{F}, R_{G} und R_{H} unabhängig ausgewählt ist aus H, C₁₋₄-Alkyl, C₃₋₄-Alkenyl, C₃₋₄-Alkinyl, C₃₋₆-Cycloalkyl und Phenyl; wobei jeder von NR_{C}R_{D}, NR_{E}R_{F} und NR_{G}R_{H} auch unabhängig Morpholinyl, Piperazinyl, Pyrrolidinyl oder Piperadinyl sein kann;
jeder von R_{I} und R_{J} unabhängig H, Methyl oder Ethyl darstellt;
R_{K} C₁₋₄-Alkyl, C₃₋₄-Alkenyl, C₃₋₄-Alkinyl, C₃₋₆-Cycloalkyl oder Phenyl darstellt;
X O, S oder NH darstellt und
worin jeder vorstehend genannte Kohlenwasserstoffrest oder heterocyclische Rest gegebenenfalls mit zwischen 1 und 3 Substituenten, unabhängig ausgewählt aus Halogen, C₁₋₄-Alkyl, C₃₋₆-Cycloalkyl, C₂₋₄-Alkenyl, C₂₋₄-Alkinyl, Phenyl, Hydroxyl, Amino, (Amino)sulfonyl und NO₂, substituiert ist, worin jeder Substituent Alkyl, Cycloalkyl, Alkenyl, Alkinyl oder Phenyl wiederum gegebenenfalls mit zwischen 1 und 2 Substituenten, unabhängig ausgewählt aus Halogen, C₁-C₂-Alkyl, Hydroxyl, Amino und NO₂, substituiert ist;
oder einem pharmazeutisch verträglichen Salz oder C₁₋₇-Ester davon, zur Herstellung von Arzneimitteln für die Behandlung von chronischem Schmerz.

28. Verwendung nach Anspruch 27, wobei der chronische Schmerz ausgewählt ist aus neuropathischem Schmerz, idiopathischem Schmerz und Schmerz verbunden mit chronischem Alkoholismus, Vitaminmangel, Urämie oder Hypothyroidismus.

29. Verwendung nach Anspruch 28, wobei der chronische Schmerz eine Art neuropathischer Schmerz ist.

30. Verwendung nach Anspruch 29, wobei der neuropathische Schmerz verbunden ist mit einem von den Nachstehenden: Entzündung, postoperativer Schmerz, Phantomgliederschmerz, Verbrennungsschmerz, Gicht, trigeminale Neuralgie, akuter herpetischer und postherpetischer Schmerz, Causalgie, diabetische Neuropathie, Plexusavulsion, Neurom, Vaskulitis, virale Infektion, Quetschverletzung, Konstriktionsschädigung, Gewebsschädigung, Gliedmaßenamputation, postoperativer Schmerz, Arthritisschmerz und jede andere Nervenschädigung zwischen dem peripheren Nervensystem und einschließlich des zentralen Nervensystems.

31. Verwendung nach Anspruch 28, wobei der chronische Schmerz mit chronischem Alkoholismus, Vitaminmangel, Urämie oder Hypothyroidismus verbunden ist.

32. Verwendung nach Anspruch 28, wobei der chronische Schmerz mit idiopathischem Schmerz verbunden ist.

33. Verwendung nach Anspruch 27, wobei der chronische Schmerz mit Entzündung verbunden ist.

34. Verwendung nach Anspruch 27, wobei der chronische Schmerz mit Arthritis verbunden ist.

35. Verwendung nach Anspruch 27, wobei der chronische Schmerz mit postoperativem Schmerz verbunden ist.

36. Verwendung nach Anspruch 27 mit der nachstehenden Formel (I)A: 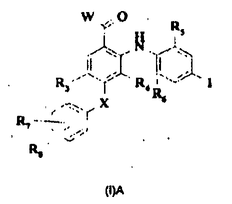 worin
W OR₁, NR₂OR₁, NR_{A}R_{B}, NR₂NR_{A}R_{B} oder NR₂(CH₂)₂₋₄NR_{A}R_{B} darstellt;
R₁ H, C₁₋₈-Alkyl, C₃₋₈-Alkenyl, C₃₋₈-Alkinyl, C₃₋₈-Cycloalkyl, Phenyl, (Phenyl)C₁₋₄-alkyl, (Phenyl)C₃₋₄-alkenyl, (Phenyl)C₃₋₄-alkinyl, (C₃₋₈-Cycloalkyl)C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (C₃₋₈-heterocyclischen Rest)C₃₋₄-alkenyl, (C₃₋₈-heterocyclischen Rest)-C₃₋₄-alkinyl oder (CH₂)₂₋₄NR_{A}R_{B} darstellt;
R₂ H, Phenyl, C₁₋₄-Alkyl, C₃₋₄-Alkenyl, C₃₋₈-Alkinyl, C₃₋₈-Cycloalkyl oder (C₃₋₈-Cycloalkyl)C₁₋₄-alkyl darstellt;
R_{A} H, C₁₋₆-Alkyl, C₃₋₈-Alkenyl, C₃₋₈-Alkinyl, C₃₋₈-Cycloalkyl, Phenyl, (C₃₋₈-Cycloalkyl)C₁₋₄-alkyl, (C₃₋₈-Cycloalkyl)-C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (Aminosulfonyl)phenyl, [(Aminosulfonyl)phenyl]C₁₋₄-alkyl, (Aminosulfonyl)C₁₋₆-alkyl, (Aminosulfonyl)C₃₋₆-cycloalkyl oder [(Aminosulfonyl)C₃₋₆-cycloalkyl]C₁₋₄-alkyl darstellt;
R_{B} H, C₁₋₈-Alkyl, C₃₋₈-Alkenyl, C₃₋₈-Alkinyl, C₃₋₈-Cycloalkyl oder C₆₋₈-Aryl darstellt;
R₃ Halogen, NO₂, SO₂NR_{I}(CH₂)₂₋₄NR_{E}R_{F}, SO₂NR_{I}R_{K} oder (CO)T darstellt;
T C₁₋₈-Alkyl, C₃₋₈-Cycloalkyl, (NR_{E}R_{F})C₁₋₄-Alkyl, OR_{F}, NR_{I}(CH₂)₂₋₄NR_{E}R_{F} oder NR_{E}R_{F} darstellt;
R₄ H oder F darstellt;
R₅ H, Methyl, Halogen oder NO₂ darstellt;
R₆ H, Methyl, Halogen oder NO₂ darstellt;
jeder von R₇ und R₈ unabhängig ausgewählt ist aus H, Halogen, C₁₋₄-Alkyl, SO₂NR_{J}(CH₂)₂₋₄NR_{G}R_{H}, (CO) (CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H} und (CO)NR_{G}R_{H};
jeder von R_{C}, R_{D}, R_{E}, R_{F}, R_{G} und R_{H} unabhängig ausgewählt ist aus H, C₁₋₄-Alkyl, C₃₋₄-Alkenyl, C₃₋₄-Alkinyl, C₃₋₆-Cycloalkyl und Phenyl; wobei jeder von NR_{C}R_{D}, NR_{E}R_{F} und NR_{G}R_{H} auch unabhängig Morpholinyl, Piperazinyl, Pyrrolidinyl oder Piperadinyl sein kann;
jeder von R₁ und R_{J} unabhängig H, Methyl oder Ethyl darstellt;
R_{K} C₁₋₄-Alkyl, C₃₋₄-Alkenyl, C₃₋₄-Alkinyl, C₃₋₆-Cycloalkyl oder Phenyl darstellt;
X O, S oder NH darstellt und
worin jeder vorstehend genannte Kohlenwasserstoffrest oder heterocyclische Rest gegebenenfalls mit zwischen 1 und 3 Substituenten, unabhängig ausgewählt aus Halogen, C₁₋₄-Alkyl, C₃₋₆-Cycloalkyl, C₂₋₄-Alkenyl, C₂₋₄-Alkinyl, Phenyl, Hydroxyl, Amino, (Amino)sulfonyl und NO₂ substituiert ist, worin jeder Substituent Alkyl, Cycloalkyl, Alkenyl, Alkinyl oder Phenyl, wiederum gegebenenfalls mit zwischen 1 und 2 Substituenten, unabhängig ausgewählt aus Halogen, C₁₋₂-Alkyl, Hydroxyl, Amino und NO₂, substituiert ist;
oder ein pharmazeutisch verträgliches Salz oder C₁₋₇-Ester davon.

37. Verwendung nach Anspruch 27, wobei R₃ NO₂ darstellt.

38. Verwendung nach Anspruch 27, wobei R₄ Fluor darstellt.

39. Verwendung nach Anspruch 27, wobei jeder von R₃ und R₄ unabhängig aus H und Fluor ausgewählt ist.

40. Verwendung nach Anspruch 27, wobei R₅ Methyl, Fluor oder Chlor darstellt.

41. Verwendung nach Anspruch 27, wobei R₆ Methyl, Chlor, Fluor, Nitro oder Wasserstoff darstellt.

42. Verwendung nach Anspruch 41, wobei R₆ H darstellt.

43. Verwendung nach Anspruch 41, wobei R₆ Fluor darstellt.

44. Verwendung nach Anspruch 27, wobei R_{K} Methyl oder Ethyl darstellt.

45. Verwendung nach Anspruch 27, wobei R₁ H, Methyl, Ethyl, Propyl, Isopropyl, Isobutyl, Benzyl, Phenyl, Phenethyl, Allyl, C₂₋₅-Alkenyl, C₃₋₆-Cycloalkyl, (C₃₋₅-Cycloalkyl)-C₁₋₂-alkyl, (C₃₋₅-heterocyclischen Rest)C₁₋₂-alkyl oder (CH₂)₂₋₄NR_{C}R_{D} darstellt.

46. Verwendung nach Anspruch 45, wobei R₁ H oder (C₃₋₄-Cycloalkyl)C₁₋₂-alkyl darstellt.

47. Verwendung nach Anspruch 27, wobei R₂ H oder Methyl darstellt.

48. Verwendung nach Anspruch 27, wobei R_{A} mindestens einen Hydroxylsubstituenten aufweist.

49. Verwendung nach Anspruch 27, wobei R_{A} H, Methyl, Ethyl, Isobutyl, Hydroxyethyl, Phenyl, 2-Piperidin-1-ylethyl, 2,3-Dihydroxypropyl, 3-[4-(2-Hydroxyethyl)piperazin-1-yl]propyl, 2-Pyrrolidin-1-ylethyl oder 2-Diethylaminoethyl darstellt und R_{B} H darstellt oder wobei R_{B} Methyl darstellt und R_{A} Phenyl darstellt.

50. Verwendung nach Anspruch 27, wobei W NR_{A}R_{B} oder NR₂NR_{A}R_{B} darstellt.

51. Verwendung nach Anspruch 27, wobei W NR₂(CH₂)₂₋₄NR_{A}R_{B} oder O(CH₂)₂₋₃NR_{A}R_{B} darstellt.

52. Verwendung nach Anspruch 27, wobei W NR₂OR₁ darstellt.

53. Verwendung nach Anspruch 27, wobei W OR_{B} darstellt.

54. Verwendung nach Anspruch 27, wobei R₇ bezogen auf X in der Paraposition vorliegt.

55. Verwendung nach Anspruch 54, wobei R₇ Jod darstellt.

56. Verwendung nach Anspruch 27, wobei R₈ bezogen auf X in der Orthoposition vorliegt.

57. Verwendung nach Anspruch 27 mit der Formel 2,4-Bis(2-chlor-4-jodphenylamino)-3-fluor-5-nitrobenzoesäure.

58. Verwendung nach Anspruch 27, wobei der MEK-Inhibitor eine Struktur aufweist ausgewählt aus: 2-(2-Chlor-4-jodphenylamino)-3-fluor-5-nitro-4-(4-sulfamoylphenylamino)benzoesäure, 2-(2-Chlor-4-jodphenylamino)-3-fluor-5-nitro-4-phenylamino)benzoesäure, 2-(2-Chlor-4-jodphenylamino)-3-fluor-5-nitro-4-phenoxybenzoesäure, 2-(2-Chlor-4-jodphenylamino)-3-fluor-5-nitro-4-phenylsulfanylbenzoesäure; 2-(2-Chlor-4-jodphenylamino)-3-fluor-(4-methylphenylamino)-5-nitrobenzoesäure; 2-[(2-Chlor-4-jodphenyl)amino]-3-fluor-N-hydroxy-4- [[4-[[(2-hydroxyethyl)amino] carbonyl]phenyl]amino] -5-nitrobenzamid; 2- [(2-Chlor-4-jodphenyl)amino]-4- [[4-[(dimethylamino)carbonyl]phenyl]amino]-3-fluor-N-hydroxy-5-nitrobenzamid; 2-(2-Chlor-4-jodphenylamino)-3,5-difluor-4-phenylaminobenzoesäure; 2-(2-Chlor-4-jodphenylamino)-3-fluor-5-nitro-4-(3-sulfamoylphenylamino)benzoesäure; und 2-(2-Chlor-4-jodphenylamino)-3-fluor-5-nitro-4-(2-sulfamoylphenylamino)-benzoesäure; und den entsprechenden Hydroxamsäuren und Cyclopropylmethylhydroxamaten.

59. Verwendung eines MEK-Inhibitors, ausgewählt aus einer Verbindung der Formel (I)B: 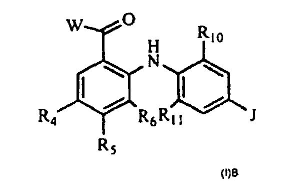 worin
W OR₁, NR₂OR₁, NR_{A}R_{B}, NR₂NR_{A}R_{B}, O(CH₂)₁₋₄NR_{A}R_{B} oder NR₂(CH₂)₁₋₄NR_{A}R_{B}; O(CH₂)₁₋₄OR₁ oder NR₂(CH₂)₁₋₄OR₁ darstellt;
R₁ H, C₁₋₈-Alkyl, C₃₋₈-Alkenyl, C₃₋₈-Alkinyl, C₃₋₈-Cycloalkyl, Phenyl, (Phenyl)C₁₋₄-alkyl, (Phenyl)C₃₋₄-alkenyl, (Phenyl)C₃₋₄-alkinyl, (C₃₋₈-Cycloalkyl)-C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (C₃₋₈heterocyclischen Rest)C₃₋₄-alkenyl oder (C₃₋₈-heterocyclischen Rest)C₃₋₄-alkinyl darstellt;
jeder von R₂ und R₃ unabhängig H, Phenyl, C₁₋₄-Alkyl, C₃₋₈-Alkinyl, C₃₋₈-Cycloalkyl oder (C₃₋₈-Cycloalkyl)C₁₋₄-alkyl darstellt;
jeder von R₄, R₅ und R₆ unabhängig H, Cl, F oder Br darstellt;
R_{A} H, C₁₋₆-Alkyl, C₃₋₈-Alkenyl, C₃₋₈-Alkinyl, C₃₋₈-Cycloalkyl, Phenyl, (C₃₋₈-Cycloalkyl)C₁₋₄-alkyl, (C₃₋₈-Cycloalkyl)-C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (Aminosulfonyl)phenyl, [(Aminosulfonyl)phenyl]C₁₋₄-alkyl, (Aminosulfonyl)C₁₋₆-alkyl, (Aminosulfonyl)C₃₋₆-cycloalkyl oder [(Aminosulfonyl) C₃₋₆-cycloalkyl]C₁₋₄-alkyl darstellt;
R_{B} H, C₁₋₈-Alkyl, C₃₋₈-Alkenyl, C₃₋₈-Alkinyl, C₃₋₈-Cycloalkyl oder Phenyl darstellt;
J SR_{C}, OR_{F}, SO₂R_{C}, SOR_{C}, SO₂NR_{D}R_{E}, C₁₋₈-Alkyl, C₃₋₈-Alkenyl, C₃₋₈-Alkinyl, C₃₋₈-Cycloalkyl, C₅₋₈-Cycloalkenyl, Phenyl, (C₃₋₈-Cycloalkyl)C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischen Rest, (C₃₋₈heterocyclischen Rest)C₂₋₄-alkyl, -M'E'G', (heterocyclischen Rest)-M'-E'-G' oder (Cycloalkyl)-M'-E'-G' darstellt;
M' O, SO, SO₂, NR_{E}, (CO)NR_{E}, NR_{E}(CO), SO₂NR_{E}, NR_{E}SO₂ oder CH₂ darstellt;
E' nicht vorliegt (eine kovalente Bindung), (CH₂)₁₋₄ oder (CH₂)ₘO(CH₂)ₚ, worin 1 ≤ (jedes von m und p unabhängig) ≤ 3 und 2 ≤ (m + p) ≤ 4;
G' OR₃, SO₂R_{C} oder NR_{F}R_{G} darstellt, mit der Maßgabe, dass, wenn p = 1, dann G' H darstellt;
jeder von R_{C}, R_{D}, R_{E}, R_{F} und R_{G} unabhängig ausgewählt ist aus H, C₁₋₆-Alkyl, C₃₋₄-Alkenyl, C₃₋₄-Alkinyl, C₃₋₆-Cycloalkyl, C₃₋₆-heterocyclischem Rest und Phenyl; NR_{F}R_{G} und NR_{D}R_{E} auch jeweils unabhängig ausgewählt sind aus Morpholinyl, Pyrazinyl, Piperazinyl, Pyrrolidinyl oder Piperadinyl;
R₁₀ H, C₁₋₄-Alkyl, Halogen, NO₂ oder SO₂NR_{H}R_{I} darstellt und
R₁₁ H, Halogen oder NO₂ darstellt ;
worin jeder vorstehend genannte Kohlenwasserstoffrest oder heterocyclische Rest gegebenenfalls mit zwischen 1 und 3 Substituenten substituiert ist, unabhängig ausgewählt aus Halogen, C₁₋₄-Alkyl, C₃₋₆-Cycloalkyl, C₂₋₄-Alkenyl, C₂₋₄-Alkinyl, Phenyl, Hydroxy, Amino, (Amino)sulfonyl und NO₂, worin jeder Substituent Alkyl, Cycloalkyl, Alkenyl, Alkinyl oder Phenyl, wiederum gegebenenfalls mit zwischen 1 und 3 Substituenten, unabhängig ausgewählt aus Halogen, C₁₋₂-Alkyl, Hydroxy, Amino und NO₂, substituiert ist;
oder einem pharmazeutisch verträglichen Salz oder C₁₋₇-Ester davon, zur Herstellung von pharmazeutischen Zusammensetzungen für die Behandlung von chronischem Schmerz.

60. Verwendung nach Anspruch 59, wobei der chronische Schmerz ausgewählt ist aus neuropathischem Schmerz, idiopathischem Schmerz und Schmerz verbunden mit chronischem Alkoholismus, Vitaminmangel, Urämie oder Hypothyroidismus.

61. Verwendung nach Anspruch 60, wobei der chronische Schmerz eine Art von neuropathischem Schmerz ist.

62. Verwendung nach Anspruch 61, wobei der neuropathische Schmerz verbunden ist mit einem von den Nachstehenden: Entzündung, postoperativer Schmerz, Phantomgliederschmerz, Verbrennungsschmerz, Gicht, trigeminale Neuralgie, akuter herpetischer und postherpetischer Schmerz, Causalgie, diabetische Neuropathie, Plexusavulsion, Neurom, Vaskulitis, virale Infektion, Quetschverletzung, Konstriktionsschädigung, Gewebsschädigung, Gliedmaßenamputation, postoperativer Schmerz, Arthritisschmerz und jede andere Nervenschädigung zwischen dem peripheren Nervensystem und einschließlich des zentralen Nervensystems.

63. Verwendung nach Anspruch 60, wobei der chronische Schmerz verbunden ist mit chronischem Alkoholismus, Vitaminmangel, Urämie oder Hypothyroidismus.

64. Verwendung nach Anspruch 60, wobei der chronische Schmerz mit idiopathischem Schmerz verbunden ist.

65. Verwendung nach Anspruch 59, wobei der chronische Schmerz mit Entzündung verbunden ist.

66. Verwendung nach Anspruch 59, wobei der chronische Schmerz mit Arthritis verbunden ist.

67. Verwendung nach Anspruch 59, wobei der chronische Schmerz mit postoperativem Schmerz verbunden ist.

68. Verwendung nach Anspruch 59, wobei R_{C} C₁₋₂-Alkyl darstellt.

69. Verwendung nach Anspruch 59, wobei W OH darstellt.

70. Verwendung nach Anspruch 59, wobei W NHOH darstellt.

71. Verwendung nach Anspruch 59, wobei W NHO(Cyclopropylmethyl) darstellt.

72. Verwendung nach Anspruch 59, wobei R₁₀ Methyl oder Chlor darstellt.

73. Verwendung nach Anspruch 59, wobei R₁₁ Fluor darstellt.

74. Verwendung nach Anspruch 59, wobei R₁₁ H darstellt.

75. Verwendung nach Anspruch 59, wobei J Trihalogenmethyl oder Methylthio darstellt.

76. Verwendung nach Anspruch 59, wobei J 1,2,5-Thiadiazol-3-yl darstellt.

77. Verwendung nach Anspruch 59, wobei J SO₂CH₃ darstellt.

78. Verwendung nach Anspruch 59, wobei J SOCH₃ darstellt:

79. Verwendung nach Anspruch 59, wobei J C₂₋₈-Alkinyl darstellt, wobei die Dreifachbindung zwischen den Kohlenstoffatomen α und β zu der Phenylgruppe ist.

80. Verwendung nach Anspruch 59, wobei R₁ mindestens einen Hydroxysubstituenten aufweist.

81. Verwendung nach Anspruch 59, wobei R₁ H, Methyl, Ethyl, Propyl, Isopropyl, Isobutyl, Benzyl, Phenethyl, Allyl, C₃₋₅-Alkenyl, C₃₋₅-Alkinyl, C₃₋₆-Cycloalkyl, (C₃₋₅-Cycloalkyl)-C₁₋₂-alkyl oder (C₃₋₅-heterocyclischen Rest)C₁₋₂-alkyl darstellt.

82. Verwendung nach Anspruch 59, wobei R₁ H oder (C₃₋₄-Cycloalkyl)C₁₋₂-alkyl darstellt.

83. Verwendung nach Anspruch 59, wobei R₂ H, Methyl, C₃₋₄-Alkinyl, C₃₋₅-Cycloalkyl oder (C₃₋₅-Cycloalkyl)methyl darstellt.

84. Verwendung nach Anspruch 59, wobei R_{A} H, Methyl, Ethyl, Isobutyl, Hydroxyethyl, Hydroxypropyl, Cyclopropylmethyl, Cyclobutylmethyl, C₂₋₄-Alkinyl, Phenyl, 2-Piperidin-1-ylethyl, 2,3-Dihydroxypropyl, 3-[4-(2-Hydroxyethyl)piperazin-1-yl]propyl, 2-Pyrrolidin-1-ylethyl oder 2-Diethylaminoethyl darstellt und R_{B} H darstellt oder wobei R_{B} Methyl darstellt und R_{A} Phenyl darstellt.

85. Verwendung nach Anspruch 59, wobei jeder von R₄ und R₆ H darstellt und R₅ F darstellt.

86. Verwendung nach Anspruch 59, wobei jeder von R₄, R₅ und R₆ F darstellt.

87. Verwendung nach Anspruch 59, wobei jeder von R₄ und R₅ F darstellt und R₆ Br darstellt.

88. Verwendung nach Anspruch 59, wobei R₅ F darstellt.

89. Verwendung nach Anspruch 59, wobei der MEK-Inhibitor eine Struktur aufweist ausgewählt aus: 4-Fluor-2-(2-methyl-4-methylsulfanylphenylamino)benzoesäure, 5-Brom-3,4-difluor-2-(2-methyl-4-methylsulfanylphenylamino)benzoesäure, 3,4-Difluor-2-(4-methansulfinyl-2-methylphenylamino)-benzoesäure, 2-(4-Methansulfinyl-2-methylphenylamino)-4-nitrobenzoesäure, 3,4,5-Trifluor-2-(4-methansulfonyl-2-methylphenylamino)benzoesäure, 3,4-Difluor-2-(2-methyl-4-methylsulfanylphenylamino)benzoesäure, 2-(2-Methyl-4-methylsulfanylphenylamino)-4-nitrobenzoesäure, 3,4,5-Trifluor-2-(4-methansulfinyl-2-methylphenylamino)benzoesäure, 4-Fluor-2-(4-methansulfinyl-2-methylphenylamino)benzoesäure, 5-Brom-3,4-difluor-2-(4-methansulfonyl-2-methylphenylamino)benzoesäure, 3,4,5-Trifluor-2-(2-methyl-4-methylsulfanylphenylamino)benzoesäure, 4-Fluor-2-(4-methansulfinyl-2-methylphenylamino)-benzoesäure, 5-Brom-3,4-difluor-2-(4-methansulfinyl-2-methylphenylamino)benzoesäure, 3,4-Difluor-2-(4-methansulfonyl-2-methylphenylamino)benzoesäure, 2-(4-Methansulfonyl-2-methylphenylamino)-4-nitrobenzoesäure, N-Cyclopropylmethoxy-4-fluor-2-(2-methyl-4-methylsulfanylphenylamino)-benzamid, 5-Brom-N-cyclopropylmethoxy-3,4-difluor-2-(2-methyl-4-metrylsulfanylphenylamino)benzamid, N-Cyclopropylmethoxy-3,4-difluor-2-(4-methansulfinyl-2-methylphenylamino)benzamid, N-Cyclopropylmethoxy-2-(4-methansulfinyl-2-methylphenylamino)-4-nitrobenzamid, N-Cyclopropylmethoxy-3,4,5-trifluor-2-(4-methansulfonyl-2-methylphenylamino)benzamid, N-Cyclopropylmethoxy-3,4-difluor-2-(2-methyl-4-methylsulfanylphenylamino)benzamid, N-Cyclopropylmethoxy-2-(2-methyl-4-methylsulfanylphenylamino)-4-nitrobenzamid, N-Cyclopropylmethoxy-3,4,5-trifluor-2-(4-methansulfinyl-2-methylphenylamino)benzamid, N-Cyclopropylmethoxy-4-fluor-2-(4-methansulfinyl-2-methylphenylamino)-benzamid, 5-Brom-N-cyclopropylmethoxy-3,4-difluor-2-(4-methansulfonyl-2-methylphenylamino)benzamid, N-Cyclopropylmethoxy-3,4,5-trifluor-2-(2-methyl-4-methylsulfanylphenylamino)-benz-amid, N-Cyclopropylmethoxy-4-fluor-2-(4-methansulfinyl-2-methylphenylamino)benzamid, 5-Brom-N-cyclopropylmethoxy-3,4-difluor-2-(4-methansulfinyl-2-methylphenylamino)benzamid, N-Cyclopropylmethoxy-3,4-difluor-2-(4-methansulfonyl-2-methylphenylamino)benzamid und N-Cyclopropylmethoxy-2-(4-methansulfonyl-2-methylphenylamino)-4-nitrobenzamid.

90. Verwendung nach Anspruch 59, wobei der MEK-Inhibitor eine Struktur aufweist ausgewählt aus: 4-Fluor-N-hydroxy-2-(2-methyl-4-methylsulfanylphenylamino)benzamid, 5-Brom-3,4-difluor-N-hydroxy-2-(2-methyl-4-methylsulfanylphenylamino)benzamid, 3,4-Difluor-N-hydroxy-2-(4-methansulfinyl-2-methylphenylamino)benzamid, N-Hydroxy-2-(4-methansulfinyl-2-methylphenylamino)-4-nitrobenzamid, 3,4,5-Trifluor-N-hydroxy-2-(4-methansulfonyl-2-methylphenylamino)benzamid, 3,4-Difluor-N-hydroxy-2-(2-methyl-4-methylsulfanylphenylamino)-benzamid, N-Hydroxy-2-(2-methyl-4-methylsulfanylphenylamino)-4-nitrobenzamid, 8:3,4,5-Trifluor-N-hydroxy-2-(4-methansulfinyl-2-methylphenylamino)benzamid, 4-Fluor-N-hydroxy-2-(4-methansulfinyl-2-methylphenylamino)benzamid, 5-Brom-3,4-difluor-N-hydroxy-2-(4-methansulfonyl-2-mathylphenylamino)benzamid, 3,4,5-Trifluor-N-hydroxy-2-(2-methyl-4-methylsulfanylphenylamino)benzamid, 4-Fluor-N-hydroxy-2-(4-methansulfinyl-2-methylphenylamino)benzamid, 5-Brom-3,4-difluor-N-hydroxy-2-(4-methansulfinyl-2-methylphenylamino)benzamid, 3,4-Difluor-N-hydroxy-2-(4-methansulfonyl-2-methylphenylamino)benzamid und N-Hydroxy-2-(4-methansulfonyl-2-methylphenylamino)-4-nitrobenzamid.

91. Verwendung nach Anspruch 59, wobei der MEK-Inhibitor eine Struktur aufweist ausgewählt aus: 3,4-Difluor-2-(4-imidazol-1-yl-2-methylphenylamino)benzoesäure, N-Cyclopropylmethoxy-3,4-difluor-2-(4-imidazol-1-yl-2-methylphenylamino)benzamid, 3,4-Difluor-N-hydroxy-2-(4-imidazol-1-yl-2-methylphenylamino)benzamid, 3,4,5-Trifluor-2-(2-methyl-4-[1,2,5]thiadiazol-3-ylphenylamino)benzoesäure, N-Cyclopropylmethoxy-3,4,5-trifluor-2-(2-methyl-4-[1,2,5]thiadiazol-3-ylphenylamino)benzamid, 3,4,5-Trifluor-N-hydroxy-2-(2-methyl-4-[1,2,5]thiadiazol-3-ylphenylamino)benzamid, 2-[4-(4-Chlor-[1,2,5]thiadiazol-3-yl)-2-methylphenylamino]-3,4,5-trifluorbenzoesäure, 2-[4-(4-Chlor-[1,2,5]thiadiazol-3-yl)-2-methylphenylamino]-N-cyclopropylmethoxy-3,4,5-trifluorbenzamid, 2-[4-(4-Chlor[1,2,5]thiadiazol-3-yl)-2-methylphenylamino]-3,4,5-trifluor-N-hydroxybenzamid, 2-{4-[4-(2-Dimethylaminoethoxy)-[1,2,5]thiadiazol-3-yl]-2-methylphenylamino}-3,4,5-trifluorbenzoesäure, N-Cyclopropylmethoxy-3,4,5-trifluor-2-{2-methyl-4-[4-(2-piperidin-1-ylethoxy)-[1,2,5]thiadiazol-3-yl]phenylamino}benzamid und 3,4,5-Trifluor-N-hydroxy-2-{2-methyl-4-[4-(2-morpholin-4-ylethoxy)-[1,2,5]thiadiazol-3-yl]-phenylamino}benzamid.

92. Verwendung nach Anspruch 59, wobei der MEK-Inhibitor eine Struktur aufweist ausgewählt aus: 5-Brom-2-(2-chlor-4-methylsulfanylphenylamino)-3,4-difluorbenzoesäure, 2-(2-Chlor-4-methansulfinylphenylamino)-3,4-difluorbenzoesäure, 2-(2-Chlor-4-methansulfonylphenylamino)-3,4,5-trifluorbenzoesäure, 2-(2-Chlormethylsulfanylphenylamino)-3,4-difluorbenzoesäure, 5-Brom-2-(2-Chlor-4-methansulfonylphenylamino)-3,4-difluorbenzoesäure, 2-(2-Chlor-4-methansulfonylphenylamino)-3,4-difluorbenzoesäure, 5-Brom-2-(2-Chlor-4-methylsulfanylphenylamino)-N-cyclopropylmethoxy-3,4-difluorbenzamid, 2-(2-Chlor-4-methansulfinylphenylamino)-N-cyclopropylmethoxy-3,4-difluorbenzamid, 2-(2-Chlor-4-methansulfonylphenylamino)-N-cyclopropylmethoxy-3,4,5-trifluorbenzamid, 2-(2-Chlor-4-methylsulfanylphenylamino)-N-cyclopropylmethoxy-3,4-difluorbenzamid, 2-(2-Chlor-4-methansulfinylphenylamino)-N-cyclopropylmethoxy-3,4,5-trifluorbenzamid, 5-Brom-2-(2-Chlor-4-methansulfonylphenylamino)-N-cyclopropylmethoxy-3,4-difluorbenzamid, 2-(2-Chlor-4-methylsulfanylphenylamino)-N-cyclopropylmethoxy-3,4,5-trifluorbenzamid, 2-(2-Chlor-4-methansulfonylphenylamino)-N-cyclopropylmethoxy-3,4-difluorbenzamid, 2-[2-Chlor-4-(3H-imidazol-1-yl)phenylamino]-N-cyclopropylmethoxy-3,4-difluorbenzamid, 2-(2-Chlor-4-[1,2,5]thiadiazol-3-ylphenylamino)-N-cyclopropylmethoxy-3,4,5-trifluorbenzamid, 2-[4-(2-Chlor-4-chlor[1,2,5]thiadiazol-3-yl)phenylamino]-3,4,5-trifluorbenzoesäure, 2-[2-Chlor-4-(4-chlor-[1,2,5]thiadiazol-3-yl)phenylamino]-N-cyclopropylmethoxy-3,4,5-trifluorbenzamid, 2-{4-[4-(2-Dimethylaminoethoxy)-[1,2,5]thiadiazol-3-yl]-2-methylphenylamino}-3,4,5-trifluorbenzoesäure und 2-{2-Chlor-4-[4-(2-piperidin-1-ylethoxy)-[1,2,5]thiadiazol-3-yl]-phenylamino}-N-cyclopropylmethoxy-3,4,5-trifluorbenzamid.

93. Verwendung nach Anspruch 59, wobei der MEK-Inhibitor eine Struktur aufweist ausgewählt aus: 2-(4-Ethinyl-2-methylphenylamino)-4-fluorbenzoesäure, 5-Brom-2-(4-ethinyl-2-methylphenylamino)-3,4-difluorbenzoesäure, N-Cyclopropylmethoxy-2-(4-ethinyl-2-methylphenylamino)-3,4-difluorbenzamid, N-Cyclopropylmethoxy-2-(4-ethinyl-2-methylphenylamino)-4-nitrobenzamid, 2-(4-Ethinyl-2-methylphenylamino)-3,4,5-trifluor-N-hydroxybenzamid, 2-(4-Ethinyl-2-methylphenylamino)-3,4-difluorbenzoesäure, 2-(4-Ethinyl-2-methylphenylamino)-4-nitrobenzoesäure, N-Cyclopropylmethoxy-2-(4-ethinyl-2-methylphenylamino)-3,4,5-trifluorbenzamid, 4-Fluor-N-hydroxy-2-(4-methansulfinyl-2-methylphenylamino)benzamid, 5-Brom-2-(4-ethinyl-2-methylphenylamino)-3,4-difluor-N-hydroxybenzamid, 2-(4-Ethinyl-2-methylphenylamino)-3,4,5-trifluorbenzoesäure, N-Cyclopropylmethoxy-2-(4-ethinyl-2-methylphenylamino)-4-fluorbenzamid, 5-Brom-N-cyclopropylmethoxy-2-(4-ethinyl-2-methylphenylamino)-3,4-difluorbenzamid, 2-(4-Ethinyl-2-methylphenylamino)-3,4-difluor-N-hydroxybenzamid, 2-(4-Ethinyl-2-methylphenylamino)-N-hydroxy-4-nitrobenzamid, 2-(4-Ethinyl-2-methylphenylamino)-4-fluorbenzoesäure, N-Cyclopropylmethoxy-2-(4-ethinyl-2-methylphenylamino)-4-fluorbenzamid und 4-Fluor-N-hydroxy-2-(4-methansulfinyl-2-methylphenylamino)-benzamid.

94. Verwendung nach Anspruch 59, wobei der MEK-Inhibitor eine Struktur aufweist ausgewählt aus: 2-(2-Chlor-4-ethinylphenylamino)-4-fluorbenzoesäure, 5-Brom-2-(2-chlor-4-ethinylphenylamino)-3,4-difluorbenzoesäure, 2-(2-Chlor-4-ethinylphenylamino)-N-cyclopropylmethoxy-3,4-difluorbenzamid, 2-(2-Chlor-4-ethinylphenylamino)-N-cyclopropylmethoxy-4-nitrobenzamid, 2-(2-Chlor-4-ethinylphenylamino)-N-hydroxy-3,4,5-trifluorbenzamid, 2-(2-Chlor-4-ethinylphenylamino)-3,4-difluorbenzoesäure, 2-(4-Ethinyl-2-chlorphenylamino)-4-nitrobenzoesäure, 2-(2-Chlor-4-ethinylphenylamino)-N-cyclopropylmethoxy-3,4,5-trifluorbenzamid, 2-(2-Chlor-4-methansulfinylphenylamino)-4-fluor-N-hydroxybenzamid, 5-Brom-2-(4-ethinyl-2-chlorphenylamino)-3,4-difluor-N-hydroxybenzamid, 2-(2-Chlor-4-ethinylphenylamino)-3,4,5-trifluorbenzoesäure, 2-(2-Chlor-4-ethinylphenylamino)-N-cyclopropylmethoxy-4-fluorbenzamid, 5-Brom-2-(2-chlor-4-ethinylphenylamino)-N-cyclopropylmethoxy-3,4-difluorbenzamid, 2-(4-Ethinyl-2-chlorphenylamino)-3,4-difluor-N-hydroxybenzamid, 2-(4-Ethinyl-2-chlorphenylamino)-N-hydroxy-4-nitrobenzamid, 2-(2-Chlor-4-ethinylphenylamino)-4-fluorbenzoesäure, 2-(2-Chlor-4-ethinylphenylamino)-N-cyclopropylmethoxy-4-fluorbenzamid, 2-(2-Chlor-4-methansulfinylphenylamino)-4-fluor-N-hydroxybenzamid und 2-(2-Chlor-4-imidazol-1-ylphenylamino)-3,4-difluorbenzoesäure.

95. Verwendung eines MEK-Inhibitors ausgewählt aus einer Verbindung der Formel I(C): 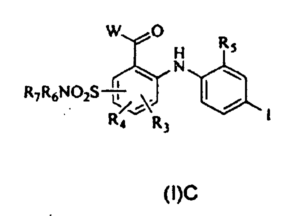 worin
W OR₁, NR₂OR₁, NR_{A}R_{B}, NR₂NR_{A}R_{B} oder NR₂(CH₂)₂₋₄NR_{A}R_{B} darstellt;
R₁ H, C₁₋₈-Alkyl, C₃₋₈-Alkenyl, C₃₋₈-Alkinyl, C₃₋₈-Cycloalkyl, Phenyl, (Phenyl)C₁₋₄-alkyl, (Phenyl)C₃₋₄-alkenyl, (Phenyl)C₃₋₄-alkinyl, (C₃₋₈-Cycloalkyl)C₁₋₄-alkyl, (C₃₋₈-Cycloalkyl)C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (C₃₋₈-heterocyclischen Rest)C₃₋₄-alkenyl, (C₃₋₈-heterocyclischen Rest)-C₃₋₄-alkinyl oder (CH₂)₂₋₄NR_{A}R_{B} darstellt;
R₂ H, Phenyl, C₁₋₄-Alkyl, C₃₋₄-Alkenyl, C₃₋₈-Alkinyl, C₃₋₈-Cycloalkyl oder (C₃₋₈-Cycloalkyl)C₁₋₄-alkyl darstellt;
R_{A} H, C₁₋₆-Alkyl, C₃₋₈-Alkenyl, C₃₋₈-Alkinyl, C₃₋₈-Cycloalkyl, Phenyl, (C₃₋₈-Cycloalkyl)C₁₋₄-alkyl, (C₃₋₈-Cycloalkyl)-C₃₋₄-alkenyl, (C₃₋₈-Cycloalkyl)C₃₋₄-alkinyl, C₃₋₈-heterocyclischen Rest, (C₃₋₈-heterocyclischen Rest)C₁₋₄-alkyl, (Aminosulfonyl)phenyl, [(Aminosulfonyl)phenyl]C₁₋₄-alkyl, (Aminosulfonyl)C₁₋₆-alkyl, (Aminosulfonyl)C₃₋₆-cycloalkyl oder [(Aminosulfonyl)C₃₋₆-cycloalkyl]C₁₋₄-alkyl darstellt;
R_{B} H, C₁₋₈-Alkyl, C₃₋₈-Alkenyl, C₃₋₈-Alkinyl, C₃₋₈-Cycloalkyl oder C₆₋₈-Aryl darstellt;
R₃ H, F, Cl, Br oder NO₂ darstellt;
R₄ H oder F darstellt;
R₅ H, Methyl oder Cl darstellt;
R₆ H, C₁₋₄-Alkyl, Hydroxyethyl, Hydroxypropyl, (CH₂)₂₋₄(NR_{C}R_{D}), Phenyl, 2-Pyridyl, 3-Pyridyl, 4-Pyridyl oder CH₂Ar darstellt, worin Ar Phenyl, 2-Pyridyl, 3-Pyridyl oder 4-Pyridyl darstellt;
R₇ H, C₁₋₄-Alkyl, Hydroxyethyl, Hydroxypropyl, (CH₂)₂₋₄(NR_{C}R_{D}), Phenyl, 2-Pyridyl, 3-Pyridyl, 4-Pyridyl oder CH₂Ar darstellt, worin Ar Phenyl, 2-Pyridyl, 3-Pyridyl oder 4-Pyridyl darstellt;
jeder von R_{C} und R_{D} unabhängig ausgewählt ist aus H, C₁₋₆-Alkyl, C₃₋₄-Alkenyl, C₃₋₄-Alkinyl, C₃₋₆-Cycloalkyl, C₃₋₆heterocyclischem Rest, und Phenyl; NR_{C}R_{D} auch ausgewählt sein kann aus Morpholinyl, Piperazinyl, Pyrrolidinyl oder Piperadinyl;
worin jeder vorstehend genannte Kohlenwasserstoffrest oder heterocyclischer Rest gegebenenfalls mit zwischen 1 und 3 Substituenten, unabhängig ausgewählt aus Halogen, C₁₋₄-Alkyl, C₃₋₆-Cycloalkyl, C₂₋₄-Alkenyl, C₂₋₄-Alkinyl, Phenyl, Hydroxy, Amino, (Amino)sulfonyl und NO₂, substituiert ist, worin jeder Substituent Alkyl, Cycloalkyl, Alkenyl, Alkinyl oder Phenyl wiederum gegebenenfalls mit zwischen 1 und 3 Substituenten unabhängig ausgewählt aus Halogen, C₁₋₂-Alkyl, Hydroxy, Amino und NO₂, substituiert ist;
oder einem pharmazeutisch verträglichen Salz oder C₁₋₆-Ester davon, zur Herstellung von Arzneimitteln für die Behandlung von chronischem Schmerz.

96. Verwendung nach Anspruch 95, wobei der chronische Schmerz ausgewählt ist aus neuropathischem Schmerz, idiopathischem Schmerz und Schmerz verbunden mit chronischem Alkoholismus, Vitaminmangel, Urämie oder Hypothyroidismus.

97. Verwendung nach Anspruch 96, wobei der chronische Schmerz eine Art von neuropathischem Schmerz ist.

98. Verwendung nach Anspruch 97, wobei der neuropathische Schmerz verbunden ist mit einem von den Nachstehenden: Entzündung, postoperativer Schmerz, Phantomgliederschmerz, Verbrennungsschmerz, Gicht, trigeminale Neuralgie, akuter herpetischer und postherpetischer Schmerz, Causalgie, diabetische Neuropathie, Plexusavulsion, Neurom, Vaskulitis, virale Infektion, Quetschverletzung, Konstriktionsschädigung, Gewebsschädigung, Gliedmaßenamputation, postoperativer Schmerz, Arthritisschmerz und jede andere Nervenschädigung zwischen dem peripheren Nervensystem und einschließlich des zentralen Nervensystems.

99. Verwendung nach Anspruch 96, wobei der chronische Schmerz verbunden ist mit chronischem Alkoholismus, Vitaminmangel, Urämie oder Hypothyroidismus.

100. Verwendung nach Anspruch 96, wobei der chronische Schmerz mit idiopathischem Schmerz verbunden ist.

101. Verwendung nach Anspruch 95, wobei der chronische Schmerz mit Entzündung verbunden ist.

102. Verwendung nach Anspruch 95, wobei der chronische Schmerz mit Arthritis verbunden ist.

103. Verwendung nach Anspruch 95, wobei der chronische Schmerz mit postoperativem Schmerz verbunden ist.

104. Verwendung nach Anspruch 95, wobei die Sulfamoylgruppe *meta* zu W(CO)- und *para* zu der Brücke NH ist.

105. Verwendung nach Anspruch 95, wobei die Sulfamoylgruppe *para* zu W(CO)- und *meta* zu der Brücke NH ist.

106. Verwendung nach Anspruch 95, wobei R₄ Fluor darstellt.

107. Verwendung nach Anspruch 95, wobei R₃ Fluor darstellt.

108. Verwendung nach Anspruch 95, wobei R₃ H darstellt.

109. Verwendung nach Anspruch 95, wobei W OH darstellt.

110. Verwendung nach Anspruch 95, wobei W NR₂OR₁ darstellt.

111. Verwendung nach Anspruch 109, wobei jeder von R₃ und R₄ Fluor darstellt.

112. Verwendung nach Anspruch 95, wobei R₁ mindestens einen Hydroxysubstituenten aufweist.

113. Verwendung nach Anspruch 95, wobei R₁ H, Methyl, Ethyl, Propyl, Isopropyl, Isobutyl, Benzyl, Phenethyl, Allyl, C₃₋₅-Alkenyl, C₃₋₅-Alkinyl, C₃₋₆-Cycloalkyl, (C₃₋₅-Cycloalkyl)-C₁₋₂-alkyl oder (C₃₋₅-heterocyclischen Rest)C₁₋₂-alkyl darstellt.

114. Verwendung nach Anspruch 113, wobei R₁ H oder (C₃₋₄-Cycloalkyl)C₁₋₂-alkyl darstellt.

115. Verwendung nach Anspruch 95, wobei R₂ H, Methyl, C₃₋₄-Alkinyl, C₃₋₅-Cycloalkyl oder (C₃₋₅-Cycloalkyl)methyl darstellt.

116. Verwendung nach Anspruch 95, wobei R_{A} H, Methyl, Ethyl, Isobutyl, Hydroxyethyl, Hydroxypropyl, Cyclopropylmethyl, Cyclobutylmethyl, C₃₋₄-Alkinyl, Phenyl, 2-Piperidin-1-ylethyl, 2,3-Dihydroxypropyl, 3-[4-(2-Hydroxyethyl)piperazin-1-yl]propyl, 2-Pyrrolidin-1-ylethyl oder 2-Diethylaminoethyl darstellt und R_{B} H darstellt oder wobei R_{B} Methyl darstellt und R_{A} Phenyl darstellt.

117. Verwendung nach Anspruch 95, wobei R₇ (CH₂)₂₋₄(NR_{C}R_{D}) darstellt.

118. Verwendung nach Anspruch 95, wobei NR_{C}R_{D} ausgewählt ist aus Morpholinyl, Piperazinyl, Pyrrolidinyl oder Piperadinyl.

119. Verwendung nach Anspruch 95, wobei R₅ Methyl oder Chlor darstellt.

120. Verwendung nach Anspruch 95, wobei der MEK-Inhibitor eine Struktur aufweist ausgewählt aus: 2-(2-Chlor-4-jodphenylamino)-4-sulfamoylbenzoesäure, 2-(2-Chlor-4-jodphenylamino)-N-hydroxy-4-sulfamoylbenzamid, 2-(2-Chlor-4-jodphenylamino)-N-cyclopropylmethoxy-4-sulfamoylbenzamid, 2-(2-Chlor-4-jodphenylamino)-4-(2-morpholin-4-ylethylsulfamoyl)-benzoesäure, 2-(2-Chlor-4-jodphenylamino)-N-hydroxy-4-(2-morpholin-4-ylethylsulfamoyl)benzamid, 2-(2-Chlor-4-jodphenylamino)-N-cyclopropylmethoxy-4-(2-morpholin-4-ylethylsulfamoyl)benzamid, 2-(2-Chlor-4-jodphenylamino)-3,4-difluor-5-sulfamoylbenzoesäure, 2-(2-Chlor-4-jodphenylamino)-3,4-difluor-N-hydroxy-5-sulfamoylbenzamid, 2-(2-Chlor-4-jodphenylamino)-N-cyclopropylmethoxy-3,4-difluor-5-sulfamoylbenzamid, 2-(2-Chlor-4-jodphenylamino)-3,4-difluor-5-(2-morpholin-4-ylethylsulfamoyl)benzoesäure, 2-(2-Chlor-4-jodphenylamino)-3,4-difluor-N-hydroxy-5-(2-morpholin-4-ylethylsulfamoyl)benzamid, 2-(2-Chlor-4-jodphenylamino)-N-cyclopropylmethoxy-3,4-difluor-5-(2-morpholin-4-ylethylsulfamoyl)benzamid, 5-(Bispyridin-3-ylmethylsulfamoyl)-3,4-difluor-2-(4-jodphenylamino)-benzoesäure, 5-(Bispyridin-3-ylmethylsulfamoyl)-N-cyclopropylmethoxy-3,4-difluor-2-(4-jodphenylamino)-benzamid, N-Cyclopropylmethoxy-3,4-difluor-2-(4-jodphenylamino)-5-(methylpyridin-3-ylmethylsulfamoyl)benzamid, N-Cyclopropylmethoxy-3,4-difluor-2-(4-jodphenylamino)-5-[(pyridin-3-ylmethyl)sulfamoyl]benzamid, N-Cyclopropylmethoxy-5-[(3-diethylaminopropyl)-pyridin-3-ylmethylsulfamoyl]-3,4-difluor-2-(4-jodphenylamino)benzamid, N-Cyclopropylmethoxy-3,4-difluor-5-[(3-hydroxypropyl)pyridin-3-ylmethylsulfamoyl]-2-(4-jodphenylamino)-benzamid, N-Cyclopropylmethoxy-5-(ethylpyridin-3-ylmethylsulfamoyl)-3,4-difluor-2-(4-jodphenylamino)benzamid, N-Cyclopropylmethoxy-3,4-difluor-5-[(2-hydroxyethyl)pyridin-3-ylmethylsulfamoyl]-2-(4-jodphenylamino)benzamid, 5-(Bispyridin-2-ylmethylsulfamoyl)-3,4-difluor-2-(4-jodphenylamino)benzoesäure, 5-(Bispyridin-2-ylmethylsulfamoyl)-N-cyclopropylmethoxy-3,4-difluor-2-(4-jodphenylamino)benzamid, N-Cyclopropylmethoxy-3,4-difluor-2-(4-jodphenylamino)-5-(methylpyridin-2-ylmethylsulfamoyl)benzamid, N-Cyclopropylmethoxy-3,4-difluor-2-(4-jodphenylamino)-5-[(pyridin-2-ylmethyl)sulfamoyl]benzamid, 5-(Bispyridin-3-ylmethylsulfamoyl)-3,4-difluor-2-(4-jod-2-methylphenylamino)benzoesäure, 5-(Bispyridin-3-ylmethylsulfamoyl)-N-cyclopropylmethoxy-3,4-difluor-2-(4-jod-2-methylphenylamino)benzamid, N-cyclopropylmethoxy-3,4-difluor-2-(4-jod-2-methylphenylamino)-5-(methylpyridin-3-ylmethylsulfamoyl)benzamid, N-Cyclopropylmethoxy-3,4-difluor-2-(4-jod-2-methylphenylamino)-5-[(pyridin-3-ylmethyl)sulfamoyl]-benzamid, N-Cyclopropylmethoxy-5-[(3-diethylaminopropyl)pyridin-3-ylmethylsulfamoyl]-3,4-difluor-2-(4-jod-2-methylphenylamino)benzamid, N-Cyclopropylmethoxy-3,4-difluor-5-[(3-hydroxypropyl)pyridin-3-ylmethylsulfamoyl]-2-(4-jod-2-methylphenylamino)benzamid, N-Cyclopropylmethoxy-5-(ethylpyridin-3-ylmethylsulfamoyl))-3,4-difluor-2-(4-jod-2-methylphenylamino)benzamid, N-Cyclopropylmethoxy-3,4-difluor-5-[(2-hydroxyethyl)pyridin-3-ylmethylsulfamoyl]-2-(4-jod-2-methylphenylamino)benzamid, 5-(Bispyridin-2-ylmethylsulfamoyl)-3,4-difluor-2-(4-jod-2-methylphenylamino)benzoesäure, 5-(Bispyridin-2-ylmethylsulfamoyl)-N-cyclopropylmethoxy-3,4-difluor-2-(4-jod-2-methylphenylamino)benzamid, N-Cyclopropylmethoxy-3,4-difluor-2-(4-jod-2-methylphenylamino)-5-(methylpyridin-2-ylmethylsulfamoyl)benzamid, N-Cyclopropylmethoxy-3,4-difluor-2-(4-jod-2-methylphenylamino)-5-[(pyridin-2-ylmethyl)sulfamoyl]benzamid, 5-(Bispyridin-3-ylmethylsulfamoyl)-2-(2-Chlor-4-jodphenylamino)-3,4-difluorbenzoesäure, 5-(Bispyridin-3-ylmethylsulfamoyl)-2-(2-chlor-4-jodphenylamino)-N-cyclopropylmethoxy-3,4-difluorbenzamid, 2-(2-Chlor-4-jodphenylamino)-N-cyclopropylmethoxy-3,4-difluor-5-(methylpyridin-3-ylmethylsulfamoyl)-benzamid, 2-(2-Chlor-4-jodphenylamino)-N-cyclopropylmethoxy-3,4-difluor-5-[(pyridin-3-ylmethyl)sulfamoyl]benzamid, 2-(2-Chlor-4-jodphenylamino)-N-cyclopropylmethoxy-5-[(3-diethylaminopropyl)pyridin-3-ylmethylsulfamoyl]-3,4-difluorbenzamid, 2-(2-Chlor-4-jodphenylamino)-N-cyclopropylmethoxy-3,4-difluor-5-[(3-hydroxypropyl)pyridin-3-ylmethylsulfamoyl]benzamid, 2-(2-Chlor-4-jodphenylamino)-N-cyclopropylmethoxy-5-(ethylpyridin-3-ylmethylsulfamoyl)-3,4-difluorbenzamid, 2-(2-Chlor-4-jodphenylamino)-N-cyclopropylmethoxy-3,4-difluor-5-[(2-hydroxyethyl)pyridin-3-ylmethylsulfamoyl]-benzamid, 5-(Bispyridin-2-ylmethylsulfamoyl)-2-(2-chlor-4-jodphenylamino)-3,4-difluorbenzoesäure, 5-(Bispyridin-2-ylmethylsulfamoyl)-2-(2-chlor-4-jodphenylamino)-N-cyclopropylmethoxy-3,4-difluorbenzamid, 2-(2-Chlor-4-jodphenylamino)-N-cyclopropylmethoxy-3,4-difluor-5-(methylpyridin-2-ylmethylsulfamoyl)benzamid, 2-(2-Chlor-4-jodphenylamino)-N-methylphenylamino)-4-phenylsulfamoylbenzamid, N-Cyclopropylmethoxy-2-(4-jod-2-methylphenylamino) -4- (pyridin-3-ylsulfamoyl)benzamid, N-Cyclopropylmethoxy-2-(4-jod-2-methylphenylamino)-4-[(pyridin-3-ylmethyl)sulfamoyl]benzamid, 4-(Bispyridin-3-ylmethylsulfamoyl)-N-cyclopropylmethoxy-2-(4-jod-2-methylphenylamino)benzamid, N-Cyclopropylmethoxy-4-[(2-hydroxyethyl)-pyridin-4-ylmethylsulfamoyl]-2-(4-jod-2-methylphenylamino)-benzamid, N-Cyclopropylmethoxy-2-(4-jod-2-methylphenylamino)-4-(methylpyridin-3-ylmethylsulfamoyl)benzamid, N-Cyclopropylmethoxy-4-[(3-diethylaminopropyl)pyridin-3-ylmethylsulfamoyl]-2-(4-jod-2-methylphenylamino)benzamid, 2-(2-Chlor-4-jodphenylamino)-N-cyclopropylmethoxy-4-phenylsulfamoylbenzamid, 2-(2-Chlor-4-jodphenylamino)-N-cyclopropylmethoxy-4-(pyridin-3-ylsulfamoyl)benzamid, 2-(2-Chlor-4-jodphenylamino)-N-cyclopropylmethoxy-4-[(pyridin-3-ylmethyl)sulfamoyl]benzamid, 4-(Bispyridin-3-ylmethylsulfamoyl)-2-(2-chlor-4-jodphenylamino)-N-cyclopropylmethoxybenzamid, 2-(2-Chlor-4-jodphenylamino)-N-cyclopropylmethoxy-4-[(2-hydroxyethyl)pyridin-4-ylmethylsulfamoyl]benzamid, 2-(2-Chlor-4-jodphenylamino) -N-cyclopropylmethoxy-4-(methylpyridin-3-ylmethylsulfamoyl)benzamid, 2-(2-Chlor-4-jodphenylamino)-N-cyclopropylmethoxy-4-[(3-diethylaminopropyl)pyridin-3-ylmethylsulfamoyl]benzamid und 5-[Bis-(4-methoxybenzyl)sulfamoyl]-2-(2-Chlor-4-jodphenylamino)-3,4-difluorbenzoesäure und 2-(2-Chlor-4-jodphenylamino)-5-dimethylsulfamoyl-3,4-difluorbenzoesäuremethylester.

121. Verwendung nach Anspruch 95, wobei der MEK-Inhibitor eine Struktur aufweist ausgewählt aus: PD 298458, N-Allyloxy-2-(2-Chlor-4-jodphenylamino)-3,4-difluor-5-(4-methylpiperazin-1-sulfonyl)benzamid; PD 298459, N-Allyloxy-2-(2-chlor-4-jodphenylamino)-3,4-difluor-5-(methylphenylsulfamoyl)benzamid; PD 298460, 5-(Allylmethylsulfamoyl)-N-allyloxy-2-(2-chlor-4-jodphenylamino)-3,4-difluorbenzamid; PD 298463, 1-[5-Allyloxycarbamoyl-4-(2-Chlor-4-jodphenylamino)-2,3-difluorbenzolsulfonyl]piperidin-3-carbonsäureamid; PD 298464, N-Allyloxy-2-(2-Chlor-4-jodphenylamino)-5-[(3-dimethylaminopropyl)methylsulfamoyl]-3,4-difluorbenzamid; PD 298465, N-Allyloxy-2-(2-chlor-4-jodphenylamino)-3,4-difluor-5-(4-pyridin-2-yl-piperazin-1-sulfonyl)benzamid und PD 298467, N-Allyloxy-2-(2-Chlor-4-jodphenylamino)-3,4-difluor-5-(methoxymethylsulfamoyl)benzamid.

122. Verwendung nach Anspruch 1, wobei der MEK-Inhibitor eine Struktur aufweist ausgewählt aus: 2-(2-Chlor-4-jodphenylamino)-*N*-cyclopropylmethoxy-3,4-difluorbenzolsulfonamid und 2-(2-Chlor-4-jodphenylamino)cyclopropylmethoxy-3,4-difluorbenzolsulfonamid.

123. Verwendung nach Anspruch 27, wobei der MEK-Inhibitor eine Struktur aufweist ausgewählt aus: 2,4-Bis-(2-chlor-4-jodphenylamino)-3-fluor-5-nitrobenzoesäure.

124. Verwendung nach Anspruch 59, wobei der MEK-Inhibitor eine. Struktur aufweist ausgewählt aus: 2-(4-Ethinyl-2-methylphenylamino)-4-fluorbenzoesäure und 2-(3',5'-Dichlorbiphenyl-4-ylamino)benzoesäure.

125. Verwendung nach Anspruch 95, wobei der MEK-Inhibitor eine Struktur aufweist ausgewählt aus: 2-(2-Chlor-4-jodphenylamino)-*N*-cyclopropylmethoxy-3,4-difluor-5-sulfamoylbenzamid, 2-(2-Chlor-4-jodphenylamino)-3,4-difluor-*N*-hydroxy-5-sulfamoylbenzamid, *C*-(2-Chlor-4-jodphenylamino)-*N*-cyclopropylmethoxydimethylsulfamoyldifluorbenzamid, *N*-Cyclopropylmethoxydimethylsulfamoyldifluor-*C*-(4-jod-2-methylphenylamino)benzamid und *C*-(2-Chlor-4-jodphenylamino)difluor(methoxymethylsulfamoyl)-*N*-(2-morpholin-4-ylethoxy)benzamid.

## Revendications

1. Utilisation d'un inhibiteur de MEK choisi parmi un composé de formule (I) : 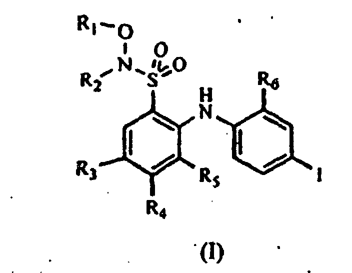 dans laquelle
R₁ est un atome d'hydrogène ou un groupe alkyle en C₁₋₈, alcényle en C₃₋₈, alcynyle en C₃₋₈, cycloalkyle en C₃₋₈, phényle, phényl-alkyle(C₁₋₄), phénylalcényle(C₃₋₄), phényl-alcynyle(C₃₋₄), cycloalkyl(C₃₋₈)-alkyle(C₁₋₄), cycloalkyl-(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), hétérocyclyl(C₃₋₈)-alcényle(C₃₋₄), hétérocyclyl-(C₃₋₈)-alcynyle(C₃₋₄), (CH₂)₂₋₄OR_{c} ou (CH₂)₂₋₄NR_{C}R_{D};
R₂ est un atome d'hydrogène ou un groupe alkyle en C₁₋₄, phényle, cycloalkyle en C₃₋₆, hétérocyclyle en C₃₋₆ ou cycloalkyl(C₃₋₆)méthyle ;
R₃ et R₄ représentent chacun, indépendamment l'un de l'autre, H, F, NO₂, Br ou Cl ;
R₅ est choisi parmi H et F ;
R₆ est H, F, Cl ou CH₃ ;
R_{C} et R_{D} représentent chacun, indépendamment l'un de l'autre, un atome d'hydrogène, un groupe alkyle en C₁₋₄, alcényle en C₃₋₄, alcynyle en C₃₋₄, cycloalkyle en C₃₋₆ ou phényle ; ou NR_{C}R_{D} peut être un cycle pipéridino, morpholino ou N-alkyl(C₁₋₆)pipérazino ;
chaque radical hydrocarboné ci-dessus portant éventuellement entre 1 et 3 substituants choisis indépendamment parmi des atomes d'halogène et des groupes hydroxy, amino, aminosulfonyle et NO₂ ; et
chaque radical hétérocyclique ci-dessus portant éventuellement entre 1 et 3 substituants choisis indépendamment parmi des atomes d'halogène et des groupes alkyle en C₁₋₄, cycloalkyle en C₃₋₆, alcényle en C₃₋₄, alcynyle en C₃₋₄, phényle, hydroxy, amino, aminosulfonyle et NO₂, chaque substituant alkyle, cycloalkyle, alcényle, alcynyle ou phényle portant à son tour éventuellement entre 1 et 2 substituants choisis indépendamment parmi des atomes d'halogène et des groupes alkyle en C₁₋₂, hydroxy, amino et NO₂,
et un sel ou ester en C₁₋₈ pharmaceutiquement acceptable d'un tel composé, pour la fabrication de produits pharmaceutiques destinés au traitement de la douleur chronique.

2. Utilisation selon la revendication 1, dans laquelle ladite douleur chronique est choisie parmi la douleur neuropathique, la douleur idiopathique et une douleur associée à l'alcoolisme chronique, une carence vitaminique, l'urémie ou l'hypothyroïdie.

3. Utilisation selon la revendication 2, dans laquelle ladite douleur chronique est un type de douleur neuropathique.

4. Utilisation selon la revendication 3, dans laquelle ladite douleur neuropathique est associée à l'un des états suivants : inflammation, douleur postopératoire, douleur des membres amputés, douleur causée par une brûlure, goutte, névralgie faciale, douleur herpétique et post-herpétique aiguë, causalgie, neuropathie diabétique, avulsion du plexus, névrome, vasculite, infection virale, syndrome d'écrasement, blessure par constriction, lésion tissulaire, amputation d'un membre, douleur postopératoire, douleur arthritique et toute autre lésion nerveuse entre le système nerveux périphérique et le système nerveux central, inclus.

5. Utilisation selon la revendication 2, dans laquelle ladite douleur chronique est associée à l'alcoolisme chronique, la carence vitaminique, l'urémie ou l'hypothyroïdie.

6. Utilisation selon la revendication 2, dans laquelle ladite douleur chronique est associée à une douleur idiopathique.

7. Utilisation selon la revendication 1, dans laquelle ladite douleur chronique est associée à l'inflammation.

8. Utilisation selon la revendication 1, dans laquelle ladite douleur chronique est associée à l'arthrite.

9. Utilisation selon la revendication 1, dans laquelle ladite douleur chronique est associée à une douleur postopératoire.

10. Utilisation selon la revendication 1, dans laquelle R₃ est un atome de brome ou de chlore.

11. Utilisation selon la revendication 1, dans laquelle R₄ est un atome de fluor.

12. Utilisation selon la revendication 1, dans laquelle R₅ est un atome d'hydrogène.

13. Utilisation selon la revendication 12, dans laquelle R₄ et R₅ représentent chacun un atome d'hydrogène.

14. Utilisation selon la revendication 1, dans laquelle R₄ et R₅ représentent chacun un atome de fluor.

15. Utilisation selon la revendication 14, dans laquelle R₃ est un atome de brome.

16. Utilisation selon la revendication 14, dans laquelle R₃ est un atome de fluor.

17. Utilisation selon la revendication 1, dans laquelle R₄ est le groupe nitro.

18. Utilisation selon la revendication 16, dans laquelle R₅ est un atome d'hydrogène.

19. Utilisation selon la revendication 1, dans laquelle R₆ est un atome de chlore.

20. Utilisation selon la revendication 1, dans laquelle R₆ est le groupe méthyle.

21. Utilisation selon la revendication 1, dans laquelle R₁ est un atome d'hydrogène ou un groupe alkyle en C₁₋₄, et R₂ est un atome d'hydrogène.

22. Utilisation selon la revendication 1, dans laquelle R₁ est un groupe cycloalkyl(C₃₋₆)méthyle.

23. Utilisation selon la revendication 1, dans laquelle R₁ est un atome d'hydrogène.

24. Utilisation selon la revendication 1, dans laquelle R₁ est un groupe (CH₂)₂₋₄OR_{C} ou (CH₂)₂₋₄NR_{C}R_{D}.

25. Utilisation selon la revendication 1, dans laquelle ledit inhibiteur de MEK est choisi parmi les composés suivants : acide 4-fluoro-2-(4-iodo-2-méthylphénylamino)benzènesulfonique, 4-fluoro-N-hydroxy-2-(4-iodo-2-méthylphénylamino)benzènesulfonamide, N-cyclopropylméthoxy-4-fluoro-2-(4-iodo-2-méthylphénylamino)benzènesulfonamide, acide 3,4-difluoro-2-(4-iodo-2-méthylphénylamino)benzènesulfonique, 3,4-difluoro-N-hydroxy-2-(4-iodo-2-méthylphénylamino)benzènesulfonamide, N-cyclopropylméthoxy-3,4-difluoro-2-(4-iodo-2-méthylphénylamino)benzènesulfonamide, acide 3,4,5-trifluoro-2-(4-iodo-2-méthylphénylamino)benzènesulfonique, 3,4,5-trifluoro-N-hydroxy-2-(4-iodo-2-méthylphénylamino)benzènesulfonamide, N-cyclopropylméthoxy-3,4,5-trifluoro-2-(4-iodo-2-méthylphénylamino)benzènesulfonamide, acide 5-bromo-3,4-difluoro-2-(4-iodo-2-méthylphénylamino)benzènesulfonique, 5-bromo-3,4-difluoro-N-hydroxy-2-(4-iodo-2-méthylphénylamino)benzènesulfonamide, 5-bromo-N-cyclopropylméthoxy-3,4-difluoro-2-(4-iodo-2-méthylphénylamino)-benzènesulfonamide, acide 2-(4-iodo-2-méthylphénylamino)-4-nitrobenzènesulfonique, N-hydroxy-2-(4-iodo-2-méthylphénylamino)-4-nitrobenzènesulfonamide et N-cyclopropylméthoxy-2-(4-iodo-2-méthylphénylamino)-4-nitrobenzènesulfonamide.

26. Utilisation selon la revendication 1, dans lequel ledit inhibiteur de MEK est choisi parmi les composés suivants : acide 2-(2-chloro-4-iodophénylamino)-4-fluorobenzènesulfonique, 2-(2-chloro-4-iodophénylamino)-4-fluoro-N-hydroxybenzènesulfonamide, 2-(2-chloro-4-iodophénylamino)-N-cyclopropylméthoxy-4-fluorobenzènesulfonamide, acide 2-(2-chloro-4-iodophénylamino)-3,4-difluorobenzènesulfonique, 2-(2-chloro-4-iodophénylamino)-3,4-difluoro-N-hydroxybenzènesulfonamide, 2-(2-chloro-4-iodophénylamino)-N-cyclopropylméthoxy-3,4-difluorobenzènesulfonamide, acide 2-(2-chloro-4-iodophénylamino)-3,4,5-trifluorobenzènesulfonique, 2-(2-chloro-4-iodophénylamino)-3,4,5-trifluoro-N-hydroxybenzènesulfonamide, 2-(2-chloro-4-iodophénylamino)-N-cyclopropylméthoxy-3,4,5-trifluorobenzènesulfonamide, acide 5-bromo-2-(2-chloro-4-iodophénylamino)-3,4-difluorobenzènesulfonique, 5-bromo-2-(2-chloro-4-iodophénylamino)-3,4-difluoro-N-hydroxybenzènesulfonamide, 5-bromo-2-(2-chloro-4-iodophénylamino)-N-cyclopropylméthoxy-3,4-difluorobenzènesulfonamide, acide 2-(2-chloro-4-iodophénylamino)-4-nitrobenzènesulfonique, 2-(2-chloro-4-iodophénylamino)-N-hydroxy-4-nitrobenzènesulfonamide et 2-(2-chloro-4-iodophénylamino)-N-cyclopropylméthoxy-4-nitrobenzènesulfonamide.

27. Utilisation d'un inhibiteur de MEK choisi parmi un composé de formule (II)A: 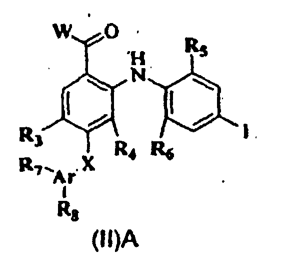 dans laquelle
W est OR₁, NR₂OR₁, NR_{A}R_{B}, NR₂NR_{A}R_{B} ou NR₂(CH₂)₂₋₄NR_{A}R_{B} ;
R₁ est un atome d'hydrogène ou un groupe alkyle en C₁₋₈, alcényle en C₃₋₈, alcynyle en C₃₋₈, cycloalkyle en C₃₋₈, phényle, phényl-alkyle(C₁₋₄), phénylalcényle(C₃₋₄), phényl-alcynyle(C₃₋₄), cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), hétérocyclyl(C₃₋₈)-alcényle(C₃₋₄), hétérocyclyl(C₃₋₈)-alcynyle(C₃₋₄) ou (CH₂)₂₋₄NR_{A}R_{B} ;
R₂ est un atome d'hydrogène ou un groupe phényle, alkyle en C₁₋₄, alcényle en C₃₋₄, alcynyle en C₃₋₈, cycloalkyle en C₃₋₈ ou cycloalkyl(C₃₋₈)-alkyle(C₁₋₄) ;
R_{A} est un atome d'hydrogène ou un groupe alkyle en C₁₋₆, alcényle en C₃₋₈, alcynyle en C₃₋₈, cycloalkyle en C₃₋₈, phényle, cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), (aminosulfonyl)phényle, [(aminosulfonyl)phényl]-alkyle(C₁₋₄), aminosulfonyl-alkyle(C₁₋₆), aminosulfonyl-cycloalkyle(C₃₋₆) ou [(aminosulfonyl)cycloalkyl(C₃₋₆)]-alkyle(C₁₋₄) ;
R_{B} est un atome d'hydrogène ou un groupe alkyle en C₁₋₈, alcényle en C₃₋₈, alcynyle en C₃₋₈, cycloalkyle en C₃₋₈ ou aryle en C₆₋₈ ;
R₃ est un atome d'halogène ou un groupe NO₂, SO₂NR₁(CH₂)₂₋₄NR_{E}R_{F}, SO₂NR_{I}R_{K} ou (CO)T ;
T est un groupe alkyle en C₁₋₈, cycloalkyle en C₃₋₈, (NR_{E}R_{F})-alkyle(C₁₋₄), OR_{F}, NR_{I}(CH₂)₂₋₄NR_{E}R_{F} ou NR_{E}R_{F} ;
R₄ est H ou F ;
R₅ est un atome d'hydrogène ou d'halogène ou le groupe méthyle ou NO₂ ;
R₆ est un atome d'hydrogène ou d'halogène ou le groupe méthyle ou NO₂ ;
Ar est le groupe phényle, 2-pyridyle, 3-pyridyle ou 4-pyridyle ;
R₇ et R₈ représentent chacun, indépendamment l'un de l'autre, un atome d'hydrogène ou d'halogène ou un groupe alkyle en C₁₋₄, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H} ou (CO)NR_{G}R_{H}, étant entendu que lorsque Ar est un groupe pyridyle, R₇ et R₈ représentent chacun un atome d'hydrogène ;
R_{C}, R_{D}, R_{E}, R_{F}, R_{G} et R_{H} représentent chacun, indépendamment les uns des autres, un atome d'hydrogène ou un groupe alkyle en C₁₋₄, alcényle en C₃₋₄, alcynyle en C₃₋₄, cycloalkyle en C₃₋₆ ou phényle ; NR_{C}R_{D}, NR_{E}R_{F} et NR_{G}R_{H} pouvant également représenter chacun indépendamment le groupe morpholinyle, pipérazinyle, pyrrolidinyle ou pipéridinyle ;
R_{I} et R_{J} représentent chacun, indépendamment l'un de l'autre, un atome d'hydrogène ou le groupe méthyle ou éthyle ;
R_{K} est un groupe alkyle en C₁₋₄, alcényle en C₃₋₄, alcynyle en C₃₋₄, cycloalkyle en C₃₋₆ ou phényle ;
X est O, S ou NH ; et
chaque radical hydrocarboné ou hétérocyclique ci-dessus portant éventuellement entre 1 et 3 substituants choisis indépendamment parmi des atomes d'halogène et des groupes alkyle en C₁₋₄, cycloalkyle en C₃₋₆, alcényle en C₂₋₄, alcynyle en C₂₋₄, phényle, hydroxy, amino, aminosulfonyle et NO₂, chaque substituant alkyle, cycloalkyle, alcényle, alcynyle ou phényle portant à son tour éventuellement 1 ou 2 substituants choisis indépendamment parmi des atomes d'halogène et des groupes alkyle en C₁₋₂, hydroxy, amino et NO₂,
et un sel ou ester en C₁₋₇ pharmaceutiquement acceptable d'un tel composé, pour la fabrication de produits pharmaceutiques destinés au traitement de la douleur chronique.

28. Utilisation selon la revendication 27, dans laquelle ladite douleur chronique est choisie parmi la douleur neuropathique, la douleur idiopathique et une douleur associée à l'alcoolisme chronique, une carence vitaminique, l'urémie ou l'hypothyroïdie.

29. Utilisation selon la revendication 28, dans laquelle ladite douleur chronique est un type de douleur neuropathique.

30. Utilisation selon la revendication 29, dans laquelle ladite douleur neuropathique est associée à l'un des états suivants : inflammation, douleur postopératoire, douleur des membres amputés, douleur causée par une brûlure, goutte, névralgie faciale, douleur herpétique et post-herpétique aiguë, causalgie, neuropathie diabétique, avulsion du plexus, névrome, vasculite, infection virale, syndrome d'écrasement, blessure par constriction, lésion tissulaire, amputation d'un membre, douleur postopératoire, douleur arthritique et toute autre lésion nerveuse entre le système nerveux périphérique et le système nerveux central, inclus.

31. Utilisation selon la revendication 28, dans laquelle ladite douleur chronique est associée à l'alcoolisme chronique, la carence vitaminique, l'urémie ou l'hypothyroïdie.

32. Utilisation selon la revendication 28, dans laquelle ladite douleur chronique est associée à une douleur idiopathique.

33. Utilisation selon la revendication 27, dans laquelle ladite douleur chronique est associée à l'inflammation.

34. Utilisation selon la revendication 27, dans laquelle ladite douleur chronique est associée à l'arthrite.

35. Utilisation selon la revendication 27, dans laquelle ladite douleur chronique est associée à une douleur postopératoire.

36. Utilisation selon la revendication 27 d'un composé de formule (I)A suivante : 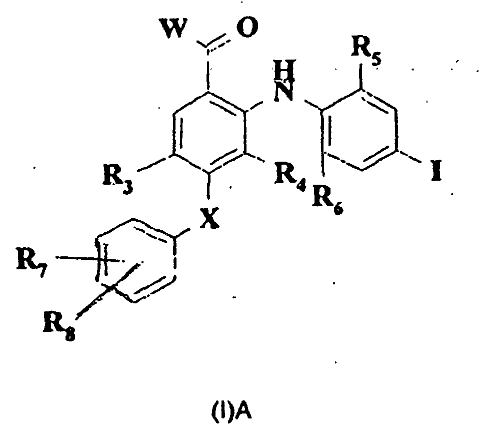 dans laquelle
W est OR₁, NR₂OR₁, NR_{A}R_{B}, NR₂NR_{A}R_{B} ou NR₂(CH₂)₂₋₄NR_{A}R_{B} ;
R₁ est un atome d'hydrogène ou un groupe alkyle en C₁₋₈, alcényle en C₃₋₈, alcynyle en C₃₋₈, cycloalkyle en C₃₋₈, phényle, phényl-alkyle(C₁₋₄), phénylalcényle(C₃₋₄), phényl-alcynyle(C₃₋₄), cycloalkyl(C₃₋₈)-alkyle(C₁₋₄), cycloalkyl-(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), hétérocyclyl(C₃₋₈)-alcényle(C₃₋₄), hétérocyclyl-(C₃₋₈)-alcynyle(C₃₋₄) ou (CH₂)₂₋₄NR_{A}R_{B} ;
R₂ est un atome d'hydrogène ou un groupe phényle, alkyle en C₁₋₄, alcényle en C₃₋₄, alcynyle en C₃₋₈, cycloalkyle en C₃₋₈ ou cycloalkyl(C₃₋₈)-alkyle(C₁₋₄) ;
R_{A} est un atome d'hydrogène ou un groupe alkyle en C₁₋₆, alcényle en C₃₋₈, alcynyle en C₃₋₈, cycloalkyle en C₃₋₈, phényle, cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), (aminosulfonyl)phényle, [(aminosulfonyl)phényl]-alkyle(C₁₋₄), aminosulfonyl-alkyle(C₁₋₆), aminosulfonyl-cycloalkyle(C₃₋₆) ou [(aminosulfonyl)cycloalkyl(C₃₋₆)]-alkyle(C₁₋₄) ;
R_{B} est un atome d'hydrogène ou un groupe alkyle en C₁₋₈, alcényle en C₃₋₈, alcynyle en C₃₋₈, cycloalkyle en C₃₋₈ ou aryle en C₆₋₈ ;
R₃ est un atome d'halogène ou un groupe NO₂, SO₂NR_{I}(CH₂)₂₋₄NR_{E}R_{F}, SO₂NR_{I}R_{K} ou (CO)T ;
T est un groupe alkyle en C₁₋₈, cycloalkyle en C₃₋₈, (NR_{E}R_{F})-alkyle(C₁₋₄), OR_{F}, NR_{I}(CH₂)₂₋₄NR_{E}R_{F} ou NR_{E}R_{F} ;
R₄ est H ou F ;
R₅ est un atome d'hydrogène ou d'halogène ou le groupe méthyle ou NO₂ ;
R₆ est un atome d'hydrogène ou d'halogène ou le groupe méthyle ou NO₂ ;
R₇ et R₈ représentent chacun, indépendamment l'un de l'autre, un atome d'hydrogène ou d'halogène ou un groupe alkyle en C₁₋₄, SO₂NR_{J}(CH₂)₂₋₄NR_{G}R_{H}, (CO)(CH₂)₂₋₄NR_{G}R_{H}, (CO)NR_{J}(CH₂)₂₋₄NR_{G}R_{H}, (CO)O(CH₂)₂₋₄NR_{G}R_{H}, SO₂NR_{G}R_{H} ou (CO)NR_{G}R_{H} ;
R_{C}, R_{D}, R_{E}, R_{F}, R_{G} et R_{H} représentent chacun, indépendamment les uns des autres, un atome d'hydrogène ou un groupe alkyle en C₁₋₄, alcényle en C₃₋₄, alcynyle en C₃₋₄, cycloalkyle en C₃₋₆ ou phényle ; NR_{C}R_{D}, NR_{E}R_{F} et NR_{G}R_{H} pouvant également représenter chacun indépendamment le groupe morpholinyle, pipérazinyle, pyrrolidinyle ou pipéridinyle ;
R_{I} et R_{J} représentent chacun, indépendamment l'un de l'autre, un atome d'hydrogène ou le groupe méthyle ou éthyle ;
R_{K} est un groupe alkyle en C₁₋₄, alcényle en C₃₋₄, alcynyle en C₃₋₄, cycloalkyle en C₃₋₆ ou phényle ;
X est O, S ou NH ; et
chaque radical hydrocarboné ou hétérocyclique ci-dessus portant éventuellement entre 1 et 3 substituants choisis indépendamment parmi des atomes d'halogène et des groupes alkyle en C₁₋₄, cycloalkyle en C₃₋₆, alcényle en C₂₋₄, alcynyle en C₂₋₄, phényle, hydroxy, amino, aminosulfonyle et NO₂, chaque substituant alkyle, cycloalkyle, alcényle, alcynyle ou phényle portant à son tour éventuellement 1 ou 2 substituants choisis indépendamment parmi des atomes d'halogène et des groupes alkyle en C₁₋₂, hydroxy, amino et NO₂,
ou d'un sel ou ester en C₁₋₇ pharmaceutiquement acceptable d'un tel composé.

37. Utilisation selon la revendication 27, dans laquelle R₃ est NO₂.

38. Utilisation selon la revendication 27, dans laquelle R₄ est un atome de fluor.

39. Utilisation selon la revendication 27, dans laquelle R₃ et R₄ représentent chacun indépendamment un atome d'hydrogène ou de fluor.

40. Utilisation selon la revendication 27, dans laquelle R₅ est le groupe méthyle ou un atome de fluor ou de chlore.

41. Utilisation selon la revendication 27, dans laquelle R₆ est un atome d'hydrogène, de chlore ou de fluor ou le groupe méthyle ou nitro.

42. Utilisation selon la revendication 41, dans laquelle R₆ est H.

43. Utilisation selon la revendication 41, dans laquelle R₆ est un atome de fluor.

44. Utilisation selon la revendication 27, dans laquelle R_{K} est le groupe méthyle ou éthyle.

45. Utilisation selon la revendication 27, dans laquelle R₁ est un atome d'hydrogène ou un groupe méthyle, éthyle, propyle, isopropyle, isobutyle, benzyle, phényle, phénéthyle, allyle, alcényle en C₂₋₅, cycloalkyle en C₃₋₆, cycloalkyl(C₃₋₅)-alkyle(C₁₋₂), hétérocyclyl(C₃₋₅)-alkyle(C₁₋₂) ou (CH₂)₂₋₄NR_{C}R_{D}.

46. Utilisation selon la revendication 45, dans laquelle R₁ est un atome d'hydrogène ou un groupe cycloalkyl(C₃₋₄)-alkyle(C₁₋₂).

47. Utilisation selon la revendication 27, dans laquelle R₂ est un atome d'hydrogène ou le groupe méthyle.

48. Utilisation selon la revendication 27, dans laquelle R_{A} comporte au moins un substituant hydroxy.

49. Utilisation selon la revendication 27, dans laquelle R_{A} est un atome d'hydrogène ou le groupe méthyle, éthyle, isobutyle, hydroxyéthyle, phényle, 2-pipéridin-1-yléthyle, 2,3-dihydroxypropyle, 3-[4-(2-hydroxyéthyl)pipérazin-1-yl]propyle, 2-pyrrolidin-1-yléthyle ou 2-diéthylaminoéthyle ; et R_{B} est un atome d'hydrogène ; ou dans laquelle R_{B} est le groupe méthyle et R_{A} est le groupe phényle.

50. Utilisation selon la revendication 27, dans laquelle W est NR_{A}R_{B} ou NR₂NR_{A}R_{B}.

51. Utilisation selon la revendication 27, dans laquelle W est NR₂(CH₂)₂₋₄NR_{A}R_{B} ou O(CH₂)₂₋₃NR_{A}R_{B}.

52. Utilisation selon la revendication 27, dans laquelle W est NR₂OR₁.

53. Utilisation selon la revendication 27, dans laquelle W est OR_{B}.

54. Utilisation selon la revendication 27, dans laquelle R₇ est en position para par rapport à X.

55. Utilisation selon la revendication 54, dans laquelle R₇ est un atome d'iode.

56. Utilisation selon la revendication 27, dans laquelle R₈ est en position ortho par rapport à X.

57. Utilisation selon la revendication 27 de l'acide 2,4-bis(2-chloro-4-iodophénylamino)-3-fluoro-5-nitrobenzoïque.

58. Utilisation selon la revendication 27, dans laquelle ledit inhibiteur de MEK est choisi parmi les composés suivants : acide 2-(2-chloro-4-iodophénylamino)-3-fluoro-5-nitro-4-(4-sulfamoylphénylamino)benzoïque, acide 2-(2-chloro-4-iodophénylamino)-3-fluoro-5-nitro-4-phénylaminobenzoïque, acide 2-(2-chloro-4-iodophénylamino)-3-fluoro-5-nitro-4-phénoxybenzoïque, acide 2-(2-chloro-4-iodophénylamino)-3-fluoro-5-nitro-4-phénylsulfanylbenzoïque, acide 2-(2-chloro-4-iodophénylamino)-3-fluoro-4-(méthylphénylamino)-5-nitrobenzoïque, 2-[(2-chloro-4-iodophényl)amino]-3-fluoro-N-hydroxy-4-[[4-[[(2-hydroxyéthyl)amino]carbonyl]phényl]amino]-5-nitrobenzamide, 2-[(2-chloro-4-iodophényl)amino]-4-[[4-[(diméthylamino)carbonyl]phényl]amino]-3-fluoro-N-hydroxy-5-nitrobenzamide, acide 2-(2-chloro-4-iodophénylamino)-3,5-difluoro-4-phénylaminobenzoïque, acide 2-(2-chloro-4-iodophénylamino)-3-fluoro-5-nitro-4-(3-sulfamoylphénylamino)benzoïque et acide 2-(2-chloro-4-iodophénylamino)-3-fluoro-5-nitro-4-(2-sulfamoylphénylamino)benzoïque et les acides hydroxamiques et hydroxamates de cyclopropylméthyle correspondants.

59. Utilisation d'un inhibiteur de MEK choisi parmi un composé de formule (I)B : 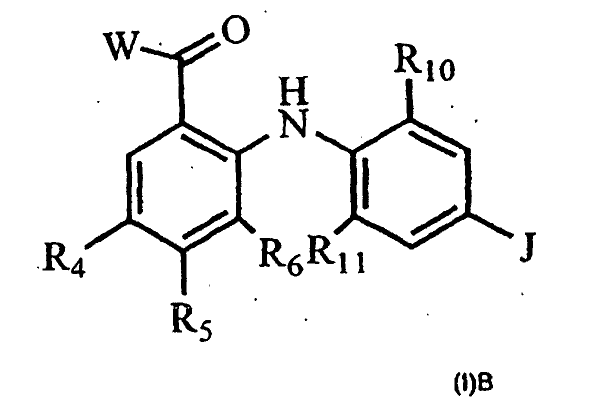 dans laquelle
W est OR₁, NR₂OR₁, NR_{A}R_{B}, NR₂NR_{A}R_{B}, O(CH₂)₁₋₄NR_{A}R_{B} ou NR₂(CH₂)₁₋₄NR_{A}R_{B}, O(CH₂)₁₋₄OR₁ ou NR₂(CH₂)₁₋₄OR₁ ;
R₁ est un atome d'hydrogène ou un groupe alkyle en C₁₋₈, alcényle en C₃₋₈, alcynyle en C₃₋₈, cycloalkyle en C₃₋₈, phényle, phényl-alkyle(C₁₋₄), phénylalcényle(C₃₋₄), phényl-alcynyle(C₃₋₄), cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), hétérocyclyl(C₃₋₈)-alcényle(C₃₋₄) ou hétérocyclyl(C₃₋₈)-alcynyle(C₃₋₄);
R₂ et R₃ représentent chacun indépendamment un atome d'hydrogène ou un groupe phényle, alkyle en C₁₋₄, alcynyle en C₃₋₈, cycloalkyle en C₃₋₈ ou cycloalkyl(C₃₋₈)-alkyle(C₁₋₄)
R₄, R₅ et R₆ représentent chacun indépendamment un atome d'hydrogène, Cl, F ou Br ;
R_{A} est un atome d'hydrogène ou un groupe alkyle en C₁₋₆, alcényle en C₃₋₈, alcynyle en C₃₋₈, cycloalkyle en C₃₋₈, phényle, cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), (aminosulfonyl)phényle, [(aminosulfonyl)phényl]-alkyle(C₁₋₄), aminosulfonyl-alkyle(C₁₋₆), aminosulfonyl-cycloalkyle(C₃₋₆) ou [(aminosulfonyl)cycloalkyl(C₃₋₆)]-alkyle(C₁₋₄) ;
R_{B} est un atome d'hydrogène ou un groupe alkyle en C₁₋₈, alcényle en C₃₋₈, alcynyle en C₃₋₈, cycloalkyle en C₃₋₈ ou phényle ;
J est un groupe SR_{C}, OR_{C}, SO₂R_{C}, SOR_{C}, SO₂NR_{D}R_{E}, alkyle en C₁₋₈, alcényle en C₃₋₈, alcynyle en C₃₋₈, cycloalkyle en C₃₋₈, cycloalcényle en C₅₋₈, phényle, cycloalkyl(C₃₋₈)-alkyle(C₁₋₄), cycloalkyl(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), -M'E'G', hétérocyclyl-M'-E'-G' ou cycloalkyl-M'-E'-G' ;
M' est O, SO, SO₂, NR_{E}, (CO)NR_{E}, NR_{E}(CO), SO₂NR_{E}, NR_{E}SO₂ ou CH₂ ;
E' est absent (une liaison covalente), (CH₂)₁₋₄ ou (CH₂)ₘO(CH₂)ₚ, où 1 ≤ (m et p chacun indépendamment) ≤ 3 et 2 ≤ (m + p) ≤ 4 ;
G' est OR₃, SO₂R_{C} ou NR_{F}R_{G} ; étant entendu que lorsque p = 1, G' est un atome d'hydrogène ;
R_{C}, R_{D}, R_{E}, R_{F} et R_{G} représentent chacun indépendamment un atome d'hydrogène ou un groupe alkyle en C₁₋₆, alcényle en C₃₋₄, alcynyle en C₃₋₄, cycloalkyle en C₃₋₆, hétérocyclyle en C₃₋₆ ou phényle ; NR_{F}R_{G} et NR_{D}R_{E} pouvant également représenter chacun, indépendamment l'un de l'autre, le groupe morpholinyle, pyrazinyle, pipérazinyle, pyrrolidinyle ou pipéridinyle ;
R₁₀ est un atome d'hydrogène ou d'halogène ou un groupe alkyle en C₁₋₄, NO₂ ou SO₂NR_{H}R_{I} ; et
R₁₁ est un atome d'hydrogène ou d'halogène ou le groupe NO₂ ;
chaque radical hydrocarboné ou hétérocyclique ci-dessus portant éventuellement entre 1 et 3 substituants choisis indépendamment parmi des atomes d'halogène et des groupes alkyle en C₁₋₄, cycloalkyle en C₃₋₆, alcényle en C₂₋₄, alcynyle en C₂₋₄, phényle, hydroxy, amino, aminosulfonyle et NO₂, chaque substituant alkyle, cycloalkyle, alcényle, alcynyle ou phényle portant à son tour éventuellement entre 1 et 3 substituants choisis indépendamment parmi des atomes d'halogène et des groupes alkyle en C₁₋₂, hydroxy, amino et NO₂,
et un sel ou ester en C₁₋₇ pharmaceutiquement acceptable d'un tel composé, pour la fabrication de compositions pharmaceutiques destinées au traitement de la douleur chronique.

60. Utilisation selon la revendication 59, dans laquelle ladite douleur chronique est choisie parmi la douleur neuropathique, la douleur idiopathique et une douleur associée à l'alcoolisme chronique, une carence vitaminique, l'urémie ou l'hypothyroïdie.

61. Utilisation selon la revendication 60, dans laquelle ladite douleur chronique est un type de douleur neuropathique.

62. Utilisation selon la revendication 61, dans laquelle ladite douleur neuropathique est associée à l'un des états suivants : inflammation, douleur postopératoire, douleur des membres amputés, douleur causée par une brûlure, goutte, névralgie faciale, douleur herpétique et post-herpétique aiguë, causalgie, neuropathie diabétique, avulsion du plexus, névrome, vasculite, affection virale, syndrome d'écrasement, blessure par constriction, lésion tissulaire, amputation d'un membre, douleur postopératoire, douleur arthritique et toute autre lésion nerveuse entre le système nerveux périphérique et le système nerveux central, inclus.

63. Utilisation selon la revendication 60, dans laquelle ladite douleur chronique est associée à l'alcoolisme chronique, la carence vitaminique, l'urémie ou l'hypothyroïdie.

64. Utilisation selon la revendication 60, dans laquelle ladite douleur chronique est associée à une douleur idiopathique.

65. Utilisation selon la revendication 59, dans laquelle ladite douleur chronique est associée à l'inflammation.

66. Utilisation selon la revendication 59, dans laquelle ladite douleur chronique est associée à l'arthrite.

67. Utilisation selon la revendication 59, dans laquelle ladite douleur chronique est associée à une douleur postopératoire.

68. Utilisation selon la revendication 59, dans laquelle R_{C} est un groupe alkyle en C₁₋₂.

69. Utilisation selon la revendication 59, dans laquelle W est OH.

70. Utilisation selon la revendication 59, dans laquelle W est NHOH.

71. Utilisation selon la revendication 59, dans laquelle W est le groupe NHO(cyclopropylméthyle).

72. Utilisation selon la revendication 59, dans laquelle R₁₀ est un atome de chlore ou le groupe méthyle.

73. Utilisation selon la revendication 59, dans laquelle R₁₁ est un atome de fluor.

74. Utilisation selon la revendication 59, dans laquelle R₁₁ est un atome d'hydrogène.

75. Utilisation selon la revendication 59, dans laquelle J est un groupe trihalogénométhyle ou méthylthio.

76. Utilisation selon la revendication 59, dans laquelle J est le groupe 1,2,5-thiadiazol-3-yle.

77. Utilisation selon la revendication 59, dans laquelle J est le groupe SO₂CH₃.

78. Utilisation selon la revendication 59, dans laquelle J est le groupe SOCH₃.

79. Utilisation selon la revendication 59, dans laquelle J est un groupe alcynyle en C₂₋₈ dans lequel la triple liaison. se trouve entre les atomes de carbone α et β par rapport au groupe phényle.

80. Utilisation selon la revendication 59, dans laquelle R₁ porte au moins un substituant hydroxy.

81. Utilisation selon la revendication 59, dans laquelle R₁ est un atome d'hydrogène ou un groupe méthyle, éthyle, propyle, isopropyle, isobutyle, benzyle, phénéthyle, allyle, alcényle en C₃₋₅, alcynyle en C₃₋₅, cycloalkyle en C₃₋₆, cycloalkyl(C₃₋₅)-alkyle(C₁₋₂) ou hétérocyclyl(C₃₋₅)-alkyle(C₁₋₂).

82. Utilisation selon la revendication 59, dans laquelle R₁ est un atome d'hydrogène ou un groupe cycloalkyl(C₃₋₄)-alkyle(C₁₋₂).

83. Utilisation selon la revendication 59, dans laquelle R₂ est un atome d'hydrogène ou un groupe méthyle, alcynyle en C₃₋₄, cycloalkyle en C₃₋₅ ou cycloalkyl(C₃₋₅)méthyle.

84. Utilisation selon la revendication 59, dans laquelle R_{A} est un atome d'hydrogène ou un groupe méthyle, éthyle, isobutyle, hydroxyéthyle, hydroxypropyle, cyclopropylméthyle, cyclobutylméthyle, alcynyle en C₂₋₄, phényle, 2-pipéridin-1-yléthyle, 2,3-dihydroxypropyle, 3-[4-(2-hydroxyéthyl)pipérazin-1-yl]propyle, 2-pyrrolidin-1-yléthyle ou 2-diéthylaminoéthyle; et R_{B} est un atome d'hydrogène ; ou dans laquelle R_{B} est le groupe méthyle et R_{A} est le groupe phényle.

85. Utilisation selon la revendication 59, dans laquelle R₄ et R₆ représentent chacun un atome d'hydrogène, et R₅ est F.

86. Utilisation selon la revendication 59, dans laquelle R₄, R₅ et R₆ représentent chacun F.

87. Utilisation selon la revendication 59, dans laquelle R₄ et R₅ représentent chacun F et R₆ est Br.

88. Utilisation selon la revendication 59, dans laquelle R₅ est F.

89. Utilisation selon la revendication 59, dans laquelle ledit inhibiteur de MEK est choisi parmi les composés suivants: acide 4-fluoro-2-(2-méthyl-4-méthylsulfanylphénylamino)benzoïque, acide 5-bromo-3,4-difluoro-2-(2-méthyl-4-méthylsulfanylphénylamino)benzoïque, acide 3,4-difluoro-2-(4-méthanesulfinyl-2-méthylphénylamino)benzoïque, acide 2-(4-méthanesulfinyl-2-méthylphénylamino)-4-nitrobenzoïque, acide 3,4,5-trifluoro-2-(4-méthanesulfonyl-2-méthylphénylamino)benzoïque, acide 3,4-difluoro-2-(2-méthyl-4-méthylsulfanylphénylamino)benzoïque, acide 2-(2-méthyl-4-méthylsulfanylphénylamino)-4-nitrobenzoïque, acide 3,4,5-trifluoro-2-(4-méthanesulfinyl-2-méthylphénylamino)benzoïque, acide 4-fluoro-2-(4-méthanesulfinyl-2-méthylphénylamino)-benzoïque, acide 5-bromo-3,4-difluoro-2-(4-méthanesulfonyl-2-méthylphénylamino)benzoïque, acide 3,4,5-trifluoro-2-(2-méthyl-4-méthylsulfanylphénylamino)benzoïque, acide 4-fluoro-2-(4-méthanesulfinyl-2-méthylphénylamino)-benzoïque, acide 5-bromo-3,4-difluoro-2-(4-méthanesulfinyl-2-méthlphénylamino)benzoïque, acide 3,4-difluoro-2-(4-méthanesulfonyl-2-méthylphénylamino)benzoïque, acide 2-4-méthanesulfonyl-2-méthylphénylamino)-4-nitrobenzoïque, N-cyclopropylméthoxy-4-fluoro-2-(2-méthyl-4-méthylsulfanylphénylamino)benzamide, 5-bromo-N-cyclopropylméthoxy-3,4-difluoro-2-(2-méthyl-4-méthylsulfanylphénylamino)benzamide, N-cyclopropylméthoxy-3,4-difluoro-2-(4-méthanesulfinyl-2-méthylphénylamino)benzamide, N-cyclopropylméthoxy-2-(4-méthanesulfinyl-2-méthylphénylamino)-4-nitrobenzamide, N-cyclopropylméthoxy-3,4,5-trifluoro-2-(4-méthanesulfonyl-2-méthylphénylamino)benzamide, N-cyclopropylméthoxy-3,4-difluoro-2-(2-méthyl-4-méthylsulfanylphénylamino)-benzamide, N-cyclopropylméthoxy-2-(2-méthyl-4-méthylsulfanylphénylamino)-4-nitrobenzamide, N-cyclopropylméthoxy-3,4,5-trifluoro-2-(4-méthanesulfinyl-2-méthylphénylamino)benzamide, N-cyclopropylméthoxy-4-fluoro-2-(4-méthanesulfinyl-2-méthylphénylamino)benzamide, 5-bromo-N-cyclopropylméthoxy-3,4-difluoro-2-(4-méthanesulfonyl-2-méthylphénylamino)benzamide, N-cyclopropylméthoxy-3,4,5-trifluoro-2-(2-méthyl-4-méthylsulfanylphénylamino)benzamide, N-cyclopropylméthoxy-4-fluoro-2-(4-méthanesulfinyl-2-méthylphénylamino)-benzamide, 5-bromo-N-cyclopropylméthoxy-3,4-difluoro-2-(4-méthanesulfinyl-2-méthylphénylamino)benzamide, N-cyclopropylméthoxy-3,4-difluoro-2-(4-méthanesulfonyl-2-méthylphénylamino)benzamide et N-cyclopropylméthoxy-2-(4-méthanesulfonyl-2-méthylphénylamino)-4-nitrobenzamide.

90. Utilisation selon la revendication 59, dans laquelle ledit inhibiteur de MEK est choisi parmi les composés suivants : 4-fluoro-N-hydroxy-2-(2-méthyl-4-méthylsulfanylphénylamino)benzamide, 5-bromo-3,4-difluoro-N-hydroxy-2-(2-méthyl-4-méthylsulfanylphénylamino)benzamide, 3,4-difluoro-N-hydroxy-2-(4-méthanesulfinyl-2-méthylphénylamino)benzamide, N-hydroxy-2-(4-méthanesulfinyl-2-méthylphénylamino)-4-nitrobenzamide, 3,4,5-trifluoro-N-hydroxy-2-(4-méthanesulfonyl-2-méthylphénylamino)benzamide, 3,4-difluoro-N-hydroxy-2-(2-méthyl-4-méthylsulfanylphénylamino)benzamide, N-hydroxy-2-(2-méthyl-4-méthylsulfanylphénylamino)-4-nitrobenzamide, 8: 3,4,5-trifluoro-N-hydroxy-2-(4-méthanesulfinyl-2-méthylphénylamino)benzamide, 4-fluoro-N-hydroxy-2-(4-méthanesulfinyl-2-méthylphénylamino)benzamide, 5-bromo-3,4-difluoro-N-hydroxy-2-(4-méthanesulfonyl-2-méthylphénylamino)benzamide, 3,4,5-trifluoro-N-hydroxy-2-(2-méthyl-4-méthylsulfanylphénylamino)benzamide, 4-fluoro-N-hydroxy-2-(4-méthanesulfinyl-2-méthylphénylamino)benzamide, 5-brome-3,4-difluoro-N-hydroxy-2-(4-méthanesulfinyl-2-méthylphénylamino)benzamide, 3,4-difluoro-N-hydroxy-2-(4-méthanesulfonyl-2-méthylphénylamino)benzamide et N-hydroxy-2-(4-méthanesulfonyl-2-méthylphénylamino)-4-nitrobenzamide.

91. Utilisation selon la revendication 59, dans laquelle ledit inhibiteur de MEK est choisi parmi les composés suivants : acide 3,4-difluoro-2-(4-imidazol-1-yl-2-méthylphénylamino)benzoïque, N-cyclopropylméthoxy-3,4-difluoro-2-(4-imidazol-1-yl-2-méthylphénylamino)benzamide, 3,4-difluoro-N-hydroxy-2-(4-imidazol-1-yl-2-méthylphénylamino)benzamide, acide 3,4,5-trifluoro-2-(2-méthyl-4-[1,2,5]thiadiazol-3-ylphénylamino)benzoïque, N-cyclopropylméthoxy-3,4,5-trifluoro-2-(2-méthyl-4-[1,2,5]thiadiazol-3-ylphénylamino)benzamide, 3,4,5-trifluoro-N-hydroxy-2-(2-méthyl-4-[1,2,5]thiadiazol-3-ylphénylamino)benzamide, acide 2-[4-(4-chloro[1,2,5]thiadiazol-3-yl)-2-méthylphénylamino]-3,4,5-trifluorobenzoïque, 2-[4-(4-chloro[1,2,5]thiadiazol-3-yl)-2-méthylphénylamino]-N-cyclopropylméthoxy-3,4,5-trifluorobenzamide, 2-[4-(4-chloro[1,2,5]thiadiazol-3-yl)-2-méthylphénylamino]-3,4,5-trifluoro-N-hydroxybenzamide, acide 2-{4-[4-(2-diméthylaminoéthoxy)-[1,2,5]thiadiazol-3-yl]-2-méthylphénylamino}-3,4,5-trifluorobenzoïque, N-cyclopropylméthoxy-3,4,5-trifluoro-2-{2-méthyl-4-[4-(2-pipéridin-1-yléthoxy)-[1,2,5]thiadiazol-3-yl]phénylamino}benzamide et 3,4,5-trifluoro-N-hydroxy-2-{2-méthyl-4-[4-(2-morpholin-4-yléthoxy)-[1,2,5]thiadiazol-3-yl]phénylamino}benzamide.

92. Utilisation selon la revendication 59, dans laquelle ledit inhibiteur de MEK est choisi parmi les composés suivants : acide 5-bromo-2-(2-chloro-4-méthylsulfanylphénylamino)-3,4-difluorobenzoïque, acide 2-(2-chloro-4-méthanesulfinylphénylamino)-3,4-difluorobenzoïque, acide 2-(2-chloro-4-méthanesulfonylphénylamino)-3,4,5-trifluorobenzoïque, acide 2-(2-chlorométhylsulfanylphénylamino)-3,4-difluorobenzoïque, acide 5-bromo-2-(2-chloro-4-méthanesulfonylphénylamino)-3,4-difluorobenzoïque, acide 2-(2-chloro-4-méthanesulfonylphénylamino)-3,4-difluorobenzoïque, 5-bromo-2-(2-chloro-4-méthylsulfanylphénylamino)-N-cyclopropylméthoxy-3,4-difluorobenzamide, 2-(2-chloro-4-méthanesulfinylphénylamino)-N-cyclopropylméthoxy-3,4-difluorobenzamide, 2-(2-chloro-4-méthanesulfonylphénylamino)-N-cyclopropylméthoxy-3,4,5-trifluorobenzamide, 2-(2-chloro-4-méthylsulfanylphénylamino)-N-cyclopropylméthoxy-3,4-difluorobenzamide, 2-(2-chloro-4-méthanesulfinylphénylamino)-N-cyclopropylméthoxy-3,4,5-trifluorobenzamide, 5-bromo-2-(2-chloro-4-méthanesulfonylphénylamino)-N-cyclopropylméthoxy-3,4-difluorobenzamide, 2-(2-chloro-4-méthylsulfanylphénylamino)-N-cyclopropylméthoxy-3,4,5-trifluorobenzamide, 2-(2-chloro-4-méthanesulfonylphénylamino)-N-cyclopropylméthoxy-3,4-difluorobenzamide, 2-[2-chloro-4-(3H-imidazol-1-yl)phénylamino]-N-cyclopropylméthoxy-3,4-difluorobenzamide, 2-(2-chloro-4-[1,2,5]thiadiazol-3-ylphénylamino)-N-cyclopropylméthoxy-3,4,5-trifluorobenzamide, acide 2-[4-(2-chloro-4-chloro[1,2,5]thiadiazol-3-yl)phénylamino]-3,4,5-trifluorobenzoïque, 2-[2-chloro-4-(4-chloro[1,2,5]thiadiazol-3-yl)phénylamino]-N-cyclopropylméthoxy-3,4,5-trifluorobenzamide, acide 2-{4-[4-(2-diméthylaminoéthoxy)-[1,2,5]thiadiazol-3-yl]-2-méthylphénylamino}-3,4,5-trifluorobenzoïque et 2-{2-chloro-4-[4-(2-pipéridin-1-yléthoxy)-[1,2,5]thiadiazol-3-yl]phénylamino}-N-cyclopropylméthoxy-3,4,5-trifluorobenzamide.

93. Utilisation selon la revendication 59, dans laquelle ledit inhibiteur de MEK est choisi parmi les composés suivants : acide 2-(4-éthynyl-2-méthylphénylamino)-4-fluorobenzoïque, acide 5-bromo-2-(4-éthynyl-2-méthylphénylamino)-3,4-difluorobenzoïque, N-cyclopropylméthoxy-2-(4-éthynyl-2-méthylphénylamino)-3,4-difluorobenzamide, N-cyclopropylméthoxy-2-(4-éthynyl-2-méthylphénylamino)-4-nitrobenzamide, 2-(4-éthynyl-2-méthylphénylamino)-3,4,5-trifluoro-N-hydroxybenzamide, acide 2-(4-éthynyl-2-méthylphénylamino)-3,4-difluorobenzoïque, acide 2-(4-éthynyl-2-méthylphénylamino)-4-nitrobenzoïque, N-cyclopropylméthoxy-2-(4-éthynyl-2-méthylphénylamino)-3,4,5-trifluorobenzamide, 4-fluoro-N-hydroxy-2-(4-méthanesulfinyl-2-méthylphénylamino)benzamide, 5-bromo-2-(4-éthynyl-2-méthylphénylamino)-3,4-difluoro-N-hydroxybenzamide, acide 2-(4-éthynyl-2-méthylphénylamino)-3,4,5-trifluorobenzoïque, N-cyclopropylméthoxy-2-(4-éthynyl-2-méthylphénylamino)-4-fluorobenzamide, 5-bromo-N-cyclopropylméthoxy-2-(4-éthynyl-2-méthylphénylamino)-3,4-difluoro-N-hydroxybenzamide, 2-(4-éthynyl-2-méthylphénylamino)-3,4-difluoro-N-hydroxybenzamide, 2-(4-éthynyl-2-méthylphénylamino)-N-hydroxy-4-nitrobenzamide, acide 2-(4-éthynyl-2-méthylphénylamino)-4-fluorobenzoïque, N-cyclopropylméthoxy-2-(4-éthynyl-2-méthylphénylamino)-4-fluorobenzamide et 4-fluoro-N-hydroxy-2-(4-méthanesulfinyl-2-méthylphénylamino)benzamide.

94. Utilisation selon la revendication 59, dans laquelle ledit inhibiteur de MEK est choisi parmi les composés suivants: acide 2-(2-chloro-4-éthynylphénylamino)-4-fluorobenzoïque, acide 5-bromo-2-(2-chloro-4-éthynylphénylamino)-3,4-difluorobenzoïque, 2-(2-chloro-4-éthynylphénylamino)-N-cyclopropylméthoxy-3,4-difluorobenzamide, 2-(2-chloro-4-éthynylphénylamino)-N-cyclopropylméthoxy-4-nitrobenzamide, 2-(2-chloro-4-éthynylphénylamino)-N-hydroxy-3,4,5-trifluorobenzamide, acide 2-(2-chloro-4-éthynylphénylamino)-3,4-difluorobenzoïque, acide 2-(4-éthynyl-2-chlorophénylamino)-4-nitrobenzoïque, 2-(2-chloro-4-éthynylphénylamino)-N-cyclopropylméthoxy-3,4,5-trifluorobenzamide, 2-(2-chloro-4-méthanesulfinylphénylamino)-4-fluoro-N-hydroxybenzamide, 5-bromo-2-(4-éthynyl-2-chlorophénylamino)-3,4-difluoro-N-hydroxybenzamide, acide 2-(2-chloro-4-éthynylphénylamino)-3,4,5-trifluorobenzoïque, 2-(2-chloro-4-éthynylphénylamino)-N-cyclopropylméthoxy-4-fluorobenzamide, 5-bromo-2-(2-chloro-4-éthynylphénylamino)-N-cyclopropylmétihoxy-3,4-difluorobenzamide, 2-(4-éthynyl-2-chlorophénylamino)-3,4-difluoro-N-hydroxybenzamide, 2-(4-éthynyl-2-chlorophénylamino)-N-hydroxy-4-nitrobenzamide, acide 2-(2-chloro-4-éthynylphénylamino)-4-fluorobenzoïque, 2-(2-chloro-4-éthynylphénylamino)-N-cyclopropylméthoxy-4-fluorobenzamide, 2-(2-chloro-4-méthanesulfinylphénylamino)-4-fluoro-N-hydroxybenzamide et acide 2-(2-chloro-4-imidazol-1-ylphénylamino)-3,4-difluorobenzoïque.

95. Utilisation d'un inhibiteur de MEK choisi parmi un composé de formule (I)C : 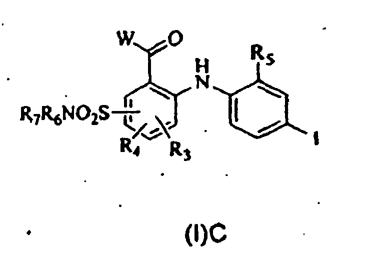 dans laquelle
W est OR₁, NR₂OR₁, NR_{A}R_{B}, NR₂R_{A}R_{B} ou NR₂(CH₂)₂₋₄NR_{A}R_{B} ;
R₁ est un atome d'hydrogène ou un groupe alkyle en C₁₋₈, alcényle en C₃₋₈, alcynyle en C₃₋₈, cycloalkyle en C₃₋₈, phényle, phényl-alkyle(C₁₋₄), phénylalcényle(C₃₋₄), phényl-alcynyle(C₃₋₄), cycloalkyl(C₃₋₈)-alkyle(C₁₋₄), cycloalkyl-(C₃₋₈)-alcényle(C₃₋₄), cycloalkyl(C₃₋₈)-alcynyle(C₃₋₄), hétérocyclyle en C₃₋₈, hétérocyclyl(C₃₋₈)-alkyle(C₁₋₄), hétérocyclyl(C₃₋₈)-alcényle(C₃₋₄), hétérocyclyl-(C₃₋₈)-alcynyle(C₃₋₄) ou (CH₂)₂₋₄NR_{A}R_{B} ;
R₂ est un atome d'hydrogène ou un groupe phényle, alkyle en C₁₋₄, alcényle en C₃₋₄, alcynyle en C₃₋₈, cycloalkyle en C₃₋₈ ou cycloalkyl(C₃₋₈)-alkyle(C₁₋₄) ;
R_{A} est un atome d'hydrogène ou un groupe alkyle en C₁₋₆, alcényle en C₃₋₈, alcynyle en C₃₋₈, cycloalkyle en C₃₋₈, phényle, cycloalkyl(C₃₋₈)-alkyle(C₁₋₄), cycloalkyl(C₃₋₈)-alcénle(C₃₋₄), cycloalkyl(C₃₋₈)-alcynyle(C₃₋₄), hétérocyclyle en C₃₋₈, hétérocyclyl(C₃₋₈)-alkyle(C₁₋₄), (aminosulfonyl)phényle, [(aminosulfonyl)phényl]-alkyle(C₁₋₄), aminosulfonyl-alkyle(C₁₋₆), aminosulfonyl-cycloalkyle(C₃₋₆) ou [(aminosulfonyl)cycloalkyl(C₃₋₆)]-alkyle(C₁₋₄) ;
R_{B} est un atome d'hydrogène ou un groupe alkyle en C₁₋₈, alcényle en C₃₋₈, alcynyle en C₃₋₈, cycloalkyle en C₃₋₈ ou aryle en C₆₋₈ ;
R₃ est H, F, Cl, Br ou NO₂ ;
R₄ est H ou F ;
R₅ est H ou Cl ou le groupe méthyle ;
R₆ est un atome d'hydrogène ou un groupe alkyle en C₁₋₄, hydroxyéthyle, hydroxypropyle, (CH₂)₂₋₄(NR_{C}R_{D}), phényle, 2-pyridyle, 3-pyridyle, 4-pyridyle ou CH₂Ar, Ar étant le groupe phényle, 2-pyridyle, 3-pyridyle ou 4-pyridyle ;
R₇ est un atome d'hydrogène ou un groupe alkyle en C₁₋₄, hydroxyéthyle, hydroxypropyle, (CH₂)₂₋₄(NR_{C}R_{D}), phényle, 2-pyridyle, 3-pyridyle, 4-pyridyle ou CH₂Ar, Ar étant le groupe phényle, 2-pyridyle, 3-pyridyle ou 4-pyridyle ;
R_{C} et R_{D} représentent chacun indépendamment un atome d'hydrogène ou un groupe alkyle en C₁₋₆, alcényle en C₃₋₄, alcynyle en C₃₋₄, cycloalkyle en C₃₋₆, hétérocyclyle en C₃₋₆ ou phényle ; NR_{C}R_{D} peut également représenter le groupe morpholinyle, pipérazinyle, pyrrolidinyle ou pipéradinyle ;
chaque radical hydrocarboné ou hétérocyclique ci-dessus pouvant éventuellement porter entre 1 et 3 substituants choisis indépendamment parmi des atomes d'halogène et des groupes alkyle en C₁₋₄, cycloalkyle en C₃₋₆, alcényle en C₂₋₄, alcynyle en C₂₋₄, phényle, hydroxy, amino, aminosulfonyle et NO₂, chaque substituant alkyle, cycloalkyle, alcényle, alcynyle ou phényle portant à son tour éventuellement entre 1 et 3 substituants choisis indépendamment parmi des atomes d'halogène et des groupes alkyle en C₁₋₂, hydroxy, amino et NO₂,
et un sel ou ester en C₁₋₆ pharmaceutiquement acceptable d'un tel composé, pour la fabrication de produits pharmaceutiques destinés au traitement de la douleur chronique.

96. Utilisation selon la revendication 95, dans laquelle ladite douleur chronique est choisie parmi la douleur neuropathique, la douleur idiopathique et une douleur associée à l'alcoolisme chronique, une carence vitaminique, l'urémie ou l'hypothyroïdie.

97. Utilisation selon la revendication 96, dans laquelle ladite douleur chronique est un type de douleur neuropathique.

98. Utilisation selon la revendication 97, dans laquelle ladite douleur neuropathique est associée à l'un des états suivants : inflammation, douleur postopératoire, douleur des membres amputés, douleur causée par une brûlure, goutte, névralgie faciale, douleur herpétique et post-herpétique aiguë, causalgie, neuropathie diabétique, avulsion du plexus, névrome, vasculite, infection virale, syndrome d'écrasement, blessure par constriction, lésion tissulaire; amputation d'un membre, douleur postopératoire, douleur arthritique et toute autre lésion nerveuse entre le système nerveux périphérique et le système nerveux central, inclus.

99. Utilisation selon la revendication 96, dans laquelle ladite douleur chronique est associée à l'alcoolisme chronique, la carence vitaminique, l'urémie ou l'hypothyroïdie.

100. Utilisation selon la revendication 96, dans laquelle ladite douleur chronique est associée à une douleur idiopathique.

101. Utilisation selon la revendication 95, dans laquelle ladite douleur chronique est associée à l'inflammation.

102. Utilisation selon la revendication 95, dans laquelle ladite douleur chronique est associée à l'arthrite.

103. Utilisation selon la revendication 95, dans laquelle ladite douleur chronique est associée à une douleur postopératoire.

104. Utilisation selon la revendication 95, dans laquelle le groupe sulfamoyle est en position méta par rapport à W(CO)- et en position para par rapport au groupe NH pontant.

105. Utilisation selon la revendication 95, dans laquelle le groupe sulfamoyle est en position para par rapport à W(CO)- et en position méta par rapport au groupe NH pontant.

106. Utilisation selon la revendication 95, dans laquelle R₄ est un atome de fluor.

107. Utilisation selon la revendication 95, dans laquelle R₃ est un atome de fluor.

108. Utilisation selon la revendication 95, dans laquelle R₃ est un atome d'hydrogène.

109. Utilisation selon la revendication 95, dans laquelle W est OH.

110. Utilisation selon la revendication 95, dans laquelle W est NR₂OR₁.

111. Utilisation selon la revendication 109, dans laquelle R₃ et R₄ représentent chacun un atome de fluor.

112. Utilisation selon la revendication 95, dans laquelle R₁ porte au moins un substituant hydroxy.

113. Utilisation selon la revendication 95, dans laquelle R₁ est un atome d'hydrogène ou un groupe méthyle, éthyle, propyle, isopropyle, isobutyle, benzyle, phénéthyle, allyle, alcényle en C₃₋₅, alcynyle en C₃₋₅, cycloalkyle en C₃₋₆, cycloalkyl(C₃₋₅)-alkyle(C₁₋₂) ou hétérocyclyl(C₃₋₅)-alkyle(C₁₋₂).

114. Utilisation selon la revendication 113, dans laquelle R₁ est un atome d'hydrogène ou un groupe cycloalkyl(C₃₋₄)-alkyle(C₁₋₂).

115. Utilisation selon la revendication 95, dans laquelle R₂ est un atome d'hydrogène ou un groupe méthyle, alcynyle en C₃₋₄, cycloalkyle en C₃₋₅ ou cycloalkyl(C₃₋₅)méthyle.

116. Utilisation selon la revendication 95, dans laquelle R_{A} est un atome d'hydrogène ou le groupe méthyle, éthyle, isobutyle, hydroxyéthyle, hydroxypropyle, cyclopropylméthyle, cyclobutylméthyle, alcynyle en C₃₋₄, phényle, 2-pipéridin-1-yléthyle, 2,3-dihydroxypropyle, 3-[4-(2-hydroxyéthyl)pipérazin-1-yl]propyle, 2-pyrrolidin-1-yléthyle ou 2-diéthylaminoéthyle et R_{B} est un atome d'hydrogène ; ou dans laquelle R_{B} est le groupe méthyle et R_{A} est le groupe phényle.

117. Utilisation selon la revendication 95, dans laquelle R₇ est (CH₂)₂₋₄(NR_{C}R_{D}).

118. Utilisation selon la revendication 95, dans laquelle NR_{C}R_{D} représente le groupe morpholinyle, pipérazinyle, pyrrolidinyle ou pipéridinyle.

119. Utilisation selon la revendication 95, dans laquelle R₅ est le groupe méthyle ou un atome de chlore.

120. Utilisation selon la revendication 95, dans laquelle ledit inhibiteur de MEK est choisi parmi les composés suivants : acide 2-(2-chloro-4-iodophénylamino)-4-sulfamoylbenzoïque, 2-(2-chloro-4-iodophénylamino)-N-hydroxy-4-sulfamoylbenzamide, 2-(2-chloro-4-iodophénylamino)-N-cyclopropylméthoxy-4-sulfamoylbenzamide, acide 2-(2-chloro-4-iodophénylamino)-4-(2-morpholin-4-yléthylsulfamoyl)benzoïque, 2-(2-chloro-4-iodophénylamino)-N-hydroxy-4-(2-morpholin-4-yléthylsulfamoyl)benzamide, 2-(2-chlore-4-iodophénylamino)-N-cyclopropylméthoxy-4-(2-morpholin-4-yléthylsulfamoyl)benzamide, acide 2-(2-chloro-4-iodophénylamino)-3,4-difluoro-5-sulfamoylbenzoïque, 2-(2-chloro-4-iodophénylamino)-3,4-difluoro-N-hydroxy-5-sulfamoylbenzamide, 2-(2-chloro-4-iodophénylamino)-N-cyclopropylméthoxy-3,4-difluoro-5-sulfamoylbenzamide, acide 2-(2-chloro-4-iodophénylamino)-3,4-difluoro-5-(2-morpholin-4-yléthylsulfamoyl)benzoïque, 2-(2-chloro-4-iodophénylamino)-3,4-difluoro-N-hydroxy-5-(2-morpholin-4-yléthylsulfamoyl)benzamide, 2-(2-chloro-4-iodophénylamino)-N-cyclopropylméthoxy-3,4-difluoro-5-(2-morpholin-4-yléthylsulfamoyl)-benzamide, acide 5-(bis-pyridin-3-ylméthylsulfamoyl)-3,4-difluoro-2-(4-iodophénylamino)benzoïque, 5-(bis-pyridin-3-ylméthylsulfamoyl)-N-cyclopropylméthoxy-3,4-difluoro-2-(4-iodophénylamino)benzamide, N-cyclopropylméthoxy-3,4-difluoro-2-(4-iodophénylamino)-5-(méthylpyridin-3-ylméthylsulfamoyl)-benzamide, N-cyclopropylméthoxy-3,4-difluoro-2-(4-iodophénylamino)-5-[(pyridin-3-ylméthyl)sulfamoyl]benzamide, N-cyclopropylméthoxy-5-[(3-diéthylaminopropyl)pyridin-3-ylméthylsulfamoyl]-3,4-difluoro-2-(4-iodophénylamino)-benzamide, N-cyclopropylméthoxy-3,4-difluoro-5-[(3-hydroxypropyl)pyridin-3-ylméthylsulfamoyl]-2-(4-iodophénylamino)benzamide, N-cyclopropylméthoxy-5-(éthylpyridin-3-ylméthylsulfamoyl)-3,4-difluoro-2-(4-iodophénylamino)-benzamide, N-cyclopropylméthoxy-3,4-difluoro-5-[(2-hydroxyéthyl)pyridin-3-ylméthylsulfamoyl]-2-(4-iodophénylamino)benzamide, acide 5-(bis-pyridin-2-ylméthylsulfamoyl)-3,4-difluoro-2-(4-iodophénylamino)benzoïque, 5-(bis-pyridin-2-ylméthylsulfamoyl)-N-cyclopropylméthoxy-3,4-difluoro-2-(4-iodophénylamino)-benzamide, N-cyclopropylméthoxy-3,4-difluoro-2-(4-iodophénylamino)-5-(méthylpyridin-2-ylméthylsulfamoyl)benzamide, N-cyclopropylméthoxy-3,4-difluoro-2-(4-iodophénylamino)-5-[(pyridin-2-ylméthylsulfamoyl)benzamide, acide 5-(bis-pyridin-3-ylméthylsulfamoyl)-3,4-difluoro-2-(4-iodo-2-méthylphénylamino)benzoïque, 5-(bis-pyridin-3-ylméthylsulfamoyl)-N-cyclopropylméthoxy-3,4-difluoro-2-(4-iodo-2-méthylphénylamino)benzamide, N-cyclopropylméthoxy-3,4-difluoro-2-(4-iodo-2-méthylphénylamino)-5-(méthylpyridin-3-ylméthylsulfamoyl)-benzamide, N-cyclopropylméthoxy-3,4-difluoro-2-(4-iodo-2-méthylphénylamino)-5-[(pyridin-3-ylméthyl)sulfamoyl]benzamide, N-cyclopropylméthoxy-5-[(3-diéthylaminopropyl)pyridin-3-ylméthylsulfamoyl]-3,4-difluoro-2-(4-iodo-2-méthylphénylamino)benzamide, N-cyclopropylméthoxy-3,4-difluoro-5-[(3-hydroxypropyl)pyridin-3-ylméthylsulfamoyl]-2-(4-iodo-2-méthylphénylamino)benzamide, N-cyclopropylméthoxy-5-(éthylpyridin-3-ylméthylsulfamoyl)-3,4-difluoro-2-(4-iodo-2-méthylphénylamino)benzamide, N-cyclopropylméthoxy-3,4-difluoro-5-[(2-hydroxyéthyl)pyridin-3-ylméthylsulfamoyl]-2-(4-iodo-2-méthylphénylamino)-benzamide, acide 5-(bis-pyridin-2-ylméthylsulfamoyl)-3,4-difluoro-2-(4-iodo-2-méthylphénylamino)benzoïque, 5-(bis-pyridin-2-ylméthylsulfamoyl)-N-cyclopropylméthoxy-3,4-difluoro-2-(4-iodo-2-méthylphénylamino)benzamide, N-cyclopropylméthoxy-3,4-difluoro-2-(4-iodo-2-méthylphénylamino)-5-(méthylpyridin-2-ylméthylsulfamoyl)benzamide, N-cyclopropylméthoxy-3,4-difluoro-2-(4-iodo-2-méthylphénylamino)-5-[(pyridin-2-ylméthyl)sulfamoyl]benzamide, acide 5-(bis-pyridin-3-ylméthylsulfamoyl)-2-(2-chloro-4-iodophénylamino)-3,4-difluorobenzoïque, 5-(bis-pyridin-3-ylméthylsulfamoyl)-2-(2-chloro-4-iodophénylamino)-N-cyclopropylméthoxy-3,4-difluorobenzamide, 2-(2-chloro-4-iodophénylamino)-N-cyclopropylméthoxy-3,4-difluoro-5-(méthylpyridin-3-ylméthylsulfamoyl)-benzamide, 2-(2-chloro-4-iodophénylamino)-N-cyclopropylméthoxy-3,4-difluoro-5-[(pyridin-3-ylméthyl)sulfamoyl]benzamide, 2-(2-chloro-4-iodophénylamino)-N-cyclopropylméthoxy-5-[(3-diéthylaminopropyl)pyridin-3-ylméthylsulfamoyl]-3,4-difluorobenzamide, 2-(2-chloro-4-iodophénylamino)-N-cyclopropylméthoxy-3,4-difluoro-5-[(3-hydroxypropyl)pyridin-3-ylméthylsulfamoyl]benzamide, 2-(2-chloro-4-iodophénylamino)-N-cyclopropyiméthoxy-5-(éthylpyridin-3-ylméthylsulfamoyl)-3,4-difluoro-benzamide, 2-(2-chloro-4-iodophénylamino)-N-cyclopropylméthoxy-3,4-difluoro-5-[(2-hydroxyéthyl)pyridin-3-ylméthylsulfamoyl]-benzamide, acide 5-(bis-pyrldin-2-ylméthylsulfamoyl)-2-(2-chloro-4-iodophénylamino)-3,4-difluorobenzoïque, 5-(bis-pyridin-2-ylméthylsulfamoyl)-2-(2-chloro-4-iodophénylamino)-N-cyclopropylméthoxy-3,4-difluorobenzamide, 2-(2-chloro-4-iodophénylamino)-N-cyclopropylméthoxy-3,4-difluoro-5-(méthylpyridin-2-ylméthylsulfamoyl)benzamide, 2-(2-chloro-4-iodophénylamino)-N-(méthylphénylamino)-4-phénylsulfamoylbenzamide, N-cyclopropylméthoxy-2-(4-iodo-2-méthylphénylamino)-4-(pyridin-3-ylsulfamoyl)benzamide, N-cyclopropylméthoxy-2-(4-iodo-2-méthylphénylamino)-4-[(pyridin-3-ylméthyl)sulfamoyl]benzamide, 4-(bis-pyridin-3-ylméthylsulfamoyl)-N-cyclopropylméthoxy-2-(4-iodo-2-méthylphénylamino)benzamide, N-cyclopropylméthoxy-4-[(2-hydroxyéthyl)pyridin-4-ylméthylsulfamoyl]-2-(4-iodo-2-méthylphénylamino)benzamide, N-cyclopropylméthoxy-2-(4-iodo-2-méthylphénylamino)-4-(méthylpyridin-3-ylméthylsulfamoyl)benzamide, N-cyclopropylméthoxy-4-[(3-diéthylaminopropyl)pyridin-3-ylméthylsulfamoyl]-2-(4-iodo-2-méthylphénylamino)benzamide, 2-(2-chloro-4-iodophénylamino)-N-cyclopropylméthoxy-4-phénylsulfamoylbenzamide, 2-(2-chloro-4-iodophénylamino)-N-cyclopropylméthoxy-4-(pyridin-3-ylsulfamoyl)-benzamide, 2-(2-chloro-4-iodophénylamino)-N-cyclopropylméthoxy-4-[(pyridin-3-ylméthyl)sulfamoyl]benzamide, 4-(bis-pyridin-3-ylméthylsulfamoyl)-2-(2-chloro-4-iodophénylamino)-N-cyclopropylméthoxybenzamide, 2-(2-chloro-4-iodophénylamino)-N-cyclopropylméthoxy-4-[(2-hydroxyéthyl)pyridin-4-ylméthylsulfamoyl]-benzamide, 2-(2-chloro-4-iodophénylamino)-N-cyclopropylméthoxy-4-(méthylpyridin-3-ylméthylsulfamoyl)benzamide, 2-(2-chloro-4-iodophénylamino)-N-cyclopropylméthoxy-4-[(3-diéthylaminopropyl)pyridin-3-ylméthylsulfamoyl]benzamide, acide 5-[bis-(4-méthoxybenzyl)sulfamoyl]-2-(2-chloro-4-iodophénylamino)-3,4-difluorobenzoïque et 2-(2-chloro-4-iodophénylamino)-5-diméthylsulfamoyl-3,4-difluorobenzoate de méthyle.

121. Utilisation selon la revendication 95, dans laquelle ledit inhibiteur de MEK est choisi parmi les composés suivants : PD 298458, N-allyloxy-2-(2-chloro-4-iodophénylamino)-3,4-difluoro-5-(4-méthylpipérazine-1-sulfonyl)benzamide, PD 298459, N-allyloxy-2-(2-chloro-4-iodophénylamino)-3,4-difluoro-5-(méthylphénylsulfamoyl)benzamide, PD 298460, 5-(allylméthylsulfamoyl)-N-allyloxy-2-(2-chloro-4-iodophénylamino)-3,4-difluorobenzamide, PD 298463, 1-[5-allyloxycarbamoyl-4-(2-chloro-4-iodophénylamino)-2,3-difluorobenzènesulfonyl]-pipéridine-3-carboxamide, PD 298464, N-allyloxy-2-(2-chloro-4-iodophénylamino)-5-[(3-diméthylaminopropyl)méthylsulfamoyl]-3,4-difluorobenzamide, PD 298465, N-allyloxy-2-(2-chloro-4-iodophénylamino)-3,4-difluoro-5-(4-pyridin-2-ylpipérazine-1-sulfonyl)benzamide et PD 298467, N-allyloxy-2-(2-chloro-4-iodophénylamino)-3,4-difluoro-5-(méthoxyméthylsulfamoyl)benzamide.

122. Utilisation selon la revendication 1, dans laquelle ledit inhibiteur de MEK est choisi parmi le 2-(2-chloro-4-iodophénylamino)-N-cyclopropylméthoxy-3,4-difluorobenzènesulfonamide et le 2-(2-chloro-4-iodophénylamino)cyclopropylméthoxy-3,4-difluorobenzènesulfonamide.

123. Utilisation selon la revendication 27, dans laquelle ledit inhibiteur de MEK est l'acide 2,4-bis(2-chloro-4-iodophénylamino)-3-fluoro-5-nitrobenzoïque.

124. Utilisation selon la revendication 59, dans laquelle ledit inhibiteur de MEK est choisi parmi l'acide 2-(4-éthynyl-2-méthylphénylamino)-4-fluorobenzoïque et l'acide 2-(3',5'-dichlorobiphényl-4-ylamino)benzoïque.

125. Utilisation selon la revendication 95, dans laquelle ledit inhibiteur de MEK est choisi parmi les composés suivants : 2-(2-chloro-4-iodophénylamino)-N-cyclopropylméthoxy-3,4-difluoro-5-sulfamoylbenzamide, 2-(2-chloro-4-iodophénylamino)-3,4-difluoro-N-hydroxy-5-sulfamoylbenzamide, C-(2-chloro-4-iodophénylamino)-N-cyclopropylméthoxydiméthylsulfamoyl-difluorobenzamide, N-cyclopropylméthoxydiméthylsulfamoyl-difluoro-C-(4-iodo-2-méthylphénylamino)benzamide et C-(2-chloro-4-iodophénylamino)difluoro(méthoxyméthylsulfamoyl)-N-(2-morpholin-4-yléthoxy)benzamide.
